# EUROPEAN PATENT APPLICATION

(11) **EP 2 277 893 A2**
(43) Date of publication of application: **26.01.2011**
(21) Application number: 10179569.8
(22) Date of filing: 08.12.1999
(51) Int. Cl.: C07K 14/295, C07K 19/00, C12N 15/62, A61K 39/118

(54) **Compounds and methods for treatment and diagnosis of chlamydial infection**

(30) Priority: 08.12.1998 US 208277; 08.04.1999 US 288594; 01.10.1999 US 410568; 22.10.1999 US 426571
(62) Divisional of application: 10163839.3
(71) Applicant: Corixa Corporation, Wilmington, DE 19808 (US)
(72) Inventor: Probst, Peter, Seattle, WA 98117 (US); Bhatia, Ajay, Seattle, WA 98104 (US); Skeiky, Yasir A. W., Silver Sping, MD 20904 (US); Fling, Steven P., Bainbridge Island, WA 98110 (US); Jen, Shyian, Seattle, WA 98122 (US); Stromberg, Erica Jean, Seattle, WA 98103 (US)
(74) Representative: Goodall, Scott

(57) **Abstract**

Compounds and methods for the diagnosis and treatment of Chlamydial infection are disclosed. The compounds provided include polypeptides that contain at least one antigenic portion of a Chlamydia antigen and DNA sequences encoding such polypeptides. Pharmaceutical compositions and vaccines comprising such polypeptides or DNA sequences are also provided, together with antibodies directed against such polypeptides. Diagnostic kits containing such polypeptides or DNA sequences and a suitable detection reagent may be used for the detection of Chlamydial infection in patients and in biological samples.

SEQUENCE LISTING

<110> Corixa Corporation
Probst, Peter
Bhatia, Ajay
Skeiky, Yasir
Fling, Steve
Maisonneuve, Jeff

<120> COMPOSITIONS AND METHODS FOR TREATMENT AND
DIAGNOSIS OF CHLAMYDIAL INFECTION

<130> 210121.469PC

<140> PCT
<141> 1999-12-08

<160> 303

<170> FastSEQ for Windows Version 3.0/4.0

<210> 1
<211> 481
<212> DNA
<213> Chlamydia trachomatis

<400> 1
ctgaagactt ggctatgttt tttattttga cgataaacct agttaaggca taaaagagtt 60
gcgaaggaag agccctcaac ttttcttatc accttcttta actaggagtc atccatgagt 120
caaaataaga actctgcttt catgcagcct gtgaacgtat ccgctgattt agctgccatc 180
gttggtgcag gacctatgcc tcgcacagag atcattaaga aaatgtggga ttacattaag 240
gagaatagtc ttcaagatcc tacaaacaaa cgtaatatca atcccgatga taaattggct 300
aaagtttttg gaactgaaaa acctatcgat atgttccaaa tgacaaaaat ggtttctcaa 360
cacatcatta aataaaatag aaattgactc acgtgttcct cgtctttaag atgaggaact 420
agttcattct ttttgttcgt ttttgtgggt attactgtat ctttaacaac tatcttagca 480
g 481

<210> 2
<211> 183
<212> DNA
<213> Chlamydia trachomatis

<400> 2
atcgttggtg caggacctat gcctcgcaca gagatcatta agaaaatgtg ggattacatt 60
aaggagaata gtcttcaaga tcctacaaac aaacgtaata tcaatcccga tgataaattg 120
gctaaagttt ttggaactga aaaacctatc gatatgttcc aaatgacaaa aatggtttct 180
caa 183

<210> 3
<211> 110
<212> DNA
<213> Chlamydia trachomatis

<400> 3

gctgcgacat catgcgagct tgcaaaccaa catggacatc tccaatttcc ccttctaact 60
cgctctttgg aactaatgct gctaccgagt caatcacaat cacatcgacc 110

<210> 4
<211> 555
<212> DNA
<213> Chlamydia trachomatis

<400> 4
cggcacgagc ctaagatgct tatactactt taagggaggc ccttcgtatg ccgcgcatca 60
ttggaataga tattcctgcg aaaaagaaat taaaaataag tcttacatat atttatggaa 120
tagggccagc tctttctaaa gagattattg ctagattgca gttgaatccc gaagctagag 180
ctgcagagtt gactgaggaa gaggttggtc gactaaacgc tcttttacag tcggattacg 240
ttgttgaagg ggatttgcgc cgtcgtgtgc aatctgatat caaacgtctg attactatcc 300
atgcttatcg tggacaaaga catagacttt ctttgcctgt tcgtggtcag agaacaaaaa 360
caaattctcg cacgcgtaag ggtaaacgta aaactattgc aggtaagaag aaataataat 420
ttttaggaga gagtgttttg gttaaaaatc aagcgcaaaa aagaggcgta aaaagaaaac 480
aagtaaaaaa cattccttcg ggcgttgtcc atgttaaggc tacttttaat aatacaattg 540
taaccataac agacc 555

<210> 5
<211> 86
<212> PRT
<213> Chlamydia trachomatis

<400> 5
Met Ser Gln Asn Lys Asn Ser Ala Phe Met Gln Pro Val Asn Val Ser
1               5                   10                  15
Ala Asp Leu Ala Ala Ile Val Gly Ala Gly Pro Met Pro Arg Thr Glu
            20                  25                  30
Ile Ile Lys Lys Met Trp Asp Tyr Ile Lys Glu Asn Ser Leu Gln Asp
        35                  40                  45
Pro Thr Asn Lys Arg Asn Ile Asn Pro Asp Asp Lys Leu Ala Lys Val
    50                  55                  60
Phe Gly Thr Glu Lys Pro Ile Asp Met Phe Gln Met Thr Lys Met Val
65                  70                  75                  80
Ser Gln His Ile Ile Lys
                85

<210> 6
<211> 61
<212> PRT
<213> Chlamydia trachomatis

<400> 6
Ile Val Gly Ala Gly Pro Met Pro Arg Thr Glu Ile Ile Lys Lys Met
1               5                   10                  15
Trp Asp Tyr Ile Lys Glu Asn Ser Leu Gln Asp Pro Thr Asn Lys Arg
            20                  25                  30
Asn Ile Asn Pro Asp Asp Lys Leu Ala Lys Val Phe Gly Thr Glu Lys
        35                  40                  45
Pro Ile Asp Met Phe Gln Met Thr Lys Met Val Ser Gln
    50                  55                  60

<210> 7

<211> 36
<212> PRT
<213> Chlamyida trachomatis

<400> 7
Ala Ala Thr Ser Cys Glu Leu Ala Asn Gln His Gly His Leu Gln Phe
1               5                   10                  15
Pro Leu Leu Thr Arg Ser Leu Glu Leu Met Leu Leu Pro Ser Gln Ser
            20                  25                  30
Gln Ser His Arg
        35

<210> 8
<211> 18
<212> PRT
<213> Chlamydia trachomatis

<400> 8
Leu Arg His His Ala Ser Leu Gln Thr Asn Met Asp Ile Ser Asn Phe
1               5                   10                  15
Pro Phe

<210> 9
<211> 5
<212> PRT
<213> Chlamydia trachomatis

<400> 9
Leu Ala Leu Trp Asn
1               5

<210> 10
<211> 11
<212> PRT
<213> Chlamydia trachomatis

<400> 10
Cys Cys Tyr Arg Val Asn His Asn His Ile Asp
1               5                   10

<210> 11
<211> 36
<212> PRT
<213> Chlamydia trachomatis

<400> 11
Val Asp Val Ile Val Ile Asp Ser Val Ala Ala Leu Val Pro Lys Ser
1               5                   10                  15
Glu Leu Glu Gly Glu Ile Gly Asp Val His Val Gly Leu Gln Ala Arg
            20                  25                  30
Met Met Ser Gln
        35

<210> 12

<211> 122
<212> PRT
<213> Chlamydia trachomatis

<400> 12
Met Pro Arg Ile Ile Gly Ile Asp Ile Pro Ala Lys Lys Lys Leu Lys
1               5                   10                  15
Ile Ser Leu Thr Tyr Ile Tyr Gly Ile Gly Pro Ala Leu Ser Lys Glu
            20                  25                  30
Ile Ile Ala Arg Leu Gln Leu Asn Pro Glu Ala Arg Ala Ala Glu Leu
        35                  40                  45
Thr Glu Glu Glu Val Gly Arg Leu Asn Ala Leu Leu Gln Ser Asp Tyr
    50                  55                  60
Val Val Glu Gly Asp Leu Arg Arg Arg Val Gln Ser Asp Ile Lys Arg
65                  70                  75                  80
Leu Ile Thr Ile His Ala Tyr Arg Gly Gln Arg His Arg Leu Ser Leu
                85                  90                  95
Pro Val Arg Gly Gln Arg Thr Lys Thr Asn Ser Arg Thr Arg Lys Gly
            100                 105                 110
Lys Arg Lys Thr Ile Ala Gly Lys Lys Lys
        115                 120

<210> 13
<211> 20
<212> PRT
<213> Chlamydia trachomatis

<400> 13
Asp Pro Thr Asn Lys Arg Asn Ile Asn Pro Asp Asp Lys Leu Ala Lys
1               5                   10                  15
Val Phe Gly Thr
            20

<210> 14
<211> 20
<212> PRT
<213> Chlamydia trachomatis

<400> 14
Asp Asp Lys Leu Ala Lys Val Phe Gly Thr Glu Lys Pro Ile Asp Met
1               5                   10                  15
Phe Gln Met Thr
            20

<210> 15
<211> 161
<212> DNA
<213> Chlymidia trachomatis

<400> 15
atctttgtgt gtctcataag cgcagagcgg ctgcggctgt ctgtagcttc atcggaggaa      60
ttacctacct cgcgacattc ggagctatcc gtccgattct gtttgtcaac aaaatgctgg     120
cgcaaccgtt tctttcttcc caaactaaag caaatatggg a                         161

<210> 16

<211> 897
<212> DNA
<213> Chlymidia trachomatis

<400> 16
atggcttcta tatgcggacg tttagggtct ggtacaggga atgctctaaa agcttttttt 60
acacagccca acaataaaat ggcaagggta gtaaataaga cgaagggaat ggataagact 120
attaaggttg ccaagtctgc tgccgaattg accgcaaata ttttggaaca agctggaggc 180
gcgggctctt ccgcacacat tacagcttcc caagtgtcca aaggattagg ggatgcgaga 240
actgttgtcg ctttagggaa tgcctttaac ggagcgttgc caggaacagt tcaaagtgcg 300
caaagcttct tctctcacat gaaagctgct agtcagaaaa cgcaagaagg ggatgagggg 360
ctcacagcag atctttgtgt gtctcataag cgcagagcgg ctgcggctgt ctgtagcatc 420
atcggaggaa ttacctacct cgcgacattc ggagctatcc gtccgattct gtttgtcaac 480
aaaatgctgg caaaaccgtt tctttcttcc caaactaaag caaatatggg atcttctgtt 540
agctatatta tggcggctaa ccatgcagcg tctgtggtgg gtgctggact cgctatcagt 600
gcggaaagag cagattgcga agcccgctgc gctcgtattg cgagagaaga gtcgttactc 660
gaagtgccgg gagaggaaaa tgcttgcgag aagaaagtcg ctggagagaa agccaagacg 720
ttcacgcgca tcaagtatgc actcctcact atgctcgaga agtttttgga atgcgttgcc 780
gacgttttca aattggtgcc gctgcctatt acaatgggta ttcgtgcgat tgtggctgct 840
ggatgtacgt tcacttctgc aattattgga ttgtgcactt tctgcgccag agcataa 897

<210> 17
<211> 298
<212> PRT
<213> Chlamydia trachomatis

<400> 17
Met Ala Ser Ile Cys Gly Arg Leu Gly Ser Gly Thr Gly Asn Ala Leu
1               5                   10                  15
Lys Ala Phe Phe Thr Gln Pro Asn Asn Lys Met Ala Arg Val Val Asn
            20                  25                  30
Lys Thr Lys Gly Met Asp Lys Thr Ile Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Ala Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110
Lys Thr Gln Glu Gly Asp Glu Gly Leu Thr Ala Asp Leu Cys Val Ser
        115                 120                 125
His Lys Arg Arg Ala Ala Ala Ala Val Cys Ser Ile Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Lys Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Glu Arg Ala Asp Cys Glu Ala
        195                 200                 205
Arg Cys Ala Arg Ile Ala Arg Glu Glu Ser Leu Leu Glu Val Pro Gly
    210                 215                 220

Glu Glu Asn Ala Cys Glu Lys Lys Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Ile
        275                 280                 285
Ile Gly Leu Cys Thr Phe Cys Ala Arg Ala
    290                 295

<210> 18
<211> 18
<212> PRT
<213> Chlamydia trachomatis

<400> 18
Arg Ala Ala Ala Ala Ala Ala Val Cys Ser Phe Ile Gly Gly Ile Thr
 1               5                  10                  15
Tyr Leu

<210> 19
<211> 18
<212> PRT
<213> Chlamydia trachomatis

<400> 19
Cys Ser Phe Ile Gly Gly Ile Thr Tyr Leu Ala Thr Phe Gly Ala Ile
 1               5                  10                  15
Arg Pro

<210> 20
<211> 216
<212> PRT
<213> Chlamydia trachomatis

<400> 20
Met Arg Gly Ser Gln Gln Ile Phe Val Cys Leu Ile Ser Ala Glu Arg
 1               5                  10                  15
Leu Arg Leu Ser Val Ala Ser Ser Glu Glu Leu Pro Thr Ser Arg His
            20                  25                  30
Ser Glu Leu Ser Val Arg Phe Cys Leu Ser Thr Lys Cys Trp Gln Asn
        35                  40                  45
Arg Phe Phe Leu Pro Lys Leu Lys Gln Ile Trp Asp Leu Leu Leu Ala
    50                  55                  60
Ile Leu Trp Arg Leu Thr Met Gln Arg Leu Trp Trp Val Leu Asp Ser
65                  70                  75                  80
Leu Ser Val Arg Lys Glu Gln Ile Ala Lys Pro Ala Ala Leu Val Leu
                85                  90                  95
Arg Glu Lys Ser Arg Tyr Ser Lys Cys Arg Glu Arg Lys Met Leu Ala
            100                 105                 110
Arg Arg Lys Ser Leu Glu Arg Lys Pro Arg Arg Ser Arg Ala Ser Ser
        115                 120                 125

Met His Ser Ser Leu Cys Ser Arg Ser Phe Trp Asn Ala Leu Pro Thr
    130                 135                 140
Phe Ser Asn Trp Cys Arg Cys Leu Leu Gln Trp Val Phe Val Arg Leu
145                 150                 155                 160
Trp Leu Leu Asp Val Arg Ser Leu Leu Gln Leu Leu Asp Cys Ala Leu
                165                 170                 175
Ser Ala Pro Glu His Lys Gly Phe Phe Lys Phe Leu Lys Lys Lys Ala
            180                 185                 190
Val Ser Lys Lys Lys Gln Pro Phe Leu Ser Thr Lys Cys Leu Ala Phe
        195                 200                 205
Leu Ile Val Lys Ile Val Phe Leu
    210                 215

<210> 21
<211> 1256
<212> DNA
<213> Chlamydia trachomatis

<400> 21
ctcgtgccgg cacgagcaaa gaaatccctc aaaaaatggc cattattggc ggtggtgtga      60
tcggttgcga attcgcttcc ttattccata cgttaggctc cgaagtttct gtgatcgaag     120
caagctctca aatccttgct ttgaataatc cagatatttc aaaaaccatg ttcgataaat     180
tcacccgaca aggactccgt ttcgtactag aagcctctgt atcaaatatt gaggatatag     240
gagatcgcgt tcggttaact atcaatggga atgtcgaaga atacgattac gttctcgtat     300
ctataggacg ccgtttgaat acagaaaata ttggcttgga taaagctggt gttatttgtg     360
atgaacgcgg agtcatccct accgatgcca caatgcgcac aaacgtacct aacatttatg     420
ctattggaga tatcacagga aaatggcaac ttgcccatgt agcttctcat caaggaatca     480
ttgcagcacg gaatataggt ggccataaag aggaaatcga ttactctgct gtcccttctg     540
tgatctttac cttccctgaa gtcgcttcag taggcctctc cccaacagca gctcaacaac     600
atctccttct tcgcttactt tttctgaaaa atttgataca gaagaagaat tcctcgcaca     660
cttgcgagga ggagggcgtc tggaagacca gttgaattta gctaagtttt ctgagcgttt     720
tgattctttg cgagaattat ccgctaagct tggttacgat agcgatggag agactgggga     780
tttcttcaac gaggagtacg acgacgaaga agaggaaatc aaaccgaaga aaactacgaa     840
acgtggacgt aagaagagcc gttcataagc cttgctttta aggtttggta gttttacttc     900
tctaaaatcc aaatggttgc tgtgccaaaa agtagtttgc gtttccggat agggcgtaaa     960
tgcgctgcat gaaagattgc ttcgagagcg gcatcgcgtg ggagatcccg gatactttct    1020
ttcagatacg aataagcata gctgttccca gaataaaaac ggccgacgct aggaacaaca    1080
agatttagat agagcttgtg tagcaggtaa actgggttat atgttgctgg gcgtgttagt    1140
tctagaatac ccaagtgtcc tccaggttgt aatactcgat acacttccct aagagcctct    1200
aatggatagg ataagttccg taatccatag gccatagaag ctaaacgaaa cgtatt        1256

<210> 22
<211> 601
<212> DNA
<213> Chlamydia trachomatis

<400> 22
ctcgtgccgg cacgagcaaa gaaatccctc aaaaaatggc cattattggc ggtggtgtga      60
tcggttgcga attcgcttcc ttattccata cgttaggctc cgaagtttct gtgatcgaag     120
caagctctca aatccttgct ttgaataatc cagatatttc aaaaaccatg ttcgataaat     180
tcacccgaca aggactccgt ttcgtactag aagcctctgt atcaaatatt gaggatatag     240
gagatcgcgt tcggttaact atcaatggga atgtcgaaga atacgattac gttctcgtat     300
ctataggacg ccgtttgaat acagaaaata ttggcttgga taaagctggt gttatttgtg     360
atgaacgcgg agtcatccct accgatgcca caatgcgcac aaacgtacct aacatttatg     420
ctattggaga tatcacagga aaatggcaac ttgcccatgt agcttctcat caaggaatca     480

ttgcagcacg gaatataggt ggccataaag aggaaatcga ttactctgct gtcccttctg 540
tgatctttac cttccctgaa gtcgcttcag taggcctctc cccaacagca gctcaacaac 600
a 601

<210> 23
<211> 270
<212> DNA
<213> Chlamydia trachomatis

<400> 23
acatctcctt cttcgcttac tttttctgaa aaatttgata cagaagaaga attcctcgca 60
cacttgcgag gaggagggcg tctggaagac cagttgaatt tagctaagtt ttctgagcgt 120
tttgattctt tgcgagaatt atccgctaag cttggttacg atagcgatgg agagactggg 180
gatttcttca acgaggagta cgacgacgaa gaagaggaaa tcaaaccgaa gaaaactacg 240
aaacgtggac gtaagaagag ccgttcataa 270

<210> 24
<211> 363
<212> DNA
<213> Chlamydia trachomatis

<400> 24
ttacttctct aaaatccaaa tggttgctgt gccaaaaagt agtttgcgtt tccggatagg 60
gcgtaaatgc gctgcatgaa agattgcttc gagagcggca tcgcgtggga gatcccggat 120
actttctttc agatacgaat aagcatagct gttcccagaa taaaaacggc cgacgctagg 180
aacaacaaga tttagataga gcttgtgtag caggtaaact gggttatatg ttgctgggcg 240
tgttagttct agaataccca agtgtcctcc aggttgtaat actcgataca cttccctaag 300
agcctctaat ggataggata agttccgtaa tccataggcc atagaagcta aacgaaacgt 360
att 363

<210> 25
<211> 696
<212> DNA
<213> Chlamydia trachomatis

<400> 25
gctcgtgccg gcacgagcaa agaaatccct caaaaaatgg ccattattgg cggtggtgtg 60
atcggttgcg aattcgcttc cttattccat acgttaggct ccgaagtttc tgtgatcgaa 120
gcaagctctc aaatccttgc tttgaataat ccagatattt caaaaaccat gttcgataaa 180
ttcacccgac aaggactccg tttcgtacta gaagcctctg tatcaaatat tgaggatata 240
ggagatcgcg ttcggttaac tatcaatggg aatgtcgaag aatacgatta cgttctcgta 300
tctataggac gccgtttgaa tacagaaaat attggcttgg ataaagctgg tgttatttgt 360
gatgaacgcg gagtcatccc taccgatgcc acaatgcgca caaacgtacc taacatttat 420
gctattggag atatcacagg aaaatggcaa cttgcccatg tagcttctca tcaaggaatc 480
attgcagcac ggaatatagg tggccataaa gaggaaatcg attactctgc tgtcccttct 540
gtgatcttta ccttccctga agtcgcttca gtaggcctct ccccaacagc agctcaacaa 600
catctccttc ttcgcttact tttttctgaa aatttgatac agaagaagaa ttcctcgcac 660
acttgcgagg aggagggcgt ctggaagacc agttga 696

<210> 26
<211> 231
<212> PRT
<213> Chlamydia trachomatis

<400> 26

Ala Arg Ala Gly Thr Ser Lys Glu Ile Pro Gln Lys Met Ala Ile Ile
1               5                   10                  15
Gly Gly Gly Val Ile Gly Cys Glu Phe Ala Ser Leu Phe His Thr Leu
            20                  25                  30
Gly Ser Glu Val Ser Val Ile Glu Ala Ser Ser Gln Ile Leu Ala Leu
        35                  40                  45
Asn Asn Pro Asp Ile Ser Lys Thr Met Phe Asp Lys Phe Thr Arg Gln
    50                  55                  60
Gly Leu Arg Phe Val Leu Glu Ala Ser Val Ser Asn Ile Glu Asp Ile
65                  70                  75                  80
Gly Asp Arg Val Arg Leu Thr Ile Asn Gly Asn Val Glu Glu Tyr Asp
                85                  90                  95
Tyr Val Leu Val Ser Ile Gly Arg Arg Leu Asn Thr Glu Asn Ile Gly
            100                 105                 110
Leu Asp Lys Ala Gly Val Ile Cys Asp Glu Arg Gly Val Ile Pro Thr
        115                 120                 125
Asp Ala Thr Met Arg Thr Asn Val Pro Asn Ile Tyr Ala Ile Gly Asp
    130                 135                 140
Ile Thr Gly Lys Trp Gln Leu Ala His Val Ala Ser His Gln Gly Ile
145                 150                 155                 160
Ile Ala Ala Arg Asn Ile Gly Gly His Lys Glu Glu Ile Asp Tyr Ser
                165                 170                 175
Ala Val Pro Ser Val Ile Phe Thr Phe Pro Glu Val Ala Ser Val Gly
            180                 185                 190
Leu Ser Pro Thr Ala Ala Gln Gln His Leu Leu Leu Arg Leu Leu Phe
        195                 200                 205
Leu Lys Asn Leu Ile Gln Lys Lys Asn Ser Ser His Thr Cys Glu Glu
    210                 215                 220
Glu Gly Val Trp Lys Thr Ser
225                 230

<210> 27
<211> 264
<212> DNA
<213> Chlamydia pneumoniae

<400> 27
atgagtcaaa aaaataaaaa ctctgctttt atgcatcccg tgaatatttc cacagattta      60
gcagttatag ttggcaaggg acctatgccc agaaccgaaa ttgtaaagaa agtttgggaa     120
tacattaaaa aacacaactg tcaggatcaa aaaaataaac gtaatatcct tcccgatgcg     180
aatcttgcca aagtctttgg ctctagtgat cctatcgaca tgttccaaat gaccaaagcc     240
ctttccaaac atattgtaaa ataa                                            264

<210> 28
<211> 87
<212> PRT
<213> Chlamydia pneumoniae

<400> 28
Met Ser Gln Lys Asn Lys Asn Ser Ala Phe Met His Pro Val Asn Ile
1               5                   10                  15
Ser Thr Asp Leu Ala Val Ile Val Gly Lys Gly Pro Met Pro Arg Thr
            20                  25                  30
Glu Ile Val Lys Lys Val Trp Glu Tyr Ile Lys Lys His Asn Cys Gln
        35                  40                  45

Asp Gln Lys Asn Lys Arg Asn Ile Leu Pro Asp Ala Asn Leu Ala Lys
    50                  55                  60
Val Phe Gly Ser Ser Asp Pro Ile Asp Met Phe Gln Met Thr Lys Ala
65                  70                  75                  80
Leu Ser Lys His Ile Val Lys
                85

<210> 29
<211> 369
<212> DNA
<213> Chlamydia pneumoniae

<400> 29
atgccacgca tcattggaat tgatattcct gcaaagaaaa agttaaaaat aagtctgaca      60
tatatttatg gaataggatc agctcgttct gatgaaatca ttaaaaagtt gaagttagat     120
cctgaggcaa gagcctctga attaactgaa gaagaagtag gacgactgaa ctctctgcta     180
caatcagaat ataccgtaga aggggatttg cgacgtcgtg ttcaatcgga tatcaaaaga     240
ttgatcgcca tccattctta tcgaggtcag agacatagac tttctttacc agtaagagga     300
caacgtacaa aaactaattc tcgtactcga aaaggtaaaa gaaaaacagt cgcaggtaag     360
aagaaataa                                                             369

<210> 30
<211> 122
<212> PRT
<213> Chlamydia pneumoniae

<400> 30
Met Pro Arg Ile Ile Gly Ile Asp Ile Pro Ala Lys Lys Lys Leu Lys
1               5                   10                  15
Ile Ser Leu Thr Tyr Ile Tyr Gly Ile Gly Ser Ala Arg Ser Asp Glu
            20                  25                  30
Ile Ile Lys Lys Leu Lys Leu Asp Pro Glu Ala Arg Ala Ser Glu Leu
        35                  40                  45
Thr Glu Glu Glu Val Gly Arg Leu Asn Ser Leu Leu Gln Ser Glu Tyr
    50                  55                  60
Thr Val Glu Gly Asp Leu Arg Arg Arg Val Gln Ser Asp Ile Lys Arg
65                  70                  75                  80
Leu Ile Ala Ile His Ser Tyr Arg Gly Gln Arg His Arg Leu Ser Leu
                85                  90                  95
Pro Val Arg Gly Gln Arg Thr Lys Thr Asn Ser Arg Thr Arg Lys Gly
            100                 105                 110
Lys Arg Lys Thr Val Ala Gly Lys Lys Lys
        115                 120

<210> 31
<211> 10
<212> PRT
<213> Artificial Sequence

<220>
<223> Made in the lab

<400> 31
Cys Ser Phe Ile Gly Gly Ile Thr Tyr Leu
1               5                   10

<210> 32
<211> 53
<212> PRT
<213> Chlamydia trachomatis

<400> 32
Leu Cys Val Ser His Lys Arg Arg Ala Ala Ala Ala Val Cys Ser Phe
1               5                   10                  15
Ile Gly Gly Ile Thr Tyr Leu Ala Thr Phe Gly Ala Ile Arg Pro Ile
            20                  25                  30
Leu Phe Val Asn Lys Met Leu Ala Gln Pro Phe Leu Ser Ser Gln Thr
        35                  40                  45
Lys Ala Asn Met Gly
    50

<210> 33
<211> 161
<212> DNA
<213> Chlamydia trachomatis

<400> 33
atctttgtgt gtctcataag cgcagagcgg ctgcggctgt ctgtagcatc atcggaggaa      60
ttacctacct cgcgacattc ggagctatcc gtccgattct gtttgtcaac aaaatgctgg     120
caaaaccgtt tctttcttcc caaactaaag caaatatggg a                         161

<210> 34
<211> 53
<212> PRT
<213> Chlamydia trachomatis

<400> 34
Leu Cys Val Ser His Lys Arg Arg Ala Ala Ala Ala Val Cys Ser Ile
1               5                   10                  15
Ile Gly Gly Ile Thr Tyr Leu Ala Thr Phe Gly Ala Ile Arg Pro Ile
            20                  25                  30
Leu Phe Val Asn Lys Met Leu Ala Lys Pro Phe Leu Ser Ser Gln Thr
        35                  40                  45
Lys Ala Asn Met Gly
    50

<210> 35
<211> 55
<212> DNA
<213> Chlamydia pneumoniae

<400> 35
gatatacata tgcatcacca tcaccatcac atgagtcaaa aaaaataaaa actct           55

<210> 36
<211> 33
<212> DNA
<213> Chlamydia pneumoniae

<400> 36

ctcgaggaat tcttatttta caatatgttt gga                                    33

<210> 37
<211> 53
<212> DNA
<213> Chlamydia pneumoniae

<400> 37
gatatacata tgcatcacca tcaccatcac atgccacgca tcattggaat gat             53

<210> 38
<211> 30
<212> DNA
<213> Chlamydia pneumoniae

<400> 38
ctcgaggaat tcttatttct tcttacctgc                                       30

<210> 39
<211> 16
<212> PRT
<213> Artificial Sequence

<220>
<223> Made in the lab

<400> 39
Lys Arg Asn Ile Asn Pro Asp Asp Lys Leu Ala Lys Val Phe Gly Thr
1               5                   10                  15

<210> 40
<211> 16
<212> PRT
<213> Artificial Sequence

<220>
<223> made in the lab

<400> 40
Lys Arg Asn Ile Leu Pro Asp Ala Asn Leu Ala Lys Val Phe Gly Ser
1               5                   10                  15

<210> 41
<211> 15
<212> PRT
<213> Artificial Sequence

<220>
<223> made in the lab

<400> 41
Lys Glu Tyr Ile Asn Gly Asp Lys Tyr Phe Gln Gln Ile Phe Asp
1               5                   10                  15

<210> 42
<211> 16
<212> PRT
<213> Artificial Sequence

<220>
<223> made in the lab

<400> 42
Lys Lys Ile Ile Ile Pro Asp Ser Lys Leu Gln Gly Val Ile Gly Ala
1               5                   10                  15

<210> 43
<211> 15
<212> PRT
<213> Artificial Sequence

<220>
<223> made in the lab

<400> 43
Lys Lys Leu Leu Val Pro Asp Asn Asn Leu Ala Thr Ile Ile Gly
1               5                   10                  15

<210> 44
<211> 509
<212> DNA
<213> Chlamydia

<400> 44
ggagctcgaa ttcggcacga gagtgcctat tgttttgcag gctttgtctg atgatagcga 60
taccgtacgt gagattgctg tacaagtagc tgttatgtat ggttctagtt gcttactgcg 120
cgccgtgggc gatttagcga aaaatgattc ttctattcaa gtacgcatca ctgcttatcg 180
tgctgcagcc gtgttggaga tacaagatct tgtgcctcat ttacgagttg tagtccaaaa 240
tacacaatta gatggaacgg aaagaagaga agcttggaga tctttatgtg ttcttactcg 300
gcctcatagt ggtgtattaa ctggcataga tcaagcttta atgacctgtg agatgttaaa 360
ggaatatcct gaaaagtgta cggaagaaca gattcgtaca ttattggctg cagatcatcc 420
agaagtgcag gtagctactt tacagatcat tctgagagga ggtagagtat tccggtcatc 480
ttctataatg gaatcggttc tcgtgccgg                                   509

<210> 45
<211> 481
<212> DNA
<213> Chlamydia

<220>
<221> unsure
<222> (23)
<223> n=A,T,C or G

<400> 45
gatccgaatt cggcacgagg cantatttac tcccaacatt acggttccaa ataagcgata 60
aggtcttcta ataaggaagt taatgtaaga ggctttttta ttgcttttcg taaggtagta 120
ttgcaaccgc acgcgattga atgatacgca agccatttcc atcatggaaa agaacccttg 180
gacaaaaata caaaggaggt tcactcctaa ccagaaaaag ggagagttag tttccatggg 240

ttttccttat atacacccgt ttcacacaat taggagccgc gtctagtatt tggaatacaa 300
attgtcccca agcgaatttt gttcctgttt cagggatttc tcctaattgt tctgtcagcc 360
atccgcctat ggtaacgcaa ttagctgtag taggaagatc aactccaaac aggtcataga 420
aatcagaaag ctcataggtg cctgcagcaa taacaacatt cttgtctgag tgagcgaatt 480
g 481

<210> 46
<211> 427
<212> DNA
<213> Chlamydia

<220>
<221> unsure
<222> (20)
<223> n=A,T,C or G

<400> 46
gatccgaatt cggcacgagn tttttcctgt tttttcttag tttttagtgt tcccggagca 60
ataacacaga tcaaagaacg gccattcagt ttaggctctg actcaacaaa acctatgtcc 120
tctaagccct gacacattct ttgaacaacc ttatgcccgt gttcgggata agccaactct 180
cgcccccgaa acatacaaga aacctttact ttatttcctt tctcaataaa ggctctagct 240
tgctttgctt tcgtaagaaa gtcgttatca tcgatattag gcttaagctt aacctctttg 300
atacgcactt ggtgctgtgc tttcttacta tctttttctt ttttagttat gtcgtaacga 360
tacttcccgt agtccatgat tttgcacaca ggaggctctg agtttgaagc aacctcgtgc 420
cgaattc 427

<210> 47
<211> 600
<212> DNA
<213> Chlamydia

<220>
<221> unsure
<222> (522)
<223> n=A,T,C or G

<400> 47
gatccgaatt cggcacgaga tgcttctatt acaattggtt tggatgcgga aaaagcttac 60
cagcttattc tagaaaagtt gggagatcaa attcttggtg gaattgctga tactattgtt 120
gatagtacag tccaagatat tttagacaaa atcacaacag acccttctct aggtttgttg 180
aaagctttta acaactttcc aatcactaat aaaattcaat gcaacgggtt attcactccc 240
aggaacattg aaactttatt aggaggaact gaaataggaa aattcacagt cacacccaaa 300
agctctggga gcatgttctt agtctcagca gatattattg catcaagaat ggaaggcggc 360
gttgttctag ctttggtacg agaaggtgat tctaagccct acgcgattag ttatggatac 420
tcatcaggcg ttcctaattt atgtagtcta agaaccagaa ttattaatac aggattgact 480
ccgacaacgt attcattacg tgtaggcggt ttagaaagcg gngtggtatg ggttaatgcc 540
ctttctaatg gcaatgatat tttaggaata acaaatcttc taatgtatct tttttggagg 600

<210> 48
<211> 600
<212> DNA
<213> Chlamydia

<400> 48
ggagctcgaa ttcggcacga gctctatgaa tatccaattc tctaaactgt tcggataaaa 60

atgatgcagg aattaggtcc acactatctt tttttgtttc gcaaatgatt gattttaaat 120
cgtttgatgt gtatactatg tcgtgtaagc ctttttggtt acttctgaca ctagccccca 180
atccagaaga taaattggat tgcgggtcta ggtcagcaag taacactttt ttccctaaaa 240
attgggccaa gttgcatccc acgtttagag aaagtgttgt ttttccagtt cctcccttaa 300
aagagcaaaa aactaaggtg tgcaaatcaa ctccaacgtt agagtaagtt atctattcag 360
ccttggaaaa catgtctttt ctagacaaga taagcataat caaagccttt tttagcttta 420
aactgttatc ctctaatttt tcaagaacag gagagtctgg gaataatcct aaagagtttt 480
ctatttgttg aagcagtcct agaattagtg agacactttt atggtagagt tctaagggag 540
aatttaagaa agttactttt tccttgttta ctcgtatttt taggtctaat tcggggaaat 600

<210> 49
<211> 600
<212> DNA
<213> Chlamydia

<400> 49
   gatccgaatt cggcacgaga tgcttctatt acaattggtt tggatgcgga aaaagcttac 60
   cagcttattc tagaaaagtt gggagatcaa attcttggtg gaattgctga tactattgtt 120
   gatagtacag tccaagatat tttagacaaa atcacaacag acccttctct aggtttgttg 180
   aaagctttta acaactttcc aatcactaat aaaattcaat gcaacgggtt attcactccc 240
   aggaacattg aaactttatt aggaggaact gaaataggaa aattcacagt cacacccaaa 300
   agctctggga gcatgttctt agtctcagca gatattattg catcaagaat ggaaggcggc 360
   gttgttctag ctttggtacg agaaggtgat tctaagccct acgcgattag ttatggatac 420
   tcatcaggcg ttcctaattt atgtagtcta agaaccagaa ttattaatac aggattgact 480
   ccgacaacgt attcattacg tgtaggcggt ttagaaagcg gtgtggtatg ggttaatgcc 540
   ctttctaatg gcaatgatat tttaggaata acaaatactt ctaatgtatc ttttttggag 600

<210> 50
<211> 406
<212> DNA
<213> Chlamydia

<400> 50
gatccgaatt cggcacgagt tcttagcttg cttaattacg taattaacca aactaaaggg 60
gctatcaaat agcttattca gtctttcatt agttaaacga tcttttctag ccatgactca 120
tcctatgttc ttcagctata aaaatacttc ttaaaacttg atatgctgta atcaaatcat 180
cattaaccac aacataatca aattcgctag cggcagcaat ttcgacagcg ctatgctcta 240
atctttcttt cttctggaaa tctttctctg aatcccgagc attcaaacgg cgctcaagtt 300
cttcttgaga gggagcttga ataaaaatgt gactgccggc atttgcttct tcagagccaa 360
agctccttgt acatcaatca cggctatgca gtctcgtgcc gaattc 406

<210> 51
<211> 602
<212> DNA
<213> Chlamydia

<400> 51
gatccgaatt cggcacgaga tattttagac aaaatcacaa cagacccttc tctaggtttg 60
ttgaaagctt ttaacaactt tccaatcact aataaaattc aatgcaacgg gttattcact 120
cccaggaaca ttgaaacttt attaggagga actgaaatag gaaaattcac agtcacaccc 180
aaaagctctg ggagcatgtt cttagtctca gcagatatta ttgcatcaag aatggaaggc 240
ggcgttgttc tagctttggt acgagaaggt gattctaagc cctacgcgat tagttatgga 300
tactcatcag gcgttcctaa tttatgtagt ctaagaacca gaattattaa tacaggattg 360
actccgacaa cgtattcatt acgtgtaggc ggtttagaaa gcggtgtggt atgggttaat 420
gccctttcta atggcaatga tattttagga ataacaaata cttctaatgt atcttttttg 480

gaggtaatac ctcaaacaaa cgcttaaaca atttttattg gatttttctt ataggtttta 540
tatttagaga aaaaagttcg aattacgggg tttgttatgc aaaataaact cgtgccgaat 600
tc 602

<210> 52
<211> 145
<212> DNA
<213> Chlamydia

<400> 52
gatccgaatt cggcacgagc tcgtgccgat gtgttcaaca gcatccatag gatgggcagt 60
caaatatact ccaagtaatt ctttttctct tttcaacaac tccttaggag agcgttggat 120
aacattttca gctcgtgccg aattc 145

<210> 53
<211> 450
<212> DNA
<213> Chlamydia

<400> 53
gatccgaatt cggcacgagg taatcggcac cgcactgctg acactcatct cctcgagctc 60
gatcaaaccc acacttggga caagtaccta caacataacg gtccgctaaa aacttccctt 120
cttcctcaga atacagctgt tcggtcacct gattctctac cagtccgcgt tcctgcaagt 180
ttcgatagaa atcttgcaca atagcaggat gataagcgtt cgtagttctg gaaaagaaat 240
ctacagaaat tcccaatttc ttgaaggtat ctttatgaag cttatgatac atgtcgacat 300
attcttgata ccccatgcct gccaactctg cattaagggt aattgcgatt ccgtattcat 360
cagaaccaca aatatacaaa acctctttgc cttgtagtct ctgaaaacgc gcataaacat 420
ctgcaggcaa ataagcctcg tgccgaattc 450

<210> 54
<211> 716
<212> DNA
<213> Chlamydia

<400> 54
gatcgaaatt cggcacgagc ggcacgagtt ttctgatagc gatttacaat cctttattca 60
acttttgcct agagaggcac actatactaa gaagtttctt gggtgtgtgg cacagtcctg 120
tcgtcagggg attctgctag aggggtaggg gaaaaaaccc ttattactat gaccatgcgc 180
atgtggaatt acattccata gactttcgca tcattcccaa catttacaca gctctacacc 240
tcttaagaag aggtgacgtg gattgggtgg ggcagccttg gcaccaaggg attccttttg 300
agcttcggac tacctctgct ctctacaccc attaccctgt agatggcaca ttctggctta 360
ttcttaatcc caaagatcct gtactttcct ctctatctaa tcgtcagcga ttgattgctg 420
ccatccaaaa ggaaaaactg gtgaagcaag ctttaggaac acaatatcga gtagctgaaa 480
gctctccatc tccagaggga atcatagctc atcaagaagc ttctactcct tttcctggga 540
aaattacttt gatatatccc aataatatta cgcgctgtca gcgtttggcc gaggtatcca 600
aaaaatgatc gacaaggagc acgctaaatt tgtacatacc ccaaaatcaa tcagccatct 660
aggcaaatgg aatatcaaag taaacagtat acaactgggg atctcgtgcc gaattc 716

<210> 55
<211> 463
<212> DNA
<213> Chlamydia trachomatis

<400> 55
tctcaaatcc ttgctttgaa taatccagat atttcaaaaa ccatgttcga taaattcacc 60

cgacaaggac tccgtttcgt actagaagcc tctgtatcaa atattgagga tataggagat 120
cgcgttcggt taactatcaa tgggaatgtc gaagaatacg attacgttct cgtatctata 180
ggacgccgtt tgaatacaga aaatattggc ttggataaag ctggtgttat ttgtgatgaa 240
cgcggagtca tccctaccga tgccacaatg cgcacaaacg tacctaacat ttatgctatt 300
ggagatatca caggaaaatg gcaacttgcc catgtagctt ctcatcaagg aatcattgca 360
gcacggaata taggtggcca taaagaggaa atcgattact ctgctgtccc ttctgtgatc 420
tttaccttcc ctgaagtcgc ttcagtaggc ctctccccaa cag 463

<210> 56
<211> 829
<212> DNA
<213> Chlamydia trachomatis

<400> 56
   gtactatggg atcattagtt ggaagacagg ctccggattt ttctggtaaa gccgttgttt 60
   gtggagaaga gaaagaaatc tctctagcag actttcgtgg taagtatgta gtgctcttct 120
   tttatcctaa agattttacc tatgtttgtc ctacagaatt acatgctttt caagatagat 180
   tggtagattt tgaagagcat ggtgcagtcg tccttggttg ctccgttgac gacattgaga 240
   cacattctcg ttggctcact gtagcgagag atgcaggagg gatagaggga acagaatatc 300
   ctctgttagc agacccctct tttaaaatat cagaagcttt tggtgttttg aatcctgaag 360
   gatcgctcgc tttaagagct actttcctta tcgataaaca tggggttatt cgtcatgcgg 420
   ttatcaatga tcttccttta gggcgttcca ttgacgagga attgcgtatt ttagattcat 480
   tgatcttctt tgagaaccac ggaatggttt gtccagctaa ctggcgttct ggagagcgtg 540
   gaatggtgcc ttctgaagag ggattaaaag aatacttcca gacgatggat taagcatctt 600
   tgaaagtaag aaagtcgtac agatcttgat ctgaaaagag aagaaggctt tttaattttc 660
   tgcagagagc cagcgaggct tcaataatgt tgaagtctcc gacaccaggc aatgctaagg 720
   cgacgatatt agttagtgaa gtctgagtat taaggaaatg aaggccaaag aaatagctat 780
   caataaagaa gccttcttcc ttgactctaa agaatagtat gtcgtatcc 829

<210> 57
<211> 1537
<212> DNA
<213> Chlamydia trachomatis

<400> 57
acatcaagaa atagcggact cgcctttagt gaaaaaagct gaggagcaga ttaatcaagc 60
acaacaagat attcaaacga tcacacctag tggtttggat attcctatcg ttggtccgag 120
tgggtcagct gcttccgcag gaagtgcggc aggagcgttg aaatcctcta acaattcagg 180
aagaatttcc ttgttgcttg atgatgtaga caatgaaatg gcagcgattg caatgcaagg 240
ttttcgatct atgatcgaac aatttaatgt aaacaatcct gcaacagcta aagagctaca 300
agctatggag gctcagctga ctgcgatgtc agatcaactg gttggtgcgg atggcgagct 360
cccagccgaa atacaagcaa tcaaagatgc tcttgcgcaa gctttgaaac aaccatcagc 420
agatggttta gctacagcta tgggacaagt ggcttttgca gctgccaagg ttggaggagg 480
ctccgcagga acagctggca ctgtccagat gaatgtaaaa cagctttaca agacagcgtt 540
ttcttcgact tcttccagct cttatgcagc agcactttcc gatggatatt ctgcttacaa 600
aacactgaac tctttatatt ccgaaagcag aagcggcgtg cagtcagcta ttagtcaaac 660
tgcaaatccc gcgctttcca gaagcgtttc tcgttctggc atagaaagtc aaggacgcag 720
tgcagatgct agccaaagag cagcagaaac tattgtcaga gatagccaaa cgttaggtga 780
tgtatatagc cgcttacagg ttctggattc tttgatgtct acgattgtga gcaatccgca 840
agcaaatcaa gaagagatta tgcagaagct cacggcatct attagcaaag ctccacaatt 900
tgggtatcct gctgttcaga attctgtgga tagcttgcag aagtttgctg cacaattgga 960
aagagagttt gttgatgggg aacgtagtct cgcagaatct caagagaatg cgtttagaaa 1020
acagcccgct ttcattcaac aggtgttggt aaacattgct tctctattct ctggttatct 1080
ttcttaacgt gtgattgaag tttgtgaatt gagggggagc caaaaaagaa tttctttttt 1140
ggctcttttt tcttttcaaa ggaatctcgt gtctacagaa gtcttttcaa taataagttc 1200

ttagttccaa aagaagaaaa tatataaaag aaaaaactcc taattcattt aaaaagtgct 1260
cggcagactt cgtggaaaat gtctgtaaag ctggagggga atcagcagaa agatgcaaga 1320
tatccgagaa aaaaggctca ggctcgtgcc gaattcggca cgagactacg aaagaaaggt 1380
cttttctttc ggaatctgtc attggatctg cgtaagactt aaagttcggc aacacaggct 1440
ctgtcttctc tttaggtttc ttgcgcgaga aaaattttct caagtaacaa gaagatttct 1500
ttttacagcc ggcatccggc ttctcgcgaa gtataac 1537

<210> 58
<211> 463
<212> DNA
<213> Chlamydia trachomatis

<400> 58
tctcaaatcc ttgctttgaa taatccagat atttcaaaaa ccatgttcga taaattcacc 60
cgacaaggac tccgtttcgt actagaagcc tctgtatcaa atattgagga tataggagat 120
cgcgttcggt taactatcaa tgggaatgtc gaagaatacg attacgttct cgtatctata 180
ggacgccgtt tgaatacaga aaatattggc ttggataaag ctggtgttat ttgtgatgaa 240
cgcggagtca tccctaccga tgccacaatg cgcacaaacg tacctaacat ttatgctatt 300
ggagatatca caggaaaatg gcaacttgcc catgtagctt ctcatcaagg aatcattgca 360
gcacggaata taggtggcca taaagaggaa atcgattact ctgctgtccc ttctgtgatc 420
tttaccttcc ctgaagtcgc ttcagtaggc ctctccccaa cag 463

<210> 59
<211> 552
<212> DNA
<213> Chlamydia trachomatis

<400> 59
acattcctcc tgctcctcgc ggccatccac aaattgaggt aaccttcgat attgatgcca 60
acggaatttt acacgtttct gctaaagatg ctgctagtgg acgcgaacaa aaaatccgta 120
ttgaagcaag ctctggatta aaagaagatg aaattcaaca aatgatccgc gatgcagagc 180
ttcataaaga ggaagacaaa caacgaaaag aagcttctga tgtgaaaaat gaagccgatg 240
gaatgatctt tagagccgaa aaagctgtga aagattacca cgacaaaatt cctgcagaac 300
ttgttaaaga aattgaagag catattgaga aagtacgcca agcaatcaaa gaagatgctt 360
ccacaacagc tatcaaagca gcttctgatg agttgagtac tcgtatgcaa aaaatcggag 420
aagctatgca ggctcaatcc gcatccgcag cagcatcttc tgcagcgaat gctcaaggag 480
ggccaaacat taactccgaa gatctgaaaa aacatagttt cagcacacga cctccagcag 540
gaggaagcgc ct 552

<210> 60
<211> 1180
<212> DNA
<213> Chlamydia trachomatis

<400> 60
atcctagcgg taaaactgct tactggtcag ataaaatcca tacagaagca acacgtactt 60
cttttaggag aaaaaatcta taatgctaga aaaatcctga gtaaggatca cttctcctca 120
acaacttttt catcttggat agagttagtt tttagaacta agtcttctgc ttacaatgct 180
cttgcatatt acgagctttt tataaacctc cccaaccaaa ctctacaaaa agagtttcaa 240
tcgatcccct ataaatccgc atatattttg gccgctagaa aaggcgattt aaaaaccaag 300
gtcgatgtga tagggaaagt atgtggaatc tcgtgccgaa ttcggcacga gcggcacgag 360
gatgtagagt aattagttaa agagctgcat aattatgaca aagcatggaa aacgcattcg 420
tggtatccaa gagacttacg atttagctaa gtcgtattct ttgggtgaag cgatagatat 480
tttaaaacag tgtcctactg tgcgtttcga tcaaacggtt gatgtgtctg ttaaattagg 540
gatcgatcca agaaagagtg atcagcaaat tcgtggttcg gtttctttac ctcacggtac 600

aggtaaagtt ttgcgaattt tagtttttgc tgctggagat aaggctgcag aggctattga 660
agcaggagcg gactttgttg gtagcgacga cttggtagaa aaaatcaaag gtggatgggt 720
tgacttcgat gttgcggttg ccactcccga tatgatgaga gaggtcggaa agctaggaaa 780
agttttaggt ccaagaaacc ttatgcctac gcctaaagcc ggaactgtaa caacagatgt 840
ggttaaaact attgcggaac tgcgaaaagg taaaattgaa tttaaagctg atcgagctgg 900
tgtatgcaac gtcggagttg cgaagctttc tttcgatagt gcgcaaatca aagaaaatgt 960
tgaagcgttg tgtgcagcct tagttaaagc taagcccgca actgctaaag gacaatattt 1020
agttaatttc actatttcct cgaccatggg gccaggggtt accgtggata ctagggagtt 1080
gattgcgtta taattctaag tttaaagagg aaaaatgaaa gaagagaaaa agttgctgct 1140
tcgcgaggtt gaagaaaaga taaccgcttc tcggcacgag 1180

<210> 61
<211> 1215
<212> DNA
<213> Chlamydia trachomatis

<400> 61
attacagcgt gtgcaggtaa cgacatcatt gcatgatgct tttgatggca ttgatgcggc 60
attccttata gggtcagttc ctagaggccc aggaatggag agaagagatc ttctaaagaa 120
aaatggggag attgttgcta cgcaaggaaa agctttgaac acaacagcca agcgggatgc 180
aaagattttt gttgttggga accctgtgaa taccaattgc tggatagcaa tgaatcatgc 240
tcccagatta ttgagaaaga actttcatgc gatgctacga ttggaccaga atcgtatgca 300
tagcatgtta tcgcatagag cagaagtacc tttatcggct gtatcacaag ttgtggtttg 360
gggaaatcac tccgccaaac aagtgcctga ttttacgcaa gctctgatta atgaccgtcc 420
tatcgcagag acgatagcgg atcgtgattg gttagagaat attatggtgc cttctgtaca 480
gagtcgtggt agtgcagtaa ttgaagcacg agggaagtct tcggcagctt ctgcagcacg 540
agctttagca gaggctgctc gatcaatata tcagccaaaa gaaggactcg tgccgaattc 600
ggcacgagta tcgaaattgc aggcatttct agtgaatggt cgtatgctta taaactacgt 560
ggtacagact tgagctctca aaagtttgct acagattctt acatcgcaga cccttattct 720
aagaatatct actcccctca actatttgga tcccctaaac aagaaaagga ttacgcattt 780
agttacctga aatatgagga ttttgactgg gaaggcgaca ctcctttgca ccttccaaaa 840
gaaaattact tcatttatga aatgcatgtt cggtcattca cccgagatcc gtcttcccag 900
gtttcccatc ctggaacttt ccttggtatc atcgaaaaaa tagaccacct caaacaacta 960
ggcgttcatg cagttgaact ccttcctatt ttcgaattcg atgaaaccgt ccatccattt 1020
aaaaatcagg acttccccca cctgtgtaac tattgggggt attcttcggt gaattttttc 1080
tgcccctctc gccgttatac ttatggggca gacccttgcg ctccggcccg agagttcaag 1140
actcttgtca aagcgttaca ccgtgcggga atcgaagtca ttctcgatgt cgttttcaat 1200
catacaggct ttgaa 1215

<210> 62
<211> 688
<212> DNA
<213> Chlamydia trachomatis

<400> 62
gtggatccaa aaaagaatct aaaaagccat acaaagattg cgttacttct tgcgatgcct 60
ctaacacttt atcagcgtca tctttgagaa gcatctcaat gagcgctttt tcttctctag 120
catgccgcac atccgcttct tcatgttctg tgaaatatgc atagtcttca ggattggaaa 180
atccaaagta ctcagtcaat ccacgaattt tctctctagc gatacgtgga atttgactct 240
cataagaata caaagcagcc actcctgcag ctaaagaatc tcctgtacac caccgcatga 300
aagtagctac tttcgctttt gctgcttcac taggctcatg agcctctaac tcttctggag 360
taactcctag agcaaacaca aactgcttcc acaaatcaat atgattaggg taaccgttct 420
cttcatccat caagttatct aacaataact tacgcgcctc taaatcatcg caacgactat 480
gaatcgcaga taaatattta ggaaaggctt tgatatgtaa ataatagtct ttggcacgag 540
cctgtaattg ctctttagta agctccccct tcgaccattt cacataaaac gtgtgttcta 600

gcatatgctt attttgaata attaaatcta actgatctaa aaaattcata aacacctcca 660
tcatttcttt tcttgactcc acgtaacc 688

<210> 63
<211> 269
<212> DNA
<213> Chlamydia trachomatis

<400> 63
atgttgaaat cacacaagct gttcctaaat atgctacggt aggatctccc tatcctgttg 60
aaattactgc tacaggtaaa agggattgtg ttgatgttat cattactcag caattaccat 120
gtgaagcaga gttcgtacgc agtgatccag cgacaactcc tactgctgat ggtaagctag 180
tttggaaaat tgaccgctta ggacaaggcg aaaagagtaa aattactgta tgggtaaaac 240
ctcttaaaga aggttgctgc tttacagct 269

<210> 64
<211> 1339
<212> DNA
<213> Chlamydia trachomatis

<400> 64
cttttattat ggcttctggg gatgatgtca acgatatcga cctgctatct cgaggagatt 60
ttaaaattgt tatacagacg gctccagagg agatgcatgg attagcggac tttttggctc 120
ccccggcgaa ggatcttggt attctctccg cctgggaagc tggtgagctg cgttacaaac 180
agctagttaa tccttaggaa acatttctgg acctatgccc atcacattgg ctccgtgatc 240
cacatagaga gtttctcccg taattgcgct agctagggga gagactaaga aggctgctgc 300
tgcgcctact tgctcagctt ccattggaga aggtagtgga gcccagtctt ggtagtaatc 360
caccattctc tcaataaatc caatagcttt tcctgcacgg ctagctaatg gccctgccga 420
gatagtattc actcggactc cccaacgtcg gccggcttcc caagccagta cttttgtatc 480
actttctaaa gcagcttttg ctgcgttcat tcctccgcca taccctggaa cagcacgcat 540
ggaagcaaga taagttagag agatggtgct agctcctgca ttcataattg ggccaaaatg 600
agagagaagg ctgataaagg agtagctgga tgtacttaag gcggcaagat agcctttacg 660
agaggtatca agtaatggtt tagcaatttc cggactgttt gctaaagagt gaacaagaat 720
atcaatgtgt ccaaaatctt ttttcacctg ttctacaact tcggatacag tgtacccaga 780
aagatctttg taacgtttat tttccaaaat ttcctgagga atatcttctg gggtgtcgaa 840
actggcatcc atgggataga ttttagcgaa agttagcaat tctccattgg agagttcacg 900
agatgcattg aattttccta actcccaaga ttgagagaaa attttataga taggaaccca 960
ggtccccaca agtatggttg cgcctgcttc tgctaacatt ttggcaatgc cccagccata 1020
cccgttatca tcgcctatgc cggctatgaa agcaattttt cctgttaaat caattttcaa 1080
catgagctaa ccccattttg tcttcttgag agaggagagt agcagattct ttattattga 1140
gaaacgggcc tcataataca taaggagtag attcactggc tggatccagg tttctagagt 1200
aaagagtttc cttgtcaaat tcttatatgg gtagagttaa tcaactgttt tcaagtgatt 1260
tatgtttatt ttaaaataat ttgttttaac aactgtttaa tagttttaat ttttaaagtg 1320
tgaaaaacag gttttatat 1339

<210> 65
<211> 195
<212> PRT
<213> Chlamydia trachomatis

<400> 65
Met Gly Ser Leu Val Gly Arg Gln Ala Pro Asp Phe Ser Gly Lys Ala
                5                   10                  15

Val Val Cys Gly Glu Glu Lys Glu Ile Ser Leu Ala Asp Phe Arg Gly

            20                  25                  30

Lys Tyr Val Val Leu Phe Phe Tyr Pro Lys Asp Phe Thr Tyr Val Cys
        35                  40                  45

Pro Thr Glu Leu His Ala Phe Gln Asp Arg Leu Val Asp Phe Glu Glu
    50                  55                  60

His Gly Ala Val Val Leu Gly Cys Ser Val Asp Asp Ile Glu Thr His
65                  70                  75                  80

Ser Arg Trp Leu Thr Val Ala Arg Asp Ala Gly Gly Ile Glu Gly Thr
                85                  90                  95

Glu Tyr Pro Leu Leu Ala Asp Pro Ser Phe Lys Ile Ser Glu Ala Phe
            100                 105                 110

Gly Val Leu Asn Pro Glu Gly Ser Leu Ala Leu Arg Ala Thr Phe Leu
        115                 120                 125

Ile Asp Lys His Gly Val Ile Arg His Ala Val Ile Asn Asp Leu Pro
    130                 135                 140

Leu Gly Arg Ser Ile Asp Glu Glu Leu Arg Ile Leu Asp Ser Leu Ile
145                 150                 155                 160

Phe Phe Glu Asn His Gly Met Val Cys Pro Ala Asn Trp Arg Ser Gly
                165                 170                 175

Glu Arg Gly Met Val Pro Ser Glu Glu Gly Leu Lys Glu Tyr Phe Gln
            180                 185                 190

Thr Met Asp
        195

<210> 66
<211> 520
<212> DNA
<213> Chlamydia

<400> 66
gatccgaatt cggcacgagg aggaatggaa gggccctccg attttaaatc tgctaccatg 60
ccattcacta gaaactccat aacagcggtt ttctctgatg gcgagtaaga agcaagcatt 120
tgatgtaaat tagcgcaatt agagggggat gaggttactt ggaaatataa ggagcgaagc 180
gatgaaggag atgtatttgc tctggaagca aaggtttctg aagctaacag aacattgcgt 240
cctccaacaa tcgcctgagg attctggctc atcagttgat gctttgcctg aatgagagcg 300
gacttaagtt tcccatcaga gggagctatt tgaattagat aatcaagagc tagatccttt 360
attgtgggat cagaaaattt acttgtgagc gcatcgagaa tttcgtcaga agaagaatca 420
tcatcgaacg aatttttcaa tcctcgaaaa tcttctccag agacttcgga aagatcttct 480
gtgaaacgat cttcaagagg agtatcgcct ttttcctctg 520

<210> 67
<211> 276
<212> DNA

<213> Chlamydia

<400> 67
gatccgaatt cggcacgagg tattgaagga gaaggatctg actcgatcta tgaaatcatg 60
atgcctatct atgaagttat gaatatggat ctagaaacac gaagatcttt tgcggtacag 120
caagggcact atcaggaccc aagagcttca gattatgacc tcccacgtgc tagcgactat 180
gatttgccta gaagcccata tcctactcca cctttgcctt ctagatatca gctacagaat 240
atggatgtag aagcagggtt ccgtgaggca gtttat 276

<210> 68
<211> 248
<212> DNA
<213> Chlamydia

<400> 68
gatccgaatt cggcacgagg tgttcaagaa tatgtccttc aagaatgggt taaattgaaa 60
gatctaccgg tagaagagtt gctagaaaaa cgatatcaga aattccgaac gataggtcta 120
tatgaaactt cttctgaaag cgattctgag gcataagaag catttagttt tattcggttt 180
ttctctttta tccatattag ggctaacgat aacgtctcaa gcagaaattt tttctctagg 240
tcttattg 248

<210> 69
<211> 715
<212> DNA
<213> Chlamydia

<220>
<221> unsure
<222> (34)
<223> n=A,T,C or G

<400> 69
gatccgaatt cggcacgaga aggtagatcc gatntcagca aaagtgctcc taaaggaaga 60
ttccttcggt atcctgcagc aaataaggtg gcacactcca tctcggacag tttgagcttt 120
attttcatat agttttcgac ggaactcttt attaaactcc caaaaccgaa tgttagtcgt 180
gtgggtgatg cctatatggt aagggaggtt tttggcttcg agaatattgg tgatcatttt 240
ttgtacgaca aaattagcta atgcagggac ctctgggggg aagtatgcat ctgatgttcc 300
atcttttcgg atgctagcaa cagggacaaa ataatctcct atttggtagt gggatcttaa 360
gcctccgcac atgcccaaca tgatcgctgc tgtagcattg ggaaggaaag aacacagatc 420
tacggtaaga gctgctcctg gagagcctaa tttaaaatcg atgattgagg tgtgaatttg 480
aggcgcatgc gctgccgaaa acatggatcc tcgagaaaca gggacctgat agatttcagc 540
gaaaacatcc acggtaatac ccmaaattag taagaaggag atagggctgg aactcttgaa 600
tggtagagcc ggtatagcgc tctagcatgt cacaggcgat tgtttcttcg ctgatttttt 660
tatgttgatg ggtcataaat cacagatatt ataatggtta gagaatcttt ttttc 715

<210> 70
<211> 323
<212> DNA
<213> Chlamydia

<400> 70
gatccgaatt cggcacgagc agaacgtaaa cagcacactt aaaccgtgta tgaggtttaa 60
cactgtttgg caagcaaaca accattcctc tttccacatc gttcttacca atacctctga 120
ggagcaatcc aacattctct cctgcacgac cttctgggag ttcttttctg aacatttcaa 180
ccccagtaac aatcgtttct ttagtatctc taagaccgac caactgaact ttatcggaaa 240

ctttaacaat tccacgctca atacgtccag ttactacagt tcctcgtccg gagatagaga 300
acacgtcctc aatgggcatt aag 323

<210> 71
<211> 715
<212> DNA
<213> Chlamydia

<400> 71
gatccgaatt cggcacgagg aaaaaaagat tctctaacca ttataatatc tgtgatttat 60
gacccatcaa cataaaaaaa tcagcgaaga aacaatcgcc tgtgacatgc tagagcggct 120
ataccggctc taccattcaa gagttccagc cctatctcct tcttactaat tttgggtatt 180
acgtggatgt tttcgctgaa atctatcagg tccctgtttc tcgaggatcc atgttttcgg 240
gcagcgcatg cgcctcaaat tcacacctca atcatcgatt ttaaattagg ctctccagga 300
gcagctctta ccgtagatct gtgttctttc cttcccaatg ctacagcagc gatcatgttg 360
ggcatgtgcg gaggcttaag atcccactac caaataggag attattttgt ccctgttgct 420
agcatccgaa aagatggaac atcagatgca tacttccccc cagaggtccc tgcattagct 480
aattttgtcg tacaaaaaat gatcaccaat attctcgaag ccaaaaacct cccttaccat 540
ataggcatca cccacacgac taacattcgg ttttgggagt ttaataaaga gttccgtcga 600
aaactatatg aaaataaagc tcaaactgtc gagatggagt gtgccacctt atttgctgca 660
ggataccgaa ggaatcttcc tttaggagca cttttgctga tatcggatct acctt 715

<210> 72
<211> 641
<212> DNA
<213> Chlamydia

<220>
<221> unsure
<222> (550)
<223> n=A,T,C or G
<221> unsure
<222> (559)
<223> n=A,T,C or G
<221> unsure
<222> (575)
<223> n=A,T,C or G
<221> unsure
<222> (583)
<223> n=A,T,C or G
<221> unsure
<222> (634)
<223> n=A,T,C or G
<221> unsure
<222> (638)
<223> n=A,T,C or G

<400> 72
gatccgaatt cggcacgaga tctcctcgag ctcgatcaaa cccacacttg ggacaagtac 60
ctacaacata acggtccgct aaaaacttcc cttcttcctc agaatacagc tgttcggtca 120
cctgattctc taccagtccg cgttcctgca agtttcgata gaaatcttgc acaatagcag 180
gatgataagc gttcgtagtt ctggaaaaga aatctacaga aattcccaat ttcttgaagg 240
tatctttatg aagcttatga tacatgtcga catattcttg ataccccatg cctgccaact 300
ctgcattaag ggtaattgcg attccgtatt catcagaacc acaaatatac aaaacctctt 360
tgccttgtag tctctgaaaa cgcgcataaa catctgcagg caaataagca ccggtaatat 420

gtccaaaatg caaaggacca tttgcgtaag gcaacgcaga agtaataaga atacgggaag 480
attccactat ttcacgtcgc tccagttgta cagagaagga tcttttcttc tggatgttcc 540
gaaaccttgn tctcttcgnc tctctcctgt agcanacaaa tgnctctctc gacatctctt 600
tcagcgtatt cggactgatg ccctaaagat cccnggangt t 641

<210> 73
<211> 584
<212> DNA
<213> Chlamydia

<220>
<221> unsure
<222> (460)
<223> n=A,T,C or G
<221> unsure
<222> (523)
<223> n=A,T,C or G
<221> unsure
<222> (541)
<223> n=A,T,C or G
<221> unsure
<222> (546)
<223> n=A,T,C or G

<400> 73
gaattcggca cgagacattt ctagaatgga accggcaaca aacaaaaact ttgtatctga 60
agatgacttt aagcaatctt tagataggga agattttttg gaatgggtct ttttatttgg 120
gacttattac ggaacgagta aggcggagat ttctagagtt ctgcaaaagg gtaagcactg 180
catagccgtg attgatgtac aaggagcttt ggctctgaag aagcaaatgc cggcagtcac 240
tatttttatt caagctccct ctcaagaaga acttgagcgc cgtttgaatg ctcgggattc 300
agagaaagat ttccagaaga aagaaagatt agagcatagc gctgtcgaaa ttgctgccgc 360
tagcgaattt gattatgttg tggttaatga tgatttgatt acagcatatc aagttttaag 420
aagtattttt atagctgaag aacataggat gagtcatggn tagaaaagat cgtttaacta 480
atgaaagact gaataagcta tttgatagcc cctttagttt ggntaattac gtaattaagc 540
nagctnagaa caaaattgct agaggagatg ttcgttcttc taac 584

<210> 74
<211> 465
<212> DNA
<213> Chlamydia

<400> 74
gatccgaatt cggcacgagc tcgtgccgtt tgggatcgtg taatcgcatc ggagaatggt 60
taagaaatta ttttcgagtg aaagagctag gcgtaatcat tacagatagc catactactc 120
caatgcggcg tggagtactg ggtatcgggc tgtgttggta tggattttct ccattacaca 180
actatatagg atcgctagat tgtttcggtc gtcccttaca gatgacgcaa agtaatcttg 240
tagatgcctt agcagttgcg gctgttgttt gtatgggaga ggggaatgag caaacaccgt 300
tagcggtgat agagcaggca cctaatatgg tctaccattc atatcctact tctcgagaag 360
agtattgttc tttgcgcata gatgaaacag aggacttata cggacctttt ttgcaagcgg 420
ttaccgtgga gtcaagaaaa gaaatgatgg aggtgtttat gaatt 465

<210> 75
<211> 545
<212> DNA
<213> Chlamydia

<400> 75
gaattcggca cgagatgaaa agttagcgtc acaggggatt ctcctaccaa agaattccga 60
aaagttttct tccaaaaacc tcttcctctc ttgattagtg atccctctgc aactacttta 120
ctatatgttc tgtgaaatat gcatagtctt caggattgga aaatccaaag tactcagtca 180
atccacgaat tttctctcta gcgatacgtg gaatttgact ctcataagaa tacaaagcag 240
ccactcctgc agctaaagaa tctcctgtac accaccgcat gaaagtagct actttcgctt 300
ttgctgcttc actaggctca tgagcctcta actcttctgg agtaactcct agagcaaaca 360
caaactgctt ccacaaatca atatgattag ggtaaccgtt ctcttcatcc atcaagttat 420
ctaacaataa cttacgcgcc tctaaatcat cgcaacgact atgaatcgca gataaatatt 480
taggaaaggc tttgatatgt aaataatagt ctttggcata cgcctgtaat tgctctttag 540
taagc 545

<210> 76
<211> 797
<212> DNA
<213> Chlamydia

<220>
<221> unsure
<222> (788)
<223> n=A,T,C or G
<221> unsure
<222> (789)
<223> n=A,T,C or G

<400> 76
gatccgaatt cggcacgaga tacgctagat gcgataaatg cggataatga ggattatcct 60
aaaccaggtg acttcccacg atcttccttc tctagtacgc ctcctcatgc tccagtacct 120
caatctgaga ttccaacgtc acctacctca acacagcctc catcacccta acttgtaaaa 180
actgtaataa aaagagcgcg cttcctttat gcaaaatcaa tttgaacaac tccttactga 240
attagggact caaatcaaca gccctcttac tcctgattcc aataatgcct gtatagttcg 300
ctttggatac aacaatgttg ctgtacaaat tgaagaggat ggtaattcag gatttttagt 360
tgctggagtc atgcttggaa aacttccaga gaataccttt agacaaaaaa ttttcaaagc 420
tgctttgtct atcaatggat ctccgcaatc taatattaaa ggcactctag gatacggtga 480
aatctctaac caactctatc tctgtgatcg gcttaacatg acctatctaa atggagaaaa 540
gctcgcccgt tacttagttc ttttttcgca gcatgccaat atctggatgc aatctatctc 600
aaaaggagaa cttccagatt tacatgctct aggtatgtat cacctgtaaa ttatgccgtc 660
attatcccaa tcccgacgta tcatccagca atcttccatt cgaaagattt ggaatcagat 720
agatacttct cctaagcatg ggggtatgcg taccggttat ttttctcttc atactcaaaa 780
aaagttgnng gggaata 797

<210> 77
<211> 399
<212> DNA
<213> Chlamydia

<400> 77
catatgcatc accatcacca tcacatgcca cgcatcattg gaattgatat tcctgcaaag 60
aaaaagttaa aaataagtct gacatatatt tatggaatag gatcagctcg ttctgatgaa 120
atcattaaaa agttgaagtt agatcctgag gcaagagcct ctgaattaac tgaagaagaa 180
gtaggacgac tgaactctct gctacaatca gaatataccg tagaagggga tttgcgacgt 240
cgtgttcaat cggatatcaa aagattgatc gccatccatt cttatcgagg tcagagacat 300
agactttctt taccagtaag aggacaacgt acaaaaacta attctcgtac tcgaaaaggt 360
aaaagaaaaa cagtcgcagg taagaagaaa taagaattc 399

<210> 78
<211> 285
<212> DNA
<213> Chlamydia

<400> 78
atgcatcacc atcaccatca catgagtcaa aaaaataaaa actctgcttt tatgcatccc 60
gtgaatattt ccacagattt agcagttata gttggcaagg gacctatgcc cagaaccgaa 120
attgtaaaga aagtttggga atacattaaa aaacacaact gtcaggatca aaaaaataaa 180
cgtaatatcc ttcccgatgc gaatcttgcc aaagtctttg gctctagtga tcctatcgac 240
atgttccaaa tgaccaaagc cctttccaaa catattgtaa aataa 285

<210> 79
<211> 950
<212> DNA
<213> Chlamydia

<400> 79
aaattaactc gagcacaaat tacggcaatt gctgagcaaa agatgaagga catggatgtc 60
gttcttttag agtccgccga gagaatggtt gaagggactg cccgaagcat gggtgtagat 120
gtagagtaat tagttaaaga gctgcataat tatgacaaag catggaaaac gcattcgtgg 180
tatccaagag acttacgatt tagctaagtc gtattctttg ggtgaagcga tagatatttt 240
aaaacagtgt cctactgtgc gtttcgatca aacggttgat gtgtctgtta aattagggat 300
cgatccaaga aagagtgatc agcaaattcg tggttcggtt tctttacctc acggtacagg 360
taaagttttg cgaattttag tttttgctgc tggagataag gctgcagagg ctattgaagc 420
aggagcggac tttgttggta gcgacgactt ggtagaaaaa atcaaaggtg gatgggttga 480
cttcgatgtt gcggttgcca ctcccgatat gatgagagag gtcggaaagc taggaaaagt 540
tttaggtcca agaaacctta tgcctacgcc taaagccgga actgtaacaa cagatgtggt 600
taaaactatt gcggaactgc gaaaaggtaa aattgaat[?]t aaagctgatc gagctggtgt 660
atgcaacgtc ggagttgcga agctttcttt cgatagtgcg caaatcaaag aaaatgttga 720
agcgttgtgt gcagccttag ttaaagctaa gcccgcaact gctaaaggac aatatttagt 780
taatttcact atttcctcga ccatggggcc aggggttacc gtggatacta gggagttgat 840
tgcgttataa ttctaagttt aaagaggaaa aatgaaagaa gagaaaaagt tgctgcttcg 900
cgaggttgaa gaaaagataa ccgcttctca aggttttatt ttgttgagat 950

<210> 80
<211> 395
<212> DNA
<213> Chlamydia

<400> 80
tttcaaggat tttgttttcc cgatcatctt actaaatgca gctccaacaa tcacatcatg 60
ggctggttta gcatctaagg caacagaagc tcctctgctg taataagtga attcttcaga 120
agtaggtgtt cctacttgcg atagcatcgt tcctagtcct gatatccaca ggttgttata 180
gctaacttca tcaaagcgag ctagattcat tttatcgttg agcaagcctt gtttgactgt 240
gaccattgac atttgagatc ccagaatcga gttcgcatag aaatgattgt ctctaggtac 300
ataagcccat tgtctataag agtcaaattt ccagagcgct gagatcgttc cattttgtag 360
ttgatcagga tccagagtga gtgttcctgt atatc 395

<210> 81
<211> 2085
<212> DNA
<213> Chlamydia

<400> 81
atttggcgaa ggagtttggg ctacggctat taataaatca ttcgtgttcg ctgcctccaa 60
gaccagattg tgtactttct tatgaagaat ctcctattga gcaaatgttg cgttggggag 120
agtctcagtt agaacaattt gctcaagtag gtttagatac aagttggcaa gttgttttcg 180
atccaggaat aggatttggg aagactcccg ttcagtcgat gttattgatg gatggagtaa 240
agcagtttaa acgtgtttta gagtgtcctg tattaatagg ccattctaga aaatcgtgtt 300
tgagtatgtt gggccgattt aatagtgacg atcgtgattg ggaaacgatc ggctgttctg 360
tatctcttca tgatcgagga gttgattatc tacgtgtgca tcaggttgaa ggtaacagac 420
gtgccttagc cgctgctgct tgggctggta tgtttgtatg atccaagcaa caggtatcgt 480
tgctattgat cccagaggag tgatgggagc tttaggcaag ctcccttgga gttatcccga 540
agatctacgt ttttttgcag aaaccattcg aaatcatccc atcattatgg gacgaaagac 600
ttgggagtct cttccagaca agtataagca tgggcgggat atcgttgtct tttctcgcag 660
gatgcatcca ccacaatgca taggagtttc ttcctttgca gagtatggga cactatcttt 720
gaatcatccg tttttaattg ggggagcgga gctctttgaa agttttttcc aacaaaacct 780
tctgaaagct tgttttgtca cacatatcaa aaagaaatat tggggcgata ctttcttccc 840
tatcacgcga ttatcaggat ggaagaagga atgtatttgt aatacagagg atttcagtat 900
ttattattat gaaaataact ccgatcaaaa cacgtaaagt atttgcacat gattcgcttc 960
aagagatctt gcaagaggct ttgccgcctc tgcaagaacg gagtgtggta gttgtctctt 1020
caaagattgt gagtttatgt gaaggcgctg tcgctgatgc aagaatgtgc aaagcagagt 1080
tgataaaaaa agaagcggat gcttatttgt tttgtgagaa aagcgggata tatctaacga 1140
aaaaagaagg tattttgatt ccttctgcag ggattgatga atcgaatacg gaccagcctt 1200
ttgttttata tcctaaagat attttgggat cgtgtaatcg catcggagaa tggttaagaa 1260
attattttcg agtgaaagag ctaggcgtaa tcattacaga tagccatact actccaatgc 1320
ggcgtggagt actgggtatc gggctgtgtt ggtatggatt ttctccatta cacaactata 1380
taggatcgct agattgtttc ggtcgtccct tacagatgac gcaaagtaat cttgtagatg 1440
ccttagcagt tgcggctgtt gtttgtatgg gagaggggaa tgagcaaaca ccgttagcgg 1500
tgatagagca ggcacctaat atggtctacc attcatatcc tacttctcga gaagagtatt 1560
gttctttgcg catagatgaa acagaggact tatacggacc ttttttgcaa gcggttacgt 1620
ggagtcaaga aaagaaatga tggaggtgtt tatgaatttt ttagatcagt tagatttaat 1680
tattcaaaat aagcatatgc tagaacacac gttttatgtg aaatggtcga agggggagct 1740
tactaaagag caattacagg cgtatgccaa agactattat ttacatatca aagcctttcc 1800
taaatattta tctgcgattc atagtcgttg cgatgattta gaggcgcgta agttattgtt 1860
agataacttg atggatgaag agaacggtta ccctaatcat attgatttgt ggaagcagtt 1920
tgtgtttgct ctaggagtta ctccagaaga gttagaggct catgagccta gtgaagcagc 1980
aaaagcgaaa gtagctactt tcatgcggtg gtgtacagga gattctttag ctgcaggagt 2040
ggctgctttg tattcttatg agagtcaaat tccacgtatc gcctc 2085

<210> 82
<211> 405
<212> DNA
<213> Chlamydia

<400> 82
ttcatcggtc tagttcgcta ttctactctc caatggttcc gcatttttgg gcagagcttc 60
gcaatcatta tgcaacgagt ggtttgaaaa gcgggtacaa tattgggagt accgatgggt 120
ttctccctgt cattgggcct gttatatggg agtcggaggg tcttttccgc gcttatattt 180
cttcggtgac tgatggggat ggtaagagcc ataaagtagg atttctaaga attcctacat 240
atagttggca ggacatggaa gattttgatc cttcaggacc gcctccttgg gaagaattgt 300
attggctcca taaagggagg agaaaacttc gatataggga atcgtatcaa ggtgaaagta 360
gcaaaaaata aattagctcc tccattccga actgcagaat ttgat 405

<210> 83
<211> 379
<212> DNA
<213> Chlamydia

<400> 83
tataccattc gtttgaaagt gcctttgacg ggagaaagtg tttttgaaga tcaatgcaaa 60
ggtcgtgtcg ttttcccttg ggcagatgtt gacgatcaag ttttggttaa atcagacggg 120
ttccctacgt atcactttgc taatgtagtt gatgatcatt tgatggggat tacccatgtg 180
ttgcgagggg aagagtggtt aagttctaca cctaaacacc ttcttcttta caaagctttt 240
gggtgggagc ctccgcagtt tttccatatg ccgcttcttc taaatcctga tggaagtaag 300
ctttccaaga gaaagaatcc tacttctatt ttttactatc gggatgctgg atacaaaaaa 360
gaagcgttca tgaatttcc 379

<210> 84
<211> 715
<212> DNA
<213> Chlamydia

<400> 84
tcaatcctgt attaataatt ctggttctta gactacataa attaggaacg cctgatgagt 60
atccataact aatcgcgtag ggcttagaat caccttctcg taccaaagct agaacaacgc 120
cgccttccat tcttgatgca ataatatctg ctgagactaa gaacatgctc ccagagcttt 180
tgggtgtgac tgtgaatttt cctatttcag ttcctcctaa taaagtttca atgttcctgg 240
gagtgaataa cccgttgcat tgaattttat tagtgattgg aaagttgtta aaagctttca 300
acaaacctag agaagggtct gttgtgattt tgtctaaaat atcttggact gtactatcaa 360
caatagtatc agcaattcca ccaagaattt gatctcccaa cttttctaga ataagctggt 420
aagctttttc cgcatccaaa ccaattgtaa tagaagcatt ggttgatgga ttattggaga 480
ctgttaaaga tattccatca gaagctgtca ttttggctgc gacaggtgtt gatgttgtcc 540
caaggattat ttgctggtcc ttgagcggct ctgtcatttg cccaactttg atattatcag 600
caaagacgca gttttgagtg ttatacaaat aaaaaccaga atttcccatt ttaaaactct 660
tttttatttt gagctttaaa taaattaggt ttttagtttc aagtttgcta ttaat 715

<210> 85
<211> 476
<212> DNA
<213> Chlamydia

<400> 85
ctcgtgccgc tcgtgccgct cgtgccggtc ttttagaaga gcgtgaagct ttaaataatt 60
cgattacgtt tatcatggat aagcgtaatt ggatagaaac cgagtctgaa caggtacaag 120
tggttttcag agatagtaca gcttgcttag gaggaggcgc tattgcagct caagaaattg 180
tttctattca gaacaatcag gctgggattt ccttcgaggg aggtaaggct agtttcggag 240
gaggtattgc gtgtggatct ttttcttccg caggcggtgc ttctgtttta gggactattg 300
atatttcgaa gaatttaggc gcgatttcgt tctctcgtac tttatgtacg acctcagatt 360
taggacaaat ggagtaccag ggaggaggag ctctatttgg tgaaaatatt tctctttctg 420
agaatgctgg tgtgctcacc tttaaagaca acattgtgaa gacttttgct tcgaat 476

<210> 86
<211> 1551
<212> DNA
<213> Chlamydia

<400> 86
gcgtatcgat atttcttctg ttacattctt tatagggatt ctgttggctg ttaatgcgct 60
aacctactct catgtattac gggatttatc tgtgagtatg gatgcgctgt tttctcgtaa 120
cacgcttgct gttcttttag gtttagtctc tagcgtttta gataatgtgc cattagtcgc 180
tgcaacaata ggtatgtatg acttacctat gaacgatcct ctttggaaac tcattgccta 240
tacagcaggc acagggggaa gtattctcat cattggatcc gctgcaggtg ttgcctacat 300

gggaatggaa aaagtgagtt tcggctggta tgtcaaacac gcttcttgga ttgctttagc 360
cagttatttt ggaggtctag cagtctattt tctaatggaa aattgtgtga atttgttcgt 420
ttgaggtagt cagtatggca gagtttcttt aaaaattctt ttaataaaag ggttctctgc 480
ctattctagg cccctttttg aatggaaaaa tgggtttttg gagaacatcg attatgaaaa 540
tgaataggat ttggctatta ctgcttacct tttcttctgc catacattct cctgtacgag 600
gagaaagctt ggtttgcaag aatgctcttc aagatttgag ttttttagag catttattac 660
aggttaaata tgctcctaaa acatggaaag agcaatactt aggatgggat cttgttcaaa 720
gctccgtttc tgcacagcag aagcttcgta cacaagaaaa tccatcaaca agtttttgcc 780
agcaggtcct tgctgatttt atcggaggat taaatgactt tcacgctgga gtaactttct 840
ttgcgataga aagtgcttac cttccttata ccgtacaaaa aagtagtgac ggccgtttct 900
actttgtaga tatcatgact ttttcttcag agatccgtgt tggagatgag ttgctagagg 960
tggatggggc gcctgtccaa gatgtgctcg ctactctata tggaagcaat cacaaaggga 1020
ctgcagctga agagtcggct gctttaagaa cactattttc tcgcatggcc tctttagggc 1080
acaaagtacc ttctgggcgc actactttaa agattcgtcg tccttttggt actacgagag 1140
aagttcgtgt gaaatggcgt tatgttcctg aaggtgtagg agatttggct accatagctc 1200
cttctatcag ggctccacag ttacagaaat cgatgagaag ctttttccct aagaaagatg 1260
atgcgtttca tcggtctagt tcgctattct actctccaat ggttccgcat ttttgggcag 1320
agcttcgcaa tcattatgca acgagtggtt tgaaaagcgg gtacaatatt gggagtaccg 1380
atgggtttct ccctgtcatt gggcctgtta tatgggagtc ggagggtctt ttccgcgctt 1440
atatttcttc ggtgactgat ggggatggta agagccataa agtaggattt ctaagaattc 1500
ctacatatag ttggcaggac atggaagatt ttgatccttc aggaccgcct c 1551

<210> 87
<211> 3031
<212> DNA
<213> Chlamydia

<400> 87
atgtaggccc tcaagcggtt ttattgttag accaaattcg agatctattc gttgggtcta 60
aagatagtca ggctgaagga cagtataggt taattgtagg agatccaagt tctttccaag 120
agaaagatgc agatactctt cccgggaagg tagagcaaag tactttgttc tcagtaacca 180
atcccgtggt tttccaaggt gtggaccaac aggatcaagt ctcttcccaa gggttaattt 240
gtagttttac gagcagcaac cttgattctc cccgtgacgg agaatctttt ttaggtattg 300
cttttgttgg ggatagtagt aaggctggaa tcacattaac tgacgtgaaa gcttctttgt 360
ctggagcggc tttatattct acagaagatc ttatctttga aaagattaag ggtggattgg 420
aatttgcatc atgttcttct ctagaacagg ggggagcttg tgcagctcaa agtattttga 480
ttcatgattg tcaaggattg caggttaaac actgtactac agccgtgaat gctgaggggt 540
ctagtgcgaa tgatcatctt ggatttggag gaggcgcttt ctttgttacg ggttctcttt 600
ctggagagaa aagtctctat atgcctgcag gagatatggt agttgcgaat tgtgatgggg 660
ctatatcttt tgaaggaaac agcgcgaact ttgctaatgg aggagcgatt gctgcctctg 720
ggaaagtgct ttttgtcgct aatgataaaa agacttcttt tatagagaac cgagctttgt 780
ctggaggagc gattgcagcc tcttctgata ttgcctttca aaactgcgca gaactagttt 840
tcaaaggcaa ttgtgcaatt ggaacagagg ataaaggttc tttaggtgga ggggctatat 900
cttctctagg caccgttctt ttgcaaggga atcacgggat aacttgtgat aataatgagt 960
ctgcttcgca aggaggcgcc atttttggca aaaattgtca gatttctgac aacgaggggc 1020
cagtggtttt cagagatagt acagcttgct taggaggagg cgctattgca gctcaagaaa 1080
ttgtttctat tcagaacaat caggctggga tttccttcga gggaggtaag gctagtttcg 1140
gaggaggtat tgcgtgtgga tctttttctt ccgcaggcgg tgcttctgtt ttagggacta 1200
ttgatatttc gaagaattta ggcgcgattt cgttctctcg tactttatgt acgacctcag 1260
atttaggaca aatggagtac cagggaggag gagctctatt tggtgaaaat atttctcttt 1320
ctgagaatgc tggtgtgctc acctttaaag acaacattgt gaagactttt gcttcgaatg 1380
ggaaaattct gggaggagga gcgattttag ctactggtaa ggtggaaatt accaataatt 1440
ccggaggaat ttcttttaca ggaaatgcga gagctccaca agctcttcca actcaagagg 1500
agtttccttt attcagcaaa aaagaagggc gaccactctc ttcaggatat tctgggggag 1560
gagcgatttt aggaagagaa gtagctattc tccacaacgc tgcagtagta tttgagcaaa 1620

atcgtttgca gtgcagcgaa gaagaagcga cattattagg ttgttgtgga ggaggcgctg 1680
ttcatgggat ggatagcact tcgattgttg gcaactcttc agtaagattt ggtaataatt 1740
acgcaatggg acaaggagtc tcaggaggag ctcttttatc taaaacagtg cagttagctg 1800
gaaatggaag cgtcgatttt tctcgaaata ttgctagttt gggaggacgc aatgttctgt 1860
tagcttcaga aacctttgct tccagagcaa atacatctcc ttcatcgctt cgctccttat 1920
atttccaagt aacctcatcc ccctctaatt gcgctaattt acatcaaatg cttgcttctt 1980
actcgccatc agagaaaacc gctgttatgg agtttctagt gaatggcatg gtagcagatt 2040
taaaatcgga gggcccttcc attcctcctg caaaattgca agtatatatg acggaactaa 2100
gcaatctcca agccttacac tctgtagata gctttttga tagaaatatt gggaacttgg 2160
aaaatagctt aaagcatgaa ggacatgccc ctattccatc cttaacgaca ggaaatttaa 2220
ctaaaacctt cttacaatta gtagaagata aattcccttc ctcttccaaa gctcaaaagg 2280
cattaaatga actggtaggc ccagatactg gtcctcaaac tgaagtttta aacttattct 2340
tccgcgctct taatggctgt tcgcctagaa tattctctgg agctgaaaaa aaacagcagc 2400
tggcatcggt tatcacaaat acgctagatg cgataaatgc ggataatgag gattatccta 2460
aaccaggtga cttcccacga tcttccttct ctagtacgcc tcctcatgct ccagtacctc 2520
aatctgagat tccaacgtca cctacctcaa cacagcctcc atcaccctaa cttgtaaaaa 2580
ctgtaataaa aagagcgcgc ttcctttatg caaaatcaat ttgaacaact ccttactgaa 2640
ttagggactc aaatcaacag ccctcttact cctgattcca ataatgcctg tatagttcgc 2700
tttggataca acaatgttgc tgtacaaatt gaagaggatg gtaattcagg atttttagtt 2760
gctggagtca tgcttggaaa acttccagag aataccttta gacaaaaaat tttcaaagct 2820
gctttgtcta tcaatggatc tccgcaatct aatattaaag gcactctagg atacggtgaa 2880
atctctaacc aactctatct ctgtgatcgg cttaacatga cctatctaaa tggagaaaag 2940
ctcgcccgtt acttagttct tttttcgcag catgccaata tctggatgca atctatctca 3000
aaaggagaac ttccagattt acatgctcta g 3031

<210> 88
<211> 976
<212> DNA
<213> Chlamydia

<400> 88
   aggtggatgg ggcgcctgtc caagatgtgc tcgctactct atatggaagc aatcacaaag 60
   ggactgcagc tgaagagtcg gctgctttaa gaacactatt ttctcgcatg gcctctttag 120
   ggcacaaagt accttctggg cgcactactt taaagatccg tcgtcctttt ggtactacga 180
   gagaagttcg tgtgaaatgg cgttatgttc ctgaaggtgt aggagatttg gctaccatag 240
   ctccttctat cagggctcca cagttacaga aatcgatgag aagctttttc cctaagaaag 300
   atgatgcgtt tcatcggtct agttcgctat tctactctcc aatggttccg catttttggg 360
   cagagcttcg caatcattat gcaacgagtg gtttgaaaag cgggtacaat attgggagta 420
   ccgatgggtt tctccctgtc attgggcctg ttatatggga gtcggagggt cttttccgcg 480
   cttatatttc ttcggtgact gatggggatg gtaagagcca taaagtagga tttctaagaa 540
   ttcctacata tagttggcag gacatggaag attttgatcc ttcaggaccg cctccttggg 600
   aagaatttgc taagattatt caagtatttt cttctaatac agaagctttg attatcgacc 660
   aaacgaacaa cccaggtggt agtgtccttt atctttatgc actgctttcc atgttgacag 720
   accgtccttt agaacttcct aaacatagaa tgattctgac tcaggatgaa gtggttgatg 780
   ctttagattg gttaaccctg ttggaaaacg tagacacaaa cgtggagtct cgccttgctc 840
   tgggagacaa catggaagga tatactgtgg atctacaggt tgccgagtat ttaaaaagct 900
   ttggacgtca agtattgaat tgttggagta aaggggatat cgagttatca acacctattc 960
   ctcttttggg ttttga 976

<210> 89
<211> 94
<212> PRT
<213> Chlamydia

<400> 89

Met His His His His His His Met Ser Gln Lys Asn Lys Asn Ser Ala
                    5                   10                  15

Phe Met His Pro Val Asn Ile Ser Thr Asp Leu Ala Val Ile Val Gly
            20                  25                  30

Lys Gly Pro Met Pro Arg Thr Glu Ile Val Lys Lys Val Trp Glu Tyr
        35                  40                  45

Ile Lys Lys His Asn Cys Gln Asp Gln Lys Asn Lys Arg Asn Ile Leu
    50                  55                  60

Pro Asp Ala Asn Leu Ala Lys Val Phe Gly Ser Ser Asp Pro Ile Asp
65                  70                  75                  80

Met Phe Gln Met Thr Lys Ala Leu Ser Lys His Ile Val Lys
                85                  90

<210> 90
<211> 474
<212> PRT
<213> Chlamydia

<400> 90
Met Ala Ser His His His His His His Met Asn Glu Ala Phe Asp Cys
                    5                   10                  15

Val Val Ile Gly Ala Gly Pro Gly Gly Tyr Val Ala Ala Ile Thr Ala
            20                  25                  30

Ala Gln Ala Gly Leu Lys Thr Ala Leu Ile Glu Lys Arg Glu Ala Gly
        35                  40                  45

Gly Thr Cys Leu Asn Arg Gly Cys Ile Pro Ser Lys Ala Leu Leu Ala
    50                  55                  60

Gly Ala Glu Val Val Thr Gln Ile Arg His Ala Asp Gln Phe Gly Ile
65                  70                  75                  80

His Val Glu Gly Phe Ser Ile Asn Tyr Pro Ala Met Val Gln Arg Lys
                85                  90                  95

Asp Ser Val Val Arg Ser Ile Arg Asp Gly Leu Asn Gly Leu Ile Arg
            100                 105                 110

Ser Asn Lys Ile Thr Val Phe Ser Gly Arg Gly Ser Leu Ile Ser Ser
        115                 120                 125

Thr Glu Val Lys Ile Leu Gly Glu Asn Pro Ser Val Ile Lys Ala His
    130                 135                 140

Ser Ile Ile Leu Ala Thr Gly Ser Glu Pro Arg Ala Phe Pro Gly Ile
145                 150                 155                 160

Pro Phe Ser Ala Glu Ser Pro Arg Ile Leu Cys Ser Thr Gly Val Leu
                165                 170                 175

Asn Leu Lys Glu Ile Pro Gln Lys Met Ala Ile Ile Gly Gly Gly Val
            180                 185                 190

Ile Gly Cys Glu Phe Ala Ser Leu Phe His Thr Leu Gly Ser Glu Val
        195                 200                 205

Ser Val Ile Glu Ala Ser Ser Gln Ile Leu Ala Leu Asn Asn Pro Asp
    210                 215                 220

Ile Ser Lys Thr Met Phe Asp Lys Phe Thr Arg Gln Gly Leu Arg Phe
225                 230                 235                 240

Val Leu Glu Ala Ser Val Ser Asn Ile Glu Asp Ile Gly Asp Arg Val
                245                 250                 255

Arg Leu Thr Ile Asn Gly Asn Val Glu Glu Tyr Asp Tyr Val Leu Val
            260                 265                 270

Ser Ile Gly Arg Arg Leu Asn Thr Glu Asn Ile Gly Leu Asp Lys Ala
        275                 280                 285

Gly Val Ile Cys Asp Glu Arg Gly Val Ile Pro Thr Asp Ala Thr Met
    290                 295                 300

Arg Thr Asn Val Pro Asn Ile Tyr Ala Ile Gly Asp Ile Thr Gly Lys
305                 310                 315                 320

Trp Gln Leu Ala His Val Ala Ser His Gln Gly Ile Ile Ala Ala Arg
                325                 330                 335

Asn Ile Gly Gly His Lys Glu Glu Ile Asp Tyr Ser Ala Val Pro Ser
            340                 345                 350

Val Ile Phe Thr Phe Pro Glu Val Ala Ser Val Gly Leu Ser Pro Thr
        355                 360                 365

Ala Ala Gln Gln Gln Lys Ile Pro Val Lys Val Thr Lys Phe Pro Phe
    370                 375                 380

Arg Ala Ile Gly Lys Ala Val Ala Met Gly Glu Ala Asp Gly Phe Ala
385                 390                 395                 400

Ala Ile Ile Ser His Glu Thr Thr Gln Gln Ile Leu Gly Ala Tyr Val
                405                 410                 415

Ile Gly Pro His Ala Ser Ser Leu Ile Ser Glu Ile Thr Leu Ala Val
            420                 425                 430

Arg Asn Glu Leu Thr Leu Pro Cys Ile Tyr Glu Thr Ile His Ala His
        435                 440                 445

Pro Thr Leu Ala Glu Val Trp Ala Glu Ser Ala Leu Leu Ala Val Asp

450                 455                 460

Thr Pro Leu His Met Pro Pro Ala Lys Lys
465                 470

<210> 91
<211> 129
<212> PRT
<213> Chlamydia

<400> 91
Met His His His His His His Met Pro Arg Ile Ile Gly Ile Asp Ile
                5                   10                  15

Pro Ala Lys Lys Lys Leu Lys Ile Ser Leu Thr Tyr Ile Tyr Gly Ile
            20                  25                  30

Gly Ser Ala Arg Ser Asp Glu Ile Ile Lys Lys Leu Lys Leu Asp Pro
        35                  40                  45

Glu Ala Arg Ala Ser Glu Leu Thr Glu Glu Glu Val Gly Arg Leu Asn
    50                  55                  60

Ser Leu Leu Gln Ser Glu Tyr Thr Val Glu Gly Asp Leu Arg Arg Arg
65                  70                  75                  80

Val Gln Ser Asp Ile Lys Arg Leu Ile Ala Ile His Ser Tyr Arg Gly
                85                  90                  95

Gln Arg His Arg Leu Ser Leu Pro Val Arg Gly Gln Arg Thr Lys Thr
            100                 105                 110

Asn Ser Arg Thr Arg Lys Gly Lys Arg Lys Thr Val Ala Gly Lys Lys
        115                 120                 125

Lys

<210> 92
<211> 202
<212> PRT
<213> Chlamydia

<400> 92
Met His His His His His His Met Gly Ser Leu Val Gly Arg Gln Ala
                5                   10                  15

Pro Asp Phe Ser Gly Lys Ala Val Val Cys Gly Glu Glu Lys Glu Ile
            20                  25                  30

Ser Leu Ala Asp Phe Arg Gly Lys Tyr Val Val Leu Phe Phe Tyr Pro
        35                  40                  45

Lys Asp Phe Thr Tyr Val Cys Pro Thr Glu Leu His Ala Phe Gln Asp
    50                  55                  60

Arg Leu Val Asp Phe Glu Glu His Gly Ala Val Val Leu Gly Cys Ser
65                  70                  75                  80

Val Asp Asp Ile Glu Thr His Ser Arg Trp Leu Thr Val Ala Arg Asp
                85                  90                  95

Ala Gly Gly Ile Glu Gly Thr Glu Tyr Pro Leu Leu Ala Asp Pro Ser
            100                 105                 110

Phe Lys Ile Ser Glu Ala Phe Gly Val Leu Asn Pro Glu Gly Ser Leu
        115                 120                 125

Ala Leu Arg Ala Thr Phe Leu Ile Asp Lys His Gly Val Ile Arg His
    130                 135                 140

Ala Val Ile Asn Asp Leu Pro Leu Gly Arg Ser Ile Asp Glu Glu Leu
145                 150                 155                 160

Arg Ile Leu Asp Ser Leu Ile Phe Phe Glu Asn His Gly Met Val Cys
                165                 170                 175

Pro Ala Asn Trp Arg Ser Gly Glu Arg Gly Met Val Pro Ser Glu Glu
            180                 185                 190

Gly Leu Lys Glu Tyr Phe Gln Thr Met Asp
        195                 200

<210> 93
<211> 19
<212> PRT
<213> Artificial Sequence

<220>
<223> made in a lab

<400> 93
Glu Asn Ser Leu Gln Asp Pro Thr Asn Lys Arg Asn Ile Asn Pro Asp
1               5                   10                  15
Asp Lys Leu

<210> 94
<211> 20
<212> PRT
<213> Artificial Sequence

<220>
<223> Made in a lab

<400> 94

Asp Pro Thr Asn Lys Arg Asn Ile Asn Pro Asp Asp Lys Leu Ala Lys
1               5                   10                  15
Val Phe Gly Thr
            20

<210> 95
<211> 20
<212> PRT
<213> Artificial Sequence

<220>
<223> Made in a lab

<400> 95
Lys Arg Asn Ile Asn Pro Asp Asp Lys Leu Ala Lys Val Phe Gly Thr
1               5                   10                  15
Glu Lys Pro Ile
            20

<210> 96
<211> 20
<212> PRT
<213> Artificial Sequence

<220>
<223> Made in a lab

<400> 96
Asp Asp Lys Leu Ala Lys Val Phe Gly Thr Glu Lys Pro Ile Asp Met
1               5                   10                  15
Phe Gln Met Thr
            20

<210> 97
<211> 20
<212> PRT
<213> Artificial Sequence

<220>
<223> Made in a lab

<400> 97
Lys Val Phe Gly Thr Glu Lys Pro Ile Asp Met Phe Gln Met Thr Lys
1               5                   10                  15
Met Val Ser Gln
            20

<210> 98
<211> 20
<212> PRT
<213> Artificial Sequence

<220>
<223> Made in a lab

<400> 98
Asn Lys Arg Asn Ile Asn Pro Asp Asp Lys Leu Ala Lys Val Phe Gly
1               5                   10                  15
Thr Glu Lys Pro
            20

<210> 99
<211> 16
<212> PRT
<213> Artificial Sequence

<220>
<223> Made in a lab

<400> 99
Asn Lys Arg Asn Ile Leu Pro Asp Ala Asn Leu Ala Lys Val Phe Gly
1               5                   10                  15

<210> 100
<211> 15
<212> PRT
<213> Artificial Sequence

<220>
<223> Made in a lab

<400> 100
Lys Met Trp Asp Tyr Ile Lys Glu Asn Ser Leu Gln Asp Pro Thr
1               5                   10                  15

<210> 101
<211> 20
<212> PRT
<213> Artificial Sequence

<220>
<223> Made in a lab

<400> 101
Thr Glu Ile Val Lys Lys Val Trp Glu Tyr Ile Lys Lys His Asn Cys
1               5                   10                  15
Gln Asp Gln Lys
            20

<210> 102
<211> 20
<212> PRT
<213> Artificial Sequence

<220>
<223> Made in a lab

<400> 102
Lys Val Trp Glu Tyr Ile Lys Lys His Asn Cys Gln Asp Gln Lys Asn
1               5                   10                  15

Lys Arg Asn Ile
            20

<210> 103
<211> 15
<212> PRT
<213> Artificial Sequence

<220>
<223> Made in a lab

<400> 103
Lys Val Trp Glu Tyr Ile Lys Lys His Asn Cys Gln Asp Gln Lys
1               5                   10                  15

<210> 104
<211> 20
<212> PRT
<213> Artificial Sequence

<220>
<223> Made in a lab

<400> 104
Ala Glu Leu Thr Glu Glu Glu Val Gly Arg Leu Asn Ala Leu Leu Gln
1               5                   10                  15
Ser Asp Tyr Val
            20

<210> 105
<211> 21
<212> PRT
<213> Artificial Sequence

<220>
<223> Made in a lab

<400> 105
Leu Gln Ser Asp Tyr Val Val Glu Gly Asp Leu Arg Arg Arg Val Gln
1               5                   10                  15
Ser Asp Ile Lys Arg
            20

<210> 106
<211> 20
<212> PRT
<213> Artificial Sequence

<220>
<223> Made in a lab

<400> 106
Met Pro Arg Ile Ile Gly Ile Asp Ile Pro Ala Lys Lys Lys Leu Lys
1               5                   10                  15
Ile Ser Leu Thr

            20

<210> 107
<211> 20
<212> PRT
<213> Artificial Sequence

<220>
<223> Made in a lab

<400> 107
Ala Glu Leu Thr Glu Glu Glu Val Gly Arg Leu Asn Ala Leu Leu Gln
1               5                   10                  15
Ser Asp Tyr Val
            20

<210> 108
<211> 20
<212> PRT
<213> Artificial Sequence

<220>
<223> Made in a lab

<400> 108
Leu Asn Ala Leu Leu Gln Ser Asp Tyr Val Val Glu Gly Asp Leu Arg
1               5                   10                  15
Arg Arg Val Gln
            20

<210> 109
<211> 20
<212> PRT
<213> Artificial Sequence

<220>
<223> Made in a lab

<400> 109
Leu Asn Ser Leu Leu Gln Ser Glu Tyr Thr Val Glu Gly Asp Leu Arg
1               5                   10                  15
Arg Arg Val Gln
            20

<210> 110
<211> 1461
<212> DNA
<213> Chlamydia

<400> 110
ctatctatga agttatgaat atggatctag aaacacgaag atcttttgcg gtacagcaag 60
ggcactatca ggacccaaga gcttcagatt atgacctccc acgtgctagc gactatgatt 120
tgcctagaag cccatatcct actccacctt tgccttctag atatcagcta cagaatatgg 180
atgtagaagc agggttccgt gaggcagttt atgcttcttt tgtagcagga atgtacaatt 240
atgtagtgac acagccgcaa gagcgtattc ccaatagtca gcaggtggaa gggattctgc 300

gtgatatgct taccaacggg tcacagacat ttagcaacct gatgcagcgt tgggatagag 360
aagtcgatag ggaataaact ggtatctacc ataggtttgt atcaaaaaac taagcccacc 420
aagaagaaat tctctttggt gggcttcttt ttttattcaa aaaagaaagc cctcttcaag 480
attatctcgt gccgctcgtg ccgaattcgg cacgagcggc acgaggagct gtaagtaagt 540
attgccaaga gttggaagaa aaaatattag atttgtgtaa gcgtcatgcc gcaacaattt 600
gctccattga ggaggatgct aaacaagaaa ttcgtcatca gacagaaagg tttaaacagc 660
ggttgcaaca aaatcagaac acttgcagtc aattaacagc agagttgtgt aaattgagat 720
ctgagaataa ggcattatcg gagcggctgc aggtgcaggc atcccgtcgt aaaaaataat 780
taaagactcc tcagatattg catctgagag ttaggggttc cttttgctta cggcgcttta 840
gttctgcatg ttgcggattt atagtgattt gcgagtaaag cgccgttctg atacagtttt 900
tccgctttaa aaataaaaag gtggaaaaat gagtactact attagcggag acgcttcttc 960
tttaccgttg ccaacagctt cctgcgtaga gacaaaatct acttcgtctt caacaaaagg 1020
gaatacttgt tccaaaattt tggatatagc tttagctatc gtaggcgctt tagttgttgt 1080
cgctggggta ttagctttgg ttttgtgcgc tagcaatgtc atatttactg taataggtat 1140
tcctgcatta attattggat ctgcttgtgt gggtgcggga atatctcgtc ttatgtatcg 1200
atcctcttat gctagcttag aagcaaaaaa tgttttggct gagcaacgtt tgcgtaatct 1260
ttcagaagag aaggacgctt tggcctccgt ctctttcatt aataagatgt ttctgcgagg 1320
tcttacggac gatctccaag ctttggaagc taaggtaatg gaatttgaga ttgattgttt 1380
ggacagatta gagaaaaatg agcaagcttt attgtccgat gtgcgcttag ttttatctag 1440
ctacacaaga tggttggata g 1461

<210> 111
<211> 267
<212> DNA
<213> Chlamydia

<400> 111
gtcctcttct tattatagca gaagacattg aaggcgaagc tttagctact ttggtcgtga 60
acagaattcg tggaggattc cgggtttgcg cagttaaagc tccaggcttt ggagatagaa 120
gaaaagctat gttggaagac atcgctatct taactggcgg tcaactcatt agcgaagagt 180
tgggcatgaa attagaaaac gctaacttag ctatgttagg taaagctaaa aaagttatcg 240
tttctaaaga agacacgacc atcgtcg 267

<210> 112
<211> 698
<212> DNA
<213> Chlamydia

<400> 112
tgataagcaa gcaaccgctc aactagcagc tctaactatt aaaaaaatcc tctgttttga 60
tgaaaattcc tacgagaagg agctggcatg cttagaaaag aaacgcagta gcgtacaaaa 120
agatctgagc caactgaaaa aatacacagt tctctacatc aagaagctgc tcgaaaccta 180
cagacaactc gggcatcgaa agacaaaaat tgcaaaattt gatgacctac ctaccgagag 240
agtctccgct cataagaaag caaaagaact cgctgcgctc gatcaagaag agaacttcta 300
aaacgtgact cggcccttga gatccttaaa ctctcgggcc aaaaagacta cagtcttctc 360
gagaagaaaa acggtgttag aaaatacgcg cgctaagact ttctctaaca atgactcaaa 420
aagctgtaaa cgtatacgtt taccgctctt ccataatttc taggctgact ttcacattat 480
ctcgacttgc tacggaaacc aataaagtac ggatagcctt aatagtgcgt ccttctttac 540
cgataatttt accgatatct cccttagcaa cagtcaattc gtagataatc gtattggttc 600
cctgcacctc tttcagatgc acttcctctg gcttatcaac aagatttttt acaatgtacg 660
ctaaaaactc tttcatgcga agcaaatcct acacaagc 698

<210> 113
<211> 1142
<212> DNA

<213> Chlamydia

<400> 113
   ctcttcaaag attgtgagtt tatgtgaagg cgctgtcgct gatgcaagaa tgtgcaaagc 60
   agagttgata aaaaaagaag cggatgctta tttgttttgt gagaaaagcg ggatatatct 120
   aacgaaaaaa gaaggtattt tgattccttc tgcagggatt gatgaatcga atacggacca 180
   gccttttgtt ttatatccta aagatatttt gggatcgtgt aatcgcatcg gagaatggtt 240
   aagaaattat tttcgagtga aagagctagg cgtaatcatt acagatagcc atactactcc 300
   aatgcggcgt ggagtactgg gtatcgggct gtgttggtat ggattttctc cattacacaa 360
   ctatatagga tcgctagatt gtttcggtcg tcccttacag atgacgcaaa gtaatcttgt 420
   agatgcctta gcagttgcgg ctgttgtttg tatgggagag gggaatgagc aaacaccgtt 480
   agcggtgata gagcaggcac ctaatatggt ctaccattca tatcctactt ctcgagaaga 540
   gtattgttct ttgcgcatag atgaaacaga ggacttatac ggaccttttt tgcaagcggt 600
   tacgtggagt caagaaaaga aatgatggag gtgtttatga attttttaga tcagttagat 660
   ttaattattc aaaataagca tatgctagaa cacacgtttt atgtgaaatg gtcgaagggg 720
   gagcttacta aagagcaatt acaggcgtat gccaaagact attatttaca tatcaaagcc 780
   tttcctaaat atttatctgc gattcatagt cgttgcgatg atttagaggc gcgtaagtta 840
   ttgttagata acttgatgga tgaagagaac ggttacccta atcatattga tttgtggaag 900
   cagtttgtgt ttgctctagg agttactcca gaagagttag aggctcatga gcctagtgaa 960
   gcagcaaaag cgaaagtagc tactttcatg cggtggtgta caggagattc tttagctgca 1020
   ggagtggctg ctttgtattc ttatgagagt caaattccac gtatcgctag agagaaaatt 1080
   cgtggattga ctgagtactt tggattttcc aatcctgaag actatgcata tttcacagaa 1140
   ca 1142

<210> 114
<211> 976
<212> DNA
<213> Chlamydia

<400> 114
   aggtggatgg ggcgcctgtc caagatgtgc tcgctactct atatggaagc aatcacaaag 60
   ggactgcagc tgaagagtcg gctgctttaa gaacactatt ctctcgcatg gcctctttag 120
   ggcacaaagt accttctggg cgcactactt taaagattcg tcgtcctttt ggtactacga 180
   gagaagttcg tgtgaaatgg cgttatgttc ctgaaggtgt aggagatttg gctaccatag 240
   ctccttctat cagggctcca cagttacaga aatcgatgag aagctttttc cctaagaaag 300
   atgatgcgtt tcatcggtct agttcgctat tctactctcc aatggttccg catttttggg 360
   cagagcttcg caatcattat gcaacgagtg gtttgaaaag cgggtacaat attgggagta 420
   ccgatgggtt tctccctgtc attgggcctg ttatatggga gtcggagggt cttttccgcg 480
   cttatatttc ttcggtgact gatggggatg gtaagagcca taaagtagga tttctaagaa 540
   ttcctacata tagttggcag gacatggaag attttgatcc ttcaggaccg cctccttggg 600
   aagaatttgc taagattatt caagtatttt cttctaatac agaagctttg attatcgacc 660
   aaacgaacaa cccaggtggt agtgtccttt atctttatgc actgctttcc atgttgacag 720
   accgtccttt agaacttcct aaacatagaa tgattctgac tcaggatgaa gtggttgatg 780
   ctttagattg gttaaccctg ttggaaaacg tagacacaaa cgtggagtct cgccttgctc 840
   tgggagacaa catggaagga tatactgtgg atctacaggt tgccgagtat ttaaaaagct 900
   ttggacgtca agtattgaat tgttggagta aaggggatat cgagttatca acacctattc 960
   ctcttttggg ttttga 976

<210> 115
<211> 995
<212> DNA
<213> Chlamydia

<400> 115
ttatcctaga aatttggtgt tcaatatgag cgaaaaaaga aagtctaaca aaattattgg 60

tatcgaccta gggacgacca actcttgcgt ctctgttatg gaaggtggcc aacctaaagt 120
tattgcctct tctgaaggaa ctcgtactac tccttctatc gttgctttta aaggtggcga 180
aactcttgtt ggaattcctg caaaacgtca ggcagtaacc aatcctgaaa aaacattggc 240
ttctactaag cgattcatcg gtagaaaatt ctctgaagtc gaatctgaaa ttaaaacagt 300
cccctacaaa gttgctccta actcgaaagg agatgcggtc tttgatgtgg aacaaaaact 360
gtacactcca gaagaaatcg gcgctcagat cctcatgaag atgaaggaaa ctgctgaggc 420
ttatctcgga gaaacagtaa cggaagcagt cattaccgta ccagcttact ttaacgattc 480
tcaaagagct tctacaaaag atgctggacg tatcgcagga ttagatgtta aacgcattat 540
tcctgaacca acagcggccg ctcttgctta tggtattgat aaggaaggag ataaaaaaat 600
cgccgtcttc gacttaggag gaggaacttt cgatatttct atcttggaaa tcggtgacgg 660
agtttttgaa gttctctcaa ccaacgggga tactcacttg ggaggagacg acttcgacgg 720
agtcatcatc aactggatgc ttgatgaatt caaaaaacaa gaaggcattg atctaagcaa 780
agataacatg gctttgcaaa gattgaaaga tgctgctgaa aaagcaaaaa tagaattgtc 840
tggtgtatcg tctactgaaa tcaatcagcc attcatcact atcgacgcta atggacctaa 900
acatttggct ttaactctaa ctcgcgctca attcgaacac ctagcttcct ctctcattga 960
gcgaaccaaa caaccttgtg ctcaggcttt aaaag 995

<210> 116
<211> 437
<212> DNA
<213> Chlamydia

<400> 116
gtcacagcta aaggcggtgg gctttatact gataagaatc tttcgattac taacatcaca 60
ggaattatcg aaattgcaaa taacaaagcg acagatgttg gaggtggtgc ttacgtaaaa 120
ggaaccctta cttgtaaaaa ctctcaccgt ctacaatttt tgaaaaactc ttccgataaa 180
caaggtggag gaatctacgg agaagacaac atcaccctat ctaatttgac agggaagact 240
ctattccaag agaatactgc caaaaaagag ggcggtggac tcttcataaa aggtacagat 300
aaagctctta caatgacagg actggatagt ttctgtttaa ttaataacac atcagaaaaa 360
catggtggtg gagcctttgt taccaaagaa atctctcaga cttacacctc tgatgtggaa 420
acaattccag gaatcac 437

<210> 117
<211> 446
<212> DNA
<213> Chlamydia

<400> 117
aagtttacct agaccaaact gaagatgacg aaggaaaagt tgttttatcc agagaaaaag 60
caacaagaca acgacaatgg gaatacattc ttgctcactg cgaggaaggt tctattgtta 120
agggacaaat tacccgaaaa gttaagggtg gtttgatcgt agatattggt atggaagcct 180
tccttccagg atcccaaata gacaataaga agatcaagaa cttagatgat tacgtaggca 240
aggtttgtga gttcaaaatt ctcaaaatca acgtggatcg tcggaacgtt gttgtatcta 300
gaagagaact tctcgaagct gaacgcattt ctaagaaagc agagttgatc gagcaaatca 360
ctatcggtga acgtcgcaaa ggtatcgtta agaatatcac agatttcgga gtattcttgg 420
atcttgatgg cattgacggc ctactc 446

<210> 118
<211> 951
<212> DNA
<213> Chlamydia

<400> 118
agtattgcga aatattactg tgagaagcaa tgctgagagc ggttctagta aaagtgaggg 60
gagagctgtc agaagggatc gctcaggaag cgagacaacg tgtggctgat ttattaggaa 120

gattccctct ttatcctgaa atcgatctgg aaacgctagt ttagtgggag actctatgcc 180
tgaaggggaa atgatgcata agttgcaaga tgtcatagat agaaagttgt tggattctcg 240
tcgtattttc ttctccgaac ctgtaacgga gaaaagtgct gcagaagcca tcaaaaagct 300
ttggtatttg gaactcacca atcctgggca gccaattgta tttgtcatta atagccctgg 360
agggtctgtt gatgctgggt ttgctgtttg ggaccaaatt aaaatgatct cttctccttt 420
gactacagtt gttacaggtt tagcagcatc tatgggatct gtattgagtt tgtgtgctgt 480
tccaggaaga cgttttgcta cgcctcatgc gcgcattatg attcaccagc cttctattgg 540
aggaaccatt actggtcaag ccacggactt ggatattcat gctcgtgaaa ttttaaaaac 600
aaaagcacgc attattgatg tgtatgtcga ggcaactgga caatctccag aggtgataga 660
gaaagctatc gatcgagata tgtggatgag tgcaaatgaa gcaatggagt ttggactgtt 720
agatgggatt ctcttctctt ttaacgactt gtagatatct tttatattct ggagcaggaa 780
acagtttcat tttgggagaa tcgatgcctt ctcttgagga tgttctgttt ttatgccagg 840
aagagatggt tgatgggttt ttatgtgtag agtcttctga aatagcagat gctaaactca 900
ctgtttttaa tagtgatgga tctatcgcgt ctatgtgcgg gaatgggttg c 951

<210> 119
<211> 953
<212> DNA
<213> Chlamydia

<400> 119
atatcaaagt tgggcaaatg acagagccgc tcaaggacca gcaaataatc cttgggacaa 60
catcaacacc tgtcgcagcc aaaatgacag cttctgatgg aatatcttta acagtctcca 120
ataatccatc aaccaatgct tctattacaa ttggtttgga tgcggaaaaa gcttaccagc 180
ttattctaga aaagttggga gatcaaattc ttggtggaat tgctgatact attgttgata 240
gtacagtcca agatatttta gacaaaatca caacagaccc ttctctaggt ttgttgaaag 300
cttttaacaa ctttccaatc actaataaaa ttcaatgcaa cgggttattc actcccagga 360
acattgaaac tttattagga ggaactgaaa taggaaaatt cacagtcaca cccaaaagct 420
ctgggagcat gttcttagtc tcagcagata ttattgcatc aagaatggaa ggcggcgttg 480
ttctagcttt ggtacgagaa ggtgattcta agccctacgc gattagttat ggatactcat 540
caggcgttcc taatttatgt agtctaagaa ccagaattat taatacagga ttgactccga 600
caacgtattc attacgtgta ggcggtttag aaagcggtgt ggtatgggtt aatgcccttt 660
ctaatggcaa tgatatttta ggaataacaa atacttctaa tgtatctttt ttggaggtaa 720
tacctcaaac aaacgcttaa acaattttta ttggattttt cttataggtt ttatatttag 780
agaaaaaagt tcgaattacg gggtttgtta tgcaaaataa aagcaaagtg agggacgatt 840
ttattaaaat tgttaaagat tcctggtatc ggtctgcgat tccgactcgt ccaacatcaa 900
tacaacctat taatttcccc tcgtcaaaaa taaggttatc aagtgagaaa tca 953

<210> 120
<211> 897
<212> DNA
<213> Chlamydia

<400> 120
atggcttcta tatgcggacg tttagggtct ggtacaggga atgctctaaa agcttttttt 60
acacagccca gcaataaaat ggcaagggta gtaaataaga cgaagggaat ggataagact 120
gttaaggtcg ccaagtctgc tgccgaattg accgcaaata ttttggaaca agctggaggc 180
gcgggctctt ccgcacacat tacagcttcc caagtgtcca aaggattagg ggatgcgaga 240
actgttctcg ctttagggaa tgcctttaac ggagcgttgc caggaacagt tcaaagtgcg 300
caaagcttct tctcttacat gaaagctgct agtcagaaac cgcaagaagg ggatgagggg 360
ctcgtagcag atctttgtgt gtctcataag cgcanagcgg ctgcggctgt ctgtagcttc 420
atcggaggaa ttacctacct cgcgacattc ggagctatcc gtccgattct gtttgtcaac 480
aaaatgctgg cgcaaccgtt tctttcttcc caaattaaag caaatatggg atcttctgtt 540
agctatatta tggcggctaa ccatgcagcg tttgtggtgg gttctggact cgctatcagt 600

gcggaaagag cagattgcga agcccgctgc gctcgtattg cgagagaaga gtcgtcactc 660
gaattgtcgg gagaggaaaa tgcttgcgag aggagagtcg ctggagagaa agccaagacg 720
ttcacgcgca tcaagtatgc actcctcact atgctcgaga agtttttgga atgcgttgcc 780
gacgttttca aattggtgcc gttgcctatt acaatgggta ttcgtgcaat tgtggctgcg 840
ggatgtacgt tcacttctgc agttattgga ttgtggactt tctgcgccag agcataa 897

<210> 121
<211> 298
<212> PRT
<213> Chlamydia

<400> 121
Met Ala Ser Ile Cys Gly Arg Leu Gly Ser Gly Thr Gly Asn Ala Leu
1               5                   10                  15
Lys Ala Phe Phe Thr Gln Pro Ser Asn Lys Met Ala Arg Val Val Asn
            20                  25                  30
Lys Thr Lys Gly Met Asp Lys Thr Val Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Ala Arg
65                  70                  75                  80
Thr Val Leu Ala Leu Gly Asn Ala Phe Asn Gly Ala Leu Pro Gly Thr
                85                  90                  95
Val Gln Ser Ala Gln Ser Phe Phe Ser Tyr Met Lys Ala Ala Ser Gln
            100                 105                 110
Lys Pro Gln Glu Gly Asp Glu Gly Leu Val Ala Asp Leu Cys Val Ser
        115                 120                 125
His Lys Arg Arg Ala Ala Ala Ala Val Cys Ser Phe Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Gln Pro Phe Leu Ser Ser Gln Ile Lys Ala Asn Met
                165                 170                 175
Gly Ser Ser Val Ser Tyr Ile Met Ala Ala Asn His Ala Ala Phe Val
            180                 185                 190
Val Gly Ser Gly Leu Ala Ile Ser Ala Glu Arg Ala Asp Cys Glu Ala
        195                 200                 205
Arg Cys Ala Arg Ile Ala Arg Glu Glu Ser Ser Leu Glu Leu Ser Gly
    210                 215                 220
Glu Glu Asn Ala Cys Glu Arg Arg Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Val
        275                 280                 285
Ile Gly Leu Trp Thr Phe Cys Ala Arg Ala
    290                 295

<210> 122
<211> 897
<212> DNA
<213> Chlamydia

<400> 122
atggcttcta tatgcggacg tttagggtct ggtacaggga atgctctaaa agcttttttt 60
acacagccca gcaataaaat ggcaagggta gtaaataaga cgaagggaat ggataagact 120
gttaaggtcg ccaagtctgc tgccgaattg accgcaaata ttttggaaca agctggaggc 180
gcgggctctt ccgcacacat tacagcttcc caagtgtcca aaggattagg ggatacgaga 240
actgttgtcg ctttagggaa tgcctttaac ggagcgttgc caggaacagt tcaaagtgcg 300
caaagcttct tctctcacat gaaagctgct agtcagaaaa cgcaagaagg ggatgagggg 360
ctcacagcag atctttgtgt gtctcataag cgcagagcgg ctgcggctgt ctgtggcttc 420
atcggaggaa ttacctacct cgcgacattc ggagttatcc gtccgattct gtttgtcaac 480
aaaatgctgg tgaacccgtt tctttcttcc caaactaaag caaatatggg atcttctgtt 540
agctatatta tggcggctaa ccatgcagcg tctgtggtgg gtgctggact cgctatcagt 600
gcggaaagag cagattgcga agcccgctgc gctcgtattg cgagagaaga gtcgttactc 660
gaagtgtcgg gagaggaaaa tgcttgcgag aagagagtcg ctggagagaa agccaagacg 720
ttcacgcgca tcaagtatgc actcctcact atgctcgaga agtttttgga atgcgttgcc 780
gacgttttca aattggtgcc gctgcctatt acaatgggta ttcgtgcgat tgtggctgct 840
ggatgtacgt tcacttctgc aattattgga ttgtgcactt tctgcgccag agcataa 897

<210> 123
<211> 298
<212> PRT
<213> Chlamydia

<400> 123
Met Ala Ser Ile Cys Gly Arg Leu Gly Ser Gly Thr Gly Asn Ala Leu
1               5                   10                  15
Lys Ala Phe Phe Thr Gln Pro Ser Asn Lys Met Ala Arg Val Val Asn
            20                  25                  30
Lys Thr Lys Gly Met Asp Lys Thr Val Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Thr Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110
Lys Thr Gln Glu Gly Asp Glu Gly Leu Thr Ala Asp Leu Cys Val Ser
        115                 120                 125
His Lys Arg Arg Ala Ala Ala Ala Val Cys Gly Phe Ile Gly Gly Ile
    130                 135                 140
Thr Tyr Leu Ala Thr Phe Gly Val Ile Arg Pro Ile Leu Phe Val Asn
145                 150                 155                 160
Lys Met Leu Val Asn Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Glu Arg Ala Asp Cys Glu Ala
        195                 200                 205
Arg Cys Ala Arg Ile Ala Arg Glu Glu Ser Leu Leu Glu Val Ser Gly
    210                 215                 220
Glu Glu Asn Ala Cys Glu Lys Arg Val Ala Gly Glu Lys Ala Lys Thr
225                 230                 235                 240
Phe Thr Arg Ile Lys Tyr Ala Leu Leu Thr Met Leu Glu Lys Phe Leu

245                 250                 255
Glu Cys Val Ala Asp Val Phe Lys Leu Val Pro Leu Pro Ile Thr Met
        260                 265                 270
Gly Ile Arg Ala Ile Val Ala Ala Gly Cys Thr Phe Thr Ser Ala Ile
    275                 280                 285
Ile Gly Leu Cys Thr Phe Cys Ala Arg Ala
    290                 295

<210> 124
<211> 897
<212> DNA
<213> Chlamydia

<400> 124
atggcttcta tatgcggacg tttagggtct ggtacaggga atgctctaaa agcttttttt      60
acacagccca acaataaaat ggcaagggta gtaaataaga cgaagggaat ggataagact     120
attaaggttg ccaagtctgc tgccgaattg accgcaaata ttttggaaca agctggaggc     180
gcgggctctt ccgcacacat tacagcttcc caagtgtcca aaggattagg ggatgcgaga     240
actgttgtcg ctttagggaa tgcctttaac ggagcgttgc caggaacagt tcaaagtgcg     300
caaagcttct tctctcacat gaaagctgct agtcagaaaa cgcaagaagg ggatgagggg     360
ctcacagcag atctttgtgt gtctcataag cgcagagcgg ctgcggctgt ctgtagcatc     420
atcggaggaa ttacctacct cgcgacattc ggagctatcc gtccgattct gtttgtcaac     480
aaaatgctgg caaaaccgtt tctttcttcc caaactaaag caaatatggg atcttctgtt     540
agctatatta tggcggctaa ccatgcagcg tctgtggtgg gtgctggact cgctatcagt     600
gcggaaagag cagattgcga agcccgctgc gctcgtattg cgagagaaga gtcgttactc     660
gaagtgccgg gagaggaaaa tgcttgcgag aagaaagtcg ctggagagaa agccaagacg     720
ttcacgcgca tcaagtatgc actcctcact atgctcgaga agtttttgga atgcgttgcc     780
gacgttttca aattggtgcc gctgcctatt acaatgggta ttcgtgcgat tgtggctgct     840
ggatgtacgt tcacttctgc aattattgga ttgtgcactt tctgcgccag agcataa        897

<210> 125
<211> 298
<212> PRT
<213> Chlamydia

<400> 125
Met Ala Ser Ile Cys Gly Arg Leu Gly Ser Gly Thr Gly Asn Ala Leu
1               5                   10                  15
Lys Ala Phe Phe Thr Gln Pro Asn Asn Lys Met Ala Arg Val Val Asn
            20                  25                  30
Lys Thr Lys Gly Met Asp Lys Thr Ile Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Ala Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110
Lys Thr Gln Glu Gly Asp Glu Gly Leu Thr Ala Asp Leu Cys Val Ser
        115                 120                 125
His Lys Arg Arg Ala Ala Ala Ala Val Cys Ser Ile Ile Gly Gly Ile
    130                 135                 140
Thr Tyr Leu Ala Thr Phe Gly Ala Ile Arg Pro Ile Leu Phe Val Asn

145                 150                 155                 160
Lys Met Leu Ala Lys Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Glu Arg Ala Asp Cys Glu Ala
        195                 200                 205
Arg Cys Ala Arg Ile Ala Arg Glu Glu Ser Leu Leu Glu Val Pro Gly
    210                 215                 220
Glu Glu Asn Ala Cys Glu Lys Lys Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Ile
        275                 280                 285
Ile Gly Leu Cys Thr Phe Cys Ala Arg Ala
    290                 295

<210> 126
<211> 897
<212> DNA
<213> Chlamydia

<400> 126
atggcttcta tatgcggacg tttagggtct ggtacaggga atgctctaaa agcttttttt      60
acacagccca acaataaaat ggcaagggta gtaaataaga cgaagggaat ggataagact     120
attaaggttg ccaagtctgc tgccgaattg accgcaaata ttttggaaca agctggaggc     180
gcgggctctt ccgcacacat tacagcttcc caagtgtcca aaggattagg ggatgcgaga     240
actgttgtcg ctttagggaa tgcctttaac ggagcgttgc caggaacagt tcaaagtgcg     300
caaagcttct tctctcacat gaaagctgct agtcagaaaa cgcaagaagg ggatgagggg     360
ctcacagcag atctttgtgt gtctcataag cgcagagcgg ctgcggctgt ctgtagcatc     420
atcggaggaa ttacctacct cgcgacattc ggagctatcc gtccgattct gtttgtcaac     480
aaaatgctgg caaaaccgtt tctttcttcc caaactaaag caaatatggg atcttctgtt     540
agctatatta tggcggctaa ccatgcagcg tctgtggtgg gtgctggact cgctatcagt     600
gcggaaagag cagattgcga agcccgctgc gctcgtattg cgagagaaga gtcgttactc     660
gaagtgccgg gagaggaaaa tgcttgcgag aagaaagtcg ctggagagaa agccaagacg     720
ttcacgcgca tcaagtatgc actcctcact atgctcgaga agtttttgga atgcgttgcc     780
gacgttttca aattggtgcc gctgcctatt acaatgggta ttcgtgcgat tgtggctgct     840
ggatgtacgt tcacttctgc aattattgga ttgtgcactt tctgcgccag agcataa        897

<210> 127
<211> 298
<212> PRT
<213> Chlamydia

<400> 127
Met Ala Ser Ile Cys Gly Arg Leu Gly Ser Gly Thr Gly Asn Ala Leu
1               5                   10                  15
Lys Ala Phe Phe Thr Gln Pro Asn Asn Lys Met Ala Arg Val Val Asn
            20                  25                  30
Lys Thr Lys Gly Met Asp Lys Thr Ile Lys Val Ala Lys Ser Ala Ala
        35                  40                  45
Glu Leu Thr Ala Asn Ile Leu Glu Gln Ala Gly Gly Ala Gly Ser Ser

    50                  55                  60
Ala His Ile Thr Ala Ser Gln Val Ser Lys Gly Leu Gly Asp Ala Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110
Lys Thr Gln Glu Gly Asp Glu Gly Leu Thr Ala Asp Leu Cys Val Ser
        115                 120                 125
His Lys Arg Arg Ala Ala Ala Ala Val Cys Ser Ile Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Lys Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Glu Arg Ala Asp Cys Glu Ala
        195                 200                 205
Arg Cys Ala Arg Ile Ala Arg Glu Glu Ser Leu Leu Glu Val Pro Gly
    210                 215                 220
Glu Glu Asn Ala Cys Glu Lys Lys Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Ile
        275                 280                 285
Ile Gly Leu Cys Thr Phe Cys Ala Arg Ala
    290                 295

<210> 128
<211> 897
<212> DNA
<213> Chlamydia

<400> 128
atggcttcta tatgtggacg tttagggtct ggtacaggga atgctctaaa agcttttttt 60
acacagccca gcaataaaat ggcaagggta gtaaataaga cgaagggaat ggataagact 120
gttaaggtcg ccaagtctgc tgccgaattg accgcaaata ttttggaaca agctggaggc 180
gcgggctctt ccgcacacat tacagcttcc caagtgtcca aaggattagg ggatacgaga 240
actgttgtcg ctttagggaa tgcctttaac ggagcgttgc caggaacagt tcaaagtgcg 300
caaagcttct tctctcacat gaaagctgct agtcagaaaa cgcaagaagg ggatgagggg 360
ctcacagcag atctttgtgt gtctcataag cgcagagcgg ctgcggctgt ctgtggcttc 420
atcggaggaa ttacctacct cgcgacattc ggagttatcc gtccgattct gtttgtcaac 480
aaaatgctgg tgaacccgtt tctttcttcc caaactaaag caaatatggg atcttctgtt 540
agctatatta tggcggctaa ccatgcagcg tctgtggtgg gtgctggact cgctatcagt 600
gcggaaagag cagattgcga agcccgctgc gctcgtattg cgagagaaga gtcgttactc 660
gaagtgtcgg gagaggaaaa tgcttgcgag aagagagtcg ctggagagaa agccaagacg 720
ttcacgcgca tcaagtatgc actcctcact atgctcgaga agtttttgga atgcgttgcc 780
gacgttttca aattggtgcc gctgcctatt acaatgggta ttcgtgcgat tgtggctgct 840
ggatgtacgt tcacttctgc aattattgga ttgtgcactt tctgcgccag agcataa 897

<210> 129

<211> 298
<212> PRT
<213> Chlamydia

<400> 129
Met Ala Ser Ile Cys Gly Arg Leu Gly Ser Gly Thr Gly Asn Ala Leu
1               5                   10                  15
Lys Ala Phe Phe Thr Gln Pro Ser Asn Lys Met Ala Arg Val Val Asn
            20                  25                  30
Lys Thr Lys Gly Met Asp Lys Thr Val Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Thr Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110
Lys Thr Gln Glu Gly Asp Glu Gly Leu Thr Ala Asp Leu Cys Val Ser
        115                 120                 125
His Lys Arg Arg Ala Ala Ala Ala Val Cys Gly Phe Ile Gly Gly Ile
    130                 135                 140
Thr Tyr Leu Ala Thr Phe Gly Val Ile Arg Pro Ile Leu Phe Val Asn
145                 150                 155                 160
Lys Met Leu Val Asn Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Glu Arg Ala Asp Cys Glu Ala
        195                 200                 205
Arg Cys Ala Arg Ile Ala Arg Glu Glu Ser Leu Leu Glu Val Ser Gly
    210                 215                 220
Glu Glu Asn Ala Cys Glu Lys Arg Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Ile
        275                 280                 285
Ile Gly Leu Cys Thr Phe Cys Ala Arg Ala
    290                 295

<210> 130
<211> 897
<212> DNA
<213> Chlamydia

<400> 130
atggctgcta tatgtggacg tttagggtct ggtacaggga atgctctaaa agcttttttt      60
acacagccca gcaataaaat ggcaagggta gtaaataaga cgaagggaat ggataagact      120
gttaaggtcg ccaagtctgc tgccgaattg accgcaaata ttttggaaca agctggaggc      180
gcgggctctt ccgcacacat tacagcttcc caagtgtcca aaggattagg ggatgcgaga      240
actgttctcg ctttagggaa tgcctttaac ggagcgttgc caggaacagt tcaaagtgcg      300

caaagcttct tctcttacat gaaagctgct agtcagaaac cgcaagaagg ggatgagggg 360
ctcgtagcag atctttgtgt gtctcataag cgcagagcgg ctgcggctgt ctgtagcttc 420
atcggaggaa ttacctacct cgcgacattc ggagctatcc gtccgattct gtttgtcaac 480
aaaatgctgg cgcaaccgtt tctttcttcc caaactaaag caaatatggg atcttctgtt 540
agctatatta tggcggctaa ccatgcagcg tttgtggtgg gttctggact cgctatcagt 600
gcggaaagag cagattgcga agcccgctgc gctcgtattg cgagagaaga gtcgtcactc 660
gaattgtcgg gagaggaaaa tgcttgcgag aggggagtcg ctggagagaa agccaagacg 720
ttcacgcgca tcaagtatgc actcctcact atgctcgaga agtttttgga atgcgttgcc 780
gacgttttca aattggtgcc gttgcctatt acaatgggta ttcgtgcaat tgtggctgcg 840
ggatgtacgt tcacttctgc agttattgga ttgtggactt tctgcaacag agtataa 897

<210> 131
<211> 298
<212> PRT
<213> Chlamydia

<400> 131
Met Ala Ala Ile Cys Gly Arg Leu Gly Ser Gly Thr Gly Asn Ala Leu
1               5                   10                  15
Lys Ala Phe Phe Thr Gln Pro Ser Asn Lys Met Ala Arg Val Val Asn
            20                  25                  30
Lys Thr Lys Gly Met Asp Lys Thr Val Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Ala Arg
65                  70                  75                  80
Thr Val Leu Ala Leu Gly Asn Ala Phe Asn Gly Ala Leu Pro Gly Thr
                85                  90                  95
Val Gln Ser Ala Gln Ser Phe Phe Ser Tyr Met Lys Ala Ala Ser Gln
            100                 105                 110
Lys Pro Gln Glu Gly Asp Glu Gly Leu Val Ala Asp Leu Cys Val Ser
        115                 120                 125
His Lys Arg Arg Ala Ala Ala Ala Val Cys Ser Phe Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Gln Pro Phe Leu Ser Ser Gln Thr Lys Ala Asn Met
                165                 170                 175
Gly Ser Ser Val Ser Tyr Ile Met Ala Ala Asn His Ala Ala Phe Val
            180                 185                 190
Val Gly Ser Gly Leu Ala Ile Ser Ala Glu Arg Ala Asp Cys Glu Ala
        195                 200                 205
Arg Cys Ala Arg Ile Ala Arg Glu Glu Ser Ser Leu Glu Leu Ser Gly
    210                 215                 220
Glu Glu Asn Ala Cys Glu Arg Gly Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Val
        275                 280                 285
Ile Gly Leu Trp Thr Phe Cys Asn Arg Val
    290                 295

<210> 132
<211> 897
<212> DNA
<213> Chlamydia

<400> 132
atggctgcta tatgcggacg tttagggtct ggtacaggga atgctctaaa agcttttttt 60
acacagccca gcaataaaat ggcaagggta gtaaataaga cgaagggaat ggataagact 120
gttaaggtcg ccaagtctgc tgccgaattg accgcaaata ttttggaaca agctggaggc 180
gcgggctctt ccgcacacat tacagcttcc caagtgtcca aaggattagg ggatgcgaga 240
actgttctcg ctttagggaa tgcctttaac ggagcgttgc caggaacagt tcaaagtgcg 300
caaagcttct tctcttacat gaaagctgct agtcagaaac cgcaagaagg ggatgagggg 360
ctcgtagcag atctttgtgt gtctcataag cgcagagcgg ctgcggctgt ctgtagcttc 420
atcggaggaa ttacctacct cgcgacattc ggagctatcc gtccgattct gtttgtcaac 480
aaaatgctgg cgcaaccgtt tctttcttcc caaactaaag caaatatggg atcttctgtt 540
agctatatta tggcggctaa ccatgcagcg tttgtggtgg gttctggact cgctatcagt 600
gcggaaagag cagattgcga agcccgctgc gctcgtattg cgagagaaga gtcgtcactc 660
gaattgtcgg gagaggaaaa tgcttgtgag aggagagtcg ctggagagaa agccaagacg 720
ttcacgcgca tcaagtatgc actcctcact atgctcgaga agtttttgga atgcgttgcc 780
gacgttttca aattggtgcc gttgcctatt acaatgggta ttcgtgcaat tgtggctgcg 840
ggatgtacgt tcacttctgc agttattgga ttgtggactt tctgcaacag agtataa 897

<210> 133
<211> 298
<212> PRT
<213> Chlamydia

<400> 133
Met Ala Ala Ile Cys Gly Arg Leu Gly Ser Gly Thr Gly Asn Ala Leu
1               5                   10                  15
Lys Ala Phe Phe Thr Gln Pro Ser Asn Lys Met Ala Arg Val Val Asn
            20                  25                  30
Lys Thr Lys Gly Met Asp Lys Thr Val Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Ala Arg
65                  70                  75                  80
Thr Val Leu Ala Leu Gly Asn Ala Phe Asn Gly Ala Leu Pro Gly Thr
                85                  90                  95
Val Gln Ser Ala Gln Ser Phe Phe Ser Tyr Met Lys Ala Ala Ser Gln
            100                 105                 110
Lys Pro Gln Glu Gly Asp Glu Gly Leu Val Ala Asp Leu Cys Val Ser
        115                 120                 125
His Lys Arg Arg Ala Ala Ala Ala Val Cys Ser Phe Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Gln Pro Phe Leu Ser Ser Gln Thr Lys Ala Asn Met
                165                 170                 175
Gly Ser Ser Val Ser Tyr Ile Met Ala Ala Asn His Ala Ala Phe Val
            180                 185                 190
Val Gly Ser Gly Leu Ala Ile Ser Ala Glu Arg Ala Asp Cys Glu Ala
        195                 200                 205

Arg Cys Ala Arg Ile Ala Arg Glu Glu Ser Ser Leu Glu Leu Ser Gly
    210                 215                 220
Glu Glu Asn Ala Cys Glu Arg Arg Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Val
        275                 280                 285
Ile Gly Leu Trp Thr Phe Cys Asn Arg Val
    290                 295

<210> 134
<211> 897
<212> DNA
<213> Chlamydia

<400> 134
atggcttcta tatgcggacg tttagggtct ggtacaggga atgctctaaa agcttttttt      60
acacagccca acaataaaat ggcaagggta gtaaataaga cgaagggaat ggataagact     120
attaaggttg ccaagtctgc tgccgaattg accgcaaata ttttggaaca agctggaggc     180
gcgggctctt ccgcacacat tacagcttcc caagtgtcca aaggattagg ggatgcgaga     240
actgttgtcg ctttagggaa tgcctttaac ggagcgttgc caggaacagt tcaaagtgcg     300
caaagcttct tctctcacat gaaagctgct agtcagaaaa cgcaagaagg ggatgagggg     360
ctcacagcag atctttgtgt gtctcataag cgcagagcgg ctgcggctgt ctgtagcatc     420
atcggaggaa ttacctacct cgcgacattc ggagctatcc gtccgattct gtttgtcaac     480
aaaatgctgg caaaaccgtt tctttcttcc caaactaaag caaatatggg atcttctgtt     540
agctatatta tggcggctaa ccatgcagcg tctgtggtgg gtgctggact cgctatcagt     600
gcggaaagag cagattgcga agcccgctgc gctcgtattg cgagagaaga gtcgttactc     660
gaaatgccgg gagaggaaaa tgcttgcgag aagaaagtcg ctggagagaa agccaagacg     720
ttcacgcgca tcaagtatgc actcctcact atgctcgaga agtttttgga atgcgttgcc     780
gacgttttca aattggtgcc gctgcctatt acaatgggta ttcgtgcgat tgtggctgct     840
ggatgtacgt tcacttctgc aattattgga ttgtgcactt tctgcgccag agcataa        897

<210> 135
<211> 298
<212> PRT
<213> Chlamydia

<400> 135
Met Ala Ser Ile Cys Gly Arg Leu Gly Ser Gly Thr Gly Asn Ala Leu
1               5                   10                  15
Lys Ala Phe Phe Thr Gln Pro Asn Asn Lys Met Ala Arg Val Val Asn
            20                  25                  30
Lys Thr Lys Gly Met Asp Lys Thr Ile Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Ala Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110

Lys Thr Gln Glu Gly Asp Glu Gly Leu Thr Ala Asp Leu Cys Val Ser
        115                 120                 125
His Lys Arg Arg Ala Ala Ala Ala Val Cys Ser Ile Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Lys Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Glu Arg Ala Asp Cys Glu Ala
        195                 200                 205
Arg Cys Ala Arg Ile Ala Arg Glu Glu Ser Leu Leu Glu Met Pro Gly
    210                 215                 220
Glu Glu Asn Ala Cys Glu Lys Lys Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Ile
        275                 280                 285
Ile Gly Leu Cys Thr Phe Cys Ala Arg Ala
    290                 295

<210> 136
<211> 882
<212> DNA
<213> Chlamydia

<400> 136
atggcttctg tatgtgggcg attaagtgct ggggtgggga acagatttaa cgcatttttc      60
acgcgtcccg gtaacaagct atcacggttt gtaaatagcg caaaaggatt agacagatca     120
ataaaggttg ggaagtctgc tgctgaatta acggcgagta ttttagagca aactgggggg     180
gcagggactg atgcacatgt tacggcggcc aaggtgtcta aagcacttgg ggacgcgcga     240
acagtaatgg ctctagggaa tgtcttcaat gggtctgtgc cagcaaccat tcaaagtgcg     300
cgaagctgtc tcgcccattt acgagcggcc ggcaaagaag aagaaacatg ctccaaggtg     360
aaagatctct gtgtttctca tagacgaaga gctgcggctg aggcttgtaa tgttattgga     420
ggagcaactt atattacaac tttcggagcg attcgtccga cattactcgt taacaagctt     480
cttgccaaac cattcctttc ctcccaagcc aaagaagggt tgggagcttc tgttggttat     540
atcatggcag cgaaccatgc ggcatctgtg cttgggtctg ctttaagtat tagcgcagaa     600
agagcagact gtgaagagcg gtgtgatcgc attcgatgta gtgaggatgg tgaaatttgc     660
gaaggcaata aattaacagc tatttcggaa gagaaggcta gatcatggac tctcattaag     720
tacagattcc ttactatgat agaaaaacta tttgagatgg tggcggatat cttcaagtta     780
attcctttgc caatttcgca tggaattcgt gctattgttg ctgcgggatg tacgttgact     840
tctgcagtta ttggcttagg tactttttgg tctagagcat aa                        882

<210> 137
<211> 293
<212> PRT
<213> Chlamydia

<400> 137
Met Ala Ser Val Cys Gly Arg Leu Ser Ala Gly Val Gly Asn Arg Phe
1               5                   10                  15

Asn Ala Phe Phe Thr Arg Pro Gly Asn Lys Leu Ser Arg Phe Val Asn
            20                  25                  30
Ser Ala Lys Gly Leu Asp Arg Ser Ile Lys Val Gly Lys Ser Ala Ala
        35                  40                  45
Glu Leu Thr Ala Ser Ile Leu Glu Gln Thr Gly Gly Ala Gly Thr Asp
    50                  55                  60
Ala His Val Thr Ala Ala Lys Val Ser Lys Ala Leu Gly Asp Ala Arg
65                  70                  75                  80
Thr Val Met Ala Leu Gly Asn Val Phe Asn Gly Ser Val Pro Ala Thr
                85                  90                  95
Ile Gln Ser Ala Arg Ser Cys Leu Ala His Leu Arg Ala Ala Gly Lys
            100                 105                 110
Glu Glu Glu Thr Cys Ser Lys Val Lys Asp Leu Cys Val Ser His Arg
        115                 120                 125
Arg Arg Ala Ala Ala Glu Ala Cys Asn Val Ile Gly Gly Ala Thr Tyr
    130                 135                 140
Ile Thr Thr Phe Gly Ala Ile Arg Pro Thr Leu Leu Val Asn Lys Leu
145                 150                 155                 160
Leu Ala Lys Pro Phe Leu Ser Ser Gln Ala Lys Glu Gly Leu Gly Ala
                165                 170                 175
Ser Val Gly Tyr Ile Met Ala Ala Asn His Ala Ala Ser Val Leu Gly
            180                 185                 190
Ser Ala Leu Ser Ile Ser Ala Glu Arg Ala Asp Cys Glu Glu Arg Cys
        195                 200                 205
Asp Arg Ile Arg Cys Ser Glu Asp Gly Glu Ile Cys Glu Gly Asn Lys
    210                 215                 220
Leu Thr Ala Ile Ser Glu Glu Lys Ala Arg Ser Trp Thr Leu Ile Lys
225                 230                 235                 240
Tyr Arg Phe Leu Thr Met Ile Glu Lys Leu Phe Glu Met Val Ala Asp
                245                 250                 255
Ile Phe Lys Leu Ile Pro Leu Pro Ile Ser His Gly Ile Arg Ala Ile
            260                 265                 270
Val Ala Ala Gly Cys Thr Leu Thr Ser Ala Val Ile Gly Leu Gly Thr
        275                 280                 285
Phe Trp Ser Arg Ala
    290

<210> 138
<211> 16
<212> PRT
<213> Artificial Sequence

<220>
<223> Made in a lab

<400> 138
Asp Leu Cys Val Ser His Lys Arg Arg Ala Ala Ala Ala Val Cys Ser
1               5                   10                  15

<210> 139
<211> 16
<212> PRT
<213> Artificial Sequence

<220>

<223> Made in a lab

<400> 139
Arg Ala Ala Ala Ala Val Cys Ser Phe Ile Gly Gly Ile Thr Tyr Leu
1               5                   10                  15

<210> 140
<211> 18
<212> PRT
<213> Artificial Sequence

<220>
<223> Made in a lab

<400> 140
Cys Ser Phe Ile Gly Gly Ile Thr Tyr Leu Ala Thr Phe Gly Ala Ile
1               5                   10                  15
Arg Pro

<210> 141
<211> 18
<212> PRT
<213> Artificial Sequence

<220>
<223> Made in a lab

<400> 14
Tyr Leu Ala Thr Phe Gly Ala Ile Arg Pro Ile Leu Phe Val Asn Lys
1               5                   10                  15
Met Leu

<210> 142
<211> 18
<212> PRT
<213> Artificial Sequence

<220>
<223> Made in a lab

<400> 142
Arg Pro Ile Leu Phe Val Asn Lys Met Leu Ala Gln Pro Phe Leu Ser
1               5                   10                  15
Ser Gln

<210> 143
<211> 17
<212> PRT
<213> Artificial Sequence

<220>
<223> Made in a lab

<400> 143
Met Leu Ala Gln Pro Phe Leu Ser Ser Gln Thr Lys Ala Asn Met Gly
1               5                   10                  15
Ser

<210> 144
<211> 10
<212> PRT
<213> Artificial Sequence

<220>
<223> Made in a lab

<400> 144
Cys Ser Phe Ile Gly Gly Ile Thr Tyr Leu
1               5                   10

<210> 145
<211> 9
<212> PRT
<213> Artificial Sequence

<220>
<223> Made in a lab

<400> 145
Ser Phe Ile Gly Gly Ile Thr Tyr Leu
1               5

<210> 146
<211> 8
<212> PRT
<213> Artificial Sequence

<220>
<223> Made in a lab

<400> 146
Phe Ile Gly Gly Ile Thr Tyr Leu
1               5

<210> 147
<211> 9
<212> PRT
<213> Artificial Sequence

<220>
<223> Made in a lab

<400> 147
Cys Ser Phe Ile Gly Gly Ile Thr Tyr
1               5

<210> 148
<211> 8
<212> PRT
<213> Artificial Sequence

<220>
<223> Made in a lab

<400> 148
Cys Ser Phe Ile Gly Gly Ile Thr
1               5

<210> 149
<211> 10
<212> PRT
<213> Artificial Sequence

<220>
<223> Made in a lab

<400> 149
Cys Ser Ile Ile Gly Gly Ile Thr Tyr Leu
1               5                   10

<210> 150
<211> 10
<212> PRT
<213> Artificial Sequence

<220>
<223> Made in a lab

<400> 150
Cys Gly Phe Ile Gly Gly Ile Thr Tyr Leu
1               5                   10

<210> 151
<211> 9
<212> PRT
<213> Artificial Sequence

<220>
<223> Made in a lab

<400> 151
Gly Phe Ile Gly Gly Ile Thr Tyr Leu
1               5

<210> 152
<211> 20
<212> PRT
<213> Artificial Sequence

<220>
<223> Made in a lab

<400> 152
Gln Ile Phe Val Cys Leu Ile Ser Ala Glu Arg Leu Arg Leu Arg Leu
1               5                   10                  15
Ser Val Ala Ser
            20

<210> 153
<211> 20
<212> PRT
<213> Artificial Sequence

<220>
<223> Made in a lab

<400> 153
Glu Arg Leu Arg Leu Arg Leu Ser Val Ala Ser Ser Glu Glu Leu Pro
1               5                   10                  15
Thr Ser Arg His
            20

<210> 154
<211> 20
<212> PRT
<213> Artificial Sequence

<220>
<223> Made in a lab

<400> 154
Ala Ser Ser Glu Glu Leu Pro Thr Ser Arg His Ser Glu Leu Ser Val
1               5                   10                  15
Arg Phe Cys Leu
            20

<210> 155
<211> 20
<212> PRT
<213> Artificial Sequence

<220>
<223> Made in a lab

<400> 155
Arg His Ser Glu Leu Ser Val Arg Phe Cys Leu Ser Thr Lys Cys Trp
1               5                   10                  15
Arg Asn Arg Phe
            20

<210> 156
<211> 20
<212> PRT
<213> Artificial Sequence

<220>

<223> Made in a lab

<400> 156
Leu Ser Thr Lys Cys Trp Arg Asn Arg Phe Phe Leu Pro Lys Leu Lys
1               5                   10                  15
Gln Ile Trp Asp
            20

<210> 157
<211> 53
<212> PRT
<213> Artificial Sequence

<220>
<223> Made in a lab

<400> 157
Ile Phe Val Cys Leu Ile Ser Ala Glu Arg Leu Arg Leu Ser Val Ala
1               5                   10                  15
Ser Ser Glu Glu Leu Pro Thr Ser Arg His Ser Glu Leu Ser Val Arg
            20                  25                  30
Phe Cys Leu Ser Thr Lys Cys Trp Arg Asn Arg Phe Phe Leu Pro Lys
        35                  40                  45
Leu Lys Gln Ile Trp
    50

<210> 158
<211> 52
<212> PRT
<213> Artificial Sequence

<220>
<223> Made in a lab

<400> 158
Leu Cys Val Ser His Lys Arg Arg Ala Ala Ala Ala Val Cys Ser Phe
1               5                   10                  15
Ile Gly Gly Ile Thr Tyr Leu Ala Thr Phe Gly Ala Ile Arg Pro Ile
            20                  25                  30
Leu Phe Val Asn Lys Met Leu Ala Gln Pro Phe Leu Ser Ser Gln Ile
        35                  40                  45
Lys Ala Asn Met
    50

<210> 159
<211> 24
<212> DNA
<213> Chlamydia

<400> 159
ttttgaagca ggtaggtgaa tatg                                          24

<210> 160
<211> 24
<212> DNA

<213> Chlamydia

<400> 160
ttaagaaatt taaaaaatcc ctta                                              24

<210> 161
<211> 24
<212> DNA
<213> Chlamydia

<400> 161
ggtataatat ctctctaaat tttg                                              24

<210> 162
<211> 19
<212> DNA
<213> Chlamydia

<400> 162
agataaaaaa ggctgtttc                                                    19

<210> 163
<211> 24
<212> DNA
<213> Chlamydia

<400> 163
ttttgaagca ggtaggtgaa tatg                                              24

<210> 164
<211> 29
<212> DNA
<213> Chlamydia

<400> 164
tttacaataa gaaaagctaa gcactttgt                                         29

<210> 165
<211> 20
<212> DNA
<213> Chlamydia

<400> 165
ccttacacag tcctgctgac                                                   20

<210> 166
<211> 20
<212> DNA
<213> Chlamydia

<400> 166
gtttccgggc cctcacattg                                                   20

<210> 167
<211> 9

<212> PRT
<213> Artificial Sequence

<220>
<223> Made in a lab

<400> 167
Ser Phe Ile Gly Gly Ile Thr Tyr Leu
1               5

<210> 168
<211> 9
<212> PRT
<213> Artificial Sequence

<220>
<223> Made in a lab

<400> 168
Ser Ile Ile Gly Gly Ile Thr Tyr Leu
1               5

<210> 169
<211> 2643
<212> DNA
<213> Chlamydia

<400> 169
gcaatcatgc gacctgatca tatgaacttc tgttgtctat gtgctgctat tttgtcatcc      60
acagcggtcc tctttggcca ggatccctta ggtgaaaccg ccctcctcac taaaaatcct     120
aatcatgtcg tctgtacatt ttttgaggac tgtaccatgg agagcctctt tcctgctctt     180
tgtgctcatg catcacaaga cgatcctttg tatgtacttg gaaattccta ctgttggttc     240
gtatctaaac tccatatcac ggaccccaaa gaggctcttt ttaaagaaaa aggagatctt     300
tccattcaaa actttcgctt cctttccttc acagattgct cttccaagga aagctctcct     360
tctattattc atcaaaagaa tggtcagtta tccttgcgca ataatggtag catgagtttc     420
tgtcgaaatc atgctgaagg ctctggagga gccatctctg cggatgcctt ttctctacag     480
cacaactatc ttttcacagc ttttgaagag aattcttcta aaggaaatgg cggagccatt     540
caggctcaaa ccttctcttt atctagaaat gtgtcgccta tttctttcgc ccgtaatcgt     600
gcggatttaa atggcggcgc tatttgctgt agtaatctta tttgttcagg gaatgtaaac     660
cctctctttt tcactggaaa ctccgccacg aatggaggcg ctatttgttg tatcagcgat     720
ctaaacacct cagaaaaagg ctctctctct cttgcttgta accaagaaac gctatttgca     780
agcaattctg ctaaagaaaa aggcggggct atttatgcca agcacatggt attgcgttat     840
aacggtcctg tttccttcat taacaacagc gctaaaatag gtggagctat cgccatccag     900
tccggaggga gtctctctat ccttgcaggt gaaggatctg ttctgttcca gaataactcc     960
caacgcacct ccgaccaagg tctagtaaga aacgccatct acttaragaa agatgcgatt    1020
ctttcttcct tagaagctcg caacggagat attcttttct ttgatcctat tgtacaagaa    1080
agtagcagca aagaatcgcc tcttccctcc tctttgcaag ccagcgtgac ttctcccacc    1140
ccagccaccg catctccttt agttattcag acaagtgcaa accgttcagt gattttctcg    1200
agcgaacgtc tttctgaaga agaaaaaact cctgataacc tcacttccca actacagcag    1260
cctatcgaac tgaaatccgg acgcttagtt ttaaaagatc gcgctgtcct ttccgcgcct    1320
tctctctctc aggatcctca agctctcctc attatggaag cgggaacttc tttaaaaact    1380
tcctctgatt tgaagttagc tacgctaagt attccccttc attccttaga tactgaaaaa    1440
agcgtaacta tccacgcccc taatctttct atccaaaaga tcttcctctc taactctgga    1500
gatgagaatt tttatgaaaa tgtagagctt ctcagtaaag agcaaaacaa tattcctctc    1560
cttactctcc ctaaagagca atctcattta catcttcctg atgggaacct ctcttctcac    1620

tttggatatc aaggagattg gactttttct tggaaagatt ctgatgaagg gcattctctg 1680
attgctaatt ggacgcctaa aaactatgtg cctcatccag aacgtcaatc tacactcgtt 1740
gcgaacactc tttggaacac ctattccgat atgcaagctg tgcagtcgat gattaataca 1800
acagcgcacg gaggagccta tctatttgga acgtggggat ctgctgtttc taatttattc 1860
tatgttcacg acagctctgg gaaacctatc gataattggc atcatagaag ccttggctac 1920
ctattcggta tcagtactca cagtttagat gaccattctt tctgcttggc tgcaggacaa 1980
ttactcggga aatcgtccga ttcctttatt acgtctacag aaacgacctc ctatatagct 2040
actgtacaag cgcaactcgc tacctctcta atgaaaatct ctgcacaggc atgctacaat 2100
gaaagtatcc atgagctaaa aacaaaatat cgctccttct ctaaagaagg attcggatcc 2160
tggcatagcg ttgcagtatc cggagaagtg tgcgcatcga ttcctattgt atccaatggt 2220
tccggactgt tcagctcctt ctctattttc tctaaactgc aaggattttc aggaacacag 2280
gacggttttg aggagagttc gggagagatt cggtcctttt ctgccagctc tttcagaaat 2340
atttcacttc ctataggaat aacatttgaa aaaaaatccc aaaaaacacg aacctactat 2400
tactttctag gagcctacat ccaagacctg aaacgtgatg tggaatcggg acctgtagtg 2460
ttactcaaaa atgccgtctc ctgggatgct cctatggcga acttggattc acgagcctac 2520
atgttccggc ttacgaatca aagagctcta cacagacttc agacgctgtt aaatgtgtct 2580
tgtgtgctgc gtgggcaaag ccatagttac tccctggatc tggggaccac ttacaggttc 2640
tag 2643

<210> 170
<211> 2949
<212> DNA
<213> Chlamydia

<400> 170
atgattcctc aaggaattta cgatggggag acgttaactg tatcatttcc ctatactgtt 60
ataggagatc cgagtgggac tactgttttt tctgcaggag agttaacatt aaaaaatctt 120
gacaattcta ttgcagcttt gcctttaagt tgttttggga acttattagg gagttttact 180
gttttaggga gaggacactc gttgactttc gagaacatac ggacttctac aaatggggca 240
gctctaagta atagcgctgc tgatggactg tttactattg agggttttaa agaattatcc 300
ttttccaatt gcaattcatt acttgccgta ctgcctgctg caacgactaa taagggtagc 360
cagactccga cgacaacatc tacaccgtct aatggtacta tttattctaa aacagatctt 420
ttgttactca ataatgagaa gttctcattc tatagtaatt tagtctctgg agatggggga 480
gctatagatg ctaagagctt aacggttcaa ggaattagca agctttgtgt cttccaagaa 540
aatactgctc aagctgatgg gggagcttgt caagtagtca ccagtttctc tgctatggct 600
aacgaggctc ctattgcctt tgtagcgaat gttgcaggag taagaggggg agggattgct 660
gctgttcagg atgggcagca gggagtgtca tcatctactt caacagaaga tccagtagta 720
agtttttcca gaaatactgc ggtagagttt gatgggaacg tagcccgagt aggaggaggg 780
atttactcct acgggaacgt tgctttcctg aataatggaa aaaccttgtt tctcaacaat 840
gttgcttctc ctgtttacat tgctgctaag caaccaacaa gtggacaggc ttctaatacg 900
agtaataatt acggagatgg aggagctatc ttctgtaaga atggtgcgca agcaggatcc 960
aataactctg gatcagtttc ctttgatgga gagggagtag ttttctttag tagcaatgta 1020
gctgctggga aagggggagc tatttatgcc aaaaagctct cggttgctaa ctgtggccct 1080
gtacaatttt taaggaatat cgctaatgat ggtggagcga tttatttagg agaatctgga 1140
gagctcagtt tatctgctga ttatggagat attattttcg atgggaatct taaaagaaca 1200
gccaaagaga atgctgccga tgttaatggc gtaactgtgt cctcacaagc catttcgatg 1260
ggatcgggag ggaaaataac gacattaaga gctaaagcag ggcatcagat tctctttaat 1320
gatcccatcg agatggcaaa cggaaataac cagccagcgc agtcttccaa acttctaaaa 1380
attaacgatg gtgaaggata cacaggggat attgtttttg ctaatggaag cagtactttg 1440
taccaaaatg ttacgataga gcaaggaagg attgttcttc gtgaaaaggc aaaattatca 1500
gtgaattctc taagtcagac aggtgggagt ctgtatatgg aagctgggag tacattggat 1560
tttgtaactc cacaaccacc acaacagcct cctgccgcta atcagttgat cacgctttcc 1620
aatctgcatt tgtctctttc ttctttgtta gcaaacaatg cagttacgaa tcctcctacc 1680
aatcctccag cgcaagattc tcatcctgca gtcattggta gcacaactgc tggttctgtt 1740
acaattagtg ggcctatctt ttttgaggat ttggatgata cagcttatga taggtatgat 1800

tggctaggtt ctaatcaaaa aatcaatgtc ctgaaattac agttagggac taagccccca 1860
gctaatgccc catcagattt gactctaggg aatgagatgc ctaagtatgg ctatcaagga 1920
agctggaagc ttgcgtggga tcctaataca gcaaataatg gtccttatac tctgaaagct 1980
acatggacta aaactgggta taatcctggg cctgagcgag tagcttcttt ggttccaaat 2040
agtttatggg gatccatttt agatatacga tctgcgcatt cagcaattca agcaagtgtg 2100
gatgggcgct cttattgtcg aggattatgg gtttctggag tttcgaattt cttctatcat 2160
gaccgcgatg ctttaggtca gggatatcgg tatattagtg gggttattcc cttaggagca 2220
aactcctact ttggatcatc gatgtttggt ctagcattta ccgaagtatt tggtagatct 2280
aaagattatg tagtgtgtcg ttccaatcat catgcttgca taggatccgt ttatctatct 2340
acccaacaag ctttatgtgg atcctatttg ttcggagatg cgtttatccg tgctagctac 2400
gggtttggga atcagcatat gaaaacctca tatacatttg cagaggagag cgatgttcgt 2460
tgggataata actgtctggc tggagagatt ggagcgggat taccgattgt gattactcca 2520
tctaagctct atttgaatga gttgcgtcct ttcgtgcaag ctgagttttc ttatgccgat 2580
catgaatctt ttacagagga aggcgatcaa gctcgggcat tcaagagcgg acatctccta 2640
aatctatcag ttcctgttgg agtgaagttt gatcgatgtt ctagtacaca tcctaataaa 2700
tatagcttta tggcggctta tatctgtgat gcttatcgca ccatctctgg tactgagaca 2760
acgctcctat cccatcaaga gacatggaca acagatgcct ttcatttagc aagacatgga 2820
gttgtggtta gaggatctat gtatgcttct ctaacaagta atatagaagt atatggccat 2880
ggaagatatg agtatcgaga tgcttctcga ggctatggtt tgagtgcagg magtaaagtc 2940
yggttctaa 2949

<210> 171
<211> 2895
<212> DNA
<213> Chlamydia

<400> 171
atgaaaaaag cgtttttctt tttccttatc ggaaactccc tatcaggact agctagagag 60
gttccttcta gaatctttct tatgcccaac tcagttccag atcctacgaa agagtcgcta 120
tcaaataaaa ttagtttgac aggagacact cacaatctca ctaactgcta tctcgataac 180
ctacgctaca tactggctat tctacaaaaa actcccaatg aaggagctgc tgtcacaata 240
acagattacc taagcttttt tgatacacaa aaagaaggta tttattttgc aaaaaatctc 300
acccctgaaa gtggtggtgc gattggttat gcgagtccca attctcctac cgtggagatt 360
cgtgatacaa taggtcctgt aatctttgaa aataatactt gttgcagact atttacatgg 420
agaaatcctt atgctgctga taaaataaga gaaggcggag ccattcatgc tcaaaatctt 480
tacataaatc ataatcatga tgtggtcgga tttatgaaga acttttctta tgtccaagga 540
ggagccatta gtaccgctaa tacctttgtt gtgagcgaga atcagtcttg ttttctcttt 600
atggacaaca tctgtattca aactaataca gcaggaaaag gtggcgctat ctatgctgga 660
acgagcaatt cttttgagag taataactgc gatctcttct tcatcaataa cgcctgttgt 720
gcaggaggag cgatcttctc ccctatctgt tctctaacag gaaatcgtgg taacatcgtt 780
ttctataaca atcgctgctt taaaaatgta gaaacagctt cttcagaagc ttctgatgga 840
ggagcaatta aagtaactac tcgcctagat gttacaggca atcgtggtag gatctttttt 900
agtgacaata tcacaaaaaa ttatggcgga gctatttacg ctcctgtagt taccctagtg 960
gataatggcc ctacctactt tataaacaat atcgccaata ataagggggg cgctatctat 1020
atagacggaa ccagtaactc caaaatttct gccgaccgcc atgctattat ttttaatgaa 1080
aatattgtga ctaatgtaac taatgcaaat ggtaccagta cgtcagctaa tcctcctaga 1140
agaaatgcaa taacagtagc aagctcctct ggtgaaattc tattaggagc agggagtagc 1200
caaaatttaa ttttttatga tcctattgaa gttagcaatg caggggtctc tgtgtccttc 1260
aataaggaag ctgatcaaac aggctctgta gtattttcag gagctactgt taattctgca 1320
gattttcatc aacgcaattt acaaacaaaa acacctgcac cccttactct cagtaatggt 1380
tttctatgta tcgaagatca tgctcagctt acagtgaatc gattcacaca aactgggggt 1440
gttgtttctc ttgggaatgg agcagttctg agttgctata aaaatggtac aggagattct 1500
gctagcaatg cctctataac actgaagcat attggattga atctttcttc cattctgaaa 1560
agtggtgctg agattccttt attgtgggta gagcctacaa ataacagcaa taactataca 1620
gcagatactg cagctacctt ttcattaagt gatgtaaaac tctcactcat tgatgactac 1680

gggaactctc cttatgaatc cacagatctg acccatgctc tgtcatcaca gcctatgcta 1740
tctatttctg aagctagcga taaccagcta caatcagaaa atatagattt ttcgggacta 1800
aatgtccctc attatggatg gcaaggactt tggacttggg gctgggcaaa aactcaagat 1860
ccagaaccag catcttcagc aacaatcact gatccacaaa aagccaatag atttcataga 1920
accttactac taacatggct tcctgccggg tatgttccta gcccaaaaca cagaagtccc 1980
ctcatagcta acaccttatg ggggaatatg ctgcttgcaa cagaaagctt aaaaaatagt 2040
gcagagctga cacctagtgg tcatcctttc tggggaatta caggaggagg actaggcatg 2100
atggtttacc aagatcctcg agaaaatcat cctggattcc atatgcgctc ttccggatac 2160
tctgcgggga tgatagcagg gcagacacac accttctcat tgaaattcag tcagacctac 2220
accaaactca atgagcgtta cgcaaaaaac aacgtatctt ctaaaaatta ctcatgccaa 2280
ggagaaatgc tcttctcatt gcaagaaggt ttcttgctga ctaaattagt tgggctttac 2340
agctatggag accataactg tcaccatttc tatactcaag gagaaaatct aacatctcaa 2400
gggacgttcc gcagtcaaac gatgggaggt gctgtctttt ttgatctccc tatgaaaccc 2460
tttggatcaa cgcatatact gacagctccc tttttaggtg ctcttggtat ttattctagc 2520
ctgtctcact ttactgaggt gggagcctat ccgcgaagct tttctacaaa gactcctttg 2580
atcaatgtcc tagtccctat tggagttaaa ggtagcttta tgaatgctac ccacagacct 2640
caagcctgga ctgtagaatt ggcataccaa cccgttctgt atagacaaga accagggatc 2700
gcgacccagc tcctagccag taaaggtatt tggtttggta gtggaagccc ctcatcgcgt 2760
catgccatgt cctataaaat ctcacagcaa acacaacctt tgagttggtt aactctccat 2820
ttccagtatc atggattcta ctcctcttca accttctgta attatctcaa tggggaaatt 2880
gctctgcgat tctag 2895

<210> 172
<211> 4593
<212> DNA
<213> Chlamydia

<400> 172
atgagttccg agaaagatat aaaaagcacc tgttctaagt tttctttgtc tgtagtagca 60
gctatccttg cctctgttag cgggttagct agttgcgtag atcttcatgc tggaggacag 120
tctgtaaatg agctggtata tgtaggccct caagcggttt tattgttaga ccaaattcga 180
gatctattcg ttgggtctaa agatagtcag gctgaaggac agtataggtt aattgtagga 240
gatccaagtt ctttccaaga gaaagatgca gatactcttc ccgggaaggt agagcaaagt 300
actttgttct cagtaaccaa tcccgtggtt ttccaaggtg tggaccaaca ggatcaagtc 360
tcttcccaag ggttaatttg tagttttacg agcagcaacc ttgattctcc ccgtgacgga 420
gaatcttttt taggtattgc ttttgttggg gatagtagta aggctggaat cacattaact 480
gacgtgaaag cttctttgtc tggagcggct ttatattcta cagaagatct tatctttgaa 540
aagattaagg gtggattgga atttgcatca tgttcttctc tagaacaggg gggagcttgt 600
gcagctcaaa gtattttgat tcatgattgt caaggattgc aggttaaaca ctgtactaca 660
gccgtgaatg ctgaggggtc tagtgcgaat gatcatcttg gatttggagg aggcgctttc 720
tttgttacgg gttctctttc tggagagaaa agtctctata tgcctgcagg agatatggta 780
gttgcgaatt gtgatggggc tatatctttt gaaggaaaca gcgcgaactt tgctaatgga 840
ggagcgattg ctgcctctgg gaaagtgctt tttgtcgcta atgataaaaa gacttctttt 900
atagagaacc gagctttgtc tggaggagcg attgcagcct cttctgatat tgcctttcaa 960
aactgcgcag aactagtttt caaaggcaat tgtgcaattg gaacagagga taaaggttct 1020
ttaggtggag gggctatatc ttctctaggc accgttcttt tgcaagggaa tcacgggata 1080
acttgtgata agaatgagtc tgcttcgcaa ggaggcgcca tttttggcaa aaattgtcag 1140
atttctgaca acgaggggcc agtggttttc agagatagta cagcttgctt aggaggaggc 1200
gctattgcag ctcaagaaat tgtttctatt cagaacaatc aggctgggat ttccttcgag 1260
ggaggtaagg ctagtttcgg aggaggtatt gcgtgtggat ctttttcttc cgcaggcggt 1320
gcttctgttt tagggactat tgatatttcg aagaatttag gcgcgatttc gttctctcgt 1380
actttatgta cgacctcaga tttaggacaa atggagtacc agggaggagg agctctattt 1440
ggtgaaaata tttctctttc tgagaatgct ggtgtgctca cctttaaaga caacattgtg 1500
aagacttttg cttcgaatgg gaaaattctg ggaggaggag cgattttagc tactggtaag 1560
gtggaaatta ccaataattc cggaggaatt tcttttacag gaaatgcgag agctccacaa 1620

gctcttccaa ctcaagagga gtttccttta ttcagcaaaa aagaagggcg accactctct 1680
tcaggatatt ctgggggagg agcgatttta ggaagagaag tagctattct ccacaacgct 1740
gcagtagtat ttgagcaaaa tcgtttgcag tgcagcgaag aagaagcgac attattaggt 1800
tgttgtggag gaggcgctgt tcatgggatg gatagcactt cgattgttgg caactcttca 1860
gtaagatttg gtaataatta cgcaatggga caaggagtct caggaggagc tcttttatct 1920
aaaacagtgc agttagctgg aaatggaagc gtcgattttt ctcgaaatat tgctagtttg 1980
ggaggaggag ctcttcaagc ttctgaagga aattgtgagc tagttgataa cggctatgtg 2040
ctattcagag ataatcgagg gagggtttat gggggtgcta tttcttgctt acgtggagat 2100
gtagtcattt ctggaaacaa gggtagagtt gaatttaaag acaacatagc aacacgtctt 2160
tatgtggaag aaactgtaga aaaggttgaa gaggtagagc cagctcctga gcaaaaagac 2220
aataatgagc tttctttctt agggagtgta gaacagagtt ttattactgc agctaatcaa 2280
gctcttttcg catctgaaga tggggattta tcacctgagt catccatttc ttctgaagaa 2340
cttgcgaaaa gaagagagtg tgctggagga gctatttttg caaaacgggt tcgtattgta 2400
gataaccaag aggccgttgt attctcgaat aacttctctg atatttatgg cggcgccatt 2460
tttacaggtt ctcttcgaga agaggataag ttagatgggc aaatccctga agtcttgatc 2520
tcaggcaatg caggggatgt tgttttttcc ggaaattcct cgaagcgtga tgagcatctt 2580
cctcatacag gtgggggagc catttgtact caaaatttga cgatttctca gaatacaggg 2640
aatgttctgt tttataacaa cgtggcctgt tcgggaggag ctgttcgtat agaggatcat 2700
ggtaatgttc ttttagaagc ttttggagga gatattgttt ttaaaggaaa ttcttctttc 2760
agagcacaag gatccgatgc tatctatttt gcaggtaaag aatcgcatat tacagccctg 2820
aatgctacgg aaggacatgc tattgttttc cacgacgcat tagtttttga aaatctaaaa 2880
gaaaggaaat ctgctgaagt attgttaatc aatagtcgag aaaatccagg ttacactgga 2940
tctattcgat ttttagaagc agaaagtaaa gttcctcaat gtattcatgt acaacaagga 3000
agccttgagt tgctaaatgg agctacatta tgtagttatg gttttaaaca agatgctgga 3060
gctaagttgg tattggctgc tggatctaaa ctgaagattt tagattcagg aactcctgta 3120
caagggcatg ctatcagtaa acctgaagca gaaatcgagt catcttctga accagagggt 3180
gcacattctc tttggattgc gaagaatgct caaacaacag ttcctatggt tgatatccat 3240
actatttctg tagatttagc ctccttctct tctagtcaac aggaggggac agtagaagct 3300
cctcaggtta ttgttcctgg aggaagttat gttcgatctg gagagcttaa tttggagtta 3360
gttaacacaa caggtactgg ttatgaaaat catgctttgt tgaagaatga ggctaaagtt 3420
ccattgatgt ctttcgttgc ttctagtgat gaagcttcag ccgaaatcag taacttgtcg 3480
gtttctgatt tacagattca tgtagcaact ccagagattg aagaagacac atacggccat 3540
atgggagatt ggtctgaggc taaaattcaa gatggaactc ttgtcattaa ttggaatcct 3600
actggatatc gattagatcc tcaaaaagca ggggctttag tatttaatgc attatgggaa 3660
gaaggggctg tcttgtctgc tctgaaaaat gcacgctttg ctcataatct cactgctcag 3720
cgtatggaat tcgattattc tacaaatgtg tggggattcg cctttggtgg tttccgaact 3780
ctatctgcag agaatctggt tgctattgat ggatacaaag gagcttatgg tggtgcttct 3840
gctggagtcg atattcaatt gatggaagat tttgttctag gagttagtgg agctgctttc 3900
ctaggtaaaa tggatagtca gaagtttgat gcggaggttt ctcggaaggg agttgttggt 3960
tctgtatata caggattttt agctggatcc tggttcttca aaggacaata tagccttgga 4020
gaaacacaga acgatatgaa aacgcgttat ggagtactag gagagtcgag tgcttcttgg 4080
acatctcgag gagtactggc agatgcttta gttgaatacc gaagtttagt tggtcctgtg 4140
agacctactt tttatgcttt gcatttcaat ccttatgtcg aagtatctta tgcttctatg 4200
aaattccctg gctttacaga acaaggaaga gaagcgcgtt cttttgaaga cgcttccctt 4260
accaatatca ccattccttt agggatgaag tttgaattgg cgttcataaa aggacagttt 4320
tcagaggtga actctttggg aataagttat gcatgggaag cttatcgaaa agtagaagga 4380
ggcgcggtgc agcttttaga agctgggttt gattgggagg gagctccaat ggatcttcct 4440
agacaggagc tgcgtgtcgc tctggaaaat aatacggaat ggagttctta cttcagcaca 4500
gtcttaggat taacagcttt ttgtggagga tttacttcta cagatagtaa actaggatat 4560
gaggcgaata ctggattgcg attgatcttt taa 4593

<210> 173
<211> 5331
<212> DNA
<213> Chlamydia

<400> 173
   gcaatcatga aatttatgtc agctactgct gtatttgctg cagtactctc ctccgttact 60
   gaggcgagct cgatccaaga tcaaataaag aataccgact gcaatgttag caaagtagga 120
   tattcaactt ctcaagcatt tactgatatg atgctagcag acaacacaga gtatcgagct 180
   gctgatagtg tttcattcta tgacttttcg acatcttccg gattacctag aaaacatctt 240
   agtagtagta gtgaagcttc tccaacgaca gaaggagtgt cttcatcttc atctggagaa 300
   aatactgaga attcacaaga ttcagctccc tcttctggag aaactgataa gaaaacagaa 360
   gaagaactag acaatggcgg aatcatttat gctagagaga aactaactat ctcagaatct 420
   caggactctc tctctaatcc aagcatagaa ctccatgaca atagtttttt cttcggagaa 480
   ggtgaagtta tctttgatca cagagttgcc ctcaaaaacg gaggagctat ttatggagag 540
   aaagaggtag tctttgaaaa cataaaatct ctactagtag aagtaaatat ctcggtcgag 600
   aaagggggta gcgtctatgc aaaagaacga gtatctttag aaaatgttac cgaagcaacc 660
   ttctcctcca atggtgggga acaaggtggt ggtggaatct attcagaaca agatatgtta 720
   atcagtgatt gcaacaatgt acatttccaa gggaatgctg caggagcaac agcagtaaaa 780
   caatgtctgg atgaagaaat gatcgtattg ctcacagaat gcgttgatag cttatccgaa 840
   gatacactgg atagcactcc agaaacggaa cagactaagt caaatggaaa tcaagatggt 900
   tcgtctgaaa caaaagatac acaagtatca gaatcaccag aatcaactcc tagccccgac 960
   gatgttttag gtaaaggtgg tggtatctat acagaaaaat ctttgaccat cactggaatt 1020
   acagggacta tagattttgt cagtaacata gctaccgatt ctggagcagg tgtattcact 1080
   aaagaaaact tgtcttgcac caacacgaat agcctacagt ttttgaaaaa ctcggcaggt 1140
   caacatggag gaggagccta cgttactcaa accatgtctg ttactaatac aactagtgaa 1200
   agtataacta ctccccctct cgtaggagaa gtgatttctt ctgaaaatac agctaaaggg 1260
   cacggtggtg gtatctgcac taacaaactt tctttatcta atttaaaaac ggtgactctc 1320
   actaaaaact ctgcaaagga gtctggagga gctattttta cagatctagc gtctatacca 1380
   acaacagata ccccagagtc ttctaccccc tcttcctcct cgcctgcaag cactcccgaa 1440
   gtagttgctt ctgctaaaat aaatcgattc tttgcctcta cggcagaacc ggcagcccct 1500
   tctctaacag aggctgagtc tgatcaaacg gatcaaacag aaacttctga tactaatagc 1560
   gatatagacg tgtcgattga gaacattttg aatgtcgcta tcaatcaaaa cacttctgcg 1620
   aaaaaaggag gggctattta cgggaaaaaa gctaaacttt cccgtattaa caatcttgaa 1680
   ctttcaggga attcatccca ggatgtagga ggaggtctct gtttaactga aagcgtagaa 1740
   tttgatgcaa ttggatcgct cttatcccac tataactctg ctgctaaaga aggtggggtt 1800
   attcattcta aaacggttac tctatctaac ctcaagtcta ccttcacttt tgcagataac 1860
   actgttaaag caatagtaga aagcactcct gaagctccag aagagattcc tccagtagaa 1920
   ggagaagagt ctacagcaac agaaaatccg aattctaata cagaaggaag ttcggctaac 1980
   actaaccttg aaggatctca aggggatact gctgatacag ggactggtgt tgttaacaat 2040
   gagtctcaag acacatcaga tactggaaac gctgaatctg gagaacaact acaagattct 2100
   acacaatcta atgaagaaaa taccttcccc aatagtagta ttgatcaatc taacgaaaac 2160
   acagacgaat catctgatag ccacactgag gaaataactg acgagagtgt ctcatcgtcc 2220
   tctaaaagtg gatcatctac tcctcaagat ggaggagcag cttcttcagg ggctccctca 2280
   ggagatcaat ctatctctgc aaacgcttgt ttagctaaaa gctatgctgc gagtactgat 2340
   agctcccctg tatctaattc ttcaggttca gacgttactg catcttctga taatccagac 2400
   tcttcctcat ctggagatag cgctggagac tctgaaggac cgactgagcc agaagctggt 2460
   tctacaacag aaactcctac tttaatagga ggaggtgcta tctatggaga aactgttaag 2520
   attgagaact tctctggcca aggaatattt tctggaaaca aagctatcga taacaccaca 2580
   gaaggctcct cttccaaatc taacgtcctc ggaggtgcgg tctatgctaa aacattgttt 2640
   aatctcgata gcgggagctc tagacgaact gtcaccttct ccgggaatac tgtctcttct 2700
   caatctacaa caggtcaggt tgctggagga gctatctact ctcctactgt aaccattgct 2760
   actcctgtag tattttctaa aaactctgca acaaacaatg ctaataacgc tacagatact 2820
   cagagaaaag acacctttgg aggagctatc ggagctactt ctgctgtttc tctatcagga 2880
   ggggctcatt tcttagaaaa cgttgctgac ctcggatctg ctattgggtt ggtgccagac 2940
   acacaaaata cagaaacagt gaaattagag tctggctcct actactttga aaaaaataaa 3000
   gctttaaaac gagctactat ttacgcacct gtcgtttcca ttaaagccta tactgcgaca 3060
   tttaaccaaa acagatctct agaagaagga agcgcgattt actttacaaa agaagcatct 3120
   attgagtctt taggctctgt tctcttcaca ggaaacttag taaccccaac gctaagcaca 3180
   actacagaag gcacaccagc cacaacctca ggagatgtaa caaaatatgg tgctgctatc 3240
   tttggacaaa tagcaagctc aaacggatct cagacggata accttcccct gaaactcatt 3300
   gcttcaggag gaaatatttg tttccgaaac aatgaatacc gtcctacttc ttctgatacc 3360
   ggaacctcta ctttctgtag tattgcggga gatgttaaat taaccatgca agctgcaaaa 3420
   gggaaaacga tcagtttctt tgatgcaatc cggacctcta ctaagaaaac aggtacacag 3480
   gcaactgcct acgatactct cgatattaat aaatctgagg attcagaaac tgtaaactct 3540
   gcgtttacag gaacgattct gttctcctct gaattacatg aaaataaatc ctatattcca 3600
   caaaacgtag ttctacacag tggatctctt gtattgaagc caaataccga gcttcatgtc 3660
   atttcttttg agcagaaaga aggctcttct ctcgttatga cacctggatc tgttctttcg 3720
   aaccagactg ttgctgatgg agctttggtc ataaataaca tgaccattga tttatccagc 3780
   gtagagaaaa atggtattgc tgaaggaaat atctttactc ctccagaatt gagaatcata 3840
   gacactacta caagtggaag cggtggaacc ccatctacag atagtgaaag taaccagaat 3900
   agtgatgata ccaaggagca aaataataat gacgcctcga atcaaggaga aagcgcgaat 3960
   ggatcgtctt ctcctgcagt agctgctgca cacacatctc gtacaagaaa ctttgccgct 4020
   gcagctacag ccacacctac gacaacacca acggctacaa ctacaacaag caaccaagta 4080
   atcctaggag gagaaatcaa actcatcgat cctaatggga ccttcttcca gaaccctgca 4140
   ttaagatccg accaacaaat ctccttgtta gtgctcccta cagactcatc aaaaatgcaa 4200
   gctcagaaaa tagtactgac gggtgatatt gctcctcaga aaggatatac aggaacactc 4260
   actctggatc ctgatcaact acaaaatgga acgatctcag cgctctggaa atttgactct 4320
   tatagacaat gggcttatgt acctagagac aatcatttct atgcgaactc gattctggga 4380
   tctcaaatgt caatggtcac agtcaaacaa ggcttgctca acgataaaat gaatctagct 4440
   cgctttgatg aagttagcta taacaacctg tggatatcag gactaggaac gatgctatcg 4500
   caagtaggaa cacctacttc tgaagaattc acttattaca gcagaggagc ttctgttgcc 4560
   ttagatgcta aaccagccca tgatgtgatt gttggagctg catttagtaa gatgatcggg 4620
   aaaacaaaat ccttgaaaag agagaataac tacactcaca aaggatccga atattcttac 4680
   caagcatcgg tatacggagg caaaccattc cactttgtaa tcaataaaaa aacggaaaaa 4740
   tcgctaccgc tattgttaca aggagtcatc tcttacggat atatcaaaca tgatacagtg 4800
   actcactatc caacgatccg tgaacgaaac caaggagaat gggaagactt aggatggctg 4860
   acagctctcc gtgtctcctc tgtcttaaga actcctgcac aaggggatac taaacgtatc 4920
   actgtttacg gagaattgga atactccagt atccgtcaga aacaattcac agaaacagaa 4980
   tacgatcctc gttacttcga caactgcacc tatagaaact tagcaattcc tatggggtta 5040
   gcattcgaag gagagctctc tggtaacgat attttgatgt acaacagatt ctctgtagca 5100
   tacatgccat caatctatcg aaattctcca acatgcaaat accaagtgct ctcttcagga 5160
   gaaggcggag aaattatttg tggagtaccg acaagaaact cagctcgcgg agaatacagc 5220
   acgcagctgt acccgggacc tttgtggact ctgtatggat cctacacgat agaagcagac 5280
   gcacatacac tagctcatat gatgaactgc ggtgctcgta tgacattcta a 5331

<210> 174
<211> 5265
<212> DNA
<213> Chlamydia

<400> 174
gcaatcatga aatggctgtc agctactgcg gtgtttgctg ctgttctccc ctcagtttca 60
gggttttgct tcccagaacc taaagaatta aatttctctc gcgtagaaac ttcttcctct 120
accactttta ctgaaacaat tggagaagct ggggcagaat atatcgtctc tggtaacgca 180
tctttcacaa aatttaccaa cattcctact accgatacaa caactcccac gaactcaaac 240
tcctctagct ctagcggaga aactgcttcc gtttctgagg atagtgactc tacaacaacg 300
actcctgatc ctaaaggtgg cggcgccttt tataacgcgc actccggagt tttgtccttt 360
atgacacgat caggaacaga aggttcctta actctgtctg agataaaaat gactggtgaa 420
ggcggtgcta tcttctctca aggagagctg ctatttacag atctgacaag tctaaccatc 480
caaaataact tatcccagct atccggagga gcgatttttg gaggatctac aatctcccta 540
tcagggatta ctaaagcgac tttctcctgc aactctgcag aagttcctgc tcctgttaag 600

aaacctacag aacctaaagc tcaaacagca agcgaaacgt cgggttctag tagttctagc 660
ggaaatgatt cggtgtcttc ccccagttcc agtagagctg aacccgcagc agctaatctt 720
caaagtcact ttatttgtgc tacagctact cctgctgctc aaaccgatac agaaacatca 780
actccctctc ataagccagg atctggggga gctatctatg ctaaaggcga ccttactatc 840
gcagactctc aagaggtact attctcaata aataaagcta ctaaagatgg aggagcgatc 900
tttgctgaga aagatgtttc tttcgagaat attacatcat taaaagtaca aactaacggt 960
gctgaagaaa agggaggagc tatctatgct aaaggtgacc tctcaattca atcttctaaa 1020
cagagtcttt ttaattctaa ctacagtaaa caaggtgggg gggctctata tgttgaagga 1080
ggtataaact tccaagatct tgaagaaatt cgcattaagt acaataaagc tggaacgttc 1140
gaaacaaaaa aaatcacttt accttcttta aaagctcaag catctgcagg aaatgcagat 1200
gcttgggcct cttcctctcc tcaatctggt tctggagcaa ctacagtctc cgactcagga 1260
gactctagct ctggctcaga ctcggatacc tcagaaacag ttccagtcac agctaaaggc 1320
ggtgggcttt atactgataa gaatctttcg attactaaca tcacaggaat tatcgaaatt 1380
gcaaataaca aagcgacaga tgttggaggt ggtgcttacg taaaaggaac ccttacttgt 1440
gaaaactctc accgtctaca atttttgaaa aactcttccg ataaacaagg tggaggaatc 1500
tacggagaag acaacatcac cctatctaat ttgacaggga agactctatt ccaagagaat 1560
actgccaaag aagagggcgg tggactcttc ataaaaggta cagataaagc tcttacaatg 1620
acaggactgg atagtttctg tttaattaat aacacatcag aaaaacatgg tggtggagcc 1680
tttgttacca aagaaatctc tcagacttac acctctgatg tggaaacaat tccaggaatc 1740
acgcctgtac atggtgaaac agtcattact ggcaataaat ctacaggagg taatggtgga 1800
ggcgtgtgta caaaacgtct tgccttatct aaccttcaaa gcatttctat atccgggaat 1860
tctgcagcag aaaatggtgg tggagcccac acatgcccag atagcttccc aacggcggat 1920
actgcagaac agcccgcagc agcttctgcc gcgacgtcta ctcccaaatc tgccccggtc 1980
tcaactgctc taagcacacc ttcatcttct accgtctctt cattaacctt actagcagcc 2040
tcttcacaag cctctcctgc aacctctaat aaggaaactc aagatcctaa tgctgataca 2100
gacttattga tcgattatgt agttgatacg actatcagca aaaacactgc taagaaaggc 2160
ggtggaatct atgctaaaaa agccaagatg tcccgcatag accaactgaa tatctctgag 2220
aactccgcta cagagatagg tggaggtatc tgctgtaaag aatctttaga actagatgct 2280
ctagtctcct tatctgtaac agagaacctt gttgggaaag aaggtggagg cttacatgct 2340
aaaactgtaa atatttctaa tctgaaatca ggcttctctt tctcgaacaa caaagcaaac 2400
tcctcatcca caggagtcgc aacaacagct tcagcacctg ctgcagctgc tgcttcccta 2460
caagcagccg cagcagccgc accatcatct ccagcaacac caacttattc aggtgtagta 2520
ggaggagcta tctatggaga aaaggttaca ttctctcaat gtagcgggac ttgtcagttc 2580
tctgggaacc aagctatcga taacaatccc tcccaatcat cgttgaacgt acaaggagga 2640
gccatctatg ccaaaacctc tttgtctatt ggatcttccg atgctggaac ctcctatatt 2700
ttctcgggga acagtgtctc cactgggaaa tctcaaacaa cagggcaaat agcgggagga 2760
gcgatctact cccctactgt tacattgaat tgtcctgcga cattctctaa caatacagcc 2820
tctatagcta caccgaagac ttcttctgaa gatggatcct caggaaattc tattaaagat 2880
accattggag gagccattgc agggacagcc attaccctat ctggagtctc tcgattttca 2940
gggaatacgg ctgatttagg agctgcaata ggaactctag ctaatgcaaa tacacccagt 3000
gcaactagcg gatctcaaaa tagcattaca gaaaaaatta ctttagaaaa cggttctttt 3060
atttttgaaa gaaaccaagc taataaacgt ggagcgattt actctcctag cgtttccatt 3120
aaagggaata atattacctt caatcaaaat acatccactc atgatggaag cgctatctac 3180
tttacaaaag atgctacgat tgagtcttta ggatctgttc tttttacagg aaataacgtt 3240
acagctacac aagctagttc tgcaacatct ggacaaaata caaatactgc caactatggg 3300
gcagccatct ttggagatcc aggaaccact caatcgtctc aaacagatgc cattttaacc 3360
cttcttgctt cttctggaaa cattactttt agcaacaaca gtttacagaa taaccaaggt 3420
gatactcccg ctagcaagtt ttgtagtatt gcaggatacg tcaaactctc tctacaagcc 3480
gctaaaggga agactattag ctttttcgat tgtgtgcaca cctctaccaa aaaaacaggt 3540
tcaacacaaa acgtttatga aactttagat attaataaag aagagaacag taatccatat 3600
acaggaacta ttgtgttctc ttctgaatta catgaaaaca aatcttacat cccacagaat 3660
gcaatccttc acaacggaac tttagttctt aaagagaaaa cagaactcca cgtagtctct 3720
tttgagcaga aagaagggtc taaattaatt atggaacccg gagctgtgtt atctaaccaa 3780
aacatagcta acggagctct agctatcaat gggttaacga ttgatctttc cagtatgggg 3840
actcctcaag caggggaaat cttctctcct ccagaattac gtatcgttgc cacgacctct 3900

agtgcatccg gaggaagcgg ggtcagcagt agtataccaa caaatcctaa aaggatttct 3960
gcagcagtgc cttcaggttc tgccgcaact actccaacta tgagcgagaa caaagttttc 4020
ctaacaggag accttacttt aatagatcct aatggaaact tttaccaaaa ccctatgtta 4080
ggaagcgatc tagatgtacc actaattaag cttccgacta acacaagtga cgtccaagtc 4140
tatgatttaa ctttatctgg ggatcttttc cctcagaaag ggtacatggg aacctggaca 4200
ttagattcta atccacaaac agggaaactt caagccagat ggacattcga tacctatcgt 4260
cgctgggtat acatacctag ggataatcat ttttatgcga actctatctt aggctcccaa 4320
aactcaatga ttgttgtgaa gcaagggctt atcaacaaca tgttgaataa tgcccgcttc 4380
gatgatatcg cttacaataa cttctgggtt tcaggagtag gaactttctt agctcaacaa 4440
ggaactcctc tttccgaaga attcagttac tacagccgcg gaacttcagt tgccatcgat 4500
gccaaaccta gacaagattt tatcctagga gctgcattta gtaagatagt ggggaaaacc 4560
aaagccatca aaaaaatgca taattacttc cataagggct ctgagtactc ttaccaagct 4620
tctgtctatg gaggtaaatt cctgtatttc ttgctcaata agcaacatgg ttgggcactt 4680
cctttcctaa tacaaggagt cgtgtcctat ggacatatta aacatgatac aacaacactt 4740
tacccttcta tccatgaaag aaataaagga gattgggaag atttaggatg gttagcggat 4800
cttcgtatct ctatggatct taaagaacct tctaaagatt cttctaaacg gatcactgtc 4860
tatggggaac tcgagtattc cagcattcgc cagaaacagt tcacagaaat cgattacgat 4920
ccaagacact tcgatgattg tgcttacaga aatctgtcgc ttcctgtggg atgcgctgtc 4980
gaaggagcta tcatgaactg taatattctt atgtataata agcttgcatt agcctacatg 5040
ccttctatct acagaaataa tcctgtctgt aaatatcggg tattgtcttc gaatgaagct 5100
ggtcaagtta tctgcggagt gccaactaga acctctgcta gagcagaata cagtactcaa 5160
ctatatcttg gtcccttctg gactctctac ggaaactata ctatcgatgt aggcatgtat 5220
acgctatcgc aaatgactag ctgcggtgct cgcatgatct tctaa 5265

<210> 175
<211> 880
<212> PRT
<213> Chlamydia

<220>
<221> VARIANT
<222> (1)...(880)
<223> Xaa = Any Amino Acid

<400> 175
Ala Ile Met Arg Pro Asp His Met Asn Phe Cys Cys Leu Cys Ala Ala
1               5                   10                  15
Ile Leu Ser Ser Thr Ala Val Leu Phe Gly Gln Asp Pro Leu Gly Glu
            20                  25                  30
Thr Ala Leu Leu Thr Lys Asn Pro Asn His Val Val Cys Thr Phe Phe
        35                  40                  45
Glu Asp Cys Thr Met Glu Ser Leu Phe Pro Ala Leu Cys Ala His Ala
    50                  55                  60
Ser Gln Asp Asp Pro Leu Tyr Val Leu Gly Asn Ser Tyr Cys Trp Phe
65                  70                  75                  80
Val Ser Lys Leu His Ile Thr Asp Pro Lys Glu Ala Leu Phe Lys Glu
                85                  90                  95
Lys Gly Asp Leu Ser Ile Gln Asn Phe Arg Phe Leu Ser Phe Thr Asp
            100                 105                 110
Cys Ser Ser Lys Glu Ser Ser Pro Ser Ile Ile His Gln Lys Asn Gly
        115                 120                 125
Gln Leu Ser Leu Arg Asn Asn Gly Ser Met Ser Phe Cys Arg Asn His
    130                 135                 140

Ala Glu Gly Ser Gly Gly Ala Ile Ser Ala Asp Ala Phe Ser Leu Gln
145                 150                 155                 160
His Asn Tyr Leu Phe Thr Ala Phe Glu Glu Asn Ser Ser Lys Gly Asn
                165                 170                 175
Gly Gly Ala Ile Gln Ala Gln Thr Phe Ser Leu Ser Arg Asn Val Ser
            180                 185                 190
Pro Ile Ser Phe Ala Arg Asn Arg Ala Asp Leu Asn Gly Gly Ala Ile
        195                 200                 205
Cys Cys Ser Asn Leu Ile Cys Ser Gly Asn Val Asn Pro Leu Phe Phe
    210                 215                 220
Thr Gly Asn Ser Ala Thr Asn Gly Gly Ala Ile Cys Cys Ile Ser Asp
225                 230                 235                 240
Leu Asn Thr Ser Glu Lys Gly Ser Leu Ser Leu Ala Cys Asn Gln Glu
                245                 250                 255
Thr Leu Phe Ala Ser Asn Ser Ala Lys Glu Lys Gly Gly Ala Ile Tyr
            260                 265                 270
Ala Lys His Met Val Leu Arg Tyr Asn Gly Pro Val Ser Phe Ile Asn
        275                 280                 285
Asn Ser Ala Lys Ile Gly Gly Ala Ile Ala Ile Gln Ser Gly Gly Ser
    290                 295                 300
Leu Ser Ile Leu Ala Gly Glu Gly Ser Val Leu Phe Gln Asn Asn Ser
305                 310                 315                 320
Gln Arg Thr Ser Asp Gln Gly Leu Val Arg Asn Ala Ile Tyr Leu Xaa
                325                 330                 335
Lys Asp Ala Ile Leu Ser Ser Leu Glu Ala Arg Asn Gly Asp Ile Leu
            340                 345                 350
Phe Phe Asp Pro Ile Val Gln Glu Ser Ser Ser Lys Glu Ser Pro Leu
        355                 360                 365
Pro Ser Ser Leu Gln Ala Ser Val Thr Ser Pro Thr Pro Ala Thr Ala
    370                 375                 380
Ser Pro Leu Val Ile Gln Thr Ser Ala Asn Arg Ser Val Ile Phe Ser
385                 390                 395                 400
Ser Glu Arg Leu Ser Glu Glu Glu Lys Thr Pro Asp Asn Leu Thr Ser
                405                 410                 415
Gln Leu Gln Gln Pro Ile Glu Leu Lys Ser Gly Arg Leu Val Leu Lys
            420                 425                 430
Asp Arg Ala Val Leu Ser Ala Pro Ser Leu Ser Gln Asp Pro Gln Ala
        435                 440                 445
Leu Leu Ile Met Glu Ala Gly Thr Ser Leu Lys Thr Ser Ser Asp Leu
    450                 455                 460
Lys Leu Ala Thr Leu Ser Ile Pro Leu His Ser Leu Asp Thr Glu Lys
465                 470                 475                 480
Ser Val Thr Ile His Ala Pro Asn Leu Ser Ile Gln Lys Ile Phe Leu
                485                 490                 495
Ser Asn Ser Gly Asp Glu Asn Phe Tyr Glu Asn Val Glu Leu Leu Ser
            500                 505                 510
Lys Glu Gln Asn Asn Ile Pro Leu Leu Thr Leu Pro Lys Glu Gln Ser
        515                 520                 525
His Leu His Leu Pro Asp Gly Asn Leu Ser Ser His Phe Gly Tyr Gln
    530                 535                 540
Gly Asp Trp Thr Phe Ser Trp Lys Asp Ser Asp Glu Gly His Ser Leu
545                 550                 555                 560
Ile Ala Asn Trp Thr Pro Lys Asn Tyr Val Pro His Pro Glu Arg Gln
                565                 570                 575
Ser Thr Leu Val Ala Asn Thr Leu Trp Asn Thr Tyr Ser Asp Met Gln

580                 585                 590
Ala Val Gln Ser Met Ile Asn Thr Thr Ala His Gly Gly Ala Tyr Leu
        595                 600                 605
Phe Gly Thr Trp Gly Ser Ala Val Ser Asn Leu Phe Tyr Val His Asp
    610                 615                 620
Ser Ser Gly Lys Pro Ile Asp Asn Trp His His Arg Ser Leu Gly Tyr
625                 630                 635                 640
Leu Phe Gly Ile Ser Thr His Ser Leu Asp Asp His Ser Phe Cys Leu
                645                 650                 655
Ala Ala Gly Gln Leu Leu Gly Lys Ser Ser Asp Ser Phe Ile Thr Ser
            660                 665                 670
Thr Glu Thr Thr Ser Tyr Ile Ala Thr Val Gln Ala Gln Leu Ala Thr
        675                 680                 685
Ser Leu Met Lys Ile Ser Ala Gln Ala Cys Tyr Asn Glu Ser Ile His
    690                 695                 700
Glu Leu Lys Thr Lys Tyr Arg Ser Phe Ser Lys Glu Gly Phe Gly Ser
705                 710                 715                 720
Trp His Ser Val Ala Val Ser Gly Glu Val Cys Ala Ser Ile Pro Ile
                725                 730                 735
Val Ser Asn Gly Ser Gly Leu Phe Ser Ser Phe Ser Ile Phe Ser Lys
            740                 745                 750
Leu Gln Gly Phe Ser Gly Thr Gln Asp Gly Phe Glu Glu Ser Ser Gly
        755                 760                 765
Glu Ile Arg Ser Phe Ser Ala Ser Ser Phe Arg Asn Ile Ser Leu Pro
    770                 775                 780
Ile Gly Ile Thr Phe Glu Lys Lys Ser Gln Lys Thr Arg Thr Tyr Tyr
785                 790                 795                 800
Tyr Phe Leu Gly Ala Tyr Ile Gln Asp Leu Lys Arg Asp Val Glu Ser
                805                 810                 815
Gly Pro Val Val Leu Leu Lys Asn Ala Val Ser Trp Asp Ala Pro Met
            820                 825                 830
Ala Asn Leu Asp Ser Arg Ala Tyr Met Phe Arg Leu Thr Asn Gln Arg
        835                 840                 845
Ala Leu His Arg Leu Gln Thr Leu Leu Asn Val Ser Cys Val Leu Arg
    850                 855                 860
Gly Gln Ser His Ser Tyr Ser Leu Asp Leu Gly Thr Thr Tyr Arg Phe
865                 870                 875                 880

<210> 176
<211> 982
<212> PRT
<213> Chlamydia

<220>
<221> VARIANT
<222> (1)...(982)
<223> Xaa = Any Amino Acid

<400> 176
Met Ile Pro Gln Gly Ile Tyr Asp Gly Glu Thr Leu Thr Val Ser Phe
 1               5                  10                  15
Pro Tyr Thr Val Ile Gly Asp Pro Ser Gly Thr Thr Val Phe Ser Ala
            20                  25                  30
Gly Glu Leu Thr Leu Lys Asn Leu Asp Asn Ser Ile Ala Ala Leu Pro
        35                  40                  45

Leu Ser Cys Phe Gly Asn Leu Leu Gly Ser Phe Thr Val Leu Gly Arg
    50                  55                  60
Gly His Ser Leu Thr Phe Glu Asn Ile Arg Thr Ser Thr Asn Gly Ala
65                  70                  75                  80
Ala Leu Ser Asn Ser Ala Ala Asp Gly Leu Phe Thr Ile Glu Gly Phe
                85                  90                  95
Lys Glu Leu Ser Phe Ser Asn Cys Asn Ser Leu Leu Ala Val Leu Pro
            100                 105                 110
Ala Ala Thr Thr Asn Lys Gly Ser Gln Thr Pro Thr Thr Thr Ser Thr
        115                 120                 125
Pro Ser Asn Gly Thr Ile Tyr Ser Lys Thr Asp Leu Leu Leu Leu Asn
    130                 135                 140
Asn Glu Lys Phe Ser Phe Tyr Ser Asn Leu Val Ser Gly Asp Gly Gly
145                 150                 155                 160
Ala Ile Asp Ala Lys Ser Leu Thr Val Gln Gly Ile Ser Lys Leu Cys
                165                 170                 175
Val Phe Gln Glu Asn Thr Ala Gln Ala Asp Gly Gly Ala Cys Gln Val
            180                 185                 190
Val Thr Ser Phe Ser Ala Met Ala Asn Glu Ala Pro Ile Ala Phe Val
        195                 200                 205
Ala Asn Val Ala Gly Val Arg Gly Gly Gly Ile Ala Ala Val Gln Asp
    210                 215                 220
Gly Gln Gln Gly Val Ser Ser Ser Thr Ser Thr Glu Asp Pro Val Val
225                 230                 235                 240
Ser Phe Ser Arg Asn Thr Ala Val Glu Phe Asp Gly Asn Val Ala Arg
                245                 250                 255
Val Gly Gly Gly Ile Tyr Ser Tyr Gly Asn Val Ala Phe Leu Asn Asn
            260                 265                 270
Gly Lys Thr Leu Phe Leu Asn Asn Val Ala Ser Pro Val Tyr Ile Ala
        275                 280                 285
Ala Lys Gln Pro Thr Ser Gly Gln Ala Ser Asn Thr Ser Asn Asn Tyr
    290                 295                 300
Gly Asp Gly Gly Ala Ile Phe Cys Lys Asn Gly Ala Gln Ala Gly Ser
305                 310                 315                 320
Asn Asn Ser Gly Ser Val Ser Phe Asp Gly Glu Gly Val Val Phe Phe
                325                 330                 335
Ser Ser Asn Val Ala Ala Gly Lys Gly Gly Ala Ile Tyr Ala Lys Lys
            340                 345                 350
Leu Ser Val Ala Asn Cys Gly Pro Val Gln Phe Leu Arg Asn Ile Ala
        355                 360                 365
Asn Asp Gly Gly Ala Ile Tyr Leu Gly Glu Ser Gly Glu Leu Ser Leu
    370                 375                 380
Ser Ala Asp Tyr Gly Asp Ile Ile Phe Asp Gly Asn Leu Lys Arg Thr
385                 390                 395                 400
Ala Lys Glu Asn Ala Ala Asp Val Asn Gly Val Thr Val Ser Ser Gln
                405                 410                 415
Ala Ile Ser Met Gly Ser Gly Gly Lys Ile Thr Thr Leu Arg Ala Lys
            420                 425                 430
Ala Gly His Gln Ile Leu Phe Asn Asp Pro Ile Glu Met Ala Asn Gly
        435                 440                 445
Asn Asn Gln Pro Ala Gln Ser Ser Lys Leu Leu Lys Ile Asn Asp Gly
    450                 455                 460
Glu Gly Tyr Thr Gly Asp Ile Val Phe Ala Asn Gly Ser Ser Thr Leu
465                 470                 475                 480
Tyr Gln Asn Val Thr Ile Glu Gln Gly Arg Ile Val Leu Arg Glu Lys

                485                 490                 495
Ala Lys Leu Ser Val Asn Ser Leu Ser Gln Thr Gly Gly Ser Leu Tyr
            500                 505                 510
Met Glu Ala Gly Ser Thr Leu Asp Phe Val Thr Pro Gln Pro Pro Gln
        515                 520                 525
Gln Pro Pro Ala Ala Asn Gln Leu Ile Thr Leu Ser Asn Leu His Leu
    530                 535                 540
Ser Leu Ser Ser Leu Leu Ala Asn Asn Ala Val Thr Asn Pro Pro Thr
545                 550                 555                 560
Asn Pro Pro Ala Gln Asp Ser His Pro Ala Val Ile Gly Ser Thr Thr
                565                 570                 575
Ala Gly Ser Val Thr Ile Ser Gly Pro Ile Phe Phe Glu Asp Leu Asp
            580                 585                 590
Asp Thr Ala Tyr Asp Arg Tyr Asp Trp Leu Gly Ser Asn Gln Lys Ile
        595                 600                 605
Asn Val Leu Lys Leu Gln Leu Gly Thr Lys Pro Pro Ala Asn Ala Pro
    610                 615                 620
Ser Asp Leu Thr Leu Gly Asn Glu Met Pro Lys Tyr Gly Tyr Gln Gly
625                 630                 635                 640
Ser Trp Lys Leu Ala Trp Asp Pro Asn Thr Ala Asn Asn Gly Pro Tyr
                645                 650                 655
Thr Leu Lys Ala Thr Trp Thr Lys Thr Gly Tyr Asn Pro Gly Pro Glu
            660                 665                 670
Arg Val Ala Ser Leu Val Pro Asn Ser Leu Trp Gly Ser Ile Leu Asp
        675                 680                 685
Ile Arg Ser Ala His Ser Ala Ile Gln Ala Ser Val Asp Gly Arg Ser
    690                 695                 700
Tyr Cys Arg Gly Leu Trp Val Ser Gly Val Ser Asn Phe Phe Tyr His
705                 710                 715                 720
Asp Arg Asp Ala Leu Gly Gln Gly Tyr Arg Tyr Ile Ser Gly Gly Tyr
                725                 730                 735
Ser Leu Gly Ala Asn Ser Tyr Phe Gly Ser Ser Met Phe Gly Leu Ala
            740                 745                 750
Phe Thr Glu Val Phe Gly Arg Ser Lys Asp Tyr Val Val Cys Arg Ser
        755                 760                 765
Asn His His Ala Cys Ile Gly Ser Val Tyr Leu Ser Thr Gln Gln Ala
    770                 775                 780
Leu Cys Gly Ser Tyr Leu Phe Gly Asp Ala Phe Ile Arg Ala Ser Tyr
785                 790                 795                 800
Gly Phe Gly Asn Gln His Met Lys Thr Ser Tyr Thr Phe Ala Glu Glu
                805                 810                 815
Ser Asp Val Arg Trp Asp Asn Asn Cys Leu Ala Gly Glu Ile Gly Ala
            820                 825                 830
Gly Leu Pro Ile Val Ile Thr Pro Ser Lys Leu Tyr Leu Asn Glu Leu
        835                 840                 845
Arg Pro Phe Val Gln Ala Glu Phe Ser Tyr Ala Asp His Glu Ser Phe
    850                 855                 860
Thr Glu Glu Gly Asp Gln Ala Arg Ala Phe Lys Ser Gly His Leu Leu
865                 870                 875                 880
Asn Leu Ser Val Pro Val Gly Val Lys Phe Asp Arg Cys Ser Ser Thr
                885                 890                 895
His Pro Asn Lys Tyr Ser Phe Met Ala Ala Tyr Ile Cys Asp Ala Tyr
            900                 905                 910
Arg Thr Ile Ser Gly Thr Glu Thr Thr Leu Leu Ser His Gln Glu Thr
        915                 920                 925

Trp Thr Thr Asp Ala Phe His Leu Ala Arg His Gly Val Val Val Arg
    930                 935                 940
Gly Ser Met Tyr Ala Ser Leu Thr Ser Asn Ile Glu Val Tyr Gly His
945                 950                 955                 960
Gly Arg Tyr Glu Tyr Arg Asp Ala Ser Arg Gly Tyr Gly Leu Ser Ala
                965                 970                 975
Gly Ser Lys Val Xaa Phe
            980

<210> 177
<211> 964
<212> PRT
<213> Chlamydia

<400> 177
Met Lys Lys Ala Phe Phe Phe Phe Leu Ile Gly Asn Ser Leu Ser Gly
1               5                   10                  15
Leu Ala Arg Glu Val Pro Ser Arg Ile Phe Leu Met Pro Asn Ser Val
            20                  25                  30
Pro Asp Pro Thr Lys Glu Ser Leu Ser Asn Lys Ile Ser Leu Thr Gly
        35                  40                  45
Asp Thr His Asn Leu Thr Asn Cys Tyr Leu Asp Asn Leu Arg Tyr Ile
    50                  55                  60
Leu Ala Ile Leu Gln Lys Thr Pro Asn Glu Gly Ala Ala Val Thr Ile
65                  70                  75                  80
Thr Asp Tyr Leu Ser Phe Phe Asp Thr Gln Lys Glu Gly Ile Tyr Phe
                85                  90                  95
Ala Lys Asn Leu Thr Pro Glu Ser Gly Gly Ala Ile Gly Tyr Ala Ser
            100                 105                 110
Pro Asn Ser Pro Thr Val Glu Ile Arg Asp Thr Ile Gly Pro Val Ile
        115                 120                 125
Phe Glu Asn Asn Thr Cys Cys Arg Leu Phe Thr Trp Arg Asn Pro Tyr
    130                 135                 140
Ala Ala Asp Lys Ile Arg Glu Gly Gly Ala Ile His Ala Gln Asn Leu
145                 150                 155                 160
Tyr Ile Asn His Asn His Asp Val Val Gly Phe Met Lys Asn Phe Ser
                165                 170                 175
Tyr Val Gln Gly Gly Ala Ile Ser Thr Ala Asn Thr Phe Val Val Ser
            180                 185                 190
Glu Asn Gln Ser Cys Phe Leu Phe Met Asp Asn Ile Cys Ile Gln Thr
        195                 200                 205
Asn Thr Ala Gly Lys Gly Gly Ala Ile Tyr Ala Gly Thr Ser Asn Ser
    210                 215                 220
Phe Glu Ser Asn Asn Cys Asp Leu Phe Phe Ile Asn Asn Ala Cys Cys
225                 230                 235                 240
Ala Gly Gly Ala Ile Phe Ser Pro Ile Cys Ser Leu Thr Gly Asn Arg
                245                 250                 255
Gly Asn Ile Val Phe Tyr Asn Asn Arg Cys Phe Lys Asn Val Glu Thr
            260                 265                 270
Ala Ser Ser Glu Ala Ser Asp Gly Gly Ala Ile Lys Val Thr Thr Arg
        275                 280                 285
Leu Asp Val Thr Gly Asn Arg Gly Arg Ile Phe Phe Ser Asp Asn Ile
    290                 295                 300
Thr Lys Asn Tyr Gly Gly Ala Ile Tyr Ala Pro Val Val Thr Leu Val
305                 310                 315                 320

Asp Asn Gly Pro Thr Tyr Phe Ile Asn Asn Ile Ala Asn Asn Lys Gly
                325                 330                 335
Gly Ala Ile Tyr Ile Asp Gly Thr Ser Asn Ser Lys Ile Ser Ala Asp
            340                 345                 350
Arg His Ala Ile Ile Phe Asn Glu Asn Ile Val Thr Asn Val Thr Asn
        355                 360                 365
Ala Asn Gly Thr Ser Thr Ser Ala Asn Pro Pro Arg Arg Asn Ala Ile
    370                 375                 380
Thr Val Ala Ser Ser Ser Gly Glu Ile Leu Leu Gly Ala Gly Ser Ser
385                 390                 395                 400
Gln Asn Leu Ile Phe Tyr Asp Pro Ile Glu Val Ser Asn Ala Gly Val
                405                 410                 415
Ser Val Ser Phe Asn Lys Glu Ala Asp Gln Thr Gly Ser Val Val Phe
            420                 425                 430
Ser Gly Ala Thr Val Asn Ser Ala Asp Phe His Gln Arg Asn Leu Gln
        435                 440                 445
Thr Lys Thr Pro Ala Pro Leu Thr Leu Ser Asn Gly Phe Leu Cys Ile
    450                 455                 460
Glu Asp His Ala Gln Leu Thr Val Asn Arg Phe Thr Gln Thr Gly Gly
465                 470                 475                 480
Val Val Ser Leu Gly Asn Gly Ala Val Leu Ser Cys Tyr Lys Asn Gly
                485                 490                 495
Thr Gly Asp Ser Ala Ser Asn Ala Ser Ile Thr Leu Lys His Ile Gly
            500                 505                 510
Leu Asn Leu Ser Ser Ile Leu Lys Ser Gly Ala Glu Ile Pro Leu Leu
        515                 520                 525
Trp Val Glu Pro Thr Asn Asn Ser Asn Asn Tyr Thr Ala Asp Thr Ala
    530                 535                 540
Ala Thr Phe Ser Leu Ser Asp Val Lys Leu Ser Leu Ile Asp Asp Tyr
545                 550                 555                 560
Gly Asn Ser Pro Tyr Glu Ser Thr Asp Leu Thr His Ala Leu Ser Ser
                565                 570                 575
Gln Pro Met Leu Ser Ile Ser Glu Ala Ser Asp Asn Gln Leu Gln Ser
            580                 585                 590
Glu Asn Ile Asp Phe Ser Gly Leu Asn Val Pro His Tyr Gly Trp Gln
        595                 600                 605
Gly Leu Trp Thr Trp Gly Trp Ala Lys Thr Gln Asp Pro Glu Pro Ala
    610                 615                 620
Ser Ser Ala Thr Ile Thr Asp Pro Gln Lys Ala Asn Arg Phe His Arg
625                 630                 635                 640
Thr Leu Leu Leu Thr Trp Leu Pro Ala Gly Tyr Val Pro Ser Pro Lys
                645                 650                 655
His Arg Ser Pro Leu Ile Ala Asn Thr Leu Trp Gly Asn Met Leu Leu
            660                 665                 670
Ala Thr Glu Ser Leu Lys Asn Ser Ala Glu Leu Thr Pro Ser Gly His
        675                 680                 685
Pro Phe Trp Gly Ile Thr Gly Gly Gly Leu Gly Met Met Val Tyr Gln
    690                 695                 700
Asp Pro Arg Glu Asn His Pro Gly Phe His Met Arg Ser Ser Gly Tyr
705                 710                 715                 720
Ser Ala Gly Met Ile Ala Gly Gln Thr His Thr Phe Ser Leu Lys Phe
                725                 730                 735
Ser Gln Thr Tyr Thr Lys Leu Asn Glu Arg Tyr Ala Lys Asn Asn Val
            740                 745                 750
Ser Ser Lys Asn Tyr Ser Cys Gln Gly Glu Met Leu Phe Ser Leu Gln

        755                 760                 765
Glu Gly Phe Leu Leu Thr Lys Leu Val Gly Leu Tyr Ser Tyr Gly Asp
    770                 775                 780
His Asn Cys His His Phe Tyr Thr Gln Gly Glu Asn Leu Thr Ser Gln
785                 790                 795                 800
Gly Thr Phe Arg Ser Gln Thr Met Gly Gly Ala Val Phe Phe Asp Leu
                805                 810                 815
Pro Met Lys Pro Phe Gly Ser Thr His Ile Leu Thr Ala Pro Phe Leu
            820                 825                 830
Gly Ala Leu Gly Ile Tyr Ser Ser Leu Ser His Phe Thr Glu Val Gly
        835                 840                 845
Ala Tyr Pro Arg Ser Phe Ser Thr Lys Thr Pro Leu Ile Asn Val Leu
    850                 855                 860
Val Pro Ile Gly Val Lys Gly Ser Phe Met Asn Ala Thr His Arg Pro
865                 870                 875                 880
Gln Ala Trp Thr Val Glu Leu Ala Tyr Gln Pro Val Leu Tyr Arg Gln
                885                 890                 895
Glu Pro Gly Ile Ala Thr Gln Leu Leu Ala Ser Lys Gly Ile Trp Phe
            900                 905                 910
Gly Ser Gly Ser Pro Ser Ser Arg His Ala Met Ser Tyr Lys Ile Ser
        915                 920                 925
Gln Gln Thr Gln Pro Leu Ser Trp Leu Thr Leu His Phe Gln Tyr His
    930                 935                 940
Gly Phe Tyr Ser Ser Ser Thr Phe Cys Asn Tyr Leu Asn Gly Glu Ile
945                 950                 955                 960
Ala Leu Arg Phe

<210> 178
<211> 1530
<212> PRT
<213> Chlamydia

<400> 178
Met Ser Ser Glu Lys Asp Ile Lys Ser Thr Cys Ser Lys Phe Ser Leu
1               5                   10                  15
Ser Val Val Ala Ala Ile Leu Ala Ser Val Ser Gly Leu Ala Ser Cys
            20                  25                  30
Val Asp Leu His Ala Gly Gly Gln Ser Val Asn Glu Leu Val Tyr Val
        35                  40                  45
Gly Pro Gln Ala Val Leu Leu Leu Asp Gln Ile Arg Asp Leu Phe Val
    50                  55                  60
Gly Ser Lys Asp Ser Gln Ala Glu Gly Gln Tyr Arg Leu Ile Val Gly
65                  70                  75                  80
Asp Pro Ser Ser Phe Gln Glu Lys Asp Ala Asp Thr Leu Pro Gly Lys
                85                  90                  95
Val Glu Gln Ser Thr Leu Phe Ser Val Thr Asn Pro Val Val Phe Gln
            100                 105                 110
Gly Val Asp Gln Gln Asp Gln Val Ser Ser Gln Gly Leu Ile Cys Ser
        115                 120                 125
Phe Thr Ser Ser Asn Leu Asp Ser Pro Arg Asp Gly Glu Ser Phe Leu
    130                 135                 140
Gly Ile Ala Phe Val Gly Asp Ser Ser Lys Ala Gly Ile Thr Leu Thr
145                 150                 155                 160
Asp Val Lys Ala Ser Leu Ser Gly Ala Ala Leu Tyr Ser Thr Glu Asp

                165                 170                 175
Leu Ile Phe Glu Lys Ile Lys Gly Gly Leu Glu Phe Ala Ser Cys Ser
            180                 185                 190
Ser Leu Glu Gln Gly Gly Ala Cys Ala Ala Gln Ser Ile Leu Ile His
        195                 200                 205
Asp Cys Gln Gly Leu Gln Val Lys His Cys Thr Thr Ala Val Asn Ala
    210                 215                 220
Glu Gly Ser Ser Ala Asn Asp His Leu Gly Phe Gly Gly Gly Ala Phe
225                 230                 235                 240
Phe Val Thr Gly Ser Leu Ser Gly Glu Lys Ser Leu Tyr Met Pro Ala
                245                 250                 255
Gly Asp Met Val Val Ala Asn Cys Asp Gly Ala Ile Ser Phe Glu Gly
            260                 265                 270
Asn Ser Ala Asn Phe Ala Asn Gly Gly Ala Ile Ala Ala Ser Gly Lys
        275                 280                 285
Val Leu Phe Val Ala Asn Asp Lys Lys Thr Ser Phe Ile Glu Asn Arg
    290                 295                 300
Ala Leu Ser Gly Gly Ala Ile Ala Ala Ser Ser Asp Ile Ala Phe Gln
305                 310                 315                 320
Asn Cys Ala Glu Leu Val Phe Lys Gly Asn Cys Ala Ile Gly Thr Glu
                325                 330                 335
Asp Lys Gly Ser Leu Gly Gly Gly Ala Ile Ser Ser Leu Gly Thr Val
            340                 345                 350
Leu Leu Gln Gly Asn His Gly Ile Thr Cys Asp Lys Asn Glu Ser Ala
        355                 360                 365
Ser Gln Gly Gly Ala Ile Phe Gly Lys Asn Cys Gln Ile Ser Asp Asn
    370                 375                 380
Glu Gly Pro Val Val Phe Arg Asp Ser Thr Ala Cys Leu Gly Gly Gly
385                 390                 395                 400
Ala Ile Ala Ala Gln Glu Ile Val Ser Ile Gln Asn Asn Gln Ala Gly
                405                 410                 415
Ile Ser Phe Glu Gly Gly Lys Ala Ser Phe Gly Gly Gly Ile Ala Cys
            420                 425                 430
Gly Ser Phe Ser Ser Ala Gly Gly Ala Ser Val Leu Gly Thr Ile Asp
        435                 440                 445
Ile Ser Lys Asn Leu Gly Ala Ile Ser Phe Ser Arg Thr Leu Cys Thr
    450                 455                 460
Thr Ser Asp Leu Gly Gln Met Glu Tyr Gln Gly Gly Gly Ala Leu Phe
465                 470                 475                 480
Gly Glu Asn Ile Ser Leu Ser Glu Asn Ala Gly Val Leu Thr Phe Lys
                485                 490                 495
Asp Asn Ile Val Lys Thr Phe Ala Ser Asn Gly Lys Ile Leu Gly Gly
            500                 505                 510
Gly Ala Ile Leu Ala Thr Gly Lys Val Glu Ile Thr Asn Asn Ser Gly
        515                 520                 525
Gly Ile Ser Phe Thr Gly Asn Ala Arg Ala Pro Gln Ala Leu Pro Thr
    530                 535                 540
Gln Glu Glu Phe Pro Leu Phe Ser Lys Lys Glu Gly Arg Pro Leu Ser
545                 550                 555                 560
Ser Gly Tyr Ser Gly Gly Gly Ala Ile Leu Gly Arg Glu Val Ala Ile
                565                 570                 575
Leu His Asn Ala Ala Val Val Phe Glu Gln Asn Arg Leu Gln Cys Ser
            580                 585                 590
Glu Glu Glu Ala Thr Leu Leu Gly Cys Cys Gly Gly Gly Ala Val His
        595                 600                 605

Gly Met Asp Ser Thr Ser Ile Val Gly Asn Ser Ser Val Arg Phe Gly
    610                 615                 620
Asn Asn Tyr Ala Met Gly Gln Gly Val Ser Gly Gly Ala Leu Leu Ser
625                 630                 635                 640
Lys Thr Val Gln Leu Ala Gly Asn Gly Ser Val Asp Phe Ser Arg Asn
                645                 650                 655
Ile Ala Ser Leu Gly Gly Gly Ala Leu Gln Ala Ser Glu Gly Asn Cys
                660                 665                 670
Glu Leu Val Asp Asn Gly Tyr Val Leu Phe Arg Asp Asn Arg Gly Arg
    675                 680                 685
Val Tyr Gly Gly Ala Ile Ser Cys Leu Arg Gly Asp Val Val Ile Ser
    690                 695                 700
Gly Asn Lys Gly Arg Val Glu Phe Lys Asp Asn Ile Ala Thr Arg Leu
705                 710                 715                 720
Tyr Val Gl[?] Glu Thr Val Glu Lys Val Glu Glu Val Glu Pro Ala Pro
                725                 730                 735
Glu Gln Lys Asp Asn Asn Glu Leu Ser Phe Leu Gly Ser Val Glu Gln
                740                 745                 750
Ser Phe Ile Thr Ala Ala Asn Gln Ala Leu Phe Ala Ser Glu Asp Gly
    755                 760                 765
Asp Leu Ser Pro Glu Ser Ser Ile Ser Ser Glu Glu Leu Ala Lys Arg
    770                 775                 780
Arg Glu Cys Ala Gly Gly Ala Ile Phe Ala Lys Arg Val Arg Ile Val
785                 790                 795                 800
Asp Asn Gln Glu Ala Val Val Phe Ser Asn Asn Phe Ser Asp Ile Tyr
                805                 810                 815
Gly Gly Ala Ile Phe Thr Gly Ser Leu Arg Glu Glu Asp Lys Leu Asp
                820                 825                 830
Gly Gln Ile Pro Glu Val Leu Ile Ser Gly Asn Ala Gly Asp Val Val
    835                 840                 845
Phe Ser Gly Asn Ser Ser Lys Arg Asp Glu His Leu Pro His Thr Gly
    850                 855                 860
Gly Gly Ala Ile Cys Thr Gln Asn Leu Thr Ile Ser Gln Asn Thr Gly
865                 870                 875                 880
Asn Val Leu Phe Tyr Asn Asn Val Ala Cys Ser Gly Gly Ala Val Arg
                885                 890                 895
Ile Glu Asp His Gly Asn Val Leu Leu Glu Ala Phe Gly Gly Asp Ile
                900                 905                 910
Val Phe Lys Gly Asn Ser Ser Phe Arg Ala Gln Gly Ser Asp Ala Ile
    915                 920                 925
Tyr Phe Ala Gly Lys Glu Ser His Ile Thr Ala Leu Asn Ala Thr Glu
    930                 935                 940
Gly His Ala Ile Val Phe His Asp Ala Leu Val Phe Glu Asn Leu Lys
945                 950                 955                 960
Glu Arg Lys Ser Ala Glu Val Leu Leu Ile Asn Ser Arg Glu Asn Pro
                965                 970                 975
Gly Tyr Thr Gly Ser Ile Arg Phe Leu Glu Ala Glu Ser Lys Val Pro
                980                 985                 990
Gln Cys Ile His Val Gln Gln Gly Ser Leu Glu Leu Leu Asn Gly Ala
    995                 1000                1005
Thr Leu Cys Ser Tyr Gly Phe Lys Gln Asp Ala Gly Ala Lys Leu Val
    1010                1015                1020
Leu Ala Ala Gly Ser Lys Leu Lys Ile Leu Asp Ser Gly Thr Pro Val
1025                1030                1035                1040
Gln Gly His Ala Ile Ser Lys Pro Glu Ala Glu Ile Glu Ser Ser Ser

1045                1050                1055
Glu Pro Glu Gly Ala His Ser Leu Trp Ile Ala Lys Asn Ala Gln Thr
            1060                1065                1070
Thr Val Pro Met Val Asp Ile His Thr Ile Ser Val Asp Leu Ala Ser
        1075                1080                1085
Phe Ser Ser Ser Gln Gln Glu Gly Thr Val Glu Ala Pro Gln Val Ile
    1090                1095                1100
Val Pro Gly Gly Ser Tyr Val Arg Ser Gly Glu Leu Asn Leu Glu Leu
1105                1110                1115                1120
Val Asn Thr Thr Gly Thr Gly Tyr Glu Asn His Ala Leu Leu Lys Asn
                1125                1130                1135
Glu Ala Lys Val Pro Leu Met Ser Phe Val Ala Ser Ser Asp Glu Ala
            1140                1145                1150
Ser Ala Glu Ile Ser Asn Leu Ser Val Ser Asp Leu Gln Ile His Val
        1155                1160                1165
Ala Thr Pro Glu Ile Glu Glu Asp Thr Tyr Gly His Met Gly Asp Trp
    1170                1175                1180
Ser Glu Ala Lys Ile Gln Asp Gly Thr Leu Val Ile Asn Trp Asn Pro
1185                1190                1195                1200
Thr Gly Tyr Arg Leu Asp Pro Gln Lys Ala Gly Ala Leu Val Phe Asn
                1205                1210                1215
Ala Leu Trp Glu Glu Gly Ala Val Leu Ser Ala Leu Lys Asn Ala Arg
            1220                1225                1230
Phe Ala His Asn Leu Thr Ala Gln Arg Met Glu Phe Asp Tyr Ser Thr
        1235                1240                1245
Asn Val Trp Gly Phe Ala Phe Gly Gly Phe Arg Thr Leu Ser Ala Glu
    1250                1255                1260
Asn Leu Val Ala Ile Asp Gly Tyr Lys Gly Ala Tyr Gly Gly Ala Ser
1265                1270                1275                1280
Ala Gly Val Asp Ile Gln Leu Met Glu Asp Phe Val Leu Gly Val Ser
                1285                1290                1295
Gly Ala Ala Phe Leu Gly Lys Met Asp Ser Gln Lys Phe Asp Ala Glu
            1300                1305                1310
Val Ser Arg Lys Gly Val Val Gly Ser Val Tyr Thr Gly Phe Leu Ala
        1315                1320                1325
Gly Ser Trp Phe Phe Lys Gly Gln Tyr Ser Leu Gly Glu Thr Gln Asn
    1330                1335                1340
Asp Met Lys Thr Arg Tyr Gly Val Leu Gly Glu Ser Ser Ala Ser Trp
1345                1350                1355                1360
Thr Ser Arg Gly Val Leu Ala Asp Ala Leu Val Glu Tyr Arg Ser Leu
                1365                1370                1375
Val Gly Pro Val Arg Pro Thr Phe Tyr Ala Leu His Phe Asn Pro Tyr
            1380                1385                1390
Val Glu Val Ser Tyr Ala Ser Met Lys Phe Pro Gly Phe Thr Glu Gln
        1395                1400                1405
Gly Arg Glu Ala Arg Ser Phe Glu Asp Ala Ser Leu Thr Asn Ile Thr
    1410                1415                1420
Ile Pro Leu Gly Met Lys Phe Glu Leu Ala Phe Ile Lys Gly Gln Phe
1425                1430                1435                1440
Ser Glu Val Asn Ser Leu Gly Ile Ser Tyr Ala Trp Glu Ala Tyr Arg
                1445                1450                1455
Lys Val Glu Gly Gly Ala Val Gln Leu Leu Glu Ala Gly Phe Asp Trp
            1460                1465                1470
Glu Gly Ala Pro Met Asp Leu Pro Arg Gln Glu Leu Arg Val Ala Leu
        1475                1480                1485

Glu Asn Asn Thr Glu Trp Ser Ser Tyr Phe Ser Thr Val Leu Gly Leu
    1490                1495                1500
Thr Ala Phe Cys Gly Gly Phe Thr Ser Thr Asp Ser Lys Leu Gly Tyr
1505                1510                1515                1520
Glu Ala Asn Thr Gly Leu Arg Leu Ile Phe
                1525                1530

<210> 179
<211> 1776
<212> PRT
<213> Chlamydia

<400> 179
Ala Ile Met Lys Phe Met Ser Ala Thr Ala Val Phe Ala Ala Val Leu
 1               5                  10                  15
Ser Ser Val Thr Glu Ala Ser Ser Ile Gln Asp Gln Ile Lys Asn Thr
            20                  25                  30
Asp Cys Asn Val Ser Lys Val Gly Tyr Ser Thr Ser Gln Ala Phe Thr
        35                  40                  45
Asp Met Met Leu Ala Asp Asn Thr Glu Tyr Arg Ala Ala Asp Ser Val
    50                  55                  60
Ser Phe Tyr Asp Phe Ser Thr Ser Ser Gly Leu Pro Arg Lys His Leu
65                  70                  75                  80
Ser Ser Ser Ser Glu Ala Ser Pro Thr Thr Glu Gly Val Ser Ser Ser
                85                  90                  95
Ser Ser Gly Glu Asn Thr Glu Asn Ser Gln Asp Ser Ala Pro Ser Ser
            100                 105                 110
Gly Glu Thr Asp Lys Lys Thr Glu Glu Glu Leu Asp Asn Gly Gly Ile
        115                 120                 125
Ile Tyr Ala Arg Glu Lys Leu Thr Ile Ser Glu Ser Gln Asp Ser Leu
    130                 135                 140
Ser Asn Pro Ser Ile Glu Leu His Asp Asn Ser Phe Phe Phe Gly Glu
145                 150                 155                 160
Gly Glu Val Ile Phe Asp His Arg Val Ala Leu Lys Asn Gly Gly Ala
                165                 170                 175
Ile Tyr Gly Glu Lys Glu Val Val Phe Glu Asn Ile Lys Ser Leu Leu
            180                 185                 190
Val Glu Val Asn Ile Ser Val Glu Lys Gly Gly Ser Val Tyr Ala Lys
        195                 200                 205
Glu Arg Val Ser Leu Glu Asn Val Thr Glu Ala Thr Phe Ser Ser Asn
    210                 215                 220
Gly Gly Glu Gln Gly Gly Gly Gly Ile Tyr Ser Glu Gln Asp Met Leu
225                 230                 235                 240
Ile Ser Asp Cys Asn Asn Val His Phe Gln Gly Asn Ala Ala Gly Ala
                245                 250                 255
Thr Ala Val Lys Gln Cys Leu Asp Glu Glu Met Ile Val Leu Leu Thr
            260                 265                 270
Glu Cys Val Asp Ser Leu Ser Glu Asp Thr Leu Asp Ser Thr Pro Glu
        275                 280                 285
Thr Glu Gln Thr Lys Ser Asn Gly Asn Gln Asp Gly Ser Ser Glu Thr
    290                 295                 300
Lys Asp Thr Gln Val Ser Glu Ser Pro Glu Ser Thr Pro Ser Pro Asp
305                 310                 315                 320
Asp Val Leu Gly Lys Gly Gly Gly Ile Tyr Thr Glu Lys Ser Leu Thr
                325                 330                 335

Ile Thr Gly Ile Thr Gly Thr Ile Asp Phe Val Ser Asn Ile Ala Thr
            340                 345                 350
Asp Ser Gly Ala Gly Val Phe Thr Lys Glu Asn Leu Ser Cys Thr Asn
        355                 360                 365
Thr Asn Ser Leu Gln Phe Leu Lys Asn Ser Ala Gly Gln His Gly Gly
    370                 375                 380
Gly Ala Tyr Val Thr Gln Thr Met Ser Val Thr Asn Thr Thr Ser Glu
385                 390                 395                 400
Ser Ile Thr Thr Pro Pro Leu Val Gly Glu Val Ile Phe Ser Glu Asn
                405                 410                 415
Thr Ala Lys Gly His Gly Gly Gly Ile Cys Thr Asn Lys Leu Ser Leu
            420                 425                 430
Ser Asn Leu Lys Thr Val Thr Leu Thr Lys Asn Ser Ala Lys Glu Ser
        435                 440                 445
Gly Gly Ala Ile Phe Thr Asp Leu Ala Ser Ile Pro Thr Thr Asp Thr
    450                 455                 460
Pro Glu Ser Ser Thr Pro Ser Ser Ser Ser Pro Ala Ser Thr Pro Glu
465                 470                 475                 480
Val Val Ala Ser Ala Lys Ile Asn Arg Phe Phe Ala Ser Thr Ala Glu
                485                 490                 495
Pro Ala Ala Pro Ser Leu Thr Glu Ala Glu Ser Asp Gln Thr Asp Gln
            500                 505                 510
Thr Glu Thr Ser Asp Thr Asn Ser Asp Ile Asp Val Ser Ile Glu Asn
        515                 520                 525
Ile Leu Asn Val Ala Ile Asn Gln Asn Thr Ser Ala Lys Lys Gly Gly
    530                 535                 540
Ala Ile Tyr Gly Lys Lys Ala Lys Leu Ser Arg Ile Asn Asn Leu Glu
545                 550                 555                 560
Leu Ser Gly Asn Ser Ser Gln Asp Val Gly Gly Gly Leu Cys Leu Thr
                565                 570                 575
Glu Ser Val Glu Phe Asp Ala Ile Gly Ser Leu Leu Ser His Tyr Asn
            580                 585                 590
Ser Ala Ala Lys Glu Gly Gly Val Ile His Ser Lys Thr Val Thr Leu
        595                 600                 605
Ser Asn Leu Lys Ser Thr Phe Thr Phe Ala Asp Asn Thr Val Lys Ala
    610                 615                 620
Ile Val Glu Ser Thr Pro Glu Ala Pro Glu Glu Ile Pro Pro Val Glu
625                 630                 635                 640
Gly Glu Glu Ser Thr Ala Thr Glu Asn Pro Asn Ser Asn Thr Glu Gly
                645                 650                 655
Ser Ser Ala Asn Thr Asn Leu Glu Gly Ser Gln Gly Asp Thr Ala Asp
            660                 665                 670
Thr Gly Thr Gly Val Val Asn Asn Glu Ser Gln Asp Thr Ser Asp Thr
        675                 680                 685
Gly Asn Ala Glu Ser Gly Glu Gln Leu Gln Asp Ser Thr Gln Ser Asn
    690                 695                 700
Glu Glu Asn Thr Leu Pro Asn Ser Ser Ile Asp Gln Ser Asn Glu Asn
705                 710                 715                 720
Thr Asp Glu Ser Ser Asp Ser His Thr Glu Glu Ile Thr Asp Glu Ser
                725                 730                 735
Val Ser Ser Ser Ser Lys Ser Gly Ser Ser Thr Pro Gln Asp Gly Gly
            740                 745                 750
Ala Ala Ser Ser Gly Ala Pro Ser Gly Asp Gln Ser Ile Ser Ala Asn
        755                 760                 765
Ala Cys Leu Ala Lys Ser Tyr Ala Ala Ser Thr Asp Ser Ser Pro Val

770                 775                 780
Ser Asn Ser Ser Gly Ser Asp Val Thr Ala Ser Ser Asp Asn Pro Asp
785                 790                 795                 800
Ser Ser Ser Ser Gly Asp Ser Ala Gly Asp Ser Glu Gly Pro Thr Glu
                805                 810                 815
Pro Glu Ala Gly Ser Thr Thr Glu Thr Pro Thr Leu Ile Gly Gly Gly
            820                 825                 830
Ala Ile Tyr Gly Glu Thr Val Lys Ile Glu Asn Phe Ser Gly Gln Gly
        835                 840                 845
Ile Phe Ser Gly Asn Lys Ala Ile Asp Asn Thr Thr Glu Gly Ser Ser
    850                 855                 860
Ser Lys Ser Asn Val Leu Gly Gly Ala Val Tyr Ala Lys Thr Leu Phe
865                 870                 875                 880
Asn Leu Asp Ser Gly Ser Ser Arg Arg Thr Val Thr Phe Ser Gly Asn
                885                 890                 895
Thr Val Ser Ser Gln Ser Thr Thr Gly Gln Val Ala Gly Gly Ala Ile
            900                 905                 910
Tyr Ser Pro Thr Val Thr Ile Ala Thr Pro Val Val Phe Ser Lys Asn
        915                 920                 925
Ser Ala Thr Asn Asn Ala Asn Asn Ala Thr Asp Thr Gln Arg Lys Asp
    930                 935                 940
Thr Phe Gly Gly Ala Ile Gly Ala Thr Ser Ala Val Ser Leu Ser Gly
945                 950                 955                 960
Gly Ala His Phe Leu Glu Asn Val Ala Asp Leu Gly Ser Ala Ile Gly
                965                 970                 975
Leu Val Pro Asp Thr Gln Asn Thr Glu Thr Val Lys Leu Glu Ser Gly
            980                 985                 990
Ser Tyr Tyr Phe Glu Lys Asn Lys Ala Leu Lys Arg Ala Thr Ile Tyr
        995                 1000                1005
Ala Pro Val Val Ser Ile Lys Ala Tyr Thr Ala Thr Phe Asn Gln Asn
    1010                1015                1020
Arg Ser Leu Glu Glu Gly Ser Ala Ile Tyr Phe Thr Lys Glu Ala Ser
1025                1030                1035                1040
Ile Glu Ser Leu Gly Ser Val Leu Phe Thr Gly Asn Leu Val Thr Pro
                1045                1050                1055
Thr Leu Ser Thr Thr Thr Glu Gly Thr Pro Ala Thr Thr Ser Gly Asp
            1060                1065                1070
Val Thr Lys Tyr Gly Ala Ala Ile Phe Gly Gln Ile Ala Ser Ser Asn
        1075                1080                1085
Gly Ser Gln Thr Asp Asn Leu Pro Leu Lys Leu Ile Ala Ser Gly Gly
    1090                1095                1100
Asn Ile Cys Phe Arg Asn Asn Glu Tyr Arg Pro Thr Ser Ser Asp Thr
1105                1110                1115                1120
Gly Thr Ser Thr Phe Cys Ser Ile Ala Gly Asp Val Lys Leu Thr Met
                1125                1130                1135
Gln Ala Ala Lys Gly Lys Thr Ile Ser Phe Phe Asp Ala Ile Arg Thr
            1140                1145                1150
Ser Thr Lys Lys Thr Gly Thr Gln Ala Thr Ala Tyr Asp Thr Leu Asp
        1155                1160                1165
Ile Asn Lys Ser Glu Asp Ser Glu Thr Val Asn Ser Ala Phe Thr Gly
    1170                1175                1180
Thr Ile Leu Phe Ser Ser Glu Leu His Glu Asn Lys Ser Tyr Ile Pro
1185                1190                1195                1200
Gln Asn Val Val Leu His Ser Gly Ser Leu Val Leu Lys Pro Asn Thr
                1205                1210                1215

Glu Leu His Val Ile Ser Phe Glu Gln Lys Glu Gly Ser Ser Leu Val
            1220                1225                1230
Met Thr Pro Gly Ser Val Leu Ser Asn Gln Thr Val Ala Asp Gly Ala
        1235                1240                1245
Leu Val Ile Asn Asn Met Thr Ile Asp Leu Ser Ser Val Glu Lys Asn
    1250                1255                1260
Gly Ile Ala Glu Gly Asn Ile Phe Thr Pro Pro Glu Leu Arg Ile Ile
1265                1270                1275                1280
Asp Thr Thr Thr Ser Gly Ser Gly Gly Thr Pro Ser Thr Asp Ser Glu
                1285                1290                1295
Ser Asn Gln Asn Ser Asp Asp Thr Lys Glu Gln Asn Asn Asn Asp Ala
            1300                1305                1310
Ser Asn Gln Gly Glu Ser Ala Asn Gly Ser Ser Ser Pro Ala Val Ala
        1315                1320                1325
Ala Ala His Thr Ser Arg Thr Arg Asn Phe Ala Ala Ala Ala Thr Ala
    1330                1335                1340
Thr Pro Thr Thr Thr Pro Thr Ala Thr Thr Thr Thr Ser Asn Gln Val
1345                1350                1355                1360
Ile Leu Gly Gly Glu Ile Lys Leu Ile Asp Pro Asn Gly Thr Phe Phe
                1365                1370                1375
Gln Asn Pro Ala Leu Arg Ser Asp Gln Gln Ile Ser Leu Leu Val Leu
            1380                1385                1390
Pro Thr Asp Ser Ser Lys Met Gln Ala Gln Lys Ile Val Leu Thr Gly
        1395                1400                1405
Asp Ile Ala Pro Gln Lys Gly Tyr Thr Gly Thr Leu Thr Leu Asp Pro
    1410                1415                1420
Asp Gln Leu Gln Asn Gly Thr Ile Ser Ala Leu Trp Lys Phe Asp Ser
1425                1430                1435                1440
Tyr Arg Gln Trp Ala Tyr Val Pro Arg Asp Asn His Phe Tyr Ala Asn
                1445                1450                1455
Ser Ile Leu Gly Ser Gln Met Ser Met Val Thr Val Lys Gln Gly Leu
            1460                1465                1470
Leu Asn Asp Lys Met Asn Leu Ala Arg Phe Asp Glu Val Ser Tyr Asn
        1475                1480                1485
Asn Leu Trp Ile Ser Gly Leu Gly Thr Met Leu Ser Gln Val Gly Thr
    1490                1495                1500
Pro Thr Ser Glu Glu Phe Thr Tyr Tyr Ser Arg Gly Ala Ser Val Ala
1505                1510                1515                1520
Leu Asp Ala Lys Pro Ala His Asp Val Ile Val Gly Ala Ala Phe Ser
                1525                1530                1535
Lys Met Ile Gly Lys Thr Lys Ser Leu Lys Arg Glu Asn Asn Tyr Thr
            1540                1545                1550
His Lys Gly Ser Glu Tyr Ser Tyr Gln Ala Ser Val Tyr Gly Gly Lys
        1555                1560                1565
Pro Phe His Phe Val Ile Asn Lys Lys Thr Glu Lys Ser Leu Pro Leu
    1570                1575                1580
Leu Leu Gln Gly Val Ile Ser Tyr Gly Tyr Ile Lys His Asp Thr Val
1585                1590                1595                1600
Thr His Tyr Pro Thr Ile Arg Glu Arg Asn Gln Gly Glu Trp Glu Asp
                1605                1610                1615
Leu Gly Trp Leu Thr Ala Leu Arg Val Ser Ser Val Leu Arg Thr Pro
            1620                1625                1630
Ala Gln Gly Asp Thr Lys Arg Ile Thr Val Tyr Gly Glu Leu Glu Tyr
        1635                1640                1645
Ser Ser Ile Arg Gln Lys Gln Phe Thr Glu Thr Glu Tyr Asp Pro Arg

1650                1655                1660
Tyr Phe Asp Asn Cys Thr Tyr Arg Asn Leu Ala Ile Pro Met Gly Leu
1665                1670                1675                1680
Ala Phe Glu Gly Glu Leu Ser Gly Asn Asp Ile Leu Met Tyr Asn Arg
                1685                1690                1695
Phe Ser Val Ala Tyr Met Pro Ser Ile Tyr Arg Asn Ser Pro Thr Cys
            1700                1705                1710
Lys Tyr Gln Val Leu Ser Ser Gly Glu Gly Gly Glu Ile Ile Cys Gly
        1715                1720                1725
Val Pro Thr Arg Asn Ser Ala Arg Gly Glu Tyr Ser Thr Gln Leu Tyr
    1730                1735                1740
Pro Gly Pro Leu Trp Thr Leu Tyr Gly Ser Tyr Thr Ile Glu Ala Asp
1745                1750                1755                1760
Ala His Thr Leu Ala His Met Met Asn Cys Gly Ala Arg Met Thr Phe
                1765                1770                1775

<210> 180
<211> 1752
<212> PRT
<213> Chlamydia

<400> 180
   Met Lys Trp Leu Ser Ala Thr Ala Val Phe Ala Ala Val Leu Pro Ser
    1               5                  10                  15
   Val Ser Gly Phe Cys Phe Pro Glu Pro Lys Glu Leu Asn Phe Ser Arg
               20                  25                  30
   Val Glu Thr Ser Ser Ser Thr Thr Phe Thr Glu Thr Ile Gly Glu Ala
           35                  40                  45
   Gly Ala Glu Tyr Ile Val Ser Gly Asn Ala Ser Phe Thr Lys Phe Thr
       50                  55                  60
   Asn Ile Pro Thr Thr Asp Thr Thr Thr Pro Thr Asn Ser Asn Ser Ser
   65                  70                  75                  80
   Ser Ser Ser Gly Glu Thr Ala Ser Val Ser Glu Asp Ser Asp Ser Thr
                   85                  90                  95
   Thr Thr Thr Pro Asp Pro Lys Gly Gly Gly Ala Phe Tyr Asn Ala His
               100                 105                 110
   Ser Gly Val Leu Ser Phe Met Thr Arg Ser Gly Thr Glu Gly Ser Leu
           115                 120                 125
   Thr Leu Ser Glu Ile Lys Met Thr Gly Glu Gly Gly Ala Ile Phe Ser
       130                 135                 140
   Gln Gly Glu Leu Leu Phe Thr Asp Leu Thr Ser Leu Thr Ile Gln Asn
   145                 150                 155                 160
   Asn Leu Ser Gln Leu Ser Gly Gly Ala Ile Phe Gly Gly Ser Thr Ile
                   165                 170                 175
   Ser Leu Ser Gly Ile Thr Lys Ala Thr Phe Ser Cys Asn Ser Ala Glu
               180                 185                 190
   Val Pro Ala Pro Val Lys Lys Pro Thr Glu Pro Lys Ala Gln Thr Ala
           195                 200                 205
   Ser Glu Thr Ser Gly Ser Ser Ser Ser Ser Gly Asn Asp Ser Val Ser
       210                 215                 220
   Ser Pro Ser Ser Ser Arg Ala Glu Pro Ala Ala Ala Asn Leu Gln Ser
   225                 230                 235                 240
   His Phe Ile Cys Ala Thr Ala Thr Pro Ala Ala Gln Thr Asp Thr Glu
                   245                 250                 255
   Thr Ser Thr Pro Ser His Lys Pro Gly Ser Gly Gly Ala Ile Tyr Ala
   260 265 270
   Lys Gly Asp Leu Thr Ile Ala Asp Ser Gln Glu Val Leu Phe Ser Ile
   275 280 285
   Asn Lys Ala Thr Lys Asp Gly Gly Ala Ile Phe Ala Glu Lys Asp Val
   290 295 300
   Ser Phe Glu Asn Ile Thr Ser Leu Lys Val Gln Thr Asn Gly Ala Glu
   305 310 315 320
   Glu Lys Gly Gly Ala Ile Tyr Ala Lys Gly Asp Leu Ser Ile Gln Ser
   325 330 335
   Ser Lys Gln Ser Leu Phe Asn Ser Asn Tyr Ser Lys Gln Gly Gly Gly
   340 345 350
   Ala Leu Tyr Val Glu Gly Gly Ile Asn Phe Gln Asp Leu Glu Glu Ile
   355 360 365
   Arg Ile Lys Tyr Asn Lys Ala Gly Thr Phe Glu Thr Lys Lys Ile Thr
   370 375 380
   Leu Pro Ser Leu Lys Ala Gln Ala Ser Ala Gly Asn Ala Asp Ala Trp
   385 390 395 400
   Ala Ser Ser Ser Pro Gln Ser Gly Ser Gly Ala Thr Thr Val Ser Asp
   405 410 415
   Ser Gly Asp Ser Ser Ser Gly Ser Asp Ser Asp Thr Ser Glu Thr Val
   420 425 430
   Pro Val Thr Ala Lys Gly Gly Gly Leu Tyr Thr Asp Lys Asn Leu Ser
   435 440 445
   Ile Thr Asn Ile Thr Gly Ile Ile Glu Ile Ala Asn Asn Lys Ala Thr
   450 455 460
   Asp Val Gly Gly Gly Ala Tyr Val Lys Gly Thr Leu Thr Cys Glu Asn
   465 470 475 480
   Ser His Arg Leu Gln Phe Leu Lys Asn Ser Ser Asp Lys Gln Gly Gly
   485 490 495
   Gly Ile Tyr Gly Glu Asp Asn Ile Thr Leu Ser Asn Leu Thr Gly Lys
   500 505 510
   Thr Leu Phe Gln Glu Asn Thr Ala Lys Glu Glu Gly Gly Gly Leu Phe
   515 520 525
   Ile Lys Gly Thr Asp Lys Ala Leu Thr Met Thr Gly Leu Asp Ser Phe
   530 535 540
   Cys Leu Ile Asn Asn Thr Ser Glu Lys His Gly Gly Gly Ala Phe Val
   545 550 555 560
   Thr Lys Glu Ile Ser Gln Thr Tyr Thr Ser Asp Val Glu Thr Ile Pro
   565 570 575
   Gly Ile Thr Pro Val His Gly Glu Thr Val Ile Thr Gly Asn Lys Ser
   580 585 590
   Thr Gly Gly Asn Gly Gly Gly Val Cys Thr Lys Arg Leu Ala Leu Ser
   595 600 605
   Asn Leu Gln Ser Ile Ser Ile Ser Gly Asn Ser Ala Ala Glu Asn Gly
   610 615 620
   Gly Gly Ala His Thr Cys Pro Asp Ser Phe Pro Thr Ala Asp Thr Ala
   625 630 635 640
   Glu Gln Pro Ala Ala Ala Ser Ala Ala Thr Ser Thr Pro Lys Ser Ala
   645 650 655
   Pro Val Ser Thr Ala Leu Ser Thr Pro Ser Ser Ser Thr Val Ser Ser
   660 665 670
   Leu Thr Leu Leu Ala Ala Ser Ser Gln Ala Ser Pro Ala Thr Ser Asn
   675 680 685
   Lys Glu Thr Gln Asp Pro Asn Ala Asp Thr Asp Leu Leu Ile Asp Tyr
   690 695 700
   Val Val Asp Thr Thr Ile Ser Lys Asn Thr Ala Lys Lys Gly Gly Gly
   705 710 715 720
   Ile Tyr Ala Lys Lys Ala Lys Met Ser Arg Ile Asp Gln Leu Asn Ile
   725 730 735
   Ser Glu Asn Ser Ala Thr Glu Ile Gly Gly Gly Ile Cys Cys Lys Glu
   740 745 750
   Ser Leu Glu Leu Asp Ala Leu Val Ser Leu Ser Val Thr Glu Asn Leu
   755 760 765
   Val Gly Lys Glu Gly Gly Gly Leu His Ala Lys Thr Val Asn Ile Ser
   770 775 780
   Asn Leu Lys Ser Gly Phe Ser Phe Ser Asn Asn Lys Ala Asn Ser Ser
   785 790 795 800
   Ser Thr Gly Val Ala Thr Thr Ala Ser Ala Pro Ala Ala Ala Ala Ala
   805 810 815
   Ser Leu Gln Ala Ala Ala Ala Ala Ala Pro Ser Ser Pro Ala Thr Pro
   820 825 830
   Thr Tyr Ser Gly Val Val Gly Gly Ala Ile Tyr Gly Glu Lys Val Thr
   835 840 845
   Phe Ser Gln Cys Ser Gly Thr Cys Gln Phe Ser Gly Asn Gln Ala Ile
   850 855 860
   Asp Asn Asn Pro Ser Gln Ser Ser Leu Asn Val Gln Gly Gly Ala Ile
   865 870 875 880
   Tyr Ala Lys Thr Ser Leu Ser Ile Gly Ser Ser Asp Ala Gly Thr Ser
   885 890 895
   Tyr Ile Phe Ser Gly Asn Ser Val Ser Thr Gly Lys Ser Gln Thr Thr
   900 905 910
   Gly Gln Ile Ala Gly Gly Ala Ile Tyr Ser Pro Thr Val Thr Leu Asn
   915 920 925
   Cys Pro Ala Thr Phe Ser Asn Asn Thr Ala Ser Ile Ala Thr Pro Lys
   930 935 940
   Thr Ser Ser Glu Asp Gly Ser Ser Gly Asn Ser Ile Lys Asp Thr Ile
   945 950 955 960
   Gly Gly Ala Ile Ala Gly Thr Ala Ile Thr Leu Ser Gly Val Ser Arg
   965 970 975
   Phe Ser Gly Asn Thr Ala Asp Leu Gly Ala Ala Ile Gly Thr Leu Ala
   980 985 990
   Asn Ala Asn Thr Pro Ser Ala Thr Ser Gly Ser Gln Asn Ser Ile Thr
   995 1000 1005
   Glu Lys Ile Thr Leu Glu Asn Gly Ser Phe Ile Phe Glu Arg Asn Gln
   1010 1015 1020
   Ala Asn Lys Arg Gly Ala Ile Tyr Ser Pro Ser Val Ser Ile Lys Gly
   1025 1030 1035 1040
   Asn Asn Ile Thr Phe Asn Gln Asn Thr Ser Thr His Asp Gly Ser Ala
   1045 1050 1055
   Ile Tyr Phe Thr Lys Asp Ala Thr Ile Glu Ser Leu Gly Ser Val Leu
   1060 1065 1070
   Phe Thr Gly Asn Asn Val Thr Ala Thr Gln Ala Ser Ser Ala Thr Ser
   1075 1080 1085
   Gly Gln Asn Thr Asn Thr Ala Asn Tyr Gly Ala Ala Ile Phe Gly Asp
   1090 1095 1100
   Pro Gly Thr Thr Gln Ser Ser Gln Thr Asp Ala Ile Leu Thr Leu Leu
   1105 1110 1115 1120
   Ala Ser Ser Gly Asn Ile Thr Phe Ser Asn Asn Ser Leu Gln Asn Asn
   1125 1130 1135
   Gln Gly Asp Thr Pro Ala Ser Lys Phe Cys Ser Ile Ala Gly Tyr Val
   1140                1145                1150
   Lys Leu Ser Leu Gln Ala Ala Lys Gly Lys Thr Ile Ser Phe Phe Asp
       1155                1160                1165
   Cys Val His Thr Ser Thr Lys Lys Thr Gly Ser Thr Gln Asn Val Tyr
       1170                1175                1180
   Glu Thr Leu Asp Ile Asn Lys Glu Glu Asn Ser Asn Pro Tyr Thr Gly
   1185                1190                1195                1200
   Thr Ile Val Phe Ser Ser Glu Leu His Glu Asn Lys Ser Tyr Ile Pro
                   1205                1210                1215
   Gln Asn Ala Ile Leu His Asn Gly Thr Leu Val Leu Lys Glu Lys Thr
               1220                1225                1230
   Glu Leu His Val Val Ser Phe Glu Gln Lys Glu Gly Ser Lys Leu Ile
           1235                1240                1245
   Met Glu Pro Gly Ala Val Leu Ser Asn Gln Asn Ile Ala Asn Gly Ala
       1250                1255                1260
   Leu Ala Ile Asn Gly Leu Thr Ile Asp Leu Ser Ser Met Gly Thr Pro
   1265                1270                1275                1280
   Gln Ala Gly Glu Ile Phe Ser Pro Pro Glu Leu Arg Ile Val Ala Thr
                   1285                1290                1295
   Thr Ser Ser Ala Ser Gly Gly Ser Gly Val Ser Ser Ser Ile Pro Thr
               1300                1305                1310
   Asn Pro Lys Arg Ile Ser Ala Ala Val Pro Ser Gly Ser Ala Ala Thr
           1315                1320                1325
   Thr Pro Thr Met Ser Glu Asn Lys Val Phe Leu Thr Gly Asp Leu Thr
       1330                1335                1340
   Leu Ile Asp Pro Asn Gly Asn Phe Tyr Gln Asn Pro Met Leu Gly Ser
   1345                1350                1355                1360
   Asp Leu Asp Val Pro Leu Ile Lys Leu Pro Thr Asn Thr Ser Asp Val
                   1365                1370                1375
   Gln Val Tyr Asp Leu Thr Leu Ser Gly Asp Leu Phe Pro Gln Lys Gly
               1380                1385                1390
   Tyr Met Gly Thr Trp Thr Leu Asp Ser Asn Pro Gln Thr Gly Lys Leu
           1395                1400                1405
   Gln Ala Arg Trp Thr Phe Asp Thr Tyr Arg Arg Trp Val Tyr Ile Pro
       1410                1415                1420
   Arg Asp Asn His Phe Tyr Ala Asn Ser Ile Leu Gly Ser Gln Asn Ser
   1425                1430                1435                1440
   Met Ile Val Val Lys Gln Gly Leu Ile Asn Asn Met Leu Asn Asn Ala
                   1445                1450                1455
   Arg Phe Asp Asp Ile Ala Tyr Asn Asn Phe Trp Val Ser Gly Val Gly
               1460                1465                1470
   Thr Phe Leu Ala Gln Gln Gly Thr Pro Leu Ser Glu Glu Phe Ser Tyr
           1475                1480                1485
   Tyr Ser Arg Gly Thr Ser Val Ala Ile Asp Ala Lys Pro Arg Gln Asp
       1490                1495                1500
   Phe Ile Leu Gly Ala Ala Phe Ser Lys Ile Val Gly Lys Thr Lys Ala
   1505                1510                1515                1520
   Ile Lys Lys Met His Asn Tyr Phe His Lys Gly Ser Glu Tyr Ser Tyr
                   1525                1530                1535
   Gln Ala Ser Val Tyr Gly Gly Lys Phe Leu Tyr Phe Leu Leu Asn Lys
               1540                1545                1550
   Gln His Gly Trp Ala Leu Pro Phe Leu Ile Gln Gly Val Val Ser Tyr
           1555                1560                1565
   Gly His Ile Lys His Asp Thr Thr Thr Leu Tyr Pro Ser Ile His Glu
       1570                1575                1580
   Arg Asn Lys Gly Asp Trp Glu Asp Leu Gly Trp Leu Ala Asp Leu Arg
   1585                1590                1595                1600
   Ile Ser Met Asp Leu Lys Glu Pro Ser Lys Asp Ser Ser Lys Arg Ile
                   1605                1610                1615
   Thr Val Tyr Gly Glu Leu Glu Tyr Ser Ser Ile Arg Gln Lys Gln Phe
               1620                1625                1630
   Thr Glu Ile Asp Tyr Asp Pro Arg His Phe Asp Asp Cys Ala Tyr Arg
           1635                1640                1645
   Asn Leu Ser Leu Pro Val Gly Cys Ala Val Glu Gly Ala Ile Met Asn
       1650                1655                1660
   Cys Asn Ile Leu Met Tyr Asn Lys Leu Ala Leu Ala Tyr Met Pro Ser
   1665                1670                1675                1680
   Ile Tyr Arg Asn Asn Pro Val Cys Lys Tyr Arg Val Leu Ser Ser Asn
                   1685                1690                1695
   Glu Ala Gly Gln Val Ile Cys Gly Val Pro Thr Arg Thr Ser Ala Arg
               1700                1705                1710
   Ala Glu Tyr Ser Thr Gln Leu Tyr Leu Gly Pro Phe Trp Thr Leu Tyr
           1715                1720                1725
   Gly Asn Tyr Thr Ile Asp Val Gly Met Tyr Thr Leu Ser Gln Met Thr
       1730                1735                1740
   Ser Cys Gly Ala Arg Met Ile Phe
   1745                1750

<210> 181
<211> 2601
<212> DNA
<213> Chlamydia

<400> 181
atggctagcc atcaccatca ccatcacctc tttggccagg atcccttagg tgaaaccgcc      60
ctcctcacta aaaatcctaa tcatgtcgtc tgtacatttt ttgaggactg taccatggag     120
agcctctttc ctgctctttg tgctcatgca tcacaagacg atcctttgta tgtacttgga     180
aattcctact gttggttcgt atctaaactc catatcacgg accccaaaga ggctcttttt     240
aaagaaaaag gagatctttc cattcaaaac tttcgcttcc tttccttcac agattgctct     300
tccaaggaaa gctctccttc tattattcat caaaagaatg gtcagttatc cttgcgcaat     360
aatggtagca tgagtttctg tcgaaatcat gctgaaggct ctggaggagc catctctgcg     420
gatgcctttt ctctacagca caactatctt ttcacagctt ttgaagagaa ttcttctaaa     480
ggaaatggcg gagccattca ggctcaaacc ttctctttat ctagaaatgt gtcgcctatt     540
tctttcgccc gtaatcgtgc ggatttaaat ggcggcgcta tttgctgtag taatcttatt     600
tgttcaggga atgtaaaccc tctctttttc actggaaact ccgccacraa tggaggcsct     660
atttgttgta tcagcgatct aaacacctca gaaaaaggct ctctctctct tgcttgtaac     720
caaraaacgc tatttgcaag caattctgct aaagaaaaag gcggggctat ttatgccaag     780
cacatggtat tgcgttataa cggtcctgtt tccttcatta acaacagcgc taaaataggt     840
ggagctatcg ccatccagtc cggagggagt ctctctatcc ttgcaggtga aggatctgtt     900
ctgttccaga ataactccca acgcacctcc gaccaaggtc tagtaagaaa cgccatctac     960
ttagagaaag atgcgattct ttcttcctta gaagctcgca acggagatat tcttttcttt    1020
gatcctattg tacaagaaag tagcagcaaa gaatcgcctc ttccctcctc tttgcaagcc    1080
agcgtgactt ctcccacccc agccaccgca tctcctttag ttattcagac aagtgcaaac    1140
cgttcagtga ttttctcgag cgaacgtctt tctgaagaag aaaaaactcc tgataacctc    1200
acttcccaac tacagcagcc tatcgaactg aaatccggac gcttagtttt aaaagatcgc    1260
gctgtccttt ccgsgccttc tctctctcag gatcctcaag ctctcctcat tatggaagcg    1320
ggaacttctt taaaaacttc ctytgatttg aagttagsta cgstaagtat tccccttcat    1380
tccttagata ctgaaaaaag cgtaactatc cacgccccta atctttctat ccaaaagatc    1440
ttcctctcta actctggaga tgagaatttt tatgaaaatg tagagcttct cagtaaagag    1500
caaaacaata ttcctctcct tactctccct aaagagcaat ctcatttaca tcttcctgat    1560

gggaacctct cttctcactt tggatatcaa ggagattgga ctttttcttg gaaagattct 1620
gatgaagggc attctctgat tgctaattgg acgcctaaaa actatgtgcc tcatccagaa 1680
cgtcaatcta cactcgttgc gaacactctt tggaacacct attccgatat gcaagctgtg 1740
cagtcgatga ttaatacaac agcgcacgga ggagcctatc tatttggaac gtggggatct 1800
gctgtttcta atttattcta tgttcacgac agctctggga aacctatcga taattggcat 1860
catagaagcc ttggctacct attcggtatc agtactcaca gtttagatga ccattctttc 1920
tgcttggctg caggacaatt actcgggaaa tcgtccgatt cctttattac gtctacagaa 1980
acgacctcct atatagctac tgtacaagcg caactcgcta cctctctaat gaaaatctct 2040
gcacaggcat gctacaatga aagtatccat gagctaaaaa caaaatatcg ctccttctct 2100
aaagaaggat tcggatcctg gcatagcgtt gcagtatccg gagaagtgtg cgcatcgatt 2160
cctattgtat ccaatggttc cggactgttc agctccttct ctattttctc taaactgcaa 2220
ggattttcag gaacacagga cggttttgag gagagttcgg gagagattcg gtccttttct 2280
gccagctctt tcagaaatat ttcacttcct ataggaataa catttgaaaa aaaatcccaa 2340
aaaacacgaa cctactatta ctttctagga gcctacatcc aagacctgaa acgtgatgtg 2400
gaatcgggac ctgtagtgtt actcaaaaat gccgtctcct gggatgctcc tatggcgaac 2460
ttggattcac gagcctacat gttccggctt acgaatcaaa gagctctaca cagacttcag 2520
acgctgttaa atgtgtcttg tgtgctgcgt gggcaaagcc atagttactc cctggatctg 2580
gggaccactt acaggttcta g 2601

<210> 182
<211> 3021
<212> DNA
<213> Chlamydia

<400> 182
atggctagca tgactggtgg acagcaaatg ggtcgggatt caagcttggt accgcatcac 60
catcaccatc acatgattcc tcaaggaatt tacgatgggg agacgttaac tgtatcattt 120
ccctatactg ttataggaga tccgagtggg actactgttt tttctgcagg agagttaaca 180
ttaaaaaatc ttgacaattc tattgcagct ttgcctttaa gttgttttgg gaacttatta 240
gggagtttta ctgttttagg gagaggacac tcgttgactt tcgagaacat acggacttct 300
acaaatgggg cagctctaag taatagcgct gctgatggac tgtttactat tgagggtttt 360
aaagaattat ccttttccaa ttgcaattca ttacttgccg tactgcctgc tgcaacgact 420
aataagggta gccagactcc gacgacaaca tctacaccgt ctaatggtac tatttattct 480
aaaacagatc ttttgttact caataatgag aagttctcat tctatagtaa tttagtctct 540
ggagatgggg gagctataga tgctaagagc ttaacggttc aaggaattag caagctttgt 600
gtcttccaag aaaatactgc tcaagctgat gggggagctt gtcaagtagt caccagtttc 660
tctgctatgg ctaacgaggc tcctattgcc tttgtagcga atgttgcagg agtaagaggg 720
ggagggattg ctgctgttca ggatgggcag cagggagtgt catcatctac ttcaacagaa 780
gatccagtag taagtttttc cagaaatact gcggtagagt ttgatgggaa cgtagcccga 840
gtaggaggag ggatttactc ctacgggaac gttgctttcc tgaataatgg aaaaaccttg 900
tttctcaaca atgttgcttc tcctgtttac attgctgcta agcaaccaac aagtggacag 960
gcttctaata cgagtaataa ttacggagat ggaggagcta tcttctgtaa gaatggtgcg 1020
caagcaggat ccaataactc tggatcagtt tcctttgatg gagagggagt agttttcttt 1080
agtagcaatg tagctgctgg gaaaggggga gctatttatg ccaaaaagct ctcggttgct 1140
aactgtggcc ctgtacaatt tttaaggaat atcgctaatg atggtggagc gatttattta 1200
ggagaatctg gagagctcag tttatctgct gattatggag atattatttt cgatgggaat 1260
cttaaaagaa cagccaaaga gaatgctgcc gatgttaatg gcgtaactgt gtcctcacaa 1320
gccatttcga tgggatcggg agggaaaata acgacattaa gagctaaagc agggcatcag 1380
attctcttta atgatcccat cgagatggca aacggaaata accagccagc gcagtcttcc 1440
aaacttctaa aaattaacga tggtgaagga tacacagggg atattgtttt tgctaatgga 1500
agcagtactt tgtaccaaaa tgttacgata gagcaaggaa ggattgttct tcgtgaaaag 1560
gcaaaattat cagtgaattc tctaagtcag acaggtggga gtctgtatat ggaagctggg 1620
agtacattgg attttgtaac tccacaacca ccacaacagc ctcctgccgc taatcagttg 1680
atcacgcttt ccaatctgca tttgtctctt tcttctttgt tagcaaacaa tgcagttacg 1740
aatcctccta ccaatcctcc agcgcaagat tctcatcctg cagtcattgg tagcacaact 1800

gctggttctg ttacaattag tgggcctatc ttttttgagg atttggatga tacagcttat 1860
gataggtatg attggctagg ttctaatcaa aaaatcaatg tcctgaaatt acagttaggg 1920
actaagcccc cagctaatgc cccatcagat ttgactctag ggaatgagat gcctaagtat 1980
ggctatcaag gaagctggaa gcttgcgtgg gatcctaata cagcaaataa tggtccttat 2040
actctgaaag ctacatggac taaaactggg tataatcctg ggcctgagcg agtagcttct 2100
ttggttccaa atagtttatg gggatccatt ttagatatac gatctgcgca ttcagcaatt 2160
caagcaagtg tggatgggcg ctcttattgt cgaggattat gggtttctgg agtttcgaat 2220
ttcttctatc atgaccgcga tgctttaggt cagggatatc ggtatattag tgggggttat 2280
tccttaggag caaactccta ctttggatca tcgatgtttg gtctagcatt taccgaagta 2340
tttggtagat ctaaagatta tgtagtgtgt cgttccaatc atcatgcttg cataggatcc 2400
gtttatctat ctacccaaca agctttatgt ggatcctatt tgttcggaga tgcgtttatc 2460
cgtgctagct acgggtttgg gaatcagcat atgaaaacct catatacatt tgcagaggag 2520
agcgatgttc gttgggataa taactgtctg gctggagaga ttggagcggg attaccgatt 2580
gtgattactc catctaagct ctatttgaat gagttgcgtc ctttcgtgca agctgagttt 2640
tcttatgccg atcatgaatc ttttacagag gaaggcgatc aagctcgggc attcaagagc 2700
ggacatctcc taaatctatc agttcctgtt ggagtgaagt ttgatcgatg ttctagtaca 2760
catcctaata aatatagctt tatggcggct tatatctgtg atgcttatcg caccatctct 2820
ggtactgaga caacgctcct atcccatcaa gagacatgga caacagatgc ctttcattta 2880
gcaagacatg gagttgtggt tagaggatct atgtatgctt ctctaacaag taatatagaa 2940
gtatatggcc atggaagata tgagtatcga gatgcttctc gaggctatgg tttgagtgca 3000
ggaagtaaag tccggttcta a 3021

<210> 183
<211> 2934
<212> DNA
<213> Chlamydia

<400> 183
atggctagca tgactggtgg acagcaaatg ggtcgggatt caagcttggt accgagctcg 60
gatccacatc accatcacca tcacggacta gctagagagg ttccttctag aatctttctt 120
atgcccaact cagttccaga tcctacgaaa gagtcgctat caaataaaat tagtttgaca 180
ggagacactc acaatctcac taactgctat ctcgataacc tacgctacat actggctatt 240
ctacaaaaaa ctcccaatga aggagctgct gtcacaataa cagattacct aagctttttt 300
gatacacaaa aagaaggtat ttattttgca aaaaatctca cccctgaaag tggtggtgcg 360
attggttatg cgagtcccaa ttctcctacc gtggagattc gtgatacaat aggtcctgta 420
atctttgaaa ataatacttg ttgcagacta tttacatgga gaaatcctta tgctgctgat 480
aaaataagag aaggcggagc cattcatgct caaaatcttt acataaatca taatcatgat 540
gtggtcggat ttatgaagaa cttttcttat gtccaaggag gagccattag taccgctaat 600
acctttgttg tgagcgagaa tcagtcttgt tttctcttta tggacaacat ctgtattcaa 660
actaatacag caggaaaagg tggcgctatc tatgctggaa cgagcaattc ttttgagagt 720
aataactgcg atctcttctt catcaataac gcctgttgtg caggaggagc gatcttctcc 780
cctatctgtt ctctaacagg aaatcgtggt aacatcgttt tctataacaa tcgctgcttt 840
aaaaatgtag aaacagcttc ttcagaagct tctgatggag gagcaattaa agtaactact 900
cgcctagatg ttacaggcaa tcgtggtagg atctttttta gtgacaatat cacaaaaaat 960
tatggcggag ctatttacgc tcctgtagtt accctagtgg ataatggccc tacctacttt 1020
ataaacaata tcgccaataa taaggggggc gctatctata tagacggaac cagtaactcc 1080
aaaatttctg ccgaccgcca tgctattatt tttaatgaaa atattgtgac taatgtaact 1140
aatgcaaatg gtaccagtac gtcagctaat cctcctagaa gaaatgcaat aacagtagca 1200
agctcctctg gtgaaattct attaggagca gggagtagcc aaaatttaat tttttatgat 1260
cctattgaag ttagcaatgc aggggtctct gtgtccttca ataaggaagc tgatcaaaca 1320
ggctctgtag tattttcagg agctactgtt aattctgcag attttcatca acgcaattta 1380
caaacaaaaa cacctgcacc ccttactctc agtaatggtt ttctatgtat cgaagatcat 1440
gctcagctta cagtgaatcg attcacacaa actgggggtg ttgtttctct tgggaatgga 1500
gcagttctga gttgctataa aaatggtaca ggagattctg ctagcaatgc ctctataaca 1560
ctgaagcata ttggattgaa tctttcttcc attctgaaaa gtggtgctga gattccttta 1620

ttgtgggtag agcctacaaa taacagcaat aactatacag cagatactgc agctaccttt 1680
tcattaagtg atgtaaaact ctcactcatt gatgactacg ggaactctcc ttatgaatcc 1740
acagatctga cccatgctct gtcatcacag cctatgctat ctatttctga agctagcgat 1800
aaccagctac aatcagaaaa tatagatttt tcgggactaa atgtccctca ttatggatgg 1860
caaggacttt ggacttgggg ctgggcaaaa actcaagatc cagaaccagc atcttcagca 1920
acaatcactg atccacaaaa agccaataga tttcatagaa ccttactact aacatggctt 1980
cctgccgggt atgttcctag cccaaaacac agaagtcccc tcatagctaa caccttatgg 2040
gggaatatgc tgcttgcaac agaaagctta aaaaatagtg cagagctgac acctagtggt 2100
catcctttct ggggaattac aggaggagga ctaggcatga tggtttacca agatcctcga 2160
gaaaatcatc ctggattcca tatgcgctct tccggatact ctgcggggat gatagcaggg 2220
cagacacaca ccttctcatt gaaattcagt cagacctaca ccaaactcaa tgagcgttac 2280
gcaaaaaaca acgtatcttc taaaaattac tcatgccaag gagaaatgct cttctcattg 2340
caagaaggtt tcttgctgac taaattagtt gggctttaca gctatggaga ccataactgt 2400
caccatttct atactcaagg agaaaatcta acatctcaag ggacgttccg cagtcaaacg 2460
atgggaggtg ctgtcttttt tgatctccct atgaaaccct ttggatcaac gcatatactg 2520
acagctccct ttttaggtgc tcttggtatt tattctagcc tgtctcactt tactgaggtg 2580
ggagcctatc cgcgaagctt ttctacaaag actcctttga tcaatgtcct agtccctatt 2640
ggagttaaag gtagctttat gaatgctacc cacagacctc aagcctggac tgtagaattg 2700
gcataccaac ccgttctgta tagacaagaa ccagggatcg cgacccagct cctagccagt 2760
aaaggtattt ggtttggtag tggaagcccc tcatcgcgtc atgccatgtc ctataaaatc 2820
tcacagcaaa cacaaccttt gagttggtta actctccatt tccagtatca tggattctac 2880
tcctcttcaa ccttctgtaa ttatctcaat ggggaaattg ctctgcgatt ctag 2934

<210> 184
<211> 2547
<212> DNA
<213> Chlamydia

<400> 184
atggctagcc atcaccatca ccatcacggt gctatttctt gcttacgtgg agatgtagtc 60
atttctggaa acaagggtag agttgaattt aaagacaaca tagcaacacg tctttatgtg 120
gaagaaactg tagaaaaggt tgaagaggta gagccagctc ctgagcaaaa agacaataat 180
gagctttctt tcttagggag tgtagaacag agttttatta ctgcagctaa tcaagctctt 240
ttcgcatctg aagatgggga tttatcacct gagtcatcca tttcttctga agaacttgcg 300
aaaagaagag agtgtgctgg aggagctatt tttgcaaaac gggttcgtat tgtagataac 360
caagaggccg ttgtattctc gaataacttc tctgatattt atggcggcgc catttttaca 420
ggttctcttc gagaagagga taagttagat gggcaaatcc ctgaagtctt gatctcaggc 480
aatgcagggg atgttgtttt ttccggaaat tcctcgaagc gtgatgagca tcttcctcat 540
acaggtgggg gagccatttg tactcaaaat ttgacgattt ctcagaatac agggaatgtt 600
ctgttttata acaacgtggc ctgttcggga ggagctgttc gtatagagga tcatggtaat 660
gttcttttag aagcttttgg aggagatatt gtttttaaag gaaattcttc tttcagagca 720
caaggatccg atgctatcta ttttgcaggt aaagaatcgc atattacagc cctgaatgct 780
acggaaggac atgctattgt tttccacgac gcattagttt ttgaaaatct aaaagaaagg 840
aaatctgctg aagtattgtt aatcaatagt cgagaaaatc caggttacac tggatctatt 900
cgatttttag aagcagaaag taaagttcct caatgtattc atgtacaaca aggaagcctt 960
gagttgctaa atggagctac attatgtagt tatggtttta aacaagatgc tggagctaag 1020
ttggtattgg ctgctggatc taaactgaag attttagatt caggaactcc tgtacaaggg 1080
catgctatca gtaaacctga agcagaaatc gagtcatctt ctgaaccaga gggtgcacat 1140
tctctttgga ttgcgaagaa tgctcaaaca acagttccta tggttgatat ccatactatt 1200
tctgtagatt tagcctcctt ctcttctagt caacaggagg ggacagtaga agctcctcag 1260
gttattgttc ctggaggaag ttatgttcga tctggagagc ttaatttgga gttagttaac 1320
acaacaggta ctggttatga aaatcatgct ttgttgaaga atgaggctaa agttccattg 1380
atgtctttcg ttgcttctag tgatgaagct tcagccgaaa tcagtaactt gtcggtttct 1440
gatttacaga ttcatgtagc aactccagag attgaagaag acacatacgg ccatatggga 1500
gattggtctg aggctaaaat tcaagatgga actcttgtca ttaattggaa tcctactgga 1560

tatcgattag atcctcaaaa agcaggggct ttagtattta atgcattatg ggaagaaggg 1620
gctgtcttgt ctgctctgaa aaatgcacgc tttgctcata atctcactgc tcagcgtatg 1680
gaattcgatt attctacaaa tgtgtgggga ttcgcctttg gtggtttccg aactctatct 1740
gcagagaatc tggttgctat tgatggatac aaaggagctt atggtggtgc ttctgctgga 1800
gtcgatattc aattgatgga agattttgtt ctaggagtta gtggagctgc tttcctaggt 1860
aaaatggata gtcagaagtt tgatgcggag gtttctcgga agggagttgt tggttctgta 1920
tatacaggat ttttagctgg atcctggttc ttcaaaggac aatatagcct tggagaaaca 1980
cagaacgata tgaaaacgcg ttatggagta ctaggagagt cgagtgcttc ttggacatct 2040
cgaggagtac tggcagatgc tttagttgaa taccgaagtt tagttggtcc tgtgagacct 2100
actttttatg ctttgcattt caatccttat gtcgaagtat cttatgcttc tatgaaattc 2160
cctggcttta cagaacaagg aagagaagcg cgttcttttg aagacgcttc ccttaccaat 2220
atcaccattc ctttagggat gaagtttgaa ttggcgttca taaaaggaca gttttcagag 2280
gtgaactctt tgggaataag ttatgcatgg gaagcttatc gaaaagtaga aggaggcgcg 2340
gtgcagcttt tagaagctgg gtttgattgg gagggagctc caatggatct tcctagacag 2400
gagctgcgtg tcgctctgga aaataatacg gaatggagtt cttacttcag cacagtctta 2460
ggattaacag ctttttgtgg aggatttact tctacagata gtaaactagg atatgaggcg 2520
aatactggat tgcgattgat cttttaa 2547

<210> 185
<211> 2337
<212> DNA
<213> Chlamydia

<400> 185
atgcatcacc atcaccatca cgggttagct agttgcgtag atcttcatgc tggaggacag 60
tctgtaaatg agctggtata tgtaggccct caagcggttt tattgttaga ccaaattcga 120
gatctattcg ttgggtctaa agatagtcag gctgaaggac agtataggtt aattgtagga 180
gatccaagtt ctttccaaga gaaagatgca gatactcttc ccgggaaggt agagcaaagt 240
actttgttct cagtaaccaa tcccgtggtt ttccaaggtg tggaccaaca ggatcaagtc 300
tcttcccaag ggttaatttg tagttttacg agcagcaacc ttgattctcc ccgtgacgga 360
gaatcttttt taggtattgc ttttgttggg gatagtagta aggctggaat cacattaact 420
gacgtgaaag cttctttgtc tggagcggct ttatattcta cagaagatct tatctttgaa 480
aagattaagg gtggattgga atttgcatca tgttcttctc tagaacaggg gggagcttgt 540
gcagctcaaa gtattttgat tcatgattgt caaggattgc aggttaaaca ctgtactaca 600
gccgtgaatg ctgaggggtc tagtgcgaat gatcatcttg gatttggagg aggcgctttc 660
tttgttacgg gttctctttc tggagagaaa agtctctata tgcctgcagg agatatggta 720
gttgcgaatt gtgatggggc tatatctttt gaaggaaaca gcgcgaactt tgctaatgga 780
ggagcgattg ctgcctctgg gaaagtgctt tttgtcgcta atgataaaaa gacttctttt 840
atagagaacc gagctttgtc tggaggagcg attgcagcct cttctgatat tgcctttcaa 900
aactgcgcag aactagtttt caaaggcaat tgtgcaattg gaacagagga taaaggttct 960
ttaggtggag gggctatatc ttctctaggc accgttcttt tgcaagggaa tcacgggata 1020
acttgtgata agaatgagtc tgcttcgcaa ggaggcgcca tttttggcaa aaattgtcag 1080
atttctgaca acgaggggcc agtggttttc agagatagta cagcttgctt aggaggaggc 1140
gctattgcag ctcaagaaat tgtttctatt cagaacaatc aggctgggat ttccttcgag 1200
ggaggtaagg ctagtttcgg aggaggtatt gcgtgtggat ctttttcttc cgcaggcggt 1260
gcttctgttt tagggactat tgatatttcg aagaatttag gcgcgatttc gttctctcgt 1320
actttatgta cgacctcaga tttaggacaa atggagtacc agggaggagg agctctattt 1380
ggtgaaaata tttctctttc tgagaatgct ggtgtgctca cctttaaaga caacattgtg 1440
aagacttttg cttcgaatgg gaaaattctg ggaggaggag cgattttagc tactggtaag 1500
gtggaaatta ccaataattc cggaggaatt tcttttacag gaaatgcgag agctccacaa 1560
gctcttccaa ctcaagagga gtttccttta ttcagcaaaa aagaagggcg accactctct 1620
tcaggatatt ctgggggagg agcgatttta ggaagagaag tagctattct ccacaacgct 1680
gcagtagtat ttgagcaaaa tcgtttgcag tgcagcgaag aagaagcgac attattaggt 1740
tgttgtggag gaggcgctgt tcatgggatg gatagcactt cgattgttgg caactcttca 1800
gtaagatttg gtaataatta cgcaatggga caaggagtct caggaggagc tcttttatct 1860

aaaacagtgc agttagctgg aaatggaagc gtcgattttt ctcgaaatat tgctagtttg 1920
ggaggaggag ctcttcaagc ttctgaagga aattgtgagc tagttgataa cggctatgtg 1980
ctattcagag ataatcgagg gagggtttat gggggtgcta tttcttgctt acgtggagat 2040
gtagtcattt ctggaaacaa gggtagagtt gaatttaaag acaacatagc aacacgtctt 2100
tatgtggaag aaactgtaga aaaggttgaa gaggtagagc cagctcctga gcaaaaagac 2160
aataatgagc tttctttctt agggagtgta gaacagagtt ttattactgc agctaatcaa 2220
gctcttttcg catctgaaga tggggattta tcacctgagt catccatttc ttctgaagaa 2280
cttgcgaaaa gaagagagtg tgctggagga gctgactcga gcagatccgg ctgctaa 2337

<210> 186
<211> 2847
<212> DNA
<213> Chlamydia

<400> 186
atggctagca tgcatcacca tcaccatcac gttaagattg agaacttctc tggccaagga 60
atattttctg gaaacaaagc tatcgataac accacagaag gctcctcttc caaatctaac 120
gtcctcggag gtgcggtcta tgctaaaaca ttgtttaatc tcgatagcgg gagctctaga 180
cgaactgtca ccttctccgg gaatactgtc tcttctcaat ctacaacagg tcaggttgct 240
ggaggagcta tctactctcc tactgtaacc attgctactc ctgtagtatt ttctaaaaac 300
tctgcaacaa acaatgctaa taacgctaca gatactcaga gaaaagacac ctttggagga 360
gctatcggag ctacttctgc tgtttctcta tcaggagggg ctcatttctt agaaaacgtt 420
gctgacctcg gatctgctat tgggttggtg ccagacacac aaaatacaga aacagtgaaa 480
ttagagtctg gctcctacta ctttgaaaaa aataaagctt taaaacgagc tactatttac 540
gcacctgtcg tttccattaa agcctatact gcgacattta accaaaacag atctctagaa 600
gaaggaagcg cgatttactt tacaaaagaa gcatctattg agtctttagg ctctgttctc 660
ttcacaggaa acttagtaac cccaacgcta agcacaacta cagaaggcac accagccaca 720
acctcaggag atgtaacaaa atatggtgct gctatctttg gacaaatagc aagctcaaac 780
ggatctcaga cggataacct tcccctgaaa ctcattgctt caggaggaaa tatttgtttc 840
cgaaacaatg aataccgtcc tacttcttct gataccggaa cctctacttt ctgtagtatt 900
gcgggagatg ttaaattaac catgcaagct gcaaaaggga aaacgatcag tttctttgat 960
gcaatccgga cctctactaa gaaaacaggt acacaggcaa ctgcctacga tactctcgat 1020
attaataaat ctgaggattc agaaactgta aactctgcgt ttacaggaac gattctgttc 1080
tcctctgaat tacatgaaaa taaatcctat attccacaaa acgtagttct acacagtgga 1140
tctcttgtat tgaagccaaa taccgagctt catgtcattt cttttgagca gaaagaaggc 1200
tcttctctcg ttatgacacc tggatctgtt ctttcgaacc agactgttgc tgatggagct 1260
ttggtcataa ataacatgac cattgattta tccagcgtag agaaaaatgg tattgctgaa 1320
ggaaatatct ttactcctcc agaattgaga atcatagaca ctactacaag tggaagcggt 1380
ggaaccccat ctacagatag tgaaagtaac cagaatagtg atgataccaa ggagcaaaat 1440
aataatgacg cctcgaatca aggagaaagc gcgaatggat cgtcttctcc tgcagtagct 1500
gctgcacaca catctcgtac aagaaacttt gccgctgcag ctacagccac acctacgaca 1560
acaccaacgg ctacaactac aacaagcaac caagtaatcc taggaggaga aatcaaactc 1620
atcgatccta atgggacctt cttccagaac cctgcattaa gatccgacca acaaatctcc 1680
ttgttagtgc tccctacaga ctcatcaaaa atgcaagctc agaaaatagt actgacgggt 1740
gatattgctc ctcagaaagg atatacagga acactcactc tggatcctga tcaactacaa 1800
aatggaacga tctcagcgct ctggaaattt gactcttata gacaatgggc ttatgtacct 1860
agagacaatc atttctatgc gaactcgatt ctgggatctc aaatgtcaat ggtcacagtc 1920
aaacaaggct tgctcaacga taaaatgaat ctagctcgct ttgatgaagt tagctataac 1980
aacctgtgga tatcaggact aggaacgatg ctatcgcaag taggaacacc tacttctgaa 2040
gaattcactt attacagcag aggagcttct gttgccttag atgctaaacc agcccatgat 2100
gtgattgttg gagctgcatt tagtaagatg atcgggaaaa caaaatcctt gaaaagagag 2160
aataactaca ctcacaaagg atccgaatat tcttaccaag catcggtata cggaggcaaa 2220
ccattccact ttgtaatcaa taaaaaaacg gaaaaatcgc taccgctatt gttacaagga 2280
gtcatctctt acggatatat caaacatgat acagtgactc actatccaac gatccgtgaa 2340
cgaaaccaag gagaatggga agacttagga tggctgacag ctctccgtgt ctcctctgtc 2400

ttaagaactc ctgcacaagg ggatactaaa cgtatcactg tttacggaga attggaatac 2460
tccagtatcc gtcagaaaca attcacagaa acagaatacg atcctcgtta cttcgacaac 2520
tgcacctata gaaacttagc aattcctatg gggttagcat tcgaaggaga gctctctggt 2580
aacgatattt tgatgtacaa cagattctct gtagcataca tgccatcaat ctatcgaaat 2640
tctccaacat gcaaatacca agtgctctct tcaggagaag gcggagaaat tatttgtgga 2700
gtaccgacaa gaaactcagc tcgcggagaa tacagcacgc agctgtaccc gggacctttg 2760
tggactctgt atggatccta cacgatagaa gcagacgcac atacactagc tcatatgatg 2820
aactgcggtg ctcgtatgac attctaa 2847

<210> 187
<211> 2466
<212> DNA
<213> Chlamydia

<400> 187
atgcatcacc atcaccatca cgaggcgagc tcgatccaag atcaaataaa gaataccgac 60
tgcaatgtta gcaaagtagg atattcaact tctcaagcat ttactgatat gatgctagca 120
gacaacacag agtatcgagc tgctgatagt gtttcattct atgacttttc gacatcttcc 180
ggattaccta gaaaacatct tagtagtagt agtgaagctt ctccaacgac agaaggagtg 240
tcttcatctt catctggaga aaatactgag aattcacaag attcagctcc ctcttctgga 300
gaaactgata agaaaacaga agaagaacta gacaatggcg gaatcattta tgctagagag 360
aaactaacta tctcagaatc tcaggactct ctctctaatc caagcataga actccatgac 420
aatagttttt tcttcggaga aggtgaagtt atctttgatc acagagttgc cctcaaaaac 480
ggaggagcta tttatggaga gaaagaggta gtctttgaaa acataaaatc tctactagta 540
gaagtaaata tctcggtcga gaaagggggt agcgtctatg caaaagaacg agtatcttta 600
gaaaatgtta ccgaagcaac cttctcctcc aatggtgggg aacaaggtgg tggtggaatc 660
tattcagaac aagatatgtt aatcagtgat tgcaacaatg tacatttcca agggaatgct 720
gcaggagcaa cagcagtaaa acaatgtctg gatgaagaaa tgatcgtatt gctcacagaa 780
tgcgttgata gcttatccga agatacactg gatagcactc cagaaacgga acagactaag 840
tcaaatggaa atcaagatgg ttcgtctgaa acaaaagata cacaagtatc agaatcacca 900
gaatcaactc ctagccccga cgatgtttta ggtaaaggtg gtggtatcta tacagaaaaa 960
tctttgacca tcactggaat tacagggact atagattttg tcagtaacat agctaccgat 1020
tctggagcag gtgtattcac taaagaaaac ttgtcttgca ccaacacgaa tagcctacag 1080
tttttgaaaa actcggcagg tcaacatgga ggaggagcct acgttactca aaccatgtct 1140
gttactaata caactagtga aagtataact actccccctc tcgtaggaga agtgattttc 1200
tctgaaaata cagctaaagg gcacggtggt ggtatctgca ctaacaaact ttctttatct 1260
aatttaaaaa cggtgactct cactaaaaac tctgcaaagg agtctggagg agctattttt 1320
acagatctag cgtctatacc aacaacagat accccagagt cttctacccc ctcttcctcc 1380
tcgcctgcaa gcactcccga agtagttgct tctgctaaaa taaatcgatt ctttgcctct 1440
acggcagaac cggcagcccc ttctctaaca gaggctgagt ctgatcaaac ggatcaaaca 1500
gaaacttctg atactaatag cgatatagac gtgtcgattg agaacatttt gaatgtcgct 1560
atcaatcaaa acacttctgc gaaaaaagga ggggctattt acgggaaaaa agctaaactt 1620
tcccgtatta acaatcttga actttcaggg aattcatccc aggatgtagg aggaggtctc 1680
tgtttaactg aaagcgtaga atttgatgca attggatcgc tcttatccca ctataactct 1740
gctgctaaag aaggtggggt tattcattct aaaacggtta ctctatctaa cctcaagtct 1800
accttcactt ttgcagataa cactgttaaa gcaatagtag aaagcactcc tgaagctcca 1860
gaagagattc ctccagtaga aggagaagag tctacagcaa cagaaaatcc gaattctaat 1920
acagaaggaa gttcggctaa cactaacctt gaaggatctc aaggggatac tgctgataca 1980
gggactggtg ttgttaacaa tgagtctcaa gacacatcag atactggaaa cgctgaatct 2040
ggagaacaac tacaagattc tacacaatct aatgaagaaa atacccttcc caatagtagt 2100
attgatcaat ctaacgaaaa cacagacgaa tcatctgata gccacactga ggaaataact 2160
gacgagagtg tctcatcgtc ctctaaaagt ggatcatcta ctcctcaaga tggaggagca 2220
gcttcttcag gggctccctc aggagatcaa tctatctctg caaacgcttg tttagctaaa 2280
agctatgctg cgagtactga tagctcccct gtatctaatt cttcaggttc agacgttact 2340
gcatcttctg ataatccaga ctcttcctca tctggagata gcgctggaga ctctgaagga 2400

ccgactgagc cagaagctgg ttctacaaca gaaactccta ctttaatagg aggaggtgct 2460
atctga 2466

<210> 188
<211> 1578
<212> DNA
<213> Chlamydia

<400> 188
atgcatcacc atcaccatca cacggccgcg tccgataact tccagctgtc ccagggtggg 60
cagggattcg ccattccgat cgggcaggcg atggcgatcg cgggccagat caagcttccc 120
accgttcata tcgggcctac cgccttcctc ggcttgggtg ttgtcgacaa caacggcaac 180
ggcgcacgag tccaacgcgt ggtcgggagc gctccggcgg caagtctcgg catctccacc 240
ggcgacgtga tcaccgcggt cgacggcgct ccgatcaact cggccaccgc gatggcggac 300
gcgcttaacg ggcatcatcc cggtgacgtc atctcggtga cctggcaaac caagtcgggc 360
ggcacgcgta cagggaacgt gacattggcc gagggacccc cggccgaatt cccgctagta 420
cctagaggtt caccgctgcc tgtggggaat ccagctgaac caagtttatt aatcgatggc 480
actatgtggg aaggtgcttc aggagatcct tgcgatcctt gcgctacttg gtgtgacgcc 540
attagcatcc gcgcaggata ctacggagat tatgttttcg atcgtgtatt aaaagttgat 600
gtgaataaaa cttttagcgg catggctgca actcctacgc aggctatagg taacgcaagt 660
aatactaatc agccagaagc aaatggcaga ccgaacatcg cttacggaag gcatatgcaa 720
gatgcagagt ggttttcaaa tgcagccttc ctagccttaa acatttggga tcgcttcgac 780
attttctgca ccttaggggc atccaatgga tacttcaaag caagttcggc tgcattcaac 840
ttggttgggt taatagggtt ttcagctgca agctcaatct ctaccgatct tccaatgcaa 900
cttcctaacg taggcattac ccaaggtgtt gtggaatttt atacagacac atcattttct 960
tggagcgtag gtgcacgtgg agctttatgg gaatgtggtt gtgcaacctt aggagctgag 1020
ttccaatacg ctcaatctaa tcctaagatt gagatgctca acgtcacttc aagcccagca 1080
caatttgtga ttcacaaacc aagaggctat aaaggagcta gctcgaattt tcctttacct 1140
ataacggctg gaacaacaga agctacagac accaaatcag ctacaattaa ataccatgaa 1200
tggcaagtag gcctcgccct gtcttacaga ttgaatatgc ttgttccata tattggcgta 1260
aactggtcaa gagcaacttt tgatgctgat actatccgca ttgctcaacc taaattaaaa 1320
tcggagattc ttaacattac tacatggaac ccaagcctta taggatcaac cactgctttg 1380
cccaataata gtggtaagga tgttctatct gatgtcttgc aaattgcttc gattcagatc 1440
aacaaaatga agtctagaaa agcttgtggt gtagctgttg gtgcaacgtt aatcgacgct 1500
gacaaatggt caatcactgg tgaagcacgc ttaatcaatg aaagagctgc tcacatgaat 1560
gcacaattcc gcttctaa 1578

<210> 189
<211> 866
<212> PRT
<213> Chlamydia

<220>
<221> VARIANT
<222> (1)...(866)
<223> Xaa = Any Amino Acid

<400> 189
Met Ala Ser His His His His His His Leu Phe Gly Gln Asp Pro Leu
1               5                   10                  15
Gly Glu Thr Ala Leu Leu Thr Lys Asn Pro Asn His Val Val Cys Thr
            20                  25                  30
Phe Phe Glu Asp Cys Thr Met Glu Ser Leu Phe Pro Ala Leu Cys Ala
        35                  40                  45
His Ala Ser Gln Asp Asp Pro Leu Tyr Val Leu Gly Asn Ser Tyr Cys

    50                  55                  60
Trp Phe Val Ser Lys Leu His Ile Thr Asp Pro Lys Glu Ala Leu Phe
65                  70                  75                  80
Lys Glu Lys Gly Asp Leu Ser Ile Gln Asn Phe Arg Phe Leu Ser Phe
                85                  90                  95
Thr Asp Cys Ser Ser Lys Glu Ser Ser Pro Ser Ile Ile His Gln Lys
            100                 105                 110
Asn Gly Gln Leu Ser Leu Arg Asn Asn Gly Ser Met Ser Phe Cys Arg
        115                 120                 125
Asn His Ala Glu Gly Ser Gly Gly Ala Ile Ser Ala Asp Ala Phe Ser
    130                 135                 140
Leu Gln His Asn Tyr Leu Phe Thr Ala Phe Glu Glu Asn Ser Ser Lys
145                 150                 155                 160
Gly Asn Gly Gly Ala Ile Gln Ala Gln Thr Phe Ser Leu Ser Arg Asn
                165                 170                 175
Val Ser Pro Ile Ser Phe Ala Arg Asn Arg Ala Asp Leu Asn Gly Gly
            180                 185                 190
Ala Ile Cys Cys Ser Asn Leu Ile Cys Ser Gly Asn Val Asn Pro Leu
        195                 200                 205
Phe Phe Thr Gly Asn Ser Ala Thr Asn Gly Gly Xaa Ile Cys Cys Ile
    210                 215                 220
Ser Asp Leu Asn Thr Ser Glu Lys Gly Ser Leu Ser Leu Ala Cys Asn
225                 230                 235                 240
Gln Xaa Thr Leu Phe Ala Ser Asn Ser Ala Lys Glu Lys Gly Gly Ala
                245                 250                 255
Ile Tyr Ala Lys His Met Val Leu Arg Tyr Asn Gly Pro Val Ser Phe
            260                 265                 270
Ile Asn Asn Ser Ala Lys Ile Gly Gly Ala Ile Ala Ile Gln Ser Gly
        275                 280                 285
Gly Ser Leu Ser Ile Leu Ala Gly Glu Gly Ser Val Leu Phe Gln Asn
    290                 295                 300
Asn Ser Gln Arg Thr Ser Asp Gln Gly Leu Val Arg Asn Ala Ile Tyr
305                 310                 315                 320
Leu Glu Lys Asp Ala Ile Leu Ser Ser Leu Glu Ala Arg Asn Gly Asp
                325                 330                 335
Ile Leu Phe Phe Asp Pro Ile Val Gln Glu Ser Ser Ser Lys Glu Ser
            340                 345                 350
Pro Leu Pro Ser Ser Leu Gln Ala Ser Val Thr Ser Pro Thr Pro Ala
        355                 360                 365
Thr Ala Ser Pro Leu Val Ile Gln Thr Ser Ala Asn Arg Ser Val Ile
    370                 375                 380
Phe Ser Ser Glu Arg Leu Ser Glu Glu Glu Lys Thr Pro Asp Asn Leu
385                 390                 395                 400
Thr Ser Gln Leu Gln Gln Pro Ile Glu Leu Lys Ser Gly Arg Leu Val
                405                 410                 415
Leu Lys Asp Arg Ala Val Leu Ser Xaa Pro Ser Leu Ser Gln Asp Pro
            420                 425                 430
Gln Ala Leu Leu Ile Met Glu Ala Gly Thr Ser Leu Lys Thr Ser Xaa
        435                 440                 445
Asp Leu Lys Leu Xaa Thr Xaa Ser Ile Pro Leu His Ser Leu Asp Thr
    450                 455                 460
Glu Lys Ser Val Thr Ile His Ala Pro Asn Leu Ser Ile Gln Lys Ile
465                 470                 475                 480
Phe Leu Ser Asn Ser Gly Asp Glu Asn Phe Tyr Glu Asn Val Glu Leu
                485                 490                 495

Leu Ser Lys Glu Gln Asn Asn Ile Pro Leu Leu Thr Leu Pro Lys Glu
            500                 505                 510
Gln Ser His Leu His Leu Pro Asp Gly Asn Leu Ser Ser His Phe Gly
        515                 520                 525
Tyr Gln Gly Asp Trp Thr Phe Ser Trp Lys Asp Ser Asp Glu Gly His
    530                 535                 540
Ser Leu Ile Ala Asn Trp Thr Pro Lys Asn Tyr Val Pro His Pro Glu
545                 550                 555                 560
Arg Gln Ser Thr Leu Val Ala Asn Thr Leu Trp Asn Thr Tyr Ser Asp
                565                 570                 575
Met Gln Ala Val Gln Ser Met Ile Asn Thr Thr Ala His Gly Gly Ala
            580                 585                 590
Tyr Leu Phe Gly Thr Trp Gly Ser Ala Val Ser Asn Leu Phe Tyr Val
        595                 600                 605
His Asp Ser Ser Gly Lys Pro Ile Asp Asn Trp His His Arg Ser Leu
    610                 615                 620
Gly Tyr Leu Phe Gly Ile Ser Thr His Ser Leu Asp Asp His Ser Phe
625                 630                 635                 640
Cys Leu Ala Ala Gly Gln Leu Leu Gly Lys Ser Ser Asp Ser Phe Ile
                645                 650                 655
Thr Ser Thr Glu Thr Thr Ser Tyr Ile Ala Thr Val Gln Ala Gln Leu
            660                 665                 670
Ala Thr Ser Leu Met Lys Ile Ser Ala Gln Ala Cys Tyr Asn Glu Ser
        675                 680                 685
Ile His Glu Leu Lys Thr Lys Tyr Arg Ser Phe Ser Lys Glu Gly Phe
    690                 695                 700
Gly Ser Trp His Ser Val Ala Val Ser Gly Glu Val Cys Ala Ser Ile
705                 710                 715                 720
Pro Ile Val Ser Asn Gly Ser Gly Leu Phe Ser Ser Phe Ser Ile Phe
                725                 730                 735
Ser Lys Leu Gln Gly Phe Ser Gly Thr Gln Asp Gly Phe Glu Glu Ser
            740                 745                 750
Ser Gly Glu Ile Arg Ser Phe Ser Ala Ser Ser Phe Arg Asn Ile Ser
        755                 760                 765
Leu Pro Ile Gly Ile Thr Phe Glu Lys Lys Ser Gln Lys Thr Arg Thr
    770                 775                 780
Tyr Tyr Tyr Phe Leu Gly Ala Tyr Ile Gln Asp Leu Lys Arg Asp Val
785                 790                 795                 800
Glu Ser Gly Pro Val Val Leu Leu Lys Asn Ala Val Ser Trp Asp Ala
                805                 810                 815
Pro Met Ala Asn Leu Asp Ser Arg Ala Tyr Met Phe Arg Leu Thr Asn
            820                 825                 830
Gln Arg Ala Leu His Arg Leu Gln Thr Leu Leu Asn Val Ser Cys Val
        835                 840                 845
Leu Arg Gly Gln Ser His Ser Tyr Ser Leu Asp Leu Gly Thr Thr Tyr
    850                 855                 860
Arg Phe
865

<210> 190
<211> 1006
<212> PRT
<213> Chlamydia

<400> 190

Met Ala Ser Met Thr Gly Gly Gln Gln Met Gly Arg Asp Ser Ser Leu
1               5                   10                  15
Val Pro His His His His His His Met Ile Pro Gln Gly Ile Tyr Asp
            20                  25                  30
Gly Glu Thr Leu Thr Val Ser Phe Pro Tyr Thr Val Ile Gly Asp Pro
        35                  40                  45
Ser Gly Thr Thr Val Phe Ser Ala Gly Glu Leu Thr Leu Lys Asn Leu
    50                  55                  60
Asp Asn Ser Ile Ala Ala Leu Pro Leu Ser Cys Phe Gly Asn Leu Leu
65                  70                  75                  80
Gly Ser Phe Thr Val Leu Gly Arg Gly His Ser Leu Thr Phe Glu Asn
                85                  90                  95
Ile Arg Thr Ser Thr Asn Gly Ala Ala Leu Ser Asn Ser Ala Ala Asp
            100                 105                 110
Gly Leu Phe Thr Ile Glu Gly Phe Lys Glu Leu Ser Phe Ser Asn Cys
        115                 120                 125
Asn Ser Leu Leu Ala Val Leu Pro Ala Ala Thr Thr Asn Lys Gly Ser
    130                 135                 140
Gln Thr Pro Thr Thr Thr Ser Thr Pro Ser Asn Gly Thr Ile Tyr Ser
145                 150                 155                 160
Lys Thr Asp Leu Leu Leu Leu Asn Asn Glu Lys Phe Ser Phe Tyr Ser
                165                 170                 175
Asn Leu Val Ser Gly Asp Gly Gly Ala Ile Asp Ala Lys Ser Leu Thr
            180                 185                 190
Val Gln Gly Ile Ser Lys Leu Cys Val Phe Gln Glu Asn Thr Ala Gln
        195                 200                 205
Ala Asp Gly Gly Ala Cys Gln Val Val Thr Ser Phe Ser Ala Met Ala
    210                 215                 220
Asn Glu Ala Pro Ile Ala Phe Val Ala Asn Val Ala Gly Val Arg Gly
225                 230                 235                 240
Gly Gly Ile Ala Ala Val Gln Asp Gly Gln Gln Gly Val Ser Ser Ser
                245                 250                 255
Thr Ser Thr Glu Asp Pro Val Val Ser Phe Ser Arg Asn Thr Ala Val
            260                 265                 270
Glu Phe Asp Gly Asn Val Ala Arg Val Gly Gly Gly Ile Tyr Ser Tyr
        275                 280                 285
Gly Asn Val Ala Phe Leu Asn Asn Gly Lys Thr Leu Phe Leu Asn Asn
    290                 295                 300
Val Ala Ser Pro Val Tyr Ile Ala Ala Lys Gln Pro Thr Ser Gly Gln
305                 310                 315                 320
Ala Ser Asn Thr Ser Asn Asn Tyr Gly Asp Gly Gly Ala Ile Phe Cys
                325                 330                 335
Lys Asn Gly Ala Gln Ala Gly Ser Asn Asn Ser Gly Ser Val Ser Phe
            340                 345                 350
Asp Gly Glu Gly Val Val Phe Phe Ser Ser Asn Val Ala Ala Gly Lys
        355                 360                 365
Gly Gly Ala Ile Tyr Ala Lys Lys Leu Ser Val Ala Asn Cys Gly Pro
    370                 375                 380
Val Gln Phe Leu Arg Asn Ile Ala Asn Asp Gly Gly Ala Ile Tyr Leu
385                 390                 395                 400
Gly Glu Ser Gly Glu Leu Ser Leu Ser Ala Asp Tyr Gly Asp Ile Ile
                405                 410                 415
Phe Asp Gly Asn Leu Lys Arg Thr Ala Lys Glu Asn Ala Ala Asp Val
            420                 425                 430
Asn Gly Val Thr Val Ser Ser Gln Ala Ile Ser Met Gly Ser Gly Gly

        435                 440                 445
Lys Ile Thr Thr Leu Arg Ala Lys Ala Gly His Gln Ile Leu Phe Asn
    450                 455                 460
Asp Pro Ile Glu Met Ala Asn Gly Asn Asn Gln Pro Ala Gln Ser Ser
465                 470                 475                 480
Lys Leu Leu Lys Ile Asn Asp Gly Glu Gly Tyr Thr Gly Asp Ile Val
                485                 490                 495
Phe Ala Asn Gly Ser Ser Thr Leu Tyr Gln Asn Val Thr Ile Glu Gln
            500                 505                 510
Gly Arg Ile Val Leu Arg Glu Lys Ala Lys Leu Ser Val Asn Ser Leu
        515                 520                 525
Ser Gln Thr Gly Gly Ser Leu Tyr Met Glu Ala Gly Ser Thr Leu Asp
    530                 535                 540
Phe Val Thr Pro Gln Pro Pro Gln Gln Prc Pro Ala Ala Asn Gln Leu
545 550 555 560
Ile Thr Leu Ser Asn Leu His Leu Ser Leu Ser Ser Leu Leu Ala Asn
565 570 575
Asn Ala Val Thr Asn Pro Pro Thr Asn Pro Pro Ala Gln Asp Ser His
580 585 590
Pro Ala Val Ile Gly Ser Thr Thr Ala Gly Ser Val Thr Ile Ser Gly
595 600 605
Pro Ile Phe Phe Glu Asp Leu Asp Asp Thr Ala Tyr Asp Arg Tyr Asp
610 615 620
Trp Leu Gly Ser Asn Gln Lys Ile Asn Val Leu Lys Leu Gln Leu Gly
625 630 635 640
Thr Lys Pro Pro Ala Asn Ala Pro Ser Asp Leu Thr Leu Gly Asn Glu
645 650 655
Met Pro Lys Tyr Gly Tyr Gln Gly Ser Trp Lys Leu Ala Trp Asp Pro
660 665 670
Asn Thr Ala Asn Asn Gly Pro Tyr Thr Leu Lys Ala Thr Trp Thr Lys
675 680 685
Thr Gly Tyr Asn Pro Gly Pro Glu Arg Val Ala Ser Leu Val Pro Asn
690 695 700
Ser Leu Trp Gly Ser Ile Leu Asp Ile Arg Ser Ala His Ser Ala Ile
705 710 715 720
Gln Ala Ser Val Asp Gly Arg Ser Tyr Cys Arg Gly Leu Trp Val Ser
725 730 735
Gly Val Ser Asn Phe Phe Tyr His Asp Arg Asp Ala Leu Gly Gln Gly
740 745 750
Tyr Arg Tyr Ile Ser Gly Gly Tyr Ser Leu Gly Ala Asn Ser Tyr Phe
755 760 765
Gly Ser Ser Met Phe Gly Leu Ala Phe Thr Glu Val Phe Gly Arg Ser
770 775 780
Lys Asp Tyr Val Val Cys Arg Ser Asn His His Ala Cys Ile Gly Ser
785 790 795 800
Val Tyr Leu Ser Thr Gln Gln Ala Leu Cys Gly Ser Tyr Leu Phe Gly
805 810 815
Asp Ala Phe Ile Arg Ala Ser Tyr Gly Phe Gly Asn Gln His Met Lys
820 825 830
Thr Ser Tyr Thr Phe Ala Glu Glu Ser Asp Val Arg Trp Asp Asn Asn
835 840 845
Cys Leu Ala Gly Glu Ile Gly Ala Gly Leu Pro Ile Val Ile Thr Pro
850 855 860
Ser Lys Leu Tyr Leu Asn Glu Leu Arg Pro Phe Val Gln Ala Glu Phe
865 870 875 880

Ser Tyr Ala Asp His Glu Ser Phe Thr Glu Glu Gly Asp Gln Ala Arg
                885                 890                 895
Ala Phe Lys Ser Gly His Leu Leu Asn Leu Ser Val Pro Val Gly Val
            900                 905                 910
Lys Phe Asp Arg Cys Ser Ser Thr His Pro Asn Lys Tyr Ser Phe Met
        915                 920                 925
Ala Ala Tyr Ile Cys Asp Ala Tyr Arg Thr Ile Ser Gly Thr Glu Thr
    930                 935                 940
Thr Leu Leu Ser His Gln Glu Thr Trp Thr Thr Asp Ala Phe His Leu
945                 950                 955                 960
Ala Arg His Gly Val Val Val Arg Gly Ser Met Tyr Ala Ser Leu Thr
                965                 970                 975
Ser Asn Ile Glu Val Tyr Gly His Gly Arg Tyr Glu Tyr Arg Asp Ala
            980                 985                 990
Ser Arg Gly Tyr Gly Leu Ser Ala Gly Ser Lys Val Arg Phe
        995                 1000                1005

<210> 191
<211> 977
<212> PRT
<213> Chlamydia

<400> 191
Met Ala Ser Met Thr Gly Gly Gln Gln Met Gly Arg Asp Ser Ser Leu
 1               5                  10                  15
Val Pro Ser Ser Asp Pro His His His His His His Gly Leu Ala Arg
            20                  25                  30
Glu Val Pro Ser Arg Ile Phe Leu Met Pro Asn Ser Val Pro Asp Pro
        35                  40                  45
Thr Lys Glu Ser Leu Ser Asn Lys Ile Ser Leu Thr Gly Asp Thr His
    50                  55                  60
Asn Leu Thr Asn Cys Tyr Leu Asp Asn Leu Arg Tyr Ile Leu Ala Ile
65                  70                  75                  80
Leu Gln Lys Thr Pro Asn Glu Gly Ala Ala Val Thr Ile Thr Asp Tyr
                85                  90                  95
Leu Ser Phe Phe Asp Thr Gln Lys Glu Gly Ile Tyr Phe Ala Lys Asn
            100                 105                 110
Leu Thr Pro Glu Ser Gly Gly Ala Ile Gly Tyr Ala Ser Pro Asn Ser
        115                 120                 125
Pro Thr Val Glu Ile Arg Asp Thr Ile Gly Pro Val Ile Phe Glu Asn
    130                 135                 140
Asn Thr Cys Cys Arg Leu Phe Thr Trp Arg Asn Pro Tyr Ala Ala Asp
145                 150                 155                 160
Lys Ile Arg Glu Gly Gly Ala Ile His Ala Gln Asn Leu Tyr Ile Asn
                165                 170                 175
His Asn His Asp Val Val Gly Phe Met Lys Asn Phe Ser Tyr Val Gln
            180                 185                 190
Gly Gly Ala Ile Ser Thr Ala Asn Thr Phe Val Val Ser Glu Asn Gln
        195                 200                 205
Ser Cys Phe Leu Phe Met Asp Asn Ile Cys Ile Gln Thr Asn Thr Ala
    210                 215                 220
Gly Lys Gly Gly Ala Ile Tyr Ala Gly Thr Ser Asn Ser Phe Glu Ser
225                 230                 235                 240
Asn Asn Cys Asp Leu Phe Phe Ile Asn Asn Ala Cys Cys Ala Gly Gly
                245                 250                 255

Ala Ile Phe Ser Pro Ile Cys Ser Leu Thr Gly Asn Arg Gly Asn Ile
            260                 265                 270
Val Phe Tyr Asn Asn Arg Cys Phe Lys Asn Val Glu Thr Ala Ser Ser
        275                 280                 285
Glu Ala Ser Asp Gly Gly Ala Ile Lys Val Thr Thr Arg Leu Asp Val
    290                 295                 300
Thr Gly Asn Arg Gly Arg Ile Phe Phe Ser Asp Asn Ile Thr Lys Asn
305                 310                 315                 320
Tyr Gly Gly Ala Ile Tyr Ala Pro Val Val Thr Leu Val Asp Asn Gly
                325                 330                 335
Pro Thr Tyr Phe Ile Asn Asn Ile Ala Asn Asn Lys Gly Gly Ala Ile
            340                 345                 350
Tyr Ile Asp Gly Thr Ser Asn Ser Lys Ile Ser Ala Asp Arg His Ala
        355                 360                 365
Ile Ile Phe Asn Glu Asn Ile Val Thr Asn Val Thr Asn Ala Asn Gly
    370                 375                 380
Thr Ser Thr Ser Ala Asn Pro Pro Arg Arg Asn Ala Ile Thr Val Ala
385                 390                 395                 400
Ser Ser Ser Gly Glu Ile Leu Leu Gly Ala Gly Ser Ser Gln Asn Leu
                405                 410                 415
Ile Phe Tyr Asp Pro Ile Glu Val Ser Asn Ala Gly Val Ser Val Ser
            420                 425                 430
Phe Asn Lys Glu Ala Asp Gln Thr Gly Ser Val Val Phe Ser Gly Ala
        435                 440                 445
Thr Val Asn Ser Ala Asp Phe His Gln Arg Asn Leu Gln Thr Lys Thr
    450                 455                 460
Pro Ala Pro Leu Thr Leu Ser Asn Gly Phe Leu Cys Ile Glu Asp His
465                 470                 475                 480
Ala Gln Leu Thr Val Asn Arg Phe Thr Gln Thr Gly Gly Val Val Ser
                485                 490                 495
Leu Gly Asn Gly Ala Val Leu Ser Cys Tyr Lys Asn Gly Thr Gly Asp
            500                 505                 510
Ser Ala Ser Asn Ala Ser Ile Thr Leu Lys His Ile Gly Leu Asn Leu
        515                 520                 525
Ser Ser Ile Leu Lys Ser Gly Ala Glu Ile Pro Leu Leu Trp Val Glu
    530                 535                 540
Pro Thr Asn Asn Ser Asn Asn Tyr Thr Ala Asp Thr Ala Ala Thr Phe
545                 550                 555                 560
Ser Leu Ser Asp Val Lys Leu Ser Leu Ile Asp Asp Tyr Gly Asn Ser
                565                 570                 575
Pro Tyr Glu Ser Thr Asp Leu Thr His Ala Leu Ser Ser Gln Pro Met
            580                 585                 590
Leu Ser Ile Ser Glu Ala Ser Asp Asn Gln Leu Gln Ser Glu Asn Ile
        595                 600                 605
Asp Phe Ser Gly Leu Asn Val Pro His Tyr Gly Trp Gln Gly Leu Trp
    610                 615                 620
Thr Trp Gly Trp Ala Lys Thr Gln Asp Pro Glu Pro Ala Ser Ser Ala
625                 630                 635                 640
Thr Ile Thr Asp Pro Gln Lys Ala Asn Arg Phe His Arg Thr Leu Leu
                645                 650                 655
Leu Thr Trp Leu Pro Ala Gly Tyr Val Pro Ser Pro Lys His Arg Ser
            660                 665                 670
Pro Leu Ile Ala Asn Thr Leu Trp Gly Asn Met Leu Leu Ala Thr Glu
        675                 680                 685
Ser Leu Lys Asn Ser Ala Glu Leu Thr Pro Ser Gly His Pro Phe Trp

690                 695                 700
Gly Ile Thr Gly Gly Gly Leu Gly Met Met Val Tyr Gln Asp Pro Arg
705                 710                 715                 720
Glu Asn His Pro Gly Phe His Met Arg Ser Ser Gly Tyr Ser Ala Gly
                725                 730                 735
Met Ile Ala Gly Gln Thr His Thr Phe Ser Leu Lys Phe Ser Gln Thr
            740                 745                 750
Tyr Thr Lys Leu Asn Glu Arg Tyr Ala Lys Asn Asn Val Ser Ser Lys
        755                 760                 765
Asn Tyr Ser Cys Gln Gly Glu Met Leu Phe Ser Leu Gln Glu Gly Phe
    770                 775                 780
Leu Leu Thr Lys Leu Val Gly Leu Tyr Ser Tyr Gly Asp His Asn Cys
785                 790                 795                 800
His His Phe Tyr Thr Gln Gly Glu Asn Leu Thr Ser Gln Gly Thr Phe
                805                 810                 815
Arg Ser Gln Thr Met Gly Gly Ala Val Phe Phe Asp Leu Pro Met Lys
            820                 825                 830
Pro Phe Gly Ser Thr His Ile Leu Thr Ala Pro Phe Leu Gly Ala Leu
        835                 840                 845
Gly Ile Tyr Ser Ser Leu Ser His Phe Thr Glu Val Gly Ala Tyr Pro
    850                 855                 860
Arg Ser Phe Ser Thr Lys Thr Pro Leu Ile Asn Val Leu Val Pro Ile
865                 870                 875                 880
Gly Val Lys Gly Ser Phe Met Asn Ala Thr His Arg Pro Gln Ala Trp
                885                 890                 895
Thr Val Glu Leu Ala Tyr Gln Pro Val Leu Tyr Arg Gln Glu Pro Gly
            900                 905                 910
Ile Ala Thr Gln Leu Leu Ala Ser Lys Gly Ile Trp Phe Gly Ser Gly
        915                 920                 925
Ser Pro Ser Ser Arg His Ala Met Ser Tyr Lys Ile Ser Gln Gln Thr
    930                 935                 940
Gln Pro Leu Ser Trp Leu Thr Leu His Phe Gln Tyr His Gly Phe Tyr
945                 950                 955                 960
Ser Ser Ser Thr Phe Cys Asn Tyr Leu Asn Gly Glu Ile Ala Leu Arg
                965                 970                 975
Phe

<210> 192
<211> 848
<212> PRT
<213> Chlamydia

<400> 192
Met Ala Ser His His His His His His Gly Ala Ile Ser Cys Leu Arg
 1               5                  10                  15
Gly Asp Val Val Ile Ser Gly Asn Lys Gly Arg Val Glu Phe Lys Asp
            20                  25                  30
Asn Ile Ala Thr Arg Leu Tyr Val Glu Glu Thr Val Glu Lys Val Glu
        35                  40                  45
Glu Val Glu Pro Ala Pro Glu Gln Lys Asp Asn Asn Glu Leu Ser Phe
    50                  55                  60
Leu Gly Ser Val Glu Gln Ser Phe Ile Thr Ala Ala Asn Gln Ala Leu
65                  70                  75                  80
Phe Ala Ser Glu Asp Gly Asp Leu Ser Pro Glu Ser Ser Ile Ser Ser

                85                  90                  95
Glu Glu Leu Ala Lys Arg Arg Glu Cys Ala Gly Gly Ala Ile Phe Ala
            100                 105                 110
Lys Arg Val Arg Ile Val Asp Asn Gln Glu Ala Val Val Phe Ser Asn
        115                 120                 125
Asn Phe Ser Asp Ile Tyr Gly Gly Ala Ile Phe Thr Gly Ser Leu Arg
    130                 135                 140
Glu Glu Asp Lys Leu Asp Gly Gln Ile Pro Glu Val Leu Ile Ser Gly
145                 150                 155                 160
Asn Ala Gly Asp Val Val Phe Ser Gly Asn Ser Ser Lys Arg Asp Glu
                165                 170                 175
His Leu Pro His Thr Gly Gly Gly Ala Ile Cys Thr Gln Asn Leu Thr
            180                 185                 190
Ile Ser Gln Asn Thr Gly Asn Val Leu Phe Tyr Asn Asn Val Ala Cys
        195                 200                 205
Ser Gly Gly Ala Val Arg Ile Glu Asp His Gly Asn Val Leu Leu Glu
    210                 215                 220
Ala Phe Gly Gly Asp Ile Val Phe Lys Gly Asn Ser Ser Phe Arg Ala
225                 230                 235                 240
Gln Gly Ser Asp Ala Ile Tyr Phe Ala Gly Lys Glu Ser His Ile Thr
                245                 250                 255
Ala Leu Asn Ala Thr Glu Gly His Ala Ile Val Phe His Asp Ala Leu
            260                 265                 270
Val Phe Glu Asn Leu Lys Glu Arg Lys Ser Ala Glu Val Leu Leu Ile
        275                 280                 285
Asn Ser Arg Glu Asn Pro Gly Tyr Thr Gly Ser Ile Arg Phe Leu Glu
    290                 295                 300
Ala Glu Ser Lys Val Pro Gln Cys Ile His Val Gln Gln Gly Ser Leu
305                 310                 315                 320
Glu Leu Leu Asn Gly Ala Thr Leu Cys Ser Tyr Gly Phe Lys Gln Asp
                325                 330                 335
Ala Gly Ala Lys Leu Val Leu Ala Ala Gly Ser Lys Leu Lys Ile Leu
            340                 345                 350
Asp Ser Gly Thr Pro Val Gln Gly His Ala Ile Ser Lys Pro Glu Ala
        355                 360                 365
Glu Ile Glu Ser Ser Ser Glu Pro Glu Gly Ala His Ser Leu Trp Ile
    370                 375                 380
Ala Lys Asn Ala Gln Thr Thr Val Pro Met Val Asp Ile His Thr Ile
385                 390                 395                 400
Ser Val Asp Leu Ala Ser Phe Ser Ser Ser Gln Gln Glu Gly Thr Val
                405                 410                 415
Glu Ala Pro Gln Val Ile Val Pro Gly Gly Ser Tyr Val Arg Ser Gly
            420                 425                 430
Glu Leu Asn Leu Glu Leu Val Asn Thr Thr Gly Thr Gly Tyr Glu Asn
        435                 440                 445
His Ala Leu Leu Lys Asn Glu Ala Lys Val Pro Leu Met Ser Phe Val
    450                 455                 460
Ala Ser Ser Asp Glu Ala Ser Ala Glu Ile Ser Asn Leu Ser Val Ser
465                 470                 475                 480
Asp Leu Gln Ile His Val Ala Thr Pro Glu Ile Glu Glu Asp Thr Tyr
                485                 490                 495
Gly His Met Gly Asp Trp Ser Glu Ala Lys Ile Gln Asp Gly Thr Leu
            500                 505                 510
Val Ile Asn Trp Asn Pro Thr Gly Tyr Arg Leu Asp Pro Gln Lys Ala
        515                 520                 525

Gly Ala Leu Val Phe Asn Ala Leu Trp Glu Glu Gly Ala Val Leu Ser
    530                 535                 540
Ala Leu Lys Asn Ala Arg Phe Ala His Asn Leu Thr Ala Gln Arg Met
545                 550                 555                 560
Glu Phe Asp Tyr Ser Thr Asn Val Trp Gly Phe Ala Phe Gly Gly Phe
                565                 570                 575
Arg Thr Leu Ser Ala Glu Asn Leu Val Ala Ile Asp Gly Tyr Lys Gly
            580                 585                 590
Ala Tyr Gly Gly Ala Ser Ala Gly Val Asp Ile Gln Leu Met Glu Asp
        595                 600                 605
Phe Val Leu Gly Val Ser Gly Ala Ala Phe Leu Gly Lys Met Asp Ser
    610                 615                 620
Gln Lys Phe Asp Ala Glu Val Ser Arg Lys Gly Val Val Gly Ser Val
625                 630                 635                 640
Tyr Thr Gly Phe Leu Ala Gly Ser Trp Phe Phe Lys Gly Gln Tyr Ser
                645                 650                 655
Leu Gly Glu Thr Gln Asn Asp Met Lys Thr Arg Tyr Gly Val Leu Gly
            660                 665                 670
Glu Ser Ser Ala Ser Trp Thr Ser Arg Gly Val Leu Ala Asp Ala Leu
        675                 680                 685
Val Glu Tyr Arg Ser Leu Val Gly Pro Val Arg Pro Thr Phe Tyr Ala
    690                 695                 700
Leu His Phe Asn Pro Tyr Val Glu Val Ser Tyr Ala Ser Met Lys Phe
705                 710                 715                 720
Pro Gly Phe Thr Glu Gln Gly Arg Glu Ala Arg Ser Phe Glu Asp Ala
                725                 730                 735
Ser Leu Thr Asn Ile Thr Ile Pro Leu Gly Met Lys Phe Glu Leu Ala
            740                 745                 750
Phe Ile Lys Gly Gln Phe Ser Glu Val Asn Ser Leu Gly Ile Ser Tyr
        755                 760                 765
Ala Trp Glu Ala Tyr Arg Lys Val Glu Gly Gly Ala Val Gln Leu Leu
    770                 775                 780
Glu Ala Gly Phe Asp Trp Glu Gly Ala Pro Met Asp Leu Pro Arg Gln
785                 790                 795                 800
Glu Leu Arg Val Ala Leu Glu Asn Asn Thr Glu Trp Ser Ser Tyr Phe
                805                 810                 815
Ser Thr Val Leu Gly Leu Thr Ala Phe Cys Gly Gly Phe Thr Ser Thr
            820                 825                 830
Asp Ser Lys Leu Gly Tyr Glu Ala Asn Thr Gly Leu Arg Leu Ile Phe
        835                 840                 845

<210> 193
<211> 778
<212> PRT
<213> Chlamydia

<400> 193
Met His His His His His His Gly Leu Ala Ser Cys Val Asp Leu His
1               5                   10                  15
Ala Gly Gly Gln Ser Val Asn Glu Leu Val Tyr Val Gly Pro Gln Ala
            20                  25                  30
Val Leu Leu Leu Asp Gln Ile Arg Asp Leu Phe Val Gly Ser Lys Asp
        35                  40                  45
Ser Gln Ala Glu Gly Gln Tyr Arg Leu Ile Val Gly Asp Pro Ser Ser
    50                  55                  60

Phe Gln Glu Lys Asp Ala Asp Thr Leu Pro Gly Lys Val Glu Gln Ser
65                  70                  75                  80
Thr Leu Phe Ser Val Thr Asn Pro Val Val Phe Gln Gly Val Asp Gln
                85                  90                  95
Gln Asp Gln Val Ser Ser Gln Gly Leu Ile Cys Ser Phe Thr Ser Ser
            100                 105                 110
Asn Leu Asp Ser Pro Arg Asp Gly Glu Ser Phe Leu Gly Ile Ala Phe
        115                 120                 125
Val Gly Asp Ser Ser Lys Ala Gly Ile Thr Leu Thr Asp Val Lys Ala
    130                 135                 140
Ser Leu Ser Gly Ala Ala Leu Tyr Ser Thr Glu Asp Leu Ile Phe Glu
145                 150                 155                 160
Lys Ile Lys Gly Gly Leu Glu Phe Ala Ser Cys Ser Ser Leu Glu Gln
                165                 170                 175
Gly Gly Ala Cys Ala Ala Gln Ser Ile Leu Ile His Asp Cys Gln Gly
            180                 185                 190
Leu Gln Val Lys His Cys Thr Thr Ala Val Asn Ala Glu Gly Ser Ser
        195                 200                 205
Ala Asn Asp His Leu Gly Phe Gly Gly Gly Ala Phe Phe Val Thr Gly
    210                 215                 220
Ser Leu Ser Gly Glu Lys Ser Leu Tyr Met Pro Ala Gly Asp Met Val
225                 230                 235                 240
Val Ala Asn Cys Asp Gly Ala Ile Ser Phe Glu Gly Asn Ser Ala Asn
                245                 250                 255
Phe Ala Asn Gly Gly Ala Ile Ala Ala Ser Gly Lys Val Leu Phe Val
            260                 265                 270
Ala Asn Asp Lys Lys Thr Ser Phe Ile Glu Asn Arg Ala Leu Ser Gly
        275                 280                 285
Gly Ala Ile Ala Ala Ser Ser Asp Ile Ala Phe Gln Asn Cys Ala Glu
    290                 295                 300
Leu Val Phe Lys Gly Asn Cys Ala Ile Gly Thr Glu Asp Lys Gly Ser
305                 310                 315                 320
Leu Gly Gly Gly Ala Ile Ser Ser Leu Gly Thr Val Leu Leu Gln Gly
                325                 330                 335
Asn His Gly Ile Thr Cys Asp Lys Asn Glu Ser Ala Ser Gln Gly Gly
            340                 345                 350
Ala Ile Phe Gly Lys Asn Cys Gln Ile Ser Asp Asn Glu Gly Pro Val
        355                 360                 365
Val Phe Arg Asp Ser Thr Ala Cys Leu Gly Gly Gly Ala Ile Ala Ala
    370                 375                 380
Gln Glu Ile Val Ser Ile Gln Asn Asn Gln Ala Gly Ile Ser Phe Glu
385                 390                 395                 400
Gly Gly Lys Ala Ser Phe Gly Gly Gly Ile Ala Cys Gly Ser Phe Ser
                405                 410                 415
Ser Ala Gly Gly Ala Ser Val Leu Gly Thr Ile Asp Ile Ser Lys Asn
            420                 425                 430
Leu Gly Ala Ile Ser Phe Ser Arg Thr Leu Cys Thr Thr Ser Asp Leu
        435                 440                 445
Gly Gln Met Glu Tyr Gln Gly Gly Gly Ala Leu Phe Gly Glu Asn Ile
    450                 455                 460
Ser Leu Ser Glu Asn Ala Gly Val Leu Thr Phe Lys Asp Asn Ile Val
465                 470                 475                 480
Lys Thr Phe Ala Ser Asn Gly Lys Ile Leu Gly Gly Gly Ala Ile Leu
                485                 490                 495
Ala Thr Gly Lys Val Glu Ile Thr Asn Asn Ser Gly Gly Ile Ser Phe

500                 505                 510
Thr Gly Asn Ala Arg Ala Pro Gln Ala Leu Pro Thr Gln Glu Glu Phe
        515                 520                 525
Pro Leu Phe Ser Lys Lys Glu Gly Arg Pro Leu Ser Ser Gly Tyr Ser
    530                 535                 540
Gly Gly Gly Ala Ile Leu Gly Arg Glu Val Ala Ile Leu His Asn Ala
545                 550                 555                 560
Ala Val Val Phe Glu Gln Asn Arg Leu Gln Cys Ser Glu Glu Glu Ala
                565                 570                 575
Thr Leu Leu Gly Cys Cys Gly Gly Gly Ala Val His Gly Met Asp Ser
            580                 585                 590
Thr Ser Ile Val Gly Asn Ser Ser Val Arg Phe Gly Asn Asn Tyr Ala
        595                 600                 605
Met Gly Gln Gly Val Ser Gly Gly Ala Leu Leu Ser Lys Thr Val Gln
    610                 615                 620
Leu Ala Gly Asn Gly Ser Val Asp Phe Ser Arg Asn Ile Ala Ser Leu
625                 630                 635                 640
Gly Gly Gly Ala Leu Gln Ala Ser Glu Gly Asn Cys Glu Leu Val Asp
                645                 650                 655
Asn Gly Tyr Val Leu Phe Arg Asp Asn Arg Gly Arg Val Tyr Gly Gly
            660                 665                 670
Ala Ile Ser Cys Leu Arg Gly Asp Val Val Ile Ser Gly Asn Lys Gly
        675                 680                 685
Arg Val Glu Phe Lys Asp Asn Ile Ala Thr Arg Leu Tyr Val Glu Glu
    690                 695                 700
Thr Val Glu Lys Val Glu Glu Val Glu Pro Ala Pro Glu Gln Lys Asp
705                 710                 715                 720
Asn Asn Glu Leu Ser Phe Leu Gly Ser Val Glu Gln Ser Phe Ile Thr
                725                 730                 735
Ala Ala Asn Gln Ala Leu Phe Ala Ser Glu Asp Gly Asp Leu Ser Pro
            740                 745                 750
Glu Ser Ser Ile Ser Ser Glu Glu Leu Ala Lys Arg Arg Glu Cys Ala
        755                 760                 765
Gly Gly Ala Asp Ser Ser Arg Ser Gly Cys
    770                 775

<210> 194
<211> 948
<212> PRT
<213> Chlamydia

<400> 194
Met Ala Ser Met His His His His His His Val Lys Ile Glu Asn Phe
 1               5                  10                  15
Ser Gly Gln Gly Ile Phe Ser Gly Asn Lys Ala Ile Asp Asn Thr Thr
            20                  25                  30
Glu Gly Ser Ser Ser Lys Ser Asn Val Leu Gly Gly Ala Val Tyr Ala
        35                  40                  45
Lys Thr Leu Phe Asn Leu Asp Ser Gly Ser Ser Arg Arg Thr Val Thr
    50                  55                  60
Phe Ser Gly Asn Thr Val Ser Ser Gln Ser Thr Thr Gly Gln Val Ala
65                  70                  75                  80
Gly Gly Ala Ile Tyr Ser Pro Thr Val Thr Ile Ala Thr Pro Val Val
                85                  90                  95
Phe Ser Lys Asn Ser Ala Thr Asn Asn Ala Asn Asn Ala Thr Asp Thr

            100                 105                 110
Gln Arg Lys Asp Thr Phe Gly Gly Ala Ile Gly Ala Thr Ser Ala Val
        115                 120                 125
Ser Leu Ser Gly Gly Ala His Phe Leu Glu Asn Val Ala Asp Leu Gly
    130                 135                 140
Ser Ala Ile Gly Leu Val Pro Asp Thr Gln Asn Thr Glu Thr Val Lys
145                 150                 155                 160
Leu Glu Ser Gly Ser Tyr Tyr Phe Glu Lys Asn Lys Ala Leu Lys Arg
                165                 170                 175
Ala Thr Ile Tyr Ala Pro Val Val Ser Ile Lys Ala Tyr Thr Ala Thr
            180                 185                 190
Phe Asn Gln Asn Arg Ser Leu Glu Glu Gly Ser Ala Ile Tyr Phe Thr
        195                 200                 205
Lys Glu Ala Ser Ile Glu Ser Leu Gly Ser Val Leu Phe Thr Gly Asn
    210                 215                 220
Leu Val Thr Pro Thr Leu Ser Thr Thr Thr Glu Gly Thr Pro Ala Thr
225                 230                 235                 240
Thr Ser Gly Asp Val Thr Lys Tyr Gly Ala Ala Ile Phe Gly Gln Ile
                245                 250                 255
Ala Ser Ser Asn Gly Ser Gln Thr Asp Asn Leu Pro Leu Lys Leu Ile
            260                 265                 270
Ala Ser Gly Gly Asn Ile Cys Phe Arg Asn Asn Glu Tyr Arg Pro Thr
        275                 280                 285
Ser Ser Asp Thr Gly Thr Ser Thr Phe Cys Ser Ile Ala Gly Asp Val
    290                 295                 300
Lys Leu Thr Met Gln Ala Ala Lys Gly Lys Thr Ile Ser Phe Phe Asp
305                 310                 315                 320
Ala Ile Arg Thr Ser Thr Lys Lys Thr Gly Thr Gln Ala Thr Ala Tyr
                325                 330                 335
Asp Thr Leu Asp Ile Asn Lys Ser Glu Asp Ser Glu Thr Val Asn Ser
            340                 345                 350
Ala Phe Thr Gly Thr Ile Leu Phe Ser Ser Glu Leu His Glu Asn Lys
        355                 360                 365
Ser Tyr Ile Pro Gln Asn Val Val Leu His Ser Gly Ser Leu Val Leu
    370                 375                 380
Lys Pro Asn Thr Glu Leu His Val Ile Ser Phe Glu Gln Lys Glu Gly
385                 390                 395                 400
Ser Ser Leu Val Met Thr Pro Gly Ser Val Leu Ser Asn Gln Thr Val
                405                 410                 415
Ala Asp Gly Ala Leu Val Ile Asn Asn Met Thr Ile Asp Leu Ser Ser
            420                 425                 430
Val Glu Lys Asn Gly Ile Ala Glu Gly Asn Ile Phe Thr Pro Pro Glu
        435                 440                 445
Leu Arg Ile Ile Asp Thr Thr Thr Ser Gly Ser Gly Gly Thr Pro Ser
    450                 455                 460
Thr Asp Ser Glu Ser Asn Gln Asn Ser Asp Asp Thr Lys Glu Gln Asn
465                 470                 475                 480
Asn Asn Asp Ala Ser Asn Gln Gly Glu Ser Ala Asn Gly Ser Ser Ser
                485                 490                 495
Pro Ala Val Ala Ala Ala His Thr Ser Arg Thr Arg Asn Phe Ala Ala
            500                 505                 510
Ala Ala Thr Ala Thr Pro Thr Thr Thr Pro Thr Ala Thr Thr Thr Thr
        515                 520                 525
Ser Asn Gln Val Ile Leu Gly Gly Glu Ile Lys Leu Ile Asp Pro Asn
    530                 535                 540

Gly Thr Phe Phe Gln Asn Pro Ala Leu Arg Ser Asp Gln Gln Ile Ser
545                 550                 555                 560
Leu Leu Val Leu Pro Thr Asp Ser Ser Lys Met Gln Ala Gln Lys Ile
                565                 570                 575
Val Leu Thr Gly Asp Ile Ala Pro Gln Lys Gly Tyr Thr Gly Thr Leu
            580                 585                 590
Thr Leu Asp Pro Asp Gln Leu Gln Asn Gly Thr Ile Ser Ala Leu Trp
        595                 600                 605
Lys Phe Asp Ser Tyr Arg Gln Trp Ala Tyr Val Pro Arg Asp Asn His
    610                 615                 620
Phe Tyr Ala Asn Ser Ile Leu Gly Ser Gln Met Ser Met Val Thr Val
625                 630                 635                 640
Lys Gln Gly Leu Leu Asn Asp Lys Met Asn Leu Ala Arg Phe Asp Glu
                645                 650                 655
Val Ser Tyr Asn Asn Leu Trp Ile Ser Gly Leu Gly Thr Met Leu Ser
            660                 665                 670
Gln Val Gly Thr Pro Thr Ser Glu Glu Phe Thr Tyr Tyr Ser Arg Gly
        675                 680                 685
Ala Ser Val Ala Leu Asp Ala Lys Pro Ala His Asp Val Ile Val Gly
    690                 695                 700
Ala Ala Phe Ser Lys Met Ile Gly Lys Thr Lys Ser Leu Lys Arg Glu
705                 710                 715                 720
Asn Asn Tyr Thr His Lys Gly Ser Glu Tyr Ser Tyr Gln Ala Ser Val
                725                 730                 735
Tyr Gly Gly Lys Pro Phe His Phe Val Ile Asn Lys Lys Thr Glu Lys
            740                 745                 750
Ser Leu Pro Leu Leu Leu Gln Gly Val Ile Ser Tyr Gly Tyr Ile Lys
        755                 760                 765
His Asp Thr Val Thr His Tyr Pro Thr Ile Arg Glu Arg Asn Gln Gly
    770                 775                 780
Glu Trp Glu Asp Leu Gly Trp Leu Thr Ala Leu Arg Val Ser Ser Val
785                 790                 795                 800
Leu Arg Thr Pro Ala Gln Gly Asp Thr Lys Arg Ile Thr Val Tyr Gly
                805                 810                 815
Glu Leu Glu Tyr Ser Ser Ile Arg Gln Lys Gln Phe Thr Glu Thr Glu
            820                 825                 830
Tyr Asp Pro Arg Tyr Phe Asp Asn Cys Thr Tyr Arg Asn Leu Ala Ile
        835                 840                 845
Pro Met Gly Leu Ala Phe Glu Gly Glu Leu Ser Gly Asn Asp Ile Leu
    850                 855                 860
Met Tyr Asn Arg Phe Ser Val Ala Tyr Met Pro Ser Ile Tyr Arg Asn
865                 870                 875                 880
Ser Pro Thr Cys Lys Tyr Gln Val Leu Ser Ser Gly Glu Gly Gly Glu
                885                 890                 895
Ile Ile Cys Gly Val Pro Thr Arg Asn Ser Ala Arg Gly Glu Tyr Ser
            900                 905                 910
Thr Gln Leu Tyr Pro Gly Pro Leu Trp Thr Leu Tyr Gly Ser Tyr Thr
        915                 920                 925
Ile Glu Ala Asp Ala His Thr Leu Ala His Met Met Asn Cys Gly Ala
    930                 935                 940
Arg Met Thr Phe
945

<210> 195
<211> 821

<212> PRT
<213> Chlamydia

<400> 195
Met His His His His His His Glu Ala Ser Ser Ile Gln Asp Gln Ile
1               5                   10                  15
Lys Asn Thr Asp Cys Asn Val Ser Lys Val Gly Tyr Ser Thr Ser Gln
            20                  25                  30
Ala Phe Thr Asp Met Met Leu Ala Asp Asn Thr Glu Tyr Arg Ala Ala
        35                  40                  45
Asp Ser Val Ser Phe Tyr Asp Phe Ser Thr Ser Ser Gly Leu Pro Arg
    50                  55                  60
Lys His Leu Ser Ser Ser Ser Glu Ala Ser Pro Thr Thr Glu Gly Val
65                  70                  75                  80
Ser Ser Ser Ser Ser Gly Glu Asn Thr Glu Asn Ser Gln Asp Ser Ala
                85                  90                  95
Pro Ser Ser Gly Glu Thr Asp Lys Lys Thr Glu Glu Glu Leu Asp Asn
            100                 105                 110
Gly Gly Ile Ile Tyr Ala Arg Glu Lys Leu Thr Ile Ser Glu Ser Gln
        115                 120                 125
Asp Ser Leu Ser Asn Pro Ser Ile Glu Leu His Asp Asn Ser Phe Phe
    130                 135                 140
Phe Gly Glu Gly Glu Val Ile Phe Asp His Arg Val Ala Leu Lys Asn
145                 150                 155                 160
Gly Gly Ala Ile Tyr Gly Glu Lys Glu Val Val Phe Glu Asn Ile Lys
                165                 170                 175
Ser Leu Leu Val Glu Val Asn Ile Ser Val Glu Lys Gly Gly Ser Val
            180                 185                 190
Tyr Ala Lys Glu Arg Val Ser Leu Glu Asn Val Thr Glu Ala Thr Phe
        195                 200                 205
Ser Ser Asn Gly Gly Glu Gln Gly Gly Gly Gly Ile Tyr Ser Glu Gln
    210                 215                 220
Asp Met Leu Ile Ser Asp Cys Asn Asn Val His Phe Gln Gly Asn Ala
225                 230                 235                 240
Ala Gly Ala Thr Ala Val Lys Gln Cys Leu Asp Glu Glu Met Ile Val
                245                 250                 255
Leu Leu Thr Glu Cys Val Asp Ser Leu Ser Glu Asp Thr Leu Asp Ser
            260                 265                 270
Thr Pro Glu Thr Glu Gln Thr Lys Ser Asn Gly Asn Gln Asp Gly Ser
        275                 280                 285
Ser Glu Thr Lys Asp Thr Gln Val Ser Glu Ser Pro Glu Ser Thr Pro
    290                 295                 300
Ser Pro Asp Asp Val Leu Gly Lys Gly Gly Gly Ile Tyr Thr Glu Lys
305                 310                 315                 320
Ser Leu Thr Ile Thr Gly Ile Thr Gly Thr Ile Asp Phe Val Ser Asn
                325                 330                 335
Ile Ala Thr Asp Ser Gly Ala Gly Val Phe Thr Lys Glu Asn Leu Ser
            340                 345                 350
Cys Thr Asn Thr Asn Ser Leu Gln Phe Leu Lys Asn Ser Ala Gly Gln
        355                 360                 365
His Gly Gly Gly Ala Tyr Val Thr Gln Thr Met Ser Val Thr Asn Thr
    370                 375                 380
Thr Ser Glu Ser Ile Thr Thr Pro Pro Leu Val Gly Glu Val Ile Phe
385                 390                 395                 400
Ser Glu Asn Thr Ala Lys Gly His Gly Gly Gly Ile Cys Thr Asn Lys

                405                 410                 415
Leu Ser Leu Ser Asn Leu Lys Thr Val Thr Leu Thr Lys Asn Ser Ala
            420                 425                 430
Lys Glu Ser Gly Gly Ala Ile Phe Thr Asp Leu Ala Ser Ile Pro Thr
        435                 440                 445
Thr Asp Thr Pro Glu Ser Ser Thr Pro Ser Ser Ser Ser Pro Ala Ser
    450                 455                 460
Thr Pro Glu Val Val Ala Ser Ala Lys Ile Asn Arg Phe Phe Ala Ser
465                 470                 475                 480
Thr Ala Glu Pro Ala Ala Pro Ser Leu Thr Glu Ala Glu Ser Asp Gln
                485                 490                 495
Thr Asp Gln Thr Glu Thr Ser Asp Thr Asn Ser Asp Ile Asp Val Ser
            500                 505                 510
Ile Glu Asn Ile Leu Asn Val Ala Ile Asn Gln Asn Thr Ser Ala Lys
        515                 520                 525
Lys Gly Gly Ala Ile Tyr Gly Lys Lys Ala Lys Leu Ser Arg Ile Asn
    530                 535                 540
Asn Leu Glu Leu Ser Gly Asn Ser Ser Gln Asp Val Gly Gly Gly Leu
545                 550                 555                 560
Cys Leu Thr Glu Ser Val Glu Phe Asp Ala Ile Gly Ser Leu Leu Ser
                565                 570                 575
His Tyr Asn Ser Ala Ala Lys Glu Gly Gly Val Ile His Ser Lys Thr
            580                 585                 590
Val Thr Leu Ser Asn Leu Lys Ser Thr Phe Thr Phe Ala Asp Asn Thr
        595                 600                 605
Val Lys Ala Ile Val Glu Ser Thr Pro Glu Ala Pro Glu Glu Ile Pro
    610                 615                 620
Pro Val Glu Gly Glu Glu Ser Thr Ala Thr Glu Asn Pro Asn Ser Asn
625                 630                 635                 640
Thr Glu Gly Ser Ser Ala Asn Thr Asn Leu Glu Gly Ser Gln Gly Asp
                645                 650                 655
Thr Ala Asp Thr Gly Thr Gly Val Val Asn Asn Glu Ser Gln Asp Thr
            660                 665                 670
Ser Asp Thr Gly Asn Ala Glu Ser Gly Glu Gln Leu Gln Asp Ser Thr
        675                 680                 685
Gln Ser Asn Glu Glu Asn Thr Leu Pro Asn Ser Ser Ile Asp Gln Ser
    690                 695                 700
Asn Glu Asn Thr Asp Glu Ser Ser Asp Ser His Thr Glu Glu Ile Thr
705                 710                 715                 720
Asp Glu Ser Val Ser Ser Ser Ser Lys Ser Gly Ser Ser Thr Pro Gln
                725                 730                 735
Asp Gly Gly Ala Ala Ser Ser Gly Ala Pro Ser Gly Asp Gln Ser Ile
            740                 745                 750
Ser Ala Asn Ala Cys Leu Ala Lys Ser Tyr Ala Ala Ser Thr Asp Ser
        755                 760                 765
Ser Pro Val Ser Asn Ser Ser Gly Ser Asp Val Thr Ala Ser Ser Asp
    770                 775                 780
Asn Pro Asp Ser Ser Ser Ser Gly Asp Ser Ala Gly Asp Ser Glu Gly
785                 790                 795                 800
Pro Thr Glu Pro Glu Ala Gly Ser Thr Thr Glu Thr Pro Thr Leu Ile
                805                 810                 815
Gly Gly Gly Ala Ile
            820

<210> 196

<211> 525
<212> PRT
<213> Chlamydia

<400> 196
Met His His His His His His Thr Ala Ala Ser Asp Asn Phe Gln Leu
1               5                   10                  15
Ser Gln Gly Gly Gln Gly Phe Ala Ile Pro Ile Gly Gln Ala Met Ala
            20                  25                  30
Ile Ala Gly Gln Ile Lys Leu Pro Thr Val His Ile Gly Pro Thr Ala
        35                  40                  45
Phe Leu Gly Leu Gly Val Val Asp Asn Asn Gly Asn Gly Ala Arg Val
    50                  55                  60
Gln Arg Val Val Gly Ser Ala Pro Ala Ala Ser Leu Gly Ile Ser Thr
65                  70                  75                  80
Gly Asp Val Ile Thr Ala Val Asp Gly Ala Pro Ile Asn Ser Ala Thr
                85                  90                  95
Ala Met Ala Asp Ala Leu Asn Gly His His Pro Gly Asp Val Ile Ser
            100                 105                 110
Val Thr Trp Gln Thr Lys Ser Gly Gly Thr Arg Thr Gly Asn Val Thr
        115                 120                 125
Leu Ala Glu Gly Pro Pro Ala Glu Phe Pro Leu Val Pro Arg Gly Ser
    130                 135                 140
Pro Leu Pro Val Gly Asn Pro Ala Glu Pro Ser Leu Leu Ile Asp Gly
145                 150                 155                 160
Thr Met Trp Glu Gly Ala Ser Gly Asp Pro Cys Asp Pro Cys Ala Thr
                165                 170                 175
Trp Cys Asp Ala Ile Ser Ile Arg Ala Gly Tyr Tyr Gly Asp Tyr Val
            180                 185                 190
Phe Asp Arg Val Leu Lys Val Asp Val Asn Lys Thr Phe Ser Gly Met
        195                 200                 205
Ala Ala Thr Pro Thr Gln Ala Ile Gly Asn Ala Ser Asn Thr Asn Gln
    210                 215                 220
Pro Glu Ala Asn Gly Arg Pro Asn Ile Ala Tyr Gly Arg His Met Gln
225                 230                 235                 240
Asp Ala Glu Trp Phe Ser Asn Ala Ala Phe Leu Ala Leu Asn Ile Trp
                245                 250                 255
Asp Arg Phe Asp Ile Phe Cys Thr Leu Gly Ala Ser Asn Gly Tyr Phe
            260                 265                 270
Lys Ala Ser Ser Ala Ala Phe Asn Leu Val Gly Leu Ile Gly Phe Ser
        275                 280                 285
Ala Ala Ser Ser Ile Ser Thr Asp Leu Pro Met Gln Leu Pro Asn Val
    290                 295                 300
Gly Ile Thr Gln Gly Val Val Glu Phe Tyr Thr Asp Thr Ser Phe Ser
305                 310                 315                 320
Trp Ser Val Gly Ala Arg Gly Ala Leu Trp Glu Cys Gly Cys Ala Thr
                325                 330                 335
Leu Gly Ala Glu Phe Gln Tyr Ala Gln Ser Asn Pro Lys Ile Glu Met
            340                 345                 350
Leu Asn Val Thr Ser Ser Pro Ala Gln Phe Val Ile His Lys Pro Arg
        355                 360                 365
Gly Tyr Lys Gly Ala Ser Ser Asn Phe Pro Leu Pro Ile Thr Ala Gly
    370                 375                 380
Thr Thr Glu Ala Thr Asp Thr Lys Ser Ala Thr Ile Lys Tyr His Glu
385                 390                 395                 400

Trp Gln Val Gly Leu Ala Leu Ser Tyr Arg Leu Asn Met Leu Val Pro
                405                 410                 415
Tyr Ile Gly Val Asn Trp Ser Arg Ala Thr Phe Asp Ala Asp Thr Ile
            420                 425                 430
Arg Ile Ala Gln Pro Lys Leu Lys Ser Glu Ile Leu Asn Ile Thr Thr
        435                 440                 445
Trp Asn Pro Ser Leu Ile Gly Ser Thr Thr Ala Leu Pro Asn Asn Ser
    450                 455                 460
Gly Lys Asp Val Leu Ser Asp Val Leu Gln Ile Ala Ser Ile Gln Ile
465                 470                 475                 480
Asn Lys Met Lys Ser Arg Lys Ala Cys Gly Val Ala Val Gly Ala Thr
                485                 490                 495
Leu Ile Asp Ala Asp Lys Trp Ser Ile Thr Gly Glu Ala Arg Leu Ile
            500                 505                 510
Asn Glu Arg Ala Ala His Met Asn Ala Gln Phe Arg Phe
        515                 520                 525

<210> 197
<211> 43
<212> DNA
<213> Chlamydia

<400> 197
gataggcgcg ccgcaatcat gaaatttatg tcagctactg ctg                43

<210> 198
<211> 34
<212> DNA
<213> Chlamydia

<400> 198
cagaacgcgt ttagaatgtc atacgagcac cgca                          34

<210> 199
<211> 6
<212> DNA
<213> Chlamydia

<400> 199
gcaatc                                                          6

<210> 200
<211> 34
<212> DNA
<213> Chlamydia

<400> 200
tgcaatcatg agttcgcaga aagatataaa aagc                          34

<210> 201
<211> 38
<212> DNA
<213> Chlamydia

<400> 201

cagagctagc ttaaaagatc aatcgcaatc cagtattc 38

<210> 202
<211> 5
<212> DNA
<213> Chlamydia

<400> 202
caatc 5

<210> 203
<211> 31
<212> DNA
<213> Chlamydia

<400> 203
tgcaatcatg aaaaaagcgt ttttcttttt c 31

<210> 204
<211> 31
<212> DNA
<213> Chlamydia

<400> 204
cagaacgcgt ctagaatcgc agagcaattt c 31

<210> 205
<211> 30
<212> DNA
<213> Chlamydia

<400> 205
gtgcaatcat gattcctcaa ggaatttacg 30

<210> 206
<211> 31
<212> DNA
<213> Chlamydia

<400> 206
cagaacgcgt ttagaaccgg actttacttc c 31

<210> 207
<211> 50
<212> DNA
<213> Chlamydia

<400> 207
cagacatatg catcaccatc accatcacga ggcgagctcg atccaagatc 50

<210> 208
<211> 40
<212> DNA
<213> Chlamydia

<400> 208
cagaggtacc tcagatagca ctctctccta ttaaagtagg 40

<210> 209
<211> 55
<212> DNA
<213> Chlamydia

<400> 209
cagagctagc atgcatcacc atcaccatca cgttaagatt gagaacttct ctggc 55

<210> 210
<211> 35
<212> DNA
<213> Chlamydia

<400> 210
cagaggtacc ttagaatgtc atacgagcac cgcag 35

<210> 211
<211> 36
<212> DNA
<213> Chlamydia

<400> 211
cagacatatg catcaccatc accatcacgg gttagc 36

<210> 212
<211> 35
<212> DNA
<213> Chlamydia

<400> 212
cagaggtacc tcagctcctc cagcacactc tcttc 35

<210> 213
<211> 51
<212> DNA
<213> Chlamydia

<400> 213
cagagctagc catcaccatc accatcacgg tgctatttct tgcttacgtg g 51

<210> 214
<211> 38
<212> DNA
<213> Chlamydia

<400> 214
cagaggtact taaaagatca atcgcaatcc agtattcg 38

<210> 215
<211> 48
<212> DNA
<213> Chlamydia

<400> 215
cagaggatcc acatcaccat caccatcacg gactagctag agaggttc                48

<210> 216
<211> 31
<212> DNA
<213> Chlamydia

<400> 216
cagagaattc ctagaatcgc agagcaattt c                                  31

<210> 217
<211> 7
<212> DNA
<213> Chlamydia

<400> 217
tgcaatc                                                              7

<210> 218
<211> 22
<212> PRT
<213> Chlamydia

<400> 218
Met Ala Ser Met Thr Gly Gly Gln Gln Met Gly Arg Asp Ser Ser Leu
 1               5                  10                  15
Val Pro Ser Ser Asp Pro
            20

<210> 219
<211> 51
<212> DNA
<213> Chlamydia

<400> 219
cagaggtacc gcatcaccat caccatcaca tgattcctca aggaatttac g            51

<210> 220
<211> 33
<212> DNA
<213> Chlamydia

<400> 220
cagagcggcc gcttagaacc ggactttact tcc                                33

<210> 221
<211> 24
<212> PRT
<213> Chlamydia

<400> 221
Met Ala Ser Met Thr Gly Gly Gln Gln Asn Gly Arg Asp Ser Ser Leu
 1               5                  10                  15

Val Pro His His His His His His
            20

<210> 222
<211> 46
<212> DNA
<213> Chlamydia

<400> 222
cagagctagc catcaccatc accatcacct ctttggccag gatccc                    46

<210> 223
<211> 30
<212> DNA
<213> Chlamydia

<400> 223
cagaactagt ctagaacctg taagtggtcc                                      30

<210> 224
<211> 20
<212> PRT
<213> Artificial Sequence

<220>
<223> Made in a lab

<400> 224
Met Ser Gln Lys Asn Lys Asn Ser Ala Phe Met His Pro Val Asn Ile
 1               5                  10                  15
Ser Thr Asp Leu
            20

<210> 225
<211> 20
<212> PRT
<213> Artificial Sequence

<220>
<223> Made in a lab

<400> 225
Lys Asn Ser Ala Phe Met His Pro Val Asn Ile Ser Thr Asp Leu Ala
 1               5                  10                  15
Val Ile Val Gly
            20

<210> 226
<211> 20
<212> PRT
<213> Artificial Sequence

<220>
<223> Made in a lab

<400> 226
His Pro Val Asn Ile Ser Thr Asp Leu Ala Val Ile Val Gly Lys Gly
1               5                   10                  15
Pro Met Pro Arg
            20

<210> 227
<211> 20
<212> PRT
<213> Artificial Sequence

<220>
<223> Made in a lab

<400> 227
Ser Thr Asp Leu Ala Val Ile Val Gly Lys Gly Pro Met Pro Arg Thr
1               5                   10                  15
Glu Ile Val Lys
            20

<210> 228
<211> 20
<212> PRT
<213> Artificial Sequence

<220>
<223> Made in a lab

<400> 228
Val Ile Val Gly Lys Gly Pro Met Pro Arg Thr Glu Ile Val Lys Lys
1               5                   10                  15
Val Trp Glu Tyr
            20

<210> 229
<211> 20
<212> PRT
<213> Artificial Sequence

<220>
<223> Made in a lab

<400> 229
Gly Pro Met Pro Arg Thr Glu Ile Val Lys Lys Val Trp Glu Tyr Ile
1               5                   10                  15
Lys Lys His Asn
            20

<210> 230
<211> 20
<212> PRT
<213> Artificial Sequence

<220>
<223> Made in a lab

<400> 230
Ile Lys Lys His Asn Cys Gln Asp Gln Lys Asn Lys Arg Asn Ile Leu
1               5                   10                  15
Pro Asp Ala Asn
            20

<210> 231
<211> 20
<212> PRT
<213> Artificial Sequence

<220>
<223> Made in a lab

<400> 231
Asn Cys Gln Asp Gln Lys Asn Lys Arg Asn Ile Leu Pro Asp Ala Asn
1               5                   10                  15
Leu Ala Lys Val
            20

<210> 232
<211> 20
<212> PRT
<213> Artificial Sequence

<220>
<223> Made in a lab

<400> 232
Lys Asn Lys Arg Asn Ile Leu Pro Asp Ala Asn Leu Ala Lys Val Phe
1               5                   10                  15
Gly Ser Ser Asp
            20

<210> 233
<211> 20
<212> PRT
<213> Artificial Sequence

<220>
<223> Made in a lab

<400> 233
Ile Leu Pro Asp Ala Asn Leu Ala Lys Val Phe Gly Ser Ser Asp Pro
1               5                   10                  15
Ile Asp Met Phe
            20

<210> 234
<211> 20
<212> PRT
<213> Artificial Sequence

<220>

<223> Made in a lab

<400> 234
Asn Leu Ala Lys Val Phe Gly Ser Ser Asp Pro Ile Asp Met Phe Gln
1               5                   10                  15
Met Thr Lys Ala
            20

<210> 235
<211> 22
<212> PRT
<213> Artificial Sequence

<220>
<223> Made in a lab

<400> 235
Phe Gly Ser Ser Asp Pro Ile Asp Met Phe Gln Met Thr Lys Ala Leu
1               5                   10                  15
Ser Lys His Ile Val Lys
            20

<210> 236
<211> 20
<212> PRT
<213> Artificial Sequence

<220>
<223> Made in a lab

<400> 236
Val Glu Ile Thr Gln Ala Val Pro Lys Tyr Ala Thr Val Gly Ser Pro
1               5                   10                  15
Tyr Pro Val Glu
            20

<210> 237
<211> 20
<212> PRT
<213> Artificial Sequence

<220>
<223> Made in a lab

<400> 237
Ala Val Pro Lys Tyr Ala Thr Val Gly Ser Pro Tyr Pro Val Glu Ile
1               5                   10                  15
Thr Ala Thr Gly
            20

<210> 238
<211> 20
<212> PRT
<213> Artificial Sequence

<220>
<223> Made in a lab

<400> 238
Ala Thr Val Gly Ser Pro Tyr Pro Val Glu Ile Thr Ala Thr Gly Lys
1               5                   10                  15
Arg Asp Cys Val
            20

<210> 239
<211> 20
<212> PRT
<213> Artificial Sequence

<220>
<223> Made in a lab

<400> 239
Pro Tyr Pro Val Glu Ile Thr Ala Thr Gly Lys Arg Asp Cys Val Asp
1               5                   10                  15
Val Ile Ile Thr
            20

<210> 240
<211> 21
<212> PRT
<213> Artificial Sequence

<220>
<223> Made in a lab

<400> 240
Ile Thr Ala Thr Gly Lys Arg Asp Cys Val Asp Val Ile Ile Thr Gln
1               5                   10                  15
Gln Leu Pro Cys Glu
            20

<210> 241
<211> 20
<212> PRT
<213> Artificial Sequence

<220>
<223> Made in a lab

<400> 241
Lys Arg Asp Cys Val Asp Val Ile Ile Thr Gln Gln Leu Pro Cys Glu
1               5                   10                  15
Ala Glu Phe Val
            20

<210> 242
<211> 20
<212> PRT
<213> Artificial Sequence

<220>
<223> Made in a lab

<400> 242
Asp Val Ile Ile Thr Gln Gln Leu Pro Cys Glu Ala Glu Phe Val Arg
1               5                   10                  15
Ser Asp Pro Ala
            20

<210> 243
<211> 20
<212> PRT
<213> Artificial Sequence

<220>
<223> Made in a lab

<400> 243
Thr Gln Gln Leu Pro Cys Glu Ala Glu Phe Val Arg Ser Asp Pro Ala
1               5                   10                  15
Thr Thr Pro Thr
            20

<210> 244
<211> 20
<212> PRT
<213> Artificial Sequence

<220>
<223> Made in a lab

<400> 244
Cys Glu Ala Glu Phe Val Arg Ser Asp Pro Ala Thr Thr Pro Thr Ala
1               5                   10                  15
Asp Gly Lys Leu
            20

<210> 245
<211> 20
<212> PRT
<213> Artificial Sequence

<220>
<223> Made in a lab

<400> 245
Val Arg Ser Asp Pro Ala Thr Thr Pro Thr Ala Asp Gly Lys Leu Val
1               5                   10                  15
Trp Lys Ile Asp
            20

<210> 246
<211> 20
<212> PRT

<213> Artificial Sequence

<220>
<223> Made in a lab

<400> 246
Ala Thr Thr Pro Thr Ala Asp Gly Lys Leu Val Trp Lys Ile Asp Arg
1               5                   10                  15
Leu Gly Gln Gly
            20

<210> 247
<211> 20
<212> PRT
<213> Artificial Sequence

<220>
<223> Made in a lab

<400> 247
Ala Asp Gly Lys Leu Val Trp Lys Ile Asp Arg Leu Gly Gln Gly Glu
1               5                   10                  15
Lys Ser Lys Ile
            20

<210> 248
<211> 20
<212> PRT
<213> Artificial Sequence

<220>
<223> Made in a lab

<400> 248
Val Trp Lys Ile Asp Arg Leu Gly Gln Gly Glu Lys Ser Lys Ile Thr
1               5                   10                  15
Val Trp Val Lys
            20

<210> 249
<211> 20
<212> PRT
<213> Artificial Sequence

<220>
<223> Made in a lab

<400> 249
Arg Leu Gly Gln Gly Glu Lys Ser Lys Ile Thr Val Trp Val Lys Pro
1               5                   10                  15
Leu Lys Glu Gly
            20

<210> 250
<211> 20

<212> PRT
<213> Artificial Sequence

<220>
<223> Made in a lab

<400> 250
Gly Glu Lys Ser Lys Ile Thr Val Trp Val Lys Pro Leu Lys Glu Gly
1               5                   10                  15
Cys Cys Phe Thr
            20

<210> 251
<211> 16
<212> PRT
<213> Artificial Sequence

<220>
<223> Made in a lab

<400> 251
Gly Glu Lys Ser Lys Ile Thr Val Trp Val Lys Pro Leu Lys Glu Gly
1               5                   10                  15

<210> 252
<211> 12
<212> PRT
<213> Artificial Sequence

<220>
<223> Made in a lab

<400> 252
Lys Ile Thr Val Trp Val Lys Pro Leu Lys Glu Gly
1               5                   10

<210> 253
<211> 16
<212> PRT
<213> Artificial Sequence

<220>
<223> Made in a lab

<400> 253
Gly Asp Lys Cys Lys Ile Thr Val Trp Val Lys Pro Leu Lys Glu Gly
1               5                   10                  15

<210> 254
<211> 20
<212> PRT
<213> Artificial Sequence

<220>
<223> Made in a lab

<400> 254
Thr Glu Tyr Pro Leu Leu Ala Asp Pro Ser Phe Lys Ile Ser Glu Ala
1               5                   10                  15
Phe Gly Val Leu
            20

<210> 255
<211> 20
<212> PRT
<213> Artificial Sequence

<220>
<223> Made in a lab

<400> 255
Leu Ala Asp Pro Ser Phe Lys Ile Ser Glu Ala Phe Gly Val Leu Asn
1               5                   10                  15
Pro Glu Gly Ser
            20

<210> 256
<211> 20
<212> PRT
<213> Artificial Sequence

<220>
<223> Made in a lab

<400> 256
Phe Lys Ile Ser Glu Ala Phe Gly Val Leu Asn Pro Glu Gly Ser Leu
1               5                   10                  15
Ala Leu Arg Ala
            20

<210> 257
<211> 20
<212> PRT
<213> Artificial Sequence

<220>
<223> Made in a lab

<400> 257
Ala Phe Gly Val Leu Asn Pro Glu Gly Ser Leu Ala Leu Arg Ala Thr
1               5                   10                  15
Phe Leu Ile Asp
            20

<210> 258
<211> 20
<212> PRT
<213> Artificial Sequence

<220>

<223> Made in a lab

<400> 258
Asn Pro Glu Gly Ser Leu Ala Leu Arg Ala Thr Phe Leu Ile Asp Lys
1               5                   10                  15
His Gly Val Ile
            20

<210> 259
<211> 20
<212> PRT
<213> Artificial Sequence

<220>
<223> Made in a lab

<400> 259
Leu Ala Leu Arg Ala Thr Phe Leu Ile Asp Lys His Gly Val Ile Arg
1               5                   10                  15
His Ala Val Ile
            20

<210> 260
<211> 20
<212> PRT
<213> Artificial Sequence

<220>
<223> Made in a lab

<400> 260
Thr Phe Leu Ile Asp Lys His Gly Val Ile Arg His Ala Val Ile Asn
1               5                   10                  15
Asp Leu Pro Leu
            20

<210> 261
<211> 20
<212> PRT
<213> Artificial Sequence

<220>
<223> Made in a lab

<400> 261
Lys His Gly Val Ile Arg His Ala Val Ile Asn Asp Leu Pro Leu Gly
1               5                   10                  15
Arg Ser Ile Asp
            20

<210> 262
<211> 20
<212> PRT
<213> Artificial Sequence

<220>
<223> Made in a lab

<400> 262
Arg His Ala Val Ile Asn Asp Leu Pro Leu Gly Arg Ser Ile Asp Glu
 1               5                  10                  15
Glu Leu Arg Ile
            20

<210> 263
<211> 897
<212> DNA
<213> Chlamydia

<220>
<221> misc_feature
<222> (1)...(897)
<223> n = A,T,C or G

<400> 263
atggcttcta tatgcggacg tttagggtct ggtacaggga atgctctaaa agcttttttt      60
acacagccca acaataaaat ggcaagggta gtaaataaga cgaagggagt ggataagact     120
attaaggttg ccaagtctgc tgccgaattg accgcaaata ttttggaaca agctggaggc     180
gcgggctctt ccgcacacat tacagcttcc caagtgtcca aaggattagg ggatgcgaga     240
actgttgtcg ctttagggaa tgcctttaac ggagcgttgc caggaacagt tcaaagtgcg     300
caaagcttct tctctcacat gaaagctgct agtcagaaaa cgcaagaagg ggatgagggg     360
ctcacagcag atctttgtgt gtctcataag cgcagagcgg ctgcggctgt ctgtagcatc     420
atcggaggaa ttacctacct cgcgacattc ggagctatcc gtccgattct gtttgtcaac     480
aaaatgctgg caaaaccgtt tctttcttcc caaactaaag caaatatggg atcttctgtt     540
agctatatta tggcggctaa ccatgcagcg tctgtggtgg gtgctggact cgctatcagt     600
gcgnaaagag cagattgcga agcccgctgc gctcgtattg cgagagaaga gtcgttactc     660
gaagtgccgg gagaggaaaa tgcttgcgag aagaaagtcg ctggagagaa agccaagacg     720
ttcacgcgca tcaagtatgc actcctcact atgctcgaga agtttttgga atgcgttgcc     780
gacgttttca aattggtgcc gctgcctatt acaatgggta ttcgtgcgat tgtggctgct     840
ggatgtacgt tcacttctgc aattattgga ttgtgcactt tctgcgccag agcataa        897

<210> 264
<211> 298
<212> PRT
<213> Chlamydia

<220>
<221> VARIANT
<222> (1)...(298)
<223> Xaa = Any Amino Acid

<400> 264
Met Ala Ser Ile Cys Gly Arg Leu Gly Ser Gly Thr Gly Asn Ala Leu
 1               5                  10                  15
Lys Ala Phe Phe Thr Gln Pro Asn Asn Lys Met Ala Arg Val Val Asn
            20                  25                  30
Lys Thr Lys Gly Val Asp Lys Thr Ile Lys Val Ala Lys Ser Ala Ala
        35                  40                  45
Glu Leu Thr Ala Asn Ile Leu Glu Gln Ala Gly Gly Ala Gly Ser Ser
    50                  55                  60

Ala His Ile Thr Ala Ser Gln Val Ser Lys Gly Leu Gly Asp Ala Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110
Lys Thr Gln Glu Gly Asp Glu Gly Leu Thr Ala Asp Leu Cys Val Ser
        115                 120                 125
His Lys Arg Arg Ala Ala Ala Ala Val Cys Ser Ile Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Lys Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Xaa Arg Ala Asp Cys Glu Ala
        195                 200                 205
Arg Cys Ala Arg Ile Ala Arg Glu Glu Ser Leu Leu Glu Val Pro Gly
    210                 215                 220
Glu Glu Asn Ala Cys Glu Lys Lys Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Ile
        275                 280                 285
Ile Gly Leu Cys Thr Phe Cys Ala Arg Ala
    290                 295

<210> 265
<211> 897
<212> DNA
<213> Chlamydia

<220>
<221> misc_feature
<222> (1)...(897)
<223> n = A,T,C or G

<400> 265
atggcttcta tatgcggacg tttagggtct ggtacaggga atgctctaaa agcttttttt      60
acacagccca acaataaaat ggcaagggta gtaaataaga cgaagggaat ggataagact     120
attaaggttg ccaagtctgc tgccgaattg accgcaaata ttttggaaca agctggaggc     180
gcgggctctt ccgcacacat tacagcttcc caagtgtcca aaggattagg ggatgcgaga     240
actgttgtcg ctttagggaa tgcctttaac ggagcgttgc caggaacagt tcaaagtgcg     300
caaagcttct tctctcacat gaaagctgct agtcagaaaa cgcaagaagg ggatgagggg     360
ctcacagcag atctttgtgt gtctcataag cgcagagcgg ctgcggctgt ctgtagcatc     420
atcggaggaa ttacctacct cgcgacattc ggagctatcc gtccgattct gtttgtcaac     480
aaaatgctgg caaaaccgtt tctttcttcc caaactaaag caaatatggg atcttctgtt     540
agctatatta tggcggctaa ccatgcagcg tctgtggtgg gtgctggact cgctatcagt     600
gcgnaaagag cagattgcga agcccgctgc gctcgtattg cgagagaaga gtcgttactc     660
gaagtgccgg gagaggaaaa tgcttgcgag aagaaagtcg ctggagagaa agccaagacg     720
ttcacgcgca tcaagtatgc actcctcact atgctcgaga agtttttgga atgcgttgcc     780

gacgttttca aattggtgcc gctgcctatt acaatgggta ttcgtgcgat tgtggctgct     840
ggatgtacgt tcacttctgc aattattgga ttgtgcactt tctgcgccag agcataa        897

<210> 266
<211> 298
<212> PRT
<213> Chlamydia

<220>
<221> VARIANT
<222> (1)...(298)
<223> Xaa = Any Amino Acid

<400> 266
Met Ala Ser Ile Cys Gly Arg Leu Gly Ser Gly Thr Gly Asn Ala Leu
 1               5                  10                  15
Lys Ala Phe Phe Thr Gln Pro Asn Asn Lys Met Ala Arg Val Val Asn
            20                  25                  30
Lys Thr Lys Gly Met Asp Lys Thr Ile Lys Val Ala Lys Ser Ala Ala
        35                  40                  45
Glu Leu Thr Ala Asn Ile Leu Glu Gln Ala Gly Gly Ala Gly Ser Ser
    50                  55                  60
Ala His Ile Thr Ala Ser Gln Val Ser Lys Gly Leu Gly Asp Ala Arg
65                  70                  75                  80
Thr Val Val Ala Leu Gly Asn Ala Phe Asn Gly Ala Leu Pro Gly Thr
                85                  90                  95
Val Gln Ser Ala Gln Ser Phe Phe Ser His Met Lys Ala Ala Ser Gln
            100                 105                 110
Lys Thr Gln Glu Gly Asp Glu Gly Leu Thr Ala Asp Leu Cys Val Ser
        115                 120                 125
His Lys Arg Arg Ala Ala Ala Ala Val Cys Ser Ile Ile Gly Gly Ile
    130                 135                 140
Thr Tyr Leu Ala Thr Phe Gly Ala Ile Arg Pro Ile Leu Phe Val Asn
145                 150                 155                 160
Lys Met Leu Ala Lys Pro Phe Leu Ser Ser Gln Thr Lys Ala Asn Met
                165                 170                 175
Gly Ser Ser Val Ser Tyr Ile Met Ala Ala Asn His Ala Ala Ser Val
            180                 185                 190
Val Gly Ala Gly Leu Ala Ile Ser Ala Xaa Arg Ala Asp Cys Glu Ala
        195                 200                 205
Arg Cys Ala Arg Ile Ala Arg Glu Glu Ser Leu Leu Glu Val Pro Gly
    210                 215                 220
Glu Glu Asn Ala Cys Glu Lys Lys Val Ala Gly Glu Lys Ala Lys Thr
225                 230                 235                 240
Phe Thr Arg Ile Lys Tyr Ala Leu Leu Thr Met Leu Glu Lys Phe Leu
                245                 250                 255
Glu Cys Val Ala Asp Val Phe Lys Leu Val Pro Leu Pro Ile Thr Met
            260                 265                 270
Gly Ile Arg Ala Ile Val Ala Ala Gly Cys Thr Phe Thr Ser Ala Ile
        275                 280                 285
Ile Gly Leu Cys Thr Phe Cys Ala Arg Ala
    290                 295

<210> 267
<211> 680

<212> DNA
<213> Chlamydia

<400> 267
tctatatcca tattgatagg aaaaaacgtc gcagaaagat tttagctatg acgtttatcc 60
gagctttagg atattcaaca gatgcagata ttattgaaga gttcttttct gtagaggagc 120
gttccttacg ttcagagaag gattttgtcg cgttagttgg taaagtttta gctgataacg 180
tagttgatgc ggattcttca ttagtttacg ggaaagctgg agagaagcta agtactgcta 240
tgctaaaacg catcttagat acgggagtcc aatctttgaa gattgctgtt ggcgcagatg 300
aaaatcaccc aattattaag atgctcgcaa aagatcctac ggattcttac gaagctgctc 360
ttaaagattt ttatcgcaga ttacgaccag gagagcctgc aactttagct aatgctcgat 420
ccacaattat gcgtttattc ttcgatgcta aacgttataa tttaggccgc gttggacgtt 480
ataaattaaa taaaaaatta ggcttcccat tagacgacga aacattatct caagtgactt 540
tgagaaaaga agatgttatc ggcgcgttga aatatttgat tcgtttgcga atgggcgatg 600
agaagacatc tatcgatgat attgaccatt tggcaaaccg acgagttcgc tctgttggag 660
aactaattca gaatcactgt 680

<210> 268
<211> 359
<212> DNA
<213> Chlamydia

<400> 268
   cttatgttct ggagaatgtt gcaacaacat attaatcgaa ccagctcctc ctagtaacat 60
   agaaaccaag cccttttgag aaaaaacctg tacttcgcat cctttagcca tttgttgaat 120
   agctcctaac aaagagctaa ttttttcctc ttccttgttt ttctgaggcg ctgtggactc 180
   taaatatagc aagtgctctt ggaacacctc atcaacaatc gcttgtccta gattaggtat 240
   agagactgtc tctccatcaa ttaaatggag tttcaaagta atatcccctt ccgtccctcc 300
   atcacaagac tctatgaaag ctatctgatt ccatcgagca gaaatgtatg gggaaatac 359

<210> 269
<211> 124
<212> DNA
<213> Chlamydia

<400> 269
gatcgaatca attgagggag ctcattaaca agaatagctg cagtttcttt gcgttcttct 60
ggaataacaa gaaataggta atcggtacca ttgatagaac gaacacgaca aatcgcagaa 120
ggtt 124

<210> 270
<211> 219
<212> DNA
<213> Chlamydia

<400> 270
gatcctgttg ggcctagtaa taatacgttg gatttcccat aactcacttg tttatcctgc 60
ataagagcac ggatacgctt atagtggtta tagacggcaa ccgaaatcgt ttttttcgcg 120
cgctcttgtc caatgacata agagtcgatg tggcgtttga tttctttagg ggttaacact 180
ctcagacttg ttggagagct tgtggaagat gttgcgatc 219

<210> 271
<211> 511
<212> DNA
<213> Chlamydia

<220>
<221> misc_feature
<222> (1)...(511)
<223> n = A,T,C or G

<400> 271
ggatccgaat tcggcacgag gagaaaatat aggaggttcc akcatcggaa gatctaatag 60
acaaagaggt tttggcatag atggctcctc cttgtacgtt caacgatgat tgggagggat 120
tgttatcgat agcttggttc ccagagaact gacaagtccc gctacattga gagaatgtaa 180
cctgttctcc atagatagct cctcctacta cacctgaata agttggtgtt gctggagatg 240
atggtgcggc tgctgcggct gcttgtaggg aagcagcagc tgcagcaggt gctgaagctg 300
ttgttgcgac tcctgtggat gaggagtttg ctttgttgtt cgagaaagag aagcctgatt 360
tcagattaga aatatttaca gttttagcat gtaagcctcc accttctttc ccaacaaggt 420
tctctgttac agataaggag actagangca tctagtttta aagatttttt acagcagata 480
cctccaccta tctctgtagc ggagttctca g 511

<210> 272
<211> 598
<212> DNA
<213> Chlamydia

<400> 272
ctcttcctct cctcaatcta gttctggagc aactacagtc tccgactcag gagactctag 60
ctctggctca aactcggata cctcaaaaac agttccagtc acagctaaag gcggtgggct 120
ttatactgat aagaatcttt cgattactaa catcacagga attatcgaaa ttgcaaataa 180
caaagcgaca gatgttggag gtggtgctta cgtaaaagga acccttactt gtaaaaactc 240
tcaccgtcta caatttttga aaaactcttc cgataaacaa ggtggaggaa tctacggaga 300
agacaacatc accctatcta atttgacagg gaagactcta ttccaagaga atactgccaa 360
aaaagagggc ggtggactct tcataaaagg tacagataaa gctcttacaa tgacaggact 420
ggatagtttc tgtttaatta ataacacatc agaaaaacat ggtggtggga gcctttgtta 480
ccaaagaaat ctctcagact tacacctctt gatgtggaaa caattccagg aatcacgcct 540
gtacatggtg aaacagtcat tactggcaat aaatctacag gaggtaatgg tggagggc 598

<210> 273
<211> 126
<212> DNA
<213> Chlamydia

<400> 273
ggatccgaat tcggcacgag atgagcctta tagtttaaca aaagcttctc acattccttc 60
gatagctttt tattagccgt ttttagcatc ctaatgagat ctcctcgttc gtaacaaata 120
cgagag 126

<210> 274
<211> 264
<212> DNA
<213> Chlamydia

<400> 274
ggatccgaat tcggcacgag ctcttttaaa tcttaattac aaaaagacaa attaattcaa 60
tttttcaaaa aagaatttaa acattaattg ttgtaaaaaa acaatattta ttctaaaata 120
ataaccatag ttacggggga atctctttca tggtttattt tagagctcat caacctaggc 180
atacgcctaa aacatttcct ttgaaagttc accattcgtt ctccgataag catcctcaaa 240
ttgctaaagc tatgtggatt acgg 264

<210> 275
<211> 359
<212> DNA
<213> Chlamydia

<400> 275
ggatccgaat tcggcacgag ataaaacctg aaccacaaca aagatctaaa acttcttgat 60
tttcagctgc aaattctttt agataaatat caaccatttc ttcagtttca tatcttggaa 120
ttaaaacttg ttctcttaaa ttaattctag tatttaagta ttcaacatag cccattatta 180
attgaattgg ataattttgc cttaataatt cacattcttt ttcagtaatt ttaggttcta 240
aaccgtaccg cttttttctt aaaattaatg tttcttcatt attcatttta taagccactt 300
tcctttattt tttgattttg ttcttctgtt agtaatgctt caataatagt taataattt 359

<210> 276
<211> 357
<212> DNA
<213> Chlamydia

<400> 276
aaaacaattg atataatttt ttttttcata acttccagac tcctttctag aaaagtcttt 60
atgggtagta gtgactctaa cgttttttat tattaagacg atccccggag atccttttaa 120
tgatgaaaac ggaaacatcc tttcgccaga aactttagca ctattaaaga atcgttacgg 180
gttagataag cctttattca cccagtatct tatctatttg aaatgtctgc taacactaga 240
tttcggggaa tctcttatct acaaagatcg aaatctcagc attattgctg ccgctcttcc 300
atcttccgct attcttggac ttgaaagctt gtgtttactc gtgccgaatt cggatcc 357

<210> 277
<211> 505
<212> DNA
<213> Chlamydia

<400> 277
ggatccgaat tcggcacgag ctcgtgccga ttgcttgctt cagtcacccc atcggtatag 60
agcactaaaa gagactcctc ttcaagaacg agagtgtaag cagggtgagg aggaacttca 120
ggtaaaaatc ctaaggccat accaggatgc gacaggaaag agatatctcc attaggagct 180
cggagacacg ctgggttgtg gccacaagaa tagtattcta gttctcgtgt tgcgtaatga 240
taacaataaa tgcatagtgt tacaaacatc ccagattcag ctgtctgttg atagaagaga 300
gcagctgttt gttgaacggc ttcttgaata gaggagagct cactcaaaaa ggtatgtaac 360
atgtttttca ggaataagga gtaggcgcac gcattgactc ctttcccgga agcatcagca 420
acgattagaa agagtttagc ttggggacct tcgcctataa caaagatatc aaagaaatct 480
cctcctaccg taactgcagg aatat 505

<210> 278
<211> 407
<212> DNA
<213> Chlamydia

<400> 278
ggatccgaat tcggcacgag aactactgag caaattgggt atccaacttc ctctttacga 60
aagaaaaaca gaaggcattc tccataccaa gatttgttgc atcgacaata aaactccaat 120
ctttggctct gctaactgga gcggtgctgg tatgattaaa aactttgaag acctattcat 180
ccttcgccca attacagaga cacagcttca ggcctttatg gacgtctggt ctcttctaga 240
aacaaatagc tcctatctgt ccccagagag cgtgcttacg gcccctactc cttcaagtag 300
acctactcaa caagatacag attctgatga cgaacaaccg agtaccagcc agcaagctat 360

ccgtatgaga aaataggatt agggaaacaa aacgacagca aaccaca 407

<210> 279
<211> 351
<212> DNA
<213> Chlamydia

<400> 279
ctcgtgccgc ttacaggagg cttgtatcct ttaaaataga gtttttctta tgaccccatg 60
tggcgatagg ccgggtctag cgccgatagt agaaatatcg gttggttttt gtccttgagg 120
ggatcgtata ctttttcaaa gtatggtccc cgtatcgatt atctggaggc tcttatgtct 180
ttttttcata ctagaaaata taagcttatc ctcagaggac tcttgtgttt agcaggctgt 240
ttcttaatga acagctgttc ctctagtcga ggaaatcaac ccgctgatga gagcatctat 300
gtcttgtcta tgaatcgcat gatttgtgat tctcgtgccg aattcggatc c 351

<210> 280
<211> 522
<212> DNA
<213> Chlamydia

<400> 280
ggatccgaat tcggcacgag cagaggaaaa aggcgatact cctcttgaag atcgtttcac 60
agaagatctt tccgaagtct ctggagaaga ttttcgagga ttgaaaaatt cgttcgatga 120
tgattcttct tctgacgaaa ttctcgatgc gctcacaagt aaattttctg atcccacaat 180
aaaggatcta gctcttgatt atctaattca aatagctccc tctgatggga aacttaagtc 240
cgctctcatt caggcaaagc atcaactgat gagccagaat cctcaggcga ttgttggagg 300
acgcaatgtt ctgttagctt cagaaacctt tgcttccaga gcaaatacat ctccttcatc 360
gcttcgctcc ttatatttcc aagtaacctc atccccctct aattgcgcta atttacatca 420
aatgcttgct tcttactcgc catcagagaa aaccgctgtt atggagtttc tagtgaatgg 480
catggtagca gatttaaaat cggagggccc ttccattcct cc 522

<210> 281
<211> 577
<212> DNA
<213> Chlamydia

<400> 281
ggatccgaat tcggcacgag atgcttctat tacaattggt ttggatgcgg aaaaagctta 60
ccagcttatt ctagaaaagt tgggagatca aattcttggt ggaattgctg atactattgt 120
tgatagtaca gtccaagata ttttagacaa aatcacaaca gacccttctc taggtttgtt 180
gaaagctttt aacaactttc caatcactaa taaaattcaa tgcaacgggt tattcactcc 240
caggaacatt gaaactttat taggaggaac tgaaatagga aaattcacag tcacacccaa 300
aagctctggg agcatgttct tagtctcagc agatattatt gcatcaagaa tggaaggcgg 360
cgttgttcta gctttggtac gagaaggtga ttctaagccc tacgcgatta gttatggata 420
ctcatcaggc gttcctaatt tatgtagtct aagaaccaga attattaata caggattgac 480
tccgacaacg tattcattac gtgtaggcgg tttagaaagc ggtgtggtat gggttaatgc 540
cctttctaat ggcaatgata ttttaggaat aacaaat 577

<210> 282
<211> 607
<212> DNA
<213> Chlamydia

<400> 282
actmatcttc cccgggctcg agtgcggccg caagcttgtc gacggagctc gatacaaaaa 60

tgtgtgcgtg tgaaccgctt cttcaaaagc ttgtcttaaa agatattgtc tcgcttccgg 120
attagttaca tgtttaaaaa ttgctagaac aatattattc ccaaccaagc tctctgcggt 180
gctgaaaaaa cctaaattca aaagaatgac tcgccgctca tcttcagaaa gacgatccga 240
cttccataat tcgatgtctt tccccatggg gatctctgta gggagccagt tatttgcgca 300
gccattcaaa taatgttccc aagcccattt gtacttaata ggaacaagtt ggttgacatc 360
gacctggttg cagttcacta gacgcttgct atttagatta acgcgtttct gttttccatc 420
taaaatatct gcttgcataa gaaccgttaa ttttattgtt aatttatatg attaattact 480
gacatgcttc acacccttct tccaaagaac agacaggtgc tttcttcgct ctttcaacaa 540
taattcctgc cgaagcagac ttattcttca tccaacgagg ctgaattcct ctcttattaa 600
tatctac 607

<210> 283
<211> 1077
<212> DNA
<213> Chlamydia

<400> 283
   ggatccgaat tcggcacgag aagttaacga tgacgatttg ttcctttggt agagaaggag 60
   caatcgaaac taaatgtgcg agagcatgtg aagactccaa tgcaggaata atcccctcat 120
   ttctagtaag caggaaaaaa gctcgtaacg cctcttcatc ggtggctaat gtataaaagg 180
   ctcgtcctga ctcatgcatt tcggcatgat ctggcccaac tgaaggataa tctaatccag 240
   cggaaatgga gtgagtttgt aatacttgtc catcgtcatc ttgaagaaga tacgaataaa 300
   atccgtggaa tactccaggt cgccctgttg caaaacgtgc tgcatgtttt cctgaagaaa 360
   tgcccagtcc tccccctccc actccaatta attggacttt tggattcggg ataaaatgat 420
   ggaaaaatcc aatagcgttg gagccacctc cgatacatgc aatcagaata tcaggatctc 480
   ttcctgcaac tgcatggatt tgctctttca cttcagcgct tataacagac tgaaaaaatc 540
   gaacgatatc gggataaggt aaaggtccta aggccgatcc taagcaatag tgagtaaatg 600
   agtgtgttgt tgcccaatct tgtagagctt gattaactgc atctttgagt ccacaagatc 660
   cttttgttac agaaacgact tcagcaccta aaaagcgcat ttctctacaa tttggtttct 720
   gtcgttccac atcttttgct cccatgtata ctacacaatc taatcctaga taagcacacg 780
   ctgttgctgt tgctactcca tgttgtcccg cacctgtttc agctacaaca cgtgttttcc 840
   caagatattt agcaagcaaa cactgaccaa gagcattatt cagtttatgt gctcctgtat 900
   gcaaaagatc ttcgcgttta agaaatactc tagggccatc aatagctcga gcaaaattct 960
   taacttcagt cagaggagtt tgtctccccg catagttttt caaaatacaa tctagttcag 1020
   ataaaaaact ttgctgagtt ttgagaatct cccattccgc ttttagattc tgtatag 1077

<210> 284
<211> 407
<212> DNA
<213> Chlamydia

<400> 284
ggatccgaat tcggcacgag aactactgag caaattgggt atccaacttc ctctttacga 60
aagaaaaaca gaaggcattc tccataccaa gatttgttgc atcgacaata aaactccaat 120
ctttggctct gctaactgga gcggtgctgg tatgattaaa aactttgaag acctattcat 180
ccttcgccca attacagaga cacagcttca ggcctttatg gacgtctggt ctcttctaga 240
aacaaatagc tcctatctgt ccccagagag cgtgcttacg gcccctactc cttcaagtag 300
acctactcaa caagatacag attctgatga cgaacaaccg agtaccagcc agcaagctat 360
ccgtatgaga aaataggatt agggaaacaa aacgacagca aaccaca 407

<210> 285
<211> 802
<212> DNA
<213> Chlamydia

<400> 285
ggatccgaat tcggcacgag ttagcttaat gtctttgtca tctctaccta catttgcagc 60
taattctaca ggcacaattg gaatcgttaa tttacgtcgc tgcctagaag agtctgctct 120
tgggaaaaaa gaatctgctg aattcgaaaa gatgaaaaac caattctcta acagcatggg 180
gaagatggag gaagaactgt cttctatcta ttccaagctc caagacgacg attacatgga 240
aggtctatcc gagaccgcag ctgccgaatt aagaaaaaaa ttcgaagatc tatctgcaga 300
atacaacaca gctcaagggc agtattacca aatattaaac caaagtaatc tcaagcgcat 360
gcaaaagatt atggaagaag tgaaaaaagc ttctgaaact gtgcgtattc aagaaggctt 420
gtcagtcctt cttaacgaag atattgtctt atctatcgat agttcggcag ataaaaccga 480
tgctgttatt aaagttcttg atgattcttt tcaaaataat taacatgcga agctagccga 540
ggagtgccgt atgtctcaat ccacttattc tcttgaacaa ttagctgatt ttttgaaagt 600
cgagtttcaa ggaaatggag ctactcttct ttccggagtt gaagagatcg aggaagcaaa 660
aacggcacac atcacattct tagataatga aaaatatgct aaacatttaa aatcatcgga 720
agctggcgct atcatcatat ctcgaacaca gtttcaaaaa tatcgagact tgaataaaaa 780
ctttcttatc acttctgagt ct 802

<210> 286
<211> 588
<212> DNA
<213> Chlamydia

<400> 286
ggatccgaat tcggcacgag gcaatattta ctcccaacat tacggttcca aataagcgat 60
aaggtcttct aataaggaag ttaatgtaag aggctttttt attgcttttc gtaaggtagt 120
attgcaaccg cacgcgattg aatgatacgc aagccatttc catcatggaa aagaaccctt 180
ggacaaaaat acaaaggagg ttcactccta accagaaaaa gggagagtta gtttccatgg 240
gttttcctta tatacacccg tttcacacaa ttaggagccg cgtctagtat ttggaataca 300
aattgtcccc aagcgaattt tgttcctgtt tcagggattt ctcctaattg ttctgtcagc 360
catccgccta tggtaacgca attagctgta gtaggaagat caactccaaa caggtcatag 420
aaatcagaaa gctcataggt gcctgcagca ataacaacat tcttgtctga gtgagcgaat 480
tgtttaaaag atgggcgatt atgagctacc tcatcagaga ctattttaaa tagatcattt 540
tgggtaatca atccttctat agacccatat tcatcaatga taatctcg 588

<210> 287
<211> 489
<212> DNA
<213> Chlamydia

<220>
<221> misc_feature
<222> (1)...(489)
<223> n = A,T,C or G

<400> 287
agtgcctatt gttttgcagg ctttgtctga tgatagcgat accgtacgtg agattgctgt 60
acaagtagct gttatgtatg gttctagttg cttactgcgc gccgtgggcg atttagcgaa 120
aaatgattct tctattcaag tacgcatcac tgcttatcgt gctgcagccg tgttggagat 180
acaagatctt gtgcctcatt tacgagttgt agtccaaaat acacaattag atggaacgga 240
aagaagagaa gcttggagat ctttatgtgt tcttactcgg cctcatagtg gtgtattaac 300
tggcatagat caagctttaa tgacctgtga gatgttaaag gaatatcctg aaaagtgtac 360
ggaagaacag attcgtacat tattggctgc agatcatcca gaagtgcagg tagctacttt 420
acagatcatt ctgagaggag gtagagtatt ccggtcatct tctataatgg aatcggttct 480
cgtgccgnt 489

<210> 288

<211> 191
<212> DNA
<213> Chlamydia

<400> 288
ggatccgaat tcaggatatg ctgttgggtt atcaataaaa agggttttgc cattttttaa 60
gacgactttg tagataacgc taggagctgt agcaataata tcgagatcaa attctctaga 120
gattctctca aagatgattt ctaagtgcag cagtcctaaa aatccacagc ggaacccaaa 180
tccgagagag t 191

<210> 289
<211> 515
<212> DNA
<213> Chlamydia

<400> 289
ggatccgaat tcggcacgag gagcgacgtg aaatagtgga atcttcccgt attcttatta 60
cttctgcgtt gccttacgca aatggtcctt tgcattttgg acatattacc ggtgcttatt 120
tgcctgcaga tgtttatgcg cgttttcaga gactacaagg caaagaggtt ttgtatattt 180
gtggttctga tgaatacgga atcgcaatta cccttaatgc agagttggca ggcatggggt 240
atcaagaata tgtcgacatg tatcataagc ttcataaaga taccttcaag aaattgggaa 300
tttctgtaga tttcttttcc agaactacga acgcttatca tcctgctatt gtgcaagatt 360
tctatcgaaa cttgcaggaa cgcggactgg tagagaatca ggtgaccgaa cagctgtatt 420
ctgaggaaga agggaagttt ttagcggacc gttatgttgt aggtacttgt cccaagtgtg 480
ggtttgatcg agctcgagga gatgagtgtc agcag 515

<210> 290
<211> 522
<212> DNA
<213> Chlamydia

<400> 290
ggatccgaat tcggcacgag ggaggaatgg aagggccctc cgattktama tctgctacca 60
tgccattcac tagaaactcc ataacagcgg ttttctctga tggcgagtaa gaagcaagca 120
tttgatgtaa attagcgcaa ttagaggggg atgaggttac ttggaaatat aaggagcgaa 180
gcgatgaagg agatgtattt gctctggaag caaaggtttc tgaagctaac agaacattgc 240
gtcctccaac aatcgcctga ggattctggc tcatcagttg atgctttgcc tgaatgagag 300
cggacttaag tttcccatca gagggagcta tttgaattag ataatcaaga gctagatcct 360
ttattgtggg atcagaaaat ttacttgtga gcgcatcgag aatttcgtca gaagaagaat 420
catcatcgaa cgaatttttc aatcctcgaa aatcttctcc agagacttcg gaaagatctt 480
ctgtgaaacg atcttcaaga ggagtatcgc ctttttccyc tg 522

<210> 291
<211> 1002
<212> DNA
<213> Chlamydia

<400> 291
atggcgacta acgcaattag atcggcagga agtgcagcaa gtaagatgct gctgccagtt 60
gccaaagaac cagcggctgt cagctccttt gctcagaaag ggatttattg tattcaacaa 120
ttttttacaa accctgggaa taagttagca aagtttgtag gggcaacaaa aagtttagat 180
aaatgcttta agctaagtaa ggcggtttct gactgtgtcg taggatcgct ggaagaggcg 240
ggatgcacag gggacgcatt gacctccgcg agaaacgccc agggtatgtt aaaaacaact 300
cgagaagttg ttgccttagc taatgtgctc aatggagctg ttccatctat cgttaactcg 360
actcagaggt gttaccaata cacacgtcaa gccttcgagt taggaagcaa gacaaaagaa 420

agaaaaacgc ctggggagta tagtaaaatg ctattaactc gaggtgatta cctattggca 480
gcttccaggg aagcttgtac ggcagtcggt gcaacgactt actcagcgac attcggtgtt 540
ttacgtccgt taatgttaat caataaactc acagcaaaac cattcttaga caaagcgact 600
gtaggcaatt ttggcacggc tgttgctgga attatgacca ttaatcatat ggcaggagtt 660
gctggtgctg ttggcggaat cgcattagaa caaaagctgt tcaaacgtgc gaaggaatcc 720
ctatacaatg agagatgtgc cttagaaaac caacaatctc agttgagtgg ggacgtgatt 780
ctaagcgcgg aaagggcatt acgtaaagaa cacgttgcta ctctaaaaag aaatgtttta 840
actcttcttg aaaaagcttt agagttggta gtggatggag tcaaactcat tcctttaccg 900
attacagtgg cttgctccgc tgcaatttct ggagccttga cggcagcatc cgcaggaatt 960
ggcttatata gcatatggca gaaaacaaag tctggcaaat aa 1002

<210> 292
<211> 333
<212> PRT
<213> Chlamydia

<400> 292
Met Ala Thr Asn Ala Ile Arg Ser Ala Gly Ser Ala Ala Ser Lys Met
1               5                   10                  15
Leu Leu Pro Val Ala Lys Glu Pro Ala Ala Val Ser Ser Phe Ala Gln
            20                  25                  30
Lys Gly Ile Tyr Cys Ile Gln Gln Phe Phe Thr Asn Pro Gly Asn Lys
        35                  40                  45
Leu Ala Lys Phe Val Gly Ala Thr Lys Ser Leu Asp Lys Cys Phe Lys
    50                  55                  60
Leu Ser Lys Ala Val Ser Asp Cys Val Val Gly Ser Leu Glu Glu Ala
65                  70                  75                  80
Gly Cys Thr Gly Asp Ala Leu Thr Ser Ala Arg Asn Ala Gln Gly Met
                85                  90                  95
Leu Lys Thr Thr Arg Glu Val Val Ala Leu Ala Asn Val Leu Asn Gly
            100                 105                 110
Ala Val Pro Ser Ile Val Asn Ser Thr Gln Arg Cys Tyr Gln Tyr Thr
        115                 120                 125
Arg Gln Ala Phe Glu Leu Gly Ser Lys Thr Lys Glu Arg Lys Thr Pro
    130                 135                 140
Gly Glu Tyr Ser Lys Met Leu Leu Thr Arg Gly Asp Tyr Leu Leu Ala
145                 150                 155                 160
Ala Ser Arg Glu Ala Cys Thr Ala Val Gly Ala Thr Thr Tyr Ser Ala
                165                 170                 175
Thr Phe Gly Val Leu Arg Pro Leu Met Leu Ile Asn Lys Leu Thr Ala
            180                 185                 190
Lys Pro Phe Leu Asp Lys Ala Thr Val Gly Asn Phe Gly Thr Ala Val
        195                 200                 205
Ala Gly Ile Met Thr Ile Asn His Met Ala Gly Val Ala Gly Ala Val
    210                 215                 220
Gly Gly Ile Ala Leu Glu Gln Lys Leu Phe Lys Arg Ala Lys Glu Ser
225                 230                 235                 240
Leu Tyr Asn Glu Arg Cys Ala Leu Glu Asn Gln Gln Ser Gln Leu Ser
                245                 250                 255
Gly Asp Val Ile Leu Ser Ala Glu Arg Ala Leu Arg Lys Glu His Val
            260                 265                 270
Ala Thr Leu Lys Arg Asn Val Leu Thr Leu Leu Glu Lys Ala Leu Glu
        275                 280                 285
Leu Val Val Asp Gly Val Lys Leu Ile Pro Leu Pro Ile Thr Val Ala
    290                 295                 300

Cys Ser Ala Ala Ile Ser Gly Ala Leu Thr Ala Ala Ser Ala Gly Ile
305                 310                 315                 320
Gly Leu Tyr Ser Ile Trp Gln Lys Thr Lys Ser Gly Lys
                325                 330

<210> 293
<211> 7
<212> DNA
<213> Chlamydia

<400> 293
tgcaatc                                                                 7

<210> 294
<211> 196
<212> PRT
<213> Chlamydia

<400> 294
Thr Met Gly Ser Leu Val Gly Arg Gln Ala Pro Asp Phe Ser Gly Lys
                5                   10                  15

Ala Val Val Cys Gly Glu Glu Lys Glu Ile Ser Leu Ala Asp Phe Arg
            20                  25                  30

Gly Lys Tyr Val Val Leu Phe Phe Tyr Pro Lys Asp Phe Thr Tyr Val
        35                  40                  45

Cys Pro Thr Glu Leu His Ala Phe Gln Asp Arg Leu Val Asp Phe Glu
    50                  55                  60

Glu His Gly Ala Val Val Leu Gly Cys Ser Val Asp Asp Ile Glu Thr
65                  70                  75                  80

His Ser Arg Trp Leu Thr Val Ala Arg Asp Ala Gly Gly Ile Glu Gly
                85                  90                  95

Thr Glu Tyr Pro Leu Leu Ala Asp Pro Ser Phe Lys Ile Ser Glu Ala
            100                 105                 110

Phe Gly Val Leu Asn Pro Glu Gly Ser Leu Ala Leu Arg Ala Thr Phe
        115                 120                 125

Leu Ile Asp Lys His Gly Val Ile Arg His Ala Val Ile Asn Asp Leu
    130                 135                 140

Pro Leu Gly Arg Ser Ile Asp Glu Glu Leu Arg Ile Leu Asp Ser Leu
145                 150                 155                 160

Ile Phe Phe Glu Asn His Gly Met Val Cys Pro Ala Asn Trp Arg Ser
                165                 170                 175

Gly Glu Arg Gly Met Val Pro Ser Glu Glu Gly Leu Lys Glu Tyr Phe
            180                 185                 190

Gln Thr Met Asp
        195

<210> 295
<211> 181
<212> PRT
<213> Chlamydia

<400> 295
Lys Gly Gly Lys Met Ser Thr Thr Ile Ser Gly Asp Ala Ser Ser Leu
                  5                  10                  15

Pro Leu Pro Thr Ala Ser Cys Val Glu Thr Lys Ser Thr Ser Ser Ser
             20                  25                  30

Thr Lys Gly Asn Thr Cys Ser Lys Ile Leu Asp Ile Ala Leu Ala Ile
         35                  40                  45

Val Gly Ala Leu Val Val Val Ala Gly Val Leu Ala Leu Val Leu Cys
     50                  55                  60

Ala Ser Asn Val Ile Phe Thr Val Ile Gly Ile Pro Ala Leu Ile Ile
 65                  70                  75                  80

Gly Ser Ala Cys Val Gly Ala Gly Ile Ser Arg Leu Met Tyr Arg Ser
                 85                  90                  95

Ser Tyr Ala Ser Leu Glu Ala Lys Asn Val Leu Ala Glu Gln Arg Leu
            100                 105                 110

Arg Asn Leu Ser Glu Glu Lys Asp Ala Leu Ala Ser Val Ser Phe Ile
        115                 120                 125

Asn Lys Met Phe Leu Arg Gly Leu Thr Asp Asp Leu Gln Ala Leu Glu
    130                 135                 140

Ala Lys Val Met Glu Phe Glu Ile Asp Cys Leu Asp Arg Leu Glu Lys
145                 150                 155                 160

Asn Glu Gln Ala Leu Leu Ser Asp Val Arg Leu Val Leu Ser Ser Tyr
                165                 170                 175

Thr Arg Trp Leu Asp
            180

<210> 296
<211> 124
<212> PRT
<213> Chlamydia

<400> 296
Ile Tyr Glu Val Met Asn Met Asp Leu Glu Thr Arg Arg Ser Phe Ala
                  5                  10                  15

Val Gln Gln Gly His Tyr Gln Asp Pro Arg Ala Ser Asp Tyr Asp Leu
            20                  25                  30

Pro Arg Ala Ser Asp Tyr Asp Leu Pro Arg Ser Pro Tyr Pro Thr Pro
        35                  40                  45

Pro Leu Pro Ser Arg Tyr Gln Leu Gln Asn Met Asp Val Glu Ala Gly
    50                  55                  60

Phe Arg Glu Ala Val Tyr Ala Ser Phe Val Ala Gly Met Tyr Asn Tyr
65                  70                  75                  80

Val Val Thr Gln Pro Gln Glu Arg Ile Pro Asn Ser Gln Gln Val Glu
                85                  90                  95

Gly Ile Leu Arg Asp Met Leu Thr Asn Gly Ser Gln Thr Phe Ser Asn
            100                 105                 110

Leu Met Gln Arg Trp Asp Arg Glu Val Asp Arg Glu
        115                 120


<210> 297
<211> 488
<212> PRT
<213> Chlamydia

<400> 297
Lys Gly Ser Leu Pro Ile Leu Gly Pro Phe Leu Asn Gly Lys Met Gly
                5                   10                  15

Phe Trp Arg Thr Ser Ile Met Lys Met Asn Arg Ile Trp Leu Leu Leu
            20                  25                  30

Leu Thr Phe Ser Ser Ala Ile His Ser Pro Val Arg Gly Glu Ser Leu
        35                  40                  45

Val Cys Lys Asn Ala Leu Gln Asp Leu Ser Phe Leu Glu His Leu Leu
    50                  55                  60

Gln Val Lys Tyr Ala Pro Lys Thr Trp Lys Glu Gln Tyr Leu Gly Trp
65                  70                  75                  80

Asp Leu Val Gln Ser Ser Val Ser Ala Gln Gln Lys Leu Arg Thr Gln
                85                  90                  95

Glu Asn Pro Ser Thr Ser Phe Cys Gln Gln Val Leu Ala Asp Phe Ile
            100                 105                 110

Gly Gly Leu Asn Asp Phe His Ala Gly Val Thr Phe Phe Ala Ile Glu
        115                 120                 125

Ser Ala Tyr Leu Pro Tyr Thr Val Gln Lys Ser Ser Asp Gly Arg Phe
    130                 135                 140

Tyr Phe Val Asp Ile Met Thr Phe Ser Ser Glu Ile Arg Val Gly Asp
145                 150                 155                 160

Glu Leu Leu Glu Val Asp Gly Ala Pro Val Gln Asp Val Leu Ala Thr
                165                 170                 175

Leu Tyr Gly Ser Asn His Lys Gly Thr Ala Ala Glu Glu Ser Ala Ala
            180                 185                 190

Leu Arg Thr Leu Phe Ser Arg Met Ala Ser Leu Gly His Lys Val Pro
        195                 200                 205

Ser Gly Arg Thr Thr Leu Lys Ile Arg Arg Pro Phe Gly Thr Thr Arg
    210                 215                 220

Glu Val Arg Val Lys Trp Arg Tyr Val Pro Glu Gly Val Gly Asp Leu
225                 230                 235                 240

Ala Thr Ile Ala Pro Ser Ile Arg Ala Pro Gln Leu Gln Lys Ser Met
                245                 250                 255

Arg Ser Phe Phe Pro Lys Lys Asp Asp Ala Phe His Arg Ser Ser Ser
            260                 265                 270

Leu Phe Tyr Ser Pro Met Val Pro His Phe Trp Ala Glu Leu Arg Asn
        275                 280                 285

His Tyr Ala Thr Ser Gly Leu Lys Ser Gly Tyr Asn Ile Gly Ser Thr
    290                 295                 300

Asp Gly Phe Leu Pro Val Ile Gly Pro Val Ile Trp Glu Ser Glu Gly
305                 310                 315                 320

Leu Phe Arg Ala Tyr Ile Ser Ser Val Thr Asp Gly Asp Gly Lys Ser
                325                 330                 335

His Lys Val Gly Phe Leu Arg Ile Pro Thr Tyr Ser Trp Gln Asp Met
            340                 345                 350

Glu Asp Phe Asp Pro Ser Gly Pro Pro Pro Trp Glu Glu Phe Ala Lys
        355                 360                 365

Ile Ile Gln Val Phe Ser Ser Asn Thr Glu Ala Leu Ile Ile Asp Gln
    370                 375                 380

Thr Asn Asn Pro Gly Gly Ser Val Leu Tyr Leu Tyr Ala Leu Leu Ser
385                 390                 395                 400

Met Leu Thr Asp Arg Pro Leu Glu Leu Pro Lys His Arg Met Ile Leu
                405                 410                 415

Thr Gln Asp Glu Val Val Asp Ala Leu Asp Trp Leu Thr Leu Leu Glu
            420                 425                 430

Asn Val Asp Thr Asn Val Glu Ser Arg Leu Ala Leu Gly Asp Asn Met
        435                 440                 445

Glu Gly Tyr Thr Val Asp Leu Gln Val Ala Glu Tyr Leu Lys Ser Phe
    450                 455                 460

Gly Arg Gln Val Leu Asn Cys Trp Ser Lys Gly Asp Ile Glu Leu Ser
465                 470                 475                 480

Thr Pro Ile Pro Leu Phe Gly Phe
                485


<210> 298
<211> 140
<212> PRT
<213> Chlamydia

<400> 298
Arg Ile Asp Ile Ser Ser Val Thr Phe Phe Ile Gly Ile Leu Leu Ala
                  5                  10                  15

Val Asn Ala Leu Thr Tyr Ser His Val Leu Arg Asp Leu Ser Val Ser
             20                  25                  30

Met Asp Ala Leu Phe Ser Arg Asn Thr Leu Ala Val Leu Leu Gly Leu
         35                  40                  45

Val Ser Ser Val Leu Asp Asn Val Pro Leu Val Ala Ala Thr Ile Gly
     50                  55                  60

Met Tyr Asp Leu Pro Met Asn Asp Pro Leu Trp Lys Leu Ile Ala Tyr
 65                  70                  75                  80

Thr Ala Gly Thr Gly Gly Ser Ile Leu Ile Ile Gly Ser Ala Ala Gly
                 85                  90                  95

Val Ala Tyr Met Gly Met Glu Lys Val Ser Phe Gly Trp Tyr Val Lys
            100                 105                 110

His Ala Ser Trp Ile Ala Leu Ala Ser Tyr Phe Gly Gly Leu Ala Val
        115                 120                 125

Tyr Phe Leu Met Glu Asn Cys Val Asn Leu Phe Val
    130                 135                 140


<210> 299
<211> 361
<212> PRT
<213> Chlamydia

<400> 299
His Gln Glu Ile Ala Asp Ser Pro Leu Val Lys Lys Ala Glu Glu Gln
                  5                  10                  15

Ile Asn Gln Ala Gln Gln Asp Ile Gln Thr Ile Thr Pro Ser Gly Leu
            20                  25                  30

Asp Ile Pro Ile Val Gly Pro Ser Gly Ser Ala Ala Ser Ala Gly Ser
        35                  40                  45

Ala Ala Gly Ala Leu Lys Ser Ser Asn Asn Ser Gly Arg Ile Ser Leu
    50                  55                  60

Leu Leu Asp Asp Val Asp Asn Glu Met Ala Ala Ile Ala Met Gln Gly
65                  70                  75                  80

Phe Arg Ser Met Ile Glu Gln Phe Asn Val Asn Asn Pro Ala Thr Ala
                85                  90                  95

Lys Glu Leu Gln Ala Met Glu Ala Gln Leu Thr Ala Met Ser Asp Gln
            100                 105                 110

Leu Val Gly Ala Asp Gly Glu Leu Pro Ala Glu Ile Gln Ala Ile Lys
        115                 120                 125

Asp Ala Leu Ala Gln Ala Leu Lys Gln Pro Ser Ala Asp Gly Leu Ala
    130                 135                 140

Thr Ala Met Gly Gln Val Ala Phe Ala Ala Ala Lys Val Gly Gly Gly
145                 150                 155                 160

Ser Ala Gly Thr Ala Gly Thr Val Gln Met Asn Val Lys Gln Leu Tyr
                165                 170                 175

Lys Thr Ala Phe Ser Ser Thr Ser Ser Ser Ser Tyr Ala Ala Ala Leu
            180                 185                 190

Ser Asp Gly Tyr Ser Ala Tyr Lys Thr Leu Asn Ser Leu Tyr Ser Glu
        195                 200                 205

Ser Arg Ser Gly Val Gln Ser Ala Ile Ser Gln Thr Ala Asn Pro Ala
    210                 215                 220

Leu Ser Arg Ser Val Ser Arg Ser Gly Ile Glu Ser Gln Gly Arg Ser
225                 230                 235                 240

Ala Asp Ala Ser Gln Arg Ala Ala Glu Thr Ile Val Arg Asp Ser Gln
                245                 250                 255

Thr Leu Gly Asp Val Tyr Ser Arg Leu Gln Val Leu Asp Ser Leu Met
            260                 265                 270

Ser Thr Ile Val Ser Asn Pro Gln Ala Asn Gln Glu Glu Ile Met Gln
        275                 280                 285

Lys Leu Thr Ala Ser Ile Ser Lys Ala Pro Gln Phe Gly Tyr Pro Ala
    290                 295                 300

Val Gln Asn Ser Val Asp Ser Leu Gln Lys Phe Ala Ala Gln Leu Glu
305                 310                 315                 320

Arg Glu Phe Val Asp Gly Glu Arg Ser Leu Ala Glu Ser Gln Glu Asn
                325                 330                 335

Ala Phe Arg Lys Gln Pro Ala Phe Ile Gln Gln Val Leu Val Asn Ile
            340                 345                 350

Ala Ser Leu Phe Ser Gly Tyr Leu Ser
        355                 360

<210> 300
<211> 207
<212> PRT
<213> Chlamydia

<400> 300
Ser Ser Lys Ile Val Ser Leu Cys Glu Gly Ala Val Ala Asp Ala Arg
                5                   10                  15

Met Cys Lys Ala Glu Leu Ile Lys Lys Glu Ala Asp Ala Tyr Leu Phe
            20                  25                  30

Cys Glu Lys Ser Gly Ile Tyr Leu Thr Lys Lys Glu Gly Ile Leu Ile
        35                  40                  45

Pro Ser Ala Gly Ile Asp Glu Ser Asn Thr Asp Gln Pro Phe Val Leu
    50                  55                  60

Tyr Pro Lys Asp Ile Leu Gly Ser Cys Asn Arg Ile Gly Glu Trp Leu
65                  70                  75                  80

Arg Asn Tyr Phe Arg Val Lys Glu Leu Gly Val Ile Ile Thr Asp Ser
                85                  90                  95

His Thr Thr Pro Met Arg Arg Gly Val Leu Gly Ile Gly Leu Cys Trp
            100                 105                 110

Tyr Gly Phe Ser Pro Leu His Asn Tyr Ile Gly Ser Leu Asp Cys Phe
        115                 120                 125

Gly Arg Pro Leu Gln Met Thr Gln Ser Asn Leu Val Asp Ala Leu Ala
    130                 135                 140

Val Ala Ala Val Val Cys Met Gly Glu Gly Asn Glu Gln Thr Pro Leu
145                 150                 155                 160

Ala Val Ile Glu Gln Ala Pro Asn Met Val Tyr His Ser Tyr Pro Thr
                165                 170                 175

Ser Arg Glu Glu Tyr Cys Ser Leu Arg Ile Asp Glu Thr Glu Asp Leu
            180                 185                 190

Tyr Gly Pro Phe Leu Gln Ala Val Thr Trp Ser Gln Glu Lys Lys
        195                 200                 205

<210> 301
<211> 183
<212> PRT
<213> Chlamydia

<400> 301
Ile Pro Pro Ala Pro Arg Gly His Pro Gln Ile Glu Val Thr Phe Asp
                5                   10                  15

Ile Asp Ala Asn Gly Ile Leu His Val Ser Ala Lys Asp Ala Ala Ser
            20                  25                  30

Gly Arg Glu Gln Lys Ile Arg Ile Glu Ala Ser Ser Gly Leu Lys Glu
        35                  40                  45

Asp Glu Ile Gln Gln Met Ile Arg Asp Ala Glu Leu His Lys Glu Glu
    50                  55                  60

Asp Lys Gln Arg Lys Glu Ala Ser Asp Val Lys Asn Glu Ala Asp Gly
65                  70                  75                  80

Met Ile Phe Arg Ala Glu Lys Ala Val Lys Asp Tyr His Asp Lys Ile
                85                  90                  95

Pro Ala Glu Leu Val Lys Glu Ile Glu Glu His Ile Glu Lys Val Arg
            100                 105                 110

Gln Ala Ile Lys Glu Asp Ala Ser Thr Thr Ala Ile Lys Ala Ala Ser
        115                 120                 125

Asp Glu Leu Ser Thr Arg Met Gln Lys Ile Gly Glu Ala Met Gln Ala
    130                 135                 140

Gln Ser Ala Ser Ala Ala Ala Ser Ser Ala Ala Asn Ala Gln Gly Gly
145                 150                 155                 160

Pro Asn Ile Asn Ser Glu Asp Leu Lys Lys His Ser Phe Ser Thr Arg
                165                 170                 175

Pro Pro Ala Gly Gly Ser Ala
            180

<210> 302
<211> 232
<212> PRT
<213> Chlamydia

<400> 302
Met Thr Lys His Gly Lys Arg Ile Arg Gly Ile Gln Glu Thr Tyr Asp
                5                   10                  15

Leu Ala Lys Ser Tyr Ser Leu Gly Glu Ala Ile Asp Ile Leu Lys Gln
            20                  25                  30

Cys Pro Thr Val Arg Phe Asp Gln Thr Val Asp Val Ser Val Lys Leu
        35                  40                  45

Gly Ile Asp Pro Arg Lys Ser Asp Gln Gln Ile Arg Gly Ser Val Ser
    50                  55                  60

Leu Pro His Gly Thr Gly Lys Val Leu Arg Ile Leu Val Phe Ala Ala
65                  70                  75                  80

Gly Asp Lys Ala Ala Glu Ala Ile Glu Ala Gly Ala Asp Phe Val Gly
                85                  90                  95

Ser Asp Asp Leu Val Glu Lys Ile Lys Gly Gly Trp Val Asp Phe Asp
            100                 105                 110

Val Ala Val Ala Thr Pro Asp Met Met Arg Glu Val Gly Lys Leu Gly
        115                 120                 125

Lys Val Leu Gly Pro Arg Asn Leu Met Pro Thr Pro Lys Ala Gly Thr
    130                 135                 140

Val Thr Thr Asp Val Val Lys Thr Ile Ala Glu Leu Arg Lys Gly Lys
145                 150                 155                 160

Ile Glu Phe Lys Ala Asp Arg Ala Gly Val Cys Asn Val Gly Val Ala
                165                 170                 175

Lys Leu Ser Phe Asp Ser Ala Gln Ile Lys Glu Asn Val Glu Ala Leu
            180                 185                 190

Cys Ala Ala Leu Val Lys Ala Lys Pro Ala Thr Ala Lys Gly Gln Tyr
        195                 200                 205

Leu Val Asn Phe Thr Ile Ser Ser Thr Met Gly Pro Gly Val Thr Val
    210                 215                 220

Asp Thr Arg Glu Leu Ile Ala Leu
225                 230

<210> 303
<211> 238
<212> PRT
<213> chlamydia

<400> 303
Ile Asn Ser Lys Leu Glu Thr Lys Asn Leu Ile Tyr Leu Lys Leu Lys
                5                   10                  15

Ile Lys Lys Ser Phe Lys Met Gly Asn Ser Gly Phe Tyr Leu Tyr Asn
            20                  25                  30

Thr Gln Asn Cys Val Phe Ala Asp Asn Ile Lys Val Gly Gln Met Thr
        35                  40                  45

Glu Pro Leu Lys Asp Gln Gln Ile Ile Leu Gly Thr Thr Ser Thr Pro
    50                  55                  60

Val Ala Ala Lys Met Thr Ala Ser Asp Gly Ile Ser Leu Thr Val Ser
65                  70                  75                  80

Asn Asn Pro Ser Thr Asn Ala Ser Ile Thr Ile Gly Leu Asp Ala Glu
                85                  90                  95

Lys Ala Tyr Gln Leu Ile Leu Glu Lys Leu Gly Asp Gln Ile Leu Gly
            100                 105                 110

Gly Ile Ala Asp Thr Ile Val Asp Ser Thr Val Gln Asp Ile Leu Asp
        115                 120                 125

Lys Ile Thr Thr Asp Pro Ser Leu Gly Leu Leu Lys Ala Phe Asn Asn
    130                 135                 140

Phe Pro Ile Thr Asn Lys Ile Gln Cys Asn Gly Leu Phe Thr Pro Arg
145                 150                 155                 160

Asn Ile Glu Thr Leu Leu Gly Gly Thr Glu Ile Gly Lys Phe Thr Val
                165                 170                 175

Thr Pro Lys Ser Ser Gly Ser Met Phe Leu Val Ser Ala Asp Ile Ile
            180                 185                 190

Ala Ser Arg Met Glu Gly Gly Val Val Leu Ala Leu Val Arg Glu Gly
        195                 200                 205

Asp Ser Lys Pro Tyr Ala Ile Ser Tyr Gly Tyr Ser Ser Gly Val Pro
    210                 215                 220

Asn Leu Cys Ser Leu Arg Thr Arg Ile Ile Asn Thr Gly Leu
225                 230                 235

## Description

### TECHNICAL FIELD

The present invention relates generally to the detection and treatment of Chlamydial infection. In particular, the invention is related to polypeptides comprising a *Chlamydia* antigen and the use of such polypeptides for the serodiagnosis and treatment of Chlamydial infection.

### BACKGROUND OF THE INVENTION

Chlamydiae are intracellular bacterial pathogens that are responsible for a wide variety of important human and animal infections. *Chlamydia trachomatis* is one of the most common causes of sexually transmitted diseases and can lead to pelvic inflammatory disease (PID), resulting in tubal obstruction and infertility. *Chlamydia trachomatis* may also play a role in male infertility. In 1990, the cost of treating PID in the US was estimated to be $4 billion. Trachoma, due to ocular infection with *Chlamydia trachomatis,* is the leading cause of preventable blindness worldwide. *Chlamydia pneumonia* is a major cause of acute respiratory tract infections in humans and is also believed to play a role in the pathogenesis of atherosclerosis and, in particular, coronary heart disease. Individuals with a high titer of antibodies to *Chlamydia pneumonia* have been shown to be at least twice as likely to suffer from coronary heart disease as seronegative individuals. Chlamydial infections thus constitute a significant health problem both in the US and worldwide.

Chlamydial infection is often asymptomatic. For example, by the time a woman seeks medical attention for PID, irreversible damage may have already occurred resulting in infertility. There thus remains a need in the art for improved vaccines and pharmaceutical compositions for the prevention and treatment of *Chlamydia* infections. The present invention fulfills this need and further provides other related advantages.

### SUMMARY OF THE INVENTION

The present invention provides compositions and methods for the diagnosis and therapy of *Chlamydia* infection. In one aspect, the present invention provides polypeptides comprising an immunogenic portion of a *Chlamydia* antigen, or a variant of such an antigen. Certain portions and other variants are immunogenic, such that the ability of the variant to react with antigen-specific antisera is not substantially diminished. Within certain embodiments,, the polypeptide comprises an amino acid sequence encoded by a polynucleotide sequence selected from the group consisting of (a) a sequence of SEQ ID NO: 1, 15, 21-25, 44-64, 66-76, 79-88, 110-119, 120, 122, 124, 126, 128, 130, 132, 134, 136, 169-174, 181-188, 263, 265 and 267-290; (b) the complements of said sequences; and (c) sequences that hybridize to a sequence of (a) or (b) under moderately stringent conditions. In specific embodiments, the polypeptides of the present invention comprise at least a portion of a *Chlamydial* protein that includes an amino acid sequence selected from the group consisting of sequences recited in SEQ ID NO: 5-14, 17-20, 26, 28, 30-32, 34, 39-43, 65, 89-109, 138-158, 167, 168, 224-262, 246, 247, 254-256, 292, and variants thereof.

The present invention further provides polynucleotides that encode a polypeptide as described above, or a portion thereof (such as a portion encoding at least 15 amino acid residues of a *Chlamydial* protein), expression vectors comprising such polynucleotides and host cells transformed or transfected with such expression vectors.

In a related aspect, polynucleotide sequences encoding the above polypeptides, recombinant expression vectors comprising one or more of these polynucleotide sequences and host cells transformed or transfected with such expression vectors are also provided.

In another aspect, the present invention provides fusion proteins comprising an inventive polypeptide, or, alternatively, an inventive polypeptide and a known *Chlamydia* antigen, as well as polynucleotides encoding such fusion proteins, in combination with a physiologically acceptable carrier or immunostimulant for use as pharmaceutical compositions and vaccines thereof.

The present invention further provides pharmaceutical compositions that comprise: (a) an antibody, both polyclonal and monoclonal, or antigen-binding fragment thereof that specifically binds to a *Chlamydial* protein; and (b) a physiologically acceptable carrier.Within other aspects, the present invention provides pharmaceutical compositions that comprise one or more *Chlamydia* polypeptides disclosed herein, or a polynucleotide molecule encoding such a polypeptide, and a physiologically acceptable carrier. The invention also provides vaccines for prophylactic and therapeutic purposes comprising one or more of the disclosed polypeptides and an immunostimulant, as defined herein, together with vaccines comprising one or more polynucleotide sequences encoding such polypeptides and an immunostimulant.

In yet another aspect, methods are provided for inducing protective immunity in a patient, comprising administering to a patient an effective amount of one or more of the above pharmaceutical compositions or vaccines.

In yet a further aspect, methods for the treatment of *Chlamydia* infection in a patient are provided, the methods comprising obtaining peripheral blood mononuclear cells (PBMC) from the patient, incubating the PBMC with a polypeptide of the present invention (or a polynucleotide that encodes such a polypeptide) to provide incubated T cells and administering the incubated T cells to the patient. The present invention additionally provides methods for the treatment of *Chlamydia* infection that comprise incubating antigen presenting cells with a polypeptide of the present invention (or a polynucleotide that encodes such a polypeptide) to provide incubated antigen presenting cells and administering the incubated antigen presenting cells to the patient. Proliferated cells may, but need not, be cloned prior to administration to the patient. In certain embodiments, the antigen presenting cells are selected from the group consisting of dendritic cells, macrophages, monocytes, B-cells, and fibroblasts. Compositions for the treatment of *Chlamydia* infection comprising T cells or antigen presenting cells that have been incubated with a polypeptide or polynucleotide of the present invention are also provided. Within related aspects, vaccines are provided that comprise: (a) an antigen presenting cell that expresses a polypeptide as described above and (b) an immunostimulant.

The present invention further provides, within other aspects, methods for removing *Chlamydial*-infected cells from a biological sample, comprising contacting a biological sample with T cells that specifically react with a *Chlamydial* protein, wherein the step of contacting is performed under conditions and for a time sufficient to permit the removal of cells expressing the protein from the sample.

Within related aspects, methods are provided for inhibiting the development of *Chlamydial* infection in a patient, comprising administering to a patient a biological sample treated as described above.In further aspects of the subject invention, methods and diagnostic kits are provided for detecting *Chlamydia* infection in a patient. In one embodiment, the method comprises: (a) contacting a biological sample with at least one of the polypeptides or fusion proteins disclosed herein; and (b) detecting in the sample the presence of binding agents that bind to the polypeptide or fusion protein, thereby detecting *Chlamydia* infection in the biological sample. Suitable biological samples include whole blood, sputum, serum, plasma, saliva, cerebrospinal fluid and urine. In one embodiment, the diagnostic kits comprise one or more of the polypeptides or fusion proteins disclosed herein in combination with a detection reagent. In yet another embodiment, the diagnostic kits comprise either a monoclonal antibody or a polyclonal antibody that binds with a polypeptide of the present invention.

The present invention also provides methods for detecting *Chlamydia* infection comprising: (a) obtaining a biological sample from a patient; (b) contacting the sample with at least two oligonucleotide primers in a polymerase chain reaction, at least one of the oligonucleotide primers being specific for a polynucleotide sequence disclosed herein; and (c) detecting in the sample a polynucleotide sequence that amplifies in the presence of the oligonucleotide primers. In one embodiment, the oligonucleotide primer comprises at least about 10 contiguous nucleotides of a polynucleotide sequence peptide disclosed herein, or of a sequence that hybridizes thereto.

In a further aspect, the present invention provides a method for detecting *Chlamydia* infection in a patient comprising: (a) obtaining a biological sample from the patient; (b) contacting the sample with an oligonucleotide probe specific for a polynucleotide sequence disclosed herein; and (c) detecting in the sample a polynucleotide sequence that hybridizes to the oligonucleotide probe. In one embodiment, the oligonucleotide probe comprises at least about 15 contiguous nucleotides of a polynucleotide sequence disclosed herein, or a sequence that hybridizes thereto.

These and other aspects of the present invention will become apparent upon reference to the following detailed description. All references disclosed herein are hereby incorporated by reference in their entirety as if each was incorporated individually.

### SEQUENCE IDENTIFIERS

SEQ ID NO: 1 is the determined DNA sequence for the *C. trachomatis* clone 1-B1-66.
SEQ ID NO: 2 is the determined DNA sequence for the *C. trachomatis* clone 4-D7-28.
SEQ ID NO: 3 is the determined DNA sequence for the *C. trachomatis* clone 3-G3-10.
SEQ ID NO: 4 is the determined DNA sequence for the *C. trachomatis* clone 10-C10-31.
SEQ ID NO: 5 is the predicted amino acid sequence for 1-B1-66.
SEQ ID NO: 6 is the predicted amino acid sequence for 4-D7-28.
SEQ ID NO: 7 is a first predicted amino acid sequence for 3-G3-10.
SEQ ID NO: 8 is a second predicted amino acid sequence for 3-G3-10.
SEQ ID NO: 9 is a third predicted amino acid sequence for 3-G3-10.
SEQ ID NO: 10 is a fourth predicted amino acid sequence for 3-G3-10.
SEQ ID NO: 11 is a fifth predicted amino acid sequence for 3-G3-10.
SEQ ID NO: 12 is the predicted amino acid sequence for 10-C10-31.
SEQ ID NO: 13 is the amino acid sequence of the synthetic peptide 1-B1-66/48-67.
SEQ ID NO: 14 is the amino acid sequence of the synthetic peptide 1-B1-66/58-77.
SEQ ID NO: 15 is the determined DNA sequence for the *C. trachomatis* serovar LGV II clone 2C7-8
SEQ ID NO: 16 is the determined DNA sequence for a first putative open reading frame from *C. trachomatis* serovar D
SEQ ID NO: 17 is the predicted amino acid sequence encoded by the first putative open reading frame from *C. trachomatis* serovar D
SEQ ID NO: 18 is the amino acid sequence of the synthetic peptide CtC7.8-12
SEQ ID NO: 19 is the amino acid sequence of the synthetic peptide CtC7.8-13
SEQ ID NO: 20 is the predicted amino acid sequence encoded by a second putative open reading from *C. trachomatis* serovar D
SEQ ID NO: 21 is the determined DNA sequence for clone 4C9-18 from *C. trachomatis* LGV II
SEQ ID NO: 22 is the determined DNA sequence homologous to Lipoamide Dehydrogenase from *C. trachomatis* LGV II
SEQ ID NO: 23 is the determined DNA sequence homologous to Hypothetical protein from *C. trachomatis* LGV II
SEQ ID NO: 24 is the determined DNA sequence homologous to Ubiquinone Mehtyltransferase from *C. trachomatis* LGV II
SEQ ID NO: 25 is the determined DNA sequence for clone 4C9-18#2 BL21 pLysS from *C. trachomatis* LGV II
SEQ ID NO: 26 is the predicted amino acid sequence for 4C9-18#2 from *C. trachomatis* LGV II
SEQ ID NO: 27 is the determined DNA sequence for Cp-SWIB from *C. pneumonia* strain TWAR
SEQ ID NO: 28 is the predicted amino acid sequence for Cp-SWIB from *C. pneumonia* strain TWAR
SEQ ID NO: 29 is the determined DNA sequence for Cp-S13 from *C. pneumonia* strain TWAR
SEQ ID NO: 30 is the predicted amino acid sequence for Cp-S13 from *C. pneumonia* strain TWAR
SEQ ID NO: 31 is the amino acid sequence for a 10mer consensus peptide from CtC7.8-12 and CtC7.8-13
SEQ ID NO: 32 is the predicted amino acid sequence for clone 2C7-8 from *C. trachomatis* LGV II
SEQ ID NO: 33 is the determined DNA sequence of a clone from *C. trachomatis* serovar D which shows homology to clone 2C7-8
SEQ ID NO: 34 is the predicted amino acid sequence encoded by the sequence of SEQ ID NO: 33
SEQ ID NO: 35 is the DNA sequence for C.p. SWIB Nde (5' primer) from *C. pneumonia*
SEQ ID NO: 36 is the DNA sequence for C.p. SWIB EcoRI (3' primer) from *C. pneumonia*
SEQ ID NO : 37 is the DNA sequence for C.p. S 13 Nde (5' primer) from *C. pneumonia*
SEQ ID NO: 38 is the DNA sequence for C.p. S13 EcoRI (3' primer) from *C. pneumonia*
SEQ ID NO: 39 is the amino acid sequence for CtSwib 52-67 peptide from *C. trachomatis* LGV II
SEQ ID NO: 40 is the amino acid sequence for CpSwib 53-68 peptide from *C. pneumonia*
SEQ ID NO: 41 is the amino acid sequence for HuSwib 288-302 peptide from Human SWI domain
SEQ ID NO: 42 is the amino acid sequence for CtSWI-T 822-837 peptide from the topoisomerase-SWIB fusion of *C. trachomatis*
SEQ ID NO: 43 is the amino acid sequence for CpSWI-T 828-842 peptide from the topoisomerase-SWIB fusion of *C. pneumonia*
SEQ ID NO: 44 is a first determined DNA sequence for the *C. trachomatis* LGV II clone 19783.3,jen.seq(1>509)CTL2#11-3', representing the 3' end.
SEQ ID NO: 45 is a second determined DNA sequence for the *C. trachomatis* LGV II clone 19783.4,jen.seq(1>481)CTL2#11-5', representing the 5' end.
SEQ ID NO: 46 is the determined DNA sequence for the *C. trachomatis* LGV II clone 19784CTL2_12consensus.seq(1>427)CTL2#12.
SEQ ID NO: 47 is the determined DNA sequence for the *C. trachomatis* LGV II clone 19785.4,jen.seq(1>600)CTL2#16-5', representing the 5' end.
SEQ ID NO: 48 is a first determined DNA sequence for the *C. trachomatis* LGV II clone 19786.3,jen.seq(1>600)CTL2#18-3', representing the 3' end.
SEQ ID NO: 49 is a second determined DNA sequence for the *C. trachomatis* LGV II clone 19786.4,jen.seq(1)600)CTL2#18-5', representing the 5' end.
SEQ ID NO: 50 is the determined DNA sequence for the *C. trachomatis* LGV II clone 19788CTL2_21consensus.seq(1>406)CTL2#21.
SEQ ID NO: 51 is the determined DNA sequence for the *C. trachomatis* LGV II clone 19790CTL2 23consensus.seq(1>602)CTL2#23.
SEQ ID NO: 52 is the determined DNA sequence for the *C. trachomatis* LGV II clone 19791 CTL2_24consensus.seq(1)145)CTL2#24.
SEQ ID NO: 53 is the determined DNA sequence for the *C. trachomatis* LGV II clone CTL2#4.
SEQ ID NO: 54 is the determined DNA sequence for the *C. trachomatis* LGV II clone CTL2#8b.
SEQ ID NO: 55 is the determined DNA sequence for the *C. trachomatis* LGV II clone15-G1-89, sharing homology to the lipoamide dehydrogenase gene CT557.
SEQ ID NO: 56 is the determined DNA sequence for the *C. trachomatis* LGV II clone 14-H1-4, sharing homology to the thiol specific antioxidant gene CT603.
SEQ ID NO: 57 is the determined DNA sequence for the *C. trachomatis* LGV II clone 12-G3-83, sharing homology to the hypothetical protein CT622.
SEQ ID NO: 58 is the determined DNA sequence for the *C. trachomatis* LGV II clone 12-B3-95, sharing homology to the lipoamide dehydrogenase gene CT557.
SEQ ID NO: 59 is the determined DNA sequence for the *C. trachomatis* LGV II clone 11-H4-28, sharing homology to the dnaK gene CT396.
SEQ ID NO: 60 is the determined DNA sequence for the *C. trachomatis* LGV II clone 11-H3-68, sharing partial homology to the PGP6-D virulence protein and L1 ribosomal gene CT318.
SEQ ID NO: 61 is the determined DNA sequence for the *C. trachomatis* LGV II clone 11-G1-34, sharing partial homology to the malate dehydrogenase gene CT376 and to the glycogen hydrolase gene CT042.
SEQ ID NO: 62 is the determined DNA sequence for the *C. trachomatis* LGV II clone 11-G10-46, sharing homology to the hypothetical protein CT610.
SEQ ID NO: 63 is the determined DNA sequence for the *C. trachomatis* LGV II clone 11-C12-91, sharing homology to the OMP2 gene CT443.
SEQ ID NO: 64 is the determined DNA sequence for the *C. trachomatis* LGV II clone 11-A3-93, sharing homology to the HAD superfamily gene CT103.
SEQ ID NO: 65 is the determined amino acid sequence for the *C. trachomatis* LGV II clone 14-H1-4, sharing homology to the thiol specific antioxidant gene CT603.
SEQ ID NO: 66 is the determined DNA sequence for the *C. trachomatis* LGV II clone CtL2#9.
SEQ ID NO: 67 is the determined DNA sequence for the *C. trachomatis* LGV II clone CtL2#7.
SEQ ID NO: 68 is the determined DNA sequence for the *C. trachomatis* LGV II clone CtL2#6.
SEQ ID NO: 69 is the determined DNA sequence for the *C. trachomatis* LGV II clone CtL2#5.
SEQ ID NO: 70 is the determined DNA sequence for the *C. trachomatis* LGV II clone CtL2#2.
SEQ ID NO: 71 is the determined DNA sequence for the *C. trachomatis* LGV II clone CtL2#1.
SEQ ID NO: 72 is a first determined DNA sequence for the *C. trachomatis* LGV II clone 23509.2CtL2#3-5', representing the 5' end.
SEQ ID NO: 73 is a second determined DNA sequence for the *C. trachomatis* LGV II clone 23509. 1CtL2#3-3', representing the 3' end.
SEQ ID NO: 74 is a first determined DNA sequence for the *C. trachomatis* LGV II clone 22121.2CtL2#10-5', representing the 5' end.
SEQ ID NO: 75 is a second determined DNA sequence for the *C. trachomatis* LGV II clone 22121.1CtL2#10-3', representing the 3' end.
SEQ ID NO: 76 is the determined DNA sequence for the *C. trachomatis* LGV II clone 19787.6CtL2#19-5', representing the 5' end.
SEQ ID NO: 77 is the determined DNA sequence for the *C. pneumoniae* LGV II clone CpS13-His.
SEQ ID NO: 78 is the determined DNA sequence for the *C. pneumoniae* LGV II clone Cp_SWIB-His.
SEQ ID NO: 79 is the determined DNA sequence for the *C. trachomatis* LGV II clone 23-G7-68, sharing partial homology to the L11, L10 and L1 ribosomal protein.
SEQ ID NO: 80 is the determined DNA sequence for the *C. trachomatis* LGV II clone 22-F8-91, sharing homology to the pmpC gene.
SEQ ID NO: 81 is the determined DNA sequence for the *C. trachomatis* LGV II clone 21-E8-95, sharing homology to the CT610-CT613 genes.
SEQ ID NO: 82 is the determined DNA sequence for the *C. trachomatis* LGV II clone 19-F12-57, sharing homology to the CT858 and recA genes.
SEQ ID NO: 83 is the determined DNA sequence for the *C. trachomatis* LGV II clone 19-F12-53, sharing homology to the CT445 gene encoding glutamyl tRNA synthetase.
SEQ ID NO: 84 is the determined DNA sequence for the *C. trachomatis* LGV II clone 19-A5-54, sharing homology to the cryptic plasmid gene.
SEQ ID NO: 85 is the determined DNA sequence for the *C. trachomatis* LGV II clone 17-E11-72, sharing partial homology to the OppC_2 and pmpD genes.
SEQ ID NO: 86 is the determined DNA sequence for the *C. trachomatis* LGV II clone 17-C1-77, sharing partial homology to the CT857 and CT858 open reading frames.
SEQ ID NO: 87 is the determined DNA sequence for the *C. trachomatis* LGV II clone 15-H2-76, sharing partial homology to the pmpD and SycE genes, and to the CT089 ORF.
SEQ ID NO: 88 is the determined DNA sequence for the *C. trachomatis* LGV II clone 15-A3-26, sharing homology to the CT858 ORF.
SEQ ID NO: 89 is the determined amino acid sequence for the *C. pnuemoniae* clone Cp_SWIB-His.
SEQ ID NO: 90 is the determined amino acid sequence for the *C. trachomatis* LGV II clone CtL2_LPDA_FL.
SEQ ID NO: 91 is the determined amino acid sequence for the *C. pnuemoniae* clone CpS13-His.
SEQ ID NO: 92 is the determined amino acid sequence for the *C. trachomatis* LGV II clone CtL2_TSA_FL.
SEQ ID NO: 93 is the amino acid sequence for Ct-Swib 43-61 peptide from *C. trachomatis* LGV II.
SEQ ID NO: 94 is the amino acid sequence for Ct-Swib 48-67 peptide from *C. trachomatis* LGV II.
SEQ ID NO: 95 is the amino acid sequence for Ct-Swib 52-71 peptide from *C. trachomatis* LGV II.
SEQ ID NO: 96 is the amino acid sequence for Ct-Swib 58-77 peptide from *C. trachomatis* LGV II.
SEQ ID NO: 97 is the amino acid sequence for Ct-Swib 63-82 peptide from *C. trachomatis* LGV II.
SEQ ID NO: 98 is the amino acid sequence for Ct-Swib 51-66 peptide from *C. trachomatis* LGV II.
SEQ ID NO: 99 is the amino acid sequence for Cp-Swib 52-67 peptide from *C. pneumonia.*
SEQ ID NO: 100 is the amino acid sequence for Cp-Swib 37-51 peptide from *C. pneumonia.*
SEQ ID NO: 101 is the amino acid sequence for Cp-Swib 32-51 peptide from *C. pneumonia.*
SEQ ID NO: 102 is the amino acid sequence for Cp-Swib 37-56 peptide from *C. pneumonia.*
SEQ ID NO: 103 is the amino acid sequence for Ct-Swib 36-50 peptide from *C. trachomatis.*
SEQ ID NO: 104 is the amino acid sequence for Ct-S 13 46-65 peptide from *C. trachomatis.*
SEQ ID NO: 105 is the amino acid sequence for Ct-S13 60-80 peptide from *C. trachomatis.*
SEQ ID NO: 106 is the amino acid sequence for Ct-S13 1-20 peptide from *C. trachomatis.*
SEQ ID NO: 107 is the amino acid sequence for Ct-S13 46-65 peptide from *C. trachomatis.*
SEQ ID NO: 108 is the amino acid sequence for Ct-S 13 56-75 peptide from *C. trachomatis.*
SEQ ID NO: 109 is the amino acid sequence for Cp-S13 56-75 peptide from *C. pneumoniae.*
SEQ ID NO: 110 is the determined DNA sequence for the *C. trachomatis* LGV II clone 21-G12-60, containing partial open reading frames for hypothetical proteins CT875, CT229 and CT228.
SEQ ID NO: 111 is the determined DNA sequence for the *C. trachomatis* LGV II clone 22-B3-53, sharing homology to the CT110 ORF of GroEL.
SEQ ID NO: 112 is the determined DNA sequence for the *C. trachomatis* LGV II clone 22-A1-49, sharing partial homology to the CT660 and CT659 ORFs.
SEQ ID NO: 113 is the determined DNA sequence for the *C. trachomatis* LGV II clone 17-E2-9, sharing partial homology to the CT611 and CT 610 ORFs.
SEQ ID NO: 114 is the determined DNA sequence for the *C. trachomatis* LGV II clone 17-C10-31, sharing partial homology to the CT858 ORF.
SEQ ID NO: 115 is the determined DNA sequence for the *C. trachomatis* LGV II clone 21-C7-66, sharing homology to the dnaK-like gene.
SEQ ID NO: 116 is the determined DNA sequence for the *C. trachomatis* LGV II clone 20-G3-45, containing part of the pmpB gene CT413.
SEQ ID NO: 117 is the determined DNA sequence for the *C. trachomatis* LGV II clone 18-C5-2, sharing homology to the S1 ribosomal protein ORF.
SEQ ID NO: 118 is the determined DNA sequence for the *C. trachomatis* LGVII clone 17-C5-19, containing part of the ORFs for CT431 and CT430.
SEQ ID NO: 119 is the determined DNA sequence for the *C. trachomatis* LGV II clone 16-D4-22, contains partial sequences of ORF3 and ORF4 of the plasmid for growth within mammalian cells.
SEQ ID NO: 120 is the determined full-length DNA sequence for the *C. trachomatis* serovar LGV II Cap1 gene CT529.
SEQ ID NO: 121 is the predicted full-length amino acid sequence for the *C. trachomatis* serovar LGV II Cap1 gene CT529.
SEQ ID NO: 122 is the determined full-length DNA sequence for the *C. trachomatis* serovar E Cap1 gene CT529.
SEQ ID NO: 123 is the predicted full-length amino acid sequence for the *C. trachomatis* serovar E Cap1 gene CT529.
SEQ ID NO: 124 is the determined full-length DNA sequence for the *C. trachomatis* serovar 1A Cap1 gene CT529.
SEQ ID NO: 125 is the predicted full-length amino acid sequence for the *C. trachomatis* serovar 1A Cap1 gene CT529.
SEQ ID NO: 126 is the determined full-length DNA sequence for the *C. trachomatis* serovar G Cap1 gene CT529.
SEQ ID NO: 127 is the predicted full-length amino acid sequence for the *C. trachomatis* serovar G Cap1 gene CT529.
SEQ ID NO: 128 is the determined full-length DNA sequence for the *C. trachomatis* serovar F1 NII Cap1 gene CT529.
SEQ ID NO: 129 is the predicted full-length amino acid sequence for the *C. trachomatis* serovar F1 NII Cap1 gene CT529.
SEQ ID NO: 130 is the determined full-length DNA sequence for the *C. trachomatis* serovar L1 Cap1 gene CT529.
SEQ ID NO: 131 is the predicted full-length amino acid sequence for the *C. trachomatis* serovar L1 Cap1 gene CT529.
SEQ ID NO: 132 is the determined full-length DNA sequence for the *C. trachomatis* serovar L3 Cap1 gene CT529.
SEQ ID NO: 133 is the predicted full-length amino acid sequence for the *C. trachomatis* serovar L3 Cap1 gene CT529.
SEQ ID NO: 134 is the determined full-length DNA sequence for the *C. trachomatis* serovar Ba Cap1 gene CT529.
SEQ ID NO: 135 is the predicted full-length amino acid sequence for the *C. trachomatis* serovar Ba Cap1 gene CT529.
SEQ ID NO: 136 is the determined full-length DNA sequence for the *C. trachomatis* serovar MOPN Cap1 gene CT529.
SEQ ID NO: 137 is the predicted full-length amino acid sequence for the *C. trachomatis* serovar MOPN Cap1 gene CT529.
SEQ ID NO: 138 is the determined amino acid sequence for the Cap1 CT529 ORF peptide #124-139 of *C. trachomatis* serovar L2.
SEQ ID NO: 139 is the determined amino acid sequence for the Cap1 CT529 ORF peptide #132-147 of *C. trachomatis* serovar L2.
SEQ ID NO: 140 is the determined amino acid sequence for the Cap1 CT529 ORF peptide #138-155 of *C. trachomatis* serovar L2.
SEQ ID NO: 141 is the determined amino acid sequence for the Cap1 CT529 ORF peptide #146-163 of *C. trachomatis* serovar L2.
SEQ ID NO: 142 is the determined amino acid sequence for the Cap1 CT529 ORF peptide #154-171 of *C. trachomatis* serovar L2.
SEQ ID NO: 143 is the determined amino acid sequence for the Cap1 CT529 ORF peptide #162-178 of *C. trachomatis* serovar L2.
SEQ ID NO: 144 is the determined amino acid sequence for the Cap1 CT529 ORF peptide #138-147 of *C. trachomatis* serovar L2.
SEQ ID NO: 145 is the determined amino acid sequence for the Cap1 CT529 ORF peptide #139-147 of *C. trachomatis* serovar L2.
SEQ ID NO: 146 is the determined amino acid sequence for the Cap1 CT529 ORF peptide #140-147 of *C. trachomatis* serovar L2.
SEQ ID NO: 147 is the determined amino acid sequence for the Cap1 CT529 ORF peptide #138-146 of *C. trachomatis* serovar L2.
SEQ ID NO: 148 is the determined amino acid sequence for the Cap1 CT529 ORF peptide #138-145 of *C. trachomatis* serovar L2.
SEQ ID NO: 149 is the determined amino acid sequence for the Cap1 CT529 ORF peptide # F140->I of *C. trachomatis* serovar L2.
SEQ ID NO: 150 is the determined amino acid sequence for the Cap1 CT529 ORF peptide # #S139>Ga of *C. trachomatis* serovar L2.
SEQ ID NO: 151 is the determined amino acid sequence for the Cap1 CT529 ORF peptide # #S139>Gb of *C. trachomatis* serovar L2.
SEQ ID NO: 152 is the determined amino acid sequence for the peptide # 2 C7.8-6 of the 216aa ORF of *C. trachomatis* serovar L2.
SEQ ID NO: 153 is the determined amino acid sequence for the peptide # 2 C7.8-7 of the 216aa ORF of *C. trachomatis* serovar L2.
SEQ ID NO: 154 is the determined amino acid sequence for the peptide # 2 C7.8-8 of the 216aa ORF of *C. trachomatis* serovar L2.
SEQ ID NO: 155 is the determined amino acid sequence for the peptide # 2 C7.8-9 of the 216aa ORF of *C. trachomatis* serovar L2.
SEQ ID NO: 156 is the determined amino acid sequence for the peptide # 2 C7.8-10 of the 216aa ORF of *C. trachomatis* serovar L2.
SEQ ID NO: 157 is the determined amino acid sequence for the 53 amino acid residue peptide of the 216aa ORF within clone 2C7.8 of *C. trachomatis* serovar L2.
SEQ ID NO: 158 is the determined amino acid sequence for the 52 amino acid residue peptide of the CT529 ORF within clone 2C7.8 of *C. trachomatis* serovar L2.
SEQ ID NO: 159 is the determined DNA sequence for the 5' (forward) primer for cloning full-length CT529 serovar L2.
SEQ ID NO: 160 is the determined DNA sequence for the 5' (reverse) primer for cloning full-length CT529 serovar L2.
SEQ ID NO: 161 is the determined DNA sequence for the 5' (forward) primer for cloning full-length CT529 for serovars other than L2 and MOPN.
SEQ ID NO: 162 is the determined DNA sequence for the 5' (reverse) primer for cloning full-length CT529 serovars other than L2 and MOPN.
SEQ ID NO: 163 is the determined DNA sequence for the 5' (forward) primer for cloning full-length CT529 serovar MOPN.
SEQ ID NO: 164 is the determined DNA sequence for the 5' (reverse) primer for cloning full-length CT529 serovar MOPN.
SEQ ID NO: 165 is the determined DNA sequence for the 5' (forward) primer for pBIB-KS.
SEQ ID NO: 166 is the determined DNA sequence for the 5' (reverse) primer for pBIB-KS.
SEQ ID NO: 167 is the determined amino acid sequence for the 9-mer epitope peptide Cap1#139-147 from serovar L2.
SEQ ID NO: 168 is the determined amino acid sequence for the 9-mer epitope peptide Cap1#139-147 from serovar D.
SEQ ID NO: 169 is the determined full-length DNA sequence for the *C. trachomatis* pmpI gene.
SEQ ID NO: 170 is the determined full-length DNA sequence for the *C. trachomatis* pmpG gene.
SEQ ID NO: 171 is the determined full-length DNA sequence for the *C. trachomatis* pmpE gene.
SEQ ID NO: 172 is the determined full-length DNA sequence for the *C. trachomatis* pmpD gene.
SEQ ID NO: 173 is the determined full-length DNA sequence for the *C. trachomatis* pmpC gene.
SEQ ID NO: 174 is the determined full-length DNA sequence for the *C. trachomatis* pmpB gene.
SEQ ID NO: 175 is the predicted full-length amino acid sequence for the *C. trachomatis* pmpI gene.
SEQ ID NO: 176 is the predicted full-length amino acid sequence for the *C. trachomatis* pmpG gene.
SEQ ID NO: 177 is the predicted full-length amino acid sequence for the *C. trachomatis* pmpE gene.
SEQ ID NO: 178 is the predicted full-length amino acid sequence for the *C. trachomatis* pmpD gene.
SEQ ID NO: 179 is the predicted full-length amino acid sequence for the *C. trachomatis* pmpC gene.
SEQ ID NO: 180 is the predicted full-length amino acid sequence for the *C. trachomatis* pmpB gene.
SEQ ID NO: 181 is the determined DNA sequence minus the signal sequence for the *C. trachomatis* pmpI gene.
SEQ ID NO: 182 is a subsequently determined full-length DNA sequence for the *C. trachomatis* pmpG gene.
SEQ ID NO: 183 is the determined DNA sequence minus the signal sequence for the *C. trachomatis* pmpE gene.
SEQ ID NO: 184 is a first determined DNA sequence representing the carboxy terminus for the *C. trachomatis* pmpD gene.
SEQ ID NO: 185 is a second determined DNA sequence representing the amino terminus minus the signal sequnce for the *C. trachomatis* pmpD gene.
SEQ ID NO: 186 is a first determined DNA sequence representing the carboxy terminus for the *C. trachomatis* pmpC gene.
SEQ ID NO: 187 is a second determined DNA sequence representing the amino terminus minus the signal sequence for the *C. trachomatis* pmpC gene.
SEQ ID NO: 188 is the determined DNA sequence representing the *C. pneumoniae* serovar MOMPS pmp gene in a fusion molecule with Ra12.
SEQ ID NO: 189 is the predicted amino acid sequence minus the signal sequence for the *C. trachomatis* pmpI gene.
SEQ ID NO: 190 is subsequently predicted amino acid sequence for the *C. trachomatis* pmpG gene.
SEQ ID NO: 191 is the predicted amino acid sequence minus the signal sequence for the *C. trachomatis* pmpE gene.
SEQ ID NO: 192 is a first predicted amino acid sequence representing the carboxy terminus for the *C. trachomatis* pmpD gene.
SEQ ID NO: 193 is a second predicted amino acid sequence representing the Amino terminus minus the signal sequence for the *C. trachomatis* pmpD gene.
SEQ ID NO: 194 is a first predicted amino acid sequence representing the Carboxy terminus for the *C. trachomatis* pmpC gene.
SEQ ID NO: 195 is a second predicted amino acid sequence representing the Amino terminus for the *C. trachomatis* pmpC gene.
SEQ ID NO: 196 is the predicted amino acid sequence representing the *C. pneumoniae* serovar MOMPS pmp gene in a fusion molecule with Ra12.
SEQ ID NO: 197 is the determined DNA sequence for the 5' oligo primer for cloning the *C. trachomatis* pmpC gene in the SKB vaccine vector.
SEQ ID NO: 198 is the determined DNA sequence for the 3' oligo primer for cloning the *C. trachomatis* pmpC gene in the SKB vaccine vector.
SEQ ID NO: 199 is the determined DNA sequence for the insertion sequence for cloning the *C. trachomatis* pmpC gene in the SKB vaccine vector.
SEQ ID NO: 200 is the determined DNA sequence for the 5' oligo primer for cloning the *C. trachomatis* pmpD gene in the SKB vaccine vector.
SEQ ID NO: 201 is the determined DNA sequence for the 3' oligo primer for cloning the *C. trachomatis* pmpD gene in the SKB vaccine vector.
SEQ ID NO: 202 is the determined DNA sequence for the insertion sequence for cloning the *C. trachomatis* pmpD gene in the SKB vaccine vector.
SEQ ID NO: 203 is the determined DNA sequence for the 5' oligo primer for cloning the *C. trachomatis* pmpE gene in the SKB vaccine vector.
SEQ ID NO: 204 is the determined DNA sequence for the 3' oligo primer for cloning the *C. trachomatis* pmpE gene in the SKB vaccine vector.
SEQ ID NO: 205 is the determined DNA sequence for the 5' oligo primer for cloning the *C. trachomatis* pmpG gene in the SKB vaccine vector.
SEQ ID NO: 206 is the determined DNA sequence for the 3' oligo primer for cloning the *C. trachomatis* pmpG gene in the SKB vaccine vector.
SEQ ID NO: 207 is the determined DNA sequence for the 5' oligo primer for cloning the amino terminus portion of the *C. trachomatis* pmpC gene in the pET17b vector.
SEQ ID NO: 208 is the determined DNA sequence for the 3' oligo primer for cloning the amino terminus portion of the *C. trachomatis* pmpC gene in the pET17b vector.
SEQ ID NO: 209 is the determined DNA sequence for the 5' oligo primer for cloning the carboxy terminus portion of the *C. trachomatis* pmpC gene in the pET17b vector.
SEQ ID NO: 210 is the determined DNA sequence for the 3' oligo primer for cloning the carboxy terminus portion of the *C. trachomatis* pmpC gene in the pET17b vector.
SEQ ID NO: 211 is the determined DNA sequence for the 5' oligo primer for cloning the amino terminus portion of the *C. trachomatis* pmpD gene in the pET17b vector.
SEQ ID NO: 212 is the determined DNA sequence for the 3' oligo primer for cloning the amino terminus portion of the *C. trachomatis* pmpD gene in the pET17b vector.
SEQ ID NO: 213 is the determined DNA sequence for the 5' oligo primer for cloning the carboxy terminus portion of the *C. trachomatis* pmpD gene in the pET17b vector.
SEQ ID NO: 214 is the determined DNA sequence for the 3' oligo primer for cloning the carboxy terminus portion of the *C. trachomatis* pmpD gene in the pET17b vector.
SEQ ID NO: 215 is the determined DNA sequence for the 5' oligo primer for cloning the *C. trachomatis* pmpE gene in the pET17b vector.
SEQ ID NO: 216 is the determined DNA sequence for the 3' oligo primer for cloning the *C. trachomatis* pmpE gene in the pET17b vector.
SEQ ID NO: 217 is the determined DNA sequence for the insertion sequence for cloning the *C. trachomatis* pmpE gene in the pET17b vector.
SEQ ID NO: 218 is the amino acid sequence for the insertion sequence for cloning the *C. trachomatis* pmpE gene in the pET17b vector.
SEQ ID NO: 219 is the determined DNA sequence for the 5' oligo primer for cloning the *C. trachomatis* pmpG gene in the pET17b vector.
SEQ ID NO: 220 is the determined DNA sequence for the 3' oligo primer for cloning the *C. trachomatis* pmpG gene in the pET17b vector.
SEQ ID NO: 221 is the amino acid sequence for the insertion sequence for cloning the *C. trachomatis* pmpG gene in the pET17b vector.
SEQ ID NO: 222 is the determined DNA sequence for the 5' oligo primer for cloning the *C. trachomatis* pmpI gene in the pET17b vector.
SEQ ID NO: 223 is the determined DNA sequence for the 3' oligo primer for cloning the *C. trachomatis* pmpI gene in the pET17b vector.
SEQ ID NO: 224 is the determined amino acid sequence for the *C. pneumoniae* Swib peptide 1-20.
SEQ ID NO: 225 is the determined amino acid sequence for the *C. pneumoniae* Swib peptide 6-25.
SEQ ID NO: 226 is the determined amino acid sequence for the *C. pneumoniae* Swib peptide 12-31.
SEQ ID NO: 227 is the determined amino acid sequence for the *C. pneumoniae* Swib peptide 17-36.
SEQ ID NO: 228 is the determined amino acid sequence for the *C. pneumoniae* Swib peptide 22-41.
SEQ ID NO: 229 is the determined amino acid sequence for the *C. pneumoniae* Swib peptide 27-46.
SEQ ID NO: 230 is the determined amino acid sequence for the *C. pneumoniae* Swib peptide 42-61.
SEQ ID NO: 231 is the determined amino acid sequence for the *C. pneumoniae* Swib peptide 46-65.
SEQ ID NO: 232 is the determined amino acid sequence for the *C. pneumoniae* Swib peptide 51-70.
SEQ ID NO: 233 is the determined amino acid sequence for the *C. pneumoniae* Swib peptide 56-75.
SEQ ID NO: 234 is the determined amino acid sequence for the *C. pneumoniae* Swib peptide 61-80.
SEQ ID NO: 235 is the determined amino acid sequence for the *C. pneumoniae* Swib peptide 66-87.
SEQ ID NO: 236 is the determined amino acid sequence for the *C. trachomatis* OMCB peptide 103-122.
SEQ ID NO: 237 is the determined amino acid sequence for the *C*. *trachomatis* OMCB peptide 108-127.
SEQ ID NO: 238 is the determined amino acid sequence for the *C*. *trachomatis* OMCB peptide 113-132.
SEQ ID NO: 239 is the determined amino acid sequence for the *C*. *trachomatis* OMCB peptide 118-137.
SEQ ID NO: 240 is the determined amino acid sequence for the *C*. *trachomatis* OMCB peptide 123-143.
SEQ ID NO: 241 is the determined amino acid sequence for the *C. trachomatis* OMCB peptide 128-147.
SEQ ID NO: 242 is the determined amino acid sequence for the *C. trachomatis* OMCB peptide 133-152.
SEQ ID NO: 243 is the determined amino acid sequence for the *C*. *trachomatis* OMCB peptide 137-156.
SEQ ID NO: 244 is the determined amino acid sequence for the *C. trachomatis* OMCB peptide 142-161.
SEQ ID NO: 245 is the determined amino acid sequence for the *C*. *trachomatis* OMCB peptide 147-166.
SEQ ID NO: 246 is the determined amino acid sequence for the *C*. *trachomatis* OMCB peptide 152-171.
SEQ ID NO: 247 is the determined amino acid sequence for the *C*. *trachomatis* OMCB peptide 157-176.
SEQ ID NO: 248 is the determined amino acid sequence for the *C*. *trachomatis* OMCB peptide 162-181.
SEQ ID NO: 249 is the determined amino acid sequence for the *C*. *trachomatis* OMCB peptide 167-186.
SEQ ID NO: 250 is the determined amino acid sequence for the *C*. *trachomatis* OMCB peptide 171-190.
SEQ ID NO: 251 is the determined amino acid sequence for the *C*. *trachomatis* OMCB peptide 171-186.
SEQ ID NO: 252 is the determined amino acid sequence for the *C*. *trachomatis* OMCB peptide 175-186.
SEQ ID NO: 252 is the determined amino acid sequence for the *C*. *trachomatis* OMCB peptide 175-186.
SEQ ID NO: 253 is the determined amino acid sequence for the *C. pneumoniae* OMCB peptide 185-198.
SEQ ID NO: 254 is the determined amino acid sequence for the *C*. *trachomatis* TSA peptide 96-115.
SEQ ID NO: 255 is the determined amino acid sequence for the *C*. *trachomatis* TSA peptide 101-120.
SEQ ID NO: 256 is the determined amino acid sequence for the *C*. *trachomatis* TSA peptide 106-125.
SEQ ID NO: 257 is the determined amino acid sequence for the *C*. *trachomatis* TSA peptide 111-130.
SEQ ID NO: 258 is the determined amino acid sequence for the *C*. *trachomatis* TSA peptide 116-135.
SEQ ID NO: 259 is the determined amino acid sequence for the *C*. *trachomatis* TSA peptide 121-140.
SEQ ID NO: 260 is the determined amino acid sequence for the *C*. *trachomatis* TSA peptide 126-145.
SEQ ID NO: 261 is the determined amino acid sequence for the *C*. *trachomatis* TSA peptide 131-150.
SEQ ID NO: 262 is the determined amino acid sequence for the *C*. *trachomatis* TSA peptide 136-155.
SEQ ID NO: 263 is the determined full-length DNA sequence for the *C*. *trachomatis* CT529/Cap 1 gene serovar 1.
SEQ ID NO: 264 is the predicted full-length amino sequence for the *C*. *trachomatis* CT529/Cap 1 gene serovar 1.
SEQ ID NO: 265 is the determined full-length DNA sequence for the *C. trachomatis* CT529/Cap 1 gene serovar K.
SEQ ID NO: 266 is the predicted full-length amino sequence for the *C*. *trachomatis* CT529/Cap 1 gene serovar K.
SEQ ID NO: 267 is the determined DNA sequence for the *C. trachomatis* clone 17-G4-36 sharing homology to part of the ORF of DNA-dirrected RNA polymerase beta subunit- CT315 in serD.
SEQ ID NO: 268 is the determined DNA sequence for the partial sequence of the C. *trachomatis* CT016 gene in clone 2E10.
SEQ ID NO: 269 is the determined DNA sequence for the partial sequence of the *C. trachomatis* tRNA syntase gene in clone 2E 10.
SEQ ID NO: 270 is the determined DNA sequence for the partial sequence for the C. *trachomatis* clpX gene in clone 2E10.
SEQ ID NO: 271 is a first determined DNA sequence for the *C. trachomatis* clone CtL2gam-30 representing the 5'end.
SEQ ID NO: 272 is a second determined DNA sequence for the *C. trachomatis* clone CtL2gam-30 representing the 3'end.
SEQ ID NO: 273 is the determined DNA sequence for the *C*. *trachomatis* clone CtL2gam-28.
SEQ ID NO: 274 is the determined DNA sequence for the *C*. *trachomatis* clone CtL2gam-27.
SEQ ID NO: 275 is the determined DNA sequence for the *C*. *trachomatis* clone CtL2gam-26.
SEQ ID NO: 276 is the determined DNA sequence for the *C*. *trachomatis* clone CtL2gam-24.
SEQ ID NO: 277 is the determined DNA sequence for the *C*. *trachomatis* clone CtL2gam-23.
SEQ ID NO: 278 is the determined DNA sequence for the *C. trachomatis* clone CtL2gam-21.
SEQ ID NO: 279 is the determined DNA sequence for the *C. trachomatis* clone CtL2gam-18.
SEQ ID NO: 280 is the determined DNA sequence for the *C*. *trachomatis* clone CtL2gam-17.
SEQ ID NO: 281 is a first determined DNA sequence for the *C. trachomatis* clone CtL2gam-15 representing the 5' end.
SEQ ID NO: 282 is a second determined DNA sequence for the *C*. *trachomatis* clone CtL2gam-15 representing the 3' end.
SEQ ID NO: 283 is the determined DNA sequence for the *C*. *trachomatis* clone CtL2gam-13.
SEQ ID NO: 284 is the determined DNA sequence for the *C*. *trachomatis* clone CtL2gam-10.
SEQ ID NO: 285 is the determined DNA sequence for the *C*. *trachomatis* clone CtL2gam-8.
SEQ ID NO: 286 is a first determined DNA sequence for the *C*. *trachomatis* clone CtL2gam-6 representing the 5' end.
SEQ ID NO: 287 is a second determined DNA sequence for the *C*. *trachomatis* clone CtL2gam-6 representing the 3' end.
SEQ ID NO: 288 is the determined DNA sequence for the *C*. *trachomatis* clone CtL2gam-5.
SEQ ID NO: 289 is the determined DNA sequence for the *C*. *trachomatis* clone CtL2gam-2.
SEQ ID NO: 290 is the determined DNA sequence for the *C. trachomatis* clone CtL2gam-1.
SEQ ID NO: 291 is the determined full-length DNA sequence for the *C*. *pneumoniae* homologue of the CT529 gene.
SEQ ID NO: 292 is the predicted full-length amino acid sequence for the *C*. *pneumoniae* homologue of the CT529 gene.
SEQ ID NO: 293 is the determined DNA sequence for the insertion sequence for cloning the *C. trachomatis* pmpG gene in the SKB vaccine vector.

### DESCRIPTION OF THE FIGURES

Fig. 1 illustrates induction of INF-γ from a *Chlamydia*-specific T cell line activated by target cells expressing clone 4C9-18#2.
Fig. 2 illustrates retroviral vectors pBIB-KS 1,2,3 modified to contain a Kosak translation initiation site and stop codons.
Fig. 3 shows specific lysis in a chromium release assay of P815 cells pulsed with *Chlamydia* peptides CtC7.8-12 (SEQ ID NO: 18) and CtC7.8-13 (SEQ ID NO: 19).
Fig. 4 shows antibody isotype titers in C57B1/6 mice immunized with *C. trachomatis* SWIB protein.
Fig. 5 shows *Chlamydia*-specific T-cell proliferative responses in splenocytes from C3H mice immunized with *C. trachomatis* SWIB protein.
Fig. 6 illustrates the 5' and 3' primer sequences designed from *C. pneumoniae* which were used to isolate the SWIB and S13 genes from *C. pneumoniae.*
Figs. 7A and 7B show induction of IFN-γ from a human anti-*chlamydia* T-cell line (TCL-8) capable of cross-reacting to *C. trachomatis* and *C. pneumonia* upon activation by monocyte-derived dendritic cells expressing chlamydial proteins.
Fig. 8 shows the identification of T cell epitopes in Chlamydial ribosomal S 13 protein with T-cell line TCL 8 EB/DC.
Fig. 9 illustrates the proliferative response of CP-21 T-cells generated against *C. pnuemoniae-*infected dendritic cells to recombinant *C. pneumonia*-SWIBprotein, but not *C. trachomatis* SWIB protein.
Fig. 10 shows the *C. trachomatis*-specific SWIB proliferative responses of a primary T-cell line (TCT-10 EB) from an asymptomatic donor.
Fig. 11 illustrates the identification of T-cell epitope in *C. trachomatis* SWIB with an antigen specific T-cell line (TCL-10 EB).

### DETAILED DESCRIPTION OF THE INVENTION

As noted above, the present invention is generally directed to compositions and methods for the diagnosis and treatment of Chlamydial infection. In one aspect, the compositions of the subject invention include polypeptides that comprise at least one immunogenic portion of a *Chlamydia* antigen, or a variant thereof.

In specific embodiments, the subject invention discloses polypeptides comprising an immunogenic portion of a *Chlamydia* antigen, wherein the *Chlamydia* antigen comprises an amino acid sequence encoded by a polynucleotide molecule including a sequence selected from the group consisting of (a) nucleotide sequences recited in SEQ ID NO: 1, 15, 21-25, 44-64, 66-76, 79-88, 110-119, 120, 122, 124, 126, 128, 130, 132, 134, 136, 169-174, 181-188, 263, 265 and 267-290 (b) the complements of said nucleotide sequences, and (c) variants of such sequences.

As used herein, the term "polypeptide" encompasses amino acid chains of any length, including full length proteins (*i.e*., antigens), wherein the amino acid residues are linked by covalent peptide bonds. Thus, a polypeptide comprising an immunogenic portion of one of the inventive antigens may consist entirely of the immunogenic portion, or may contain additional sequences. The additional sequences may be derived from the native *Chlamydia* antigen or may be heterologous, and such sequences may (but need not) be immunogenic.

The term "polynucleotide(s)," as used herein, means a single or doublestranded polymer of deoxyribonucleotide or ribonucleotide bases and includes DNA and corresponding RNA molecules, including HnRNA and mRNA molecules, both sense and anti-sense strands, and comprehends cDNA, genomic DNA and recombinant DNA, as well as wholly or partially synthesized polynucleotides. An HnRNA molecule contains introns and corresponds to a DNA molecule in a generally one-to-one manner. An mRNA molecule corresponds to an HnRNA and DNA molecule from which the introns have been excised. A polynucleotide may consist of an entire gene, or any portion thereof. Operable anti-sense polynucleotides may comprise a fragment of the corresponding polynucleotide, and the definition of "polynucleotide" therefore includes all such operable anti-sense fragments.

An "immunogenic portion" of an antigen is a portion that is capable of reacting with sera obtained from a *Chlamydia*-infected individual (*i.e*., generates an absorbance reading with sera from infected individuals that is at least three standard deviations above the absorbance obtained with sera from uninfected individuals, in a representative ELISA assay described herein). Such immunogenic portions generally comprise at least about 5 amino acid residues, more preferably at least about 10, and most preferably at least about 20 amino acid residues. Methods for preparing and identifying immunogenic portions of antigens of known sequence are well known in the art and include those summarized in Paul, Fundamental Immunology, 3rd ed., Raven Press, 1993, pp. 243-247 and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e*., they react with the protein in an ELISA or other immunoassay, and do not react detectably with unrelated proteins). Such antisera and antibodies may be prepared as described herein, and using well known techniques. An immunogenic portion of a native *Chlamydia* protein is a portion that reacts with such antisera and/or T-cells at a level that is not substantially less than the reactivity of the full length polypeptide (*e.g*., in an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

Examples of immunogenic portions of antigens contemplated by the present invention include, for example, the T cell stimulating epitopes provided in SEQ ID NO: 9, 10, 18, 19, 31, 39, 93-96, 98, 100-102, 106, 108, 138-140, 158, 167, 168, 246, 247 and 254-256. Polypeptides comprising at least an immunogenic portion of one or more *Chlamydia* antigens as described herein may generally be used, alone or in combination, to detect Chlamydial infection in a patient.

The compositions and methods of the present invention also encompass variants of the above polypeptides and polynucleotide molecules. Such variants include, but are not limited to, naturally occurring allelic variants of the inventive sequences. In particular, variants include other *Chlamydiae* serovars, such as serovars D, E and F, as well as the several LGV serovars which share homology to the inventive polypeptide and polynucleotide molecules described herein. Preferably, the serovar homologues show 95-99% homology to the corresponding polypeptide sequence(s) described herein.

A polypeptide "variant," as used herein, is a polypeptide that differs from the recited polypeptide only in conservative substitutions and/or modifications, such that the antigenic properties of the polypeptide are retained. In a preferred embodiment, variant polypeptides differ from an identified sequence by substitution, deletion or addition of five amino acids or fewer. Such variants may generally be identified by modifying one of the above polypeptide sequences, and evaluating the antigenic properties of the modified polypeptide using, for example, the representative procedures described herein. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence or transmembrane domain, have been removed. Other preferred variants include variants in which a small portion (*e.g*., 1-30 amino acids, preferably 5-15 amino acids) has been removed from the N- and/or C-terminal of the mature protein. Polypeptide variants preferably exhibit at least about 70%, more preferably at least about 90% and most preferably at least about 95% identity (determined as described below) to the identified polypeptides.

As used herein, a "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide. Variants may also, or alternatively, contain other modifications, including the deletion or addition of amino acids that have minimal influence on the antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g*., poly-His), or to enhance binding of the polypeptide to a solid support. For example, a polypeptide may be conjugated to an immunoglobulin Fc region.

A polynucleotide "variant" is a sequence that differs from the recited nucleotide sequence in having one or more nucleotide deletions, substitutions or additions such that the immunogenicity of the encoded polypeptide is not diminished, relative to the native protein. The effect on the immunogenicity of the encoded polypeptide may generally be assessed as described herein. Such modifications may be readily introduced using standard mutagenesis techniques, such as oligonucleotide-directed site-specific mutagenesis as taught, for example, by Adelman et al. (DNA, 2:183, 1983). Nucleotide variants may be naturally occurring allelic variants as discussed below, or non-naturally occurring variants. Variant nucleotide sequences preferably exhibit at least about 70%, more preferably at least about 80% and most preferably at least about 90% identity (determined as described below) to the recited sequence.

The polypeptides provided by the present invention include variants that are encoded by polynucleotide sequences which are substantially homologous to one or more of the polynucleotide sequences specifically recited herein. "Substantial homology," as used herein, refers to polynucleotide sequences that are capable of hybridizing under moderately stringent conditions. Suitable moderately stringent conditions include prewashing in a solution of 5X SSC, 0.5% SDS, 1.0 mM EDTA (pH 8.0); hybridizing at 50°C-65°C, 5X SSC, overnight or, in the event of cross-species homology, at 45°C with 0.5X SSC; followed by washing twice at 65°C for 20 minutes with each of 2X, 0.5X and 0.2X SSC containing 0.1% SDS. Such hybridizing polynucleotide sequences are also within the scope of this invention, as are nucleotide sequences that, due to code degeneracy, encode a polypeptide that is the same as a polypeptide of the present invention.

Two nucleotide or polypeptide sequences are said to be "identical" if the sequence of nucleotides or amino acid residues in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Resarch Foundaiton, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) Fast and sensitive multiple sequence alignments on a microcomputer CABIOS 5:151-153; Myers, E.W. and Muller W. (1988) Optimal alignments in linear space CABIOS 4:11-17; Robinson, E.D. (1971) Comb. Theor 11:105; Santou, N. Nes, M. (1987) The neighbor joining method. A new method for reconstructing phylogenetic trees Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Rapid similarity searches of nucleic acid and protein data banks Proc. Natl. Acad., Sci. USA 80:726-730.

Preferably, the "percentage of sequence identity" is determined by comparing two optimally aligned sequences over a window of comparison of at least 20 positions, wherein the portion of the polynucleotide sequence in the comparison window may comprise additions or deletions (i.e. gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Also included in the scope of the present invention are alleles of the genes encoding the nucleotide sequences recited in herein. As used herein, an "allele" or "allellic sequence" is an alternative form of the gene which may result from at least one mutation in the nucleic acid sequence. Alleles may result in altered mRNAs or polypeptides whose structure or function may or may not be altered. Any given gene may have none, one, or many allelic forms. Common mutational changes which give rise to alleles are generally ascribed to natural deletions, additions, or substitutions of nucleotides. Each of these types of changes may occur alone or in combination with the others, one or more times in a given sequence. In specific embodiments, the subject invention discloses polypeptides comprising at least an immunogenic portion of a *Chlamydia* antigen (or a variant of such an antigen), that comprises one or more of the amino acid sequences encoded by (a) a polynucleotide sequence selected from the group consisting of SEQ ID NO: 1-4, 15 21-25, 44-64, 66-76 and 79-88; (b) the complements of such DNA sequences or (c) DNA sequences substantially homologous to a sequence in (a) or (b). As discussed in the Examples below, several of the *Chlamydia* antigens disclosed herein recognize a T cell line that recognizes both *Chlamydia trachomatis* and *Chlamydia pneumoniae* infected monocyte-derived dendritic cells, indicating that they may represent an immunoreactive epitope shared by *Chlamydia trachomatis* and *Chlamydia pneumoniae.* The antigens may thus be employed in a vaccine for both *C. trachomatis* genital tract infections and for *C. pneumonia* infections. Further characterization of these *Chlamydia* antigens from *Chlamydia trachomatis* and *Chlamydia pneumonia* to determine the extent of cross-reactivity is provided in Example 6. Additionally, Example 4 describes cDNA fragments (SEQ ID NO: 15, 16 and 33) isolated from *C. trachomatis* which encode proteins (SEQ ID NO: 17-19 and 32) capable of stimulating a *Chlamydia*-specific murine CD8+ T cell line.

In general, *Chlamydia* antigens, and polynucleotide sequences encoding such antigens, may be prepared using any of a variety of procedures. For example, polynucleotide molecules encoding *Chlamydia* antigens may be isolated from a *Chlamydia* genomic or cDNA expression library by screening with a *Chlamydia*-specific T cell line as described below, and sequenced using techniques well known to those of skill in the art. Additionally, a polynucleotide may be identified, as described in more detail below, by screening a microarray of cDNAs for *Chlamydia*-associated expression (*i*.*e*., expression that is at least two fold greater in *Chlamydia*-infected cells than in controls, as determined using a representative assay provided herein). Such screens may be performed using a Synteni microarray (Palo Alto, CA) according to the manufacturer's instructions (and essentially as described by Schena et al., Proc. Natl. Acad Sci. USA 93:10614-10619, 1996 and Heller et al., Proc. Natl. Acad. Sci. USA 94:2150-2155, 1997). Alternatively, polypeptides may be amplified from cDNA prepared from cells expressing the proteins described herein.. Such polynucleotides may be amplified via polymerase chain reaction (PCR). For this approach, sequence-specific primers may be designed based on the sequences provided herein, and may be purchased or synthesized.

Antigens may be produced recombinantly, as described below, by inserting a polynucleotide sequence that encodes the antigen into an expression vector and expressing the antigen in an appropriate host. Antigens may be evaluated for a desired property, such as the ability to react with sera obtained from a *Chlamydia*-infected individual as described herein, and may be sequenced using, for example, traditional Edman chemistry. *See* Edman and Berg, Eur. J. Biochem. 80:116-132, 1967.

Polynucleotide sequences encoding antigens may also be obtained by screening an appropriate *Chlamydia* cDNA or genomic DNA library for polynucleotide sequences that hybridize to degenerate oligonucleotides derived from partial amino acid sequences of isolated antigens. Degenerate oligonucleotide sequences for use in such a screen may be designed and synthesized, and the screen may be performed, as described (for example) in Sambrook et al., Molecular Cloning: A Laboratory Manual, Cold Spring Harbor Laboratories, Cold Spring Harbor, NY (and references cited therein). Polymerase chain reaction (PCR) may also be employed, using the above oligonucleotides in methods well known in the art, to isolate a nucleic acid probe from a cDNA or genomic library. The library screen may then be performed using the isolated probe.

An amplified portion may be used to isolate a full length gene from a suitable library (*e.g.,* a *Chlamydia* cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e*.*g*., by nicktranslation or end-labeling with ³²P) using well known techniques. A bacterial or bacteriophage library is then screened by hybridizing filters containing denatured bacterial colonies (or lawns containing phage plaques) with the labeled probe (*see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using techniques well known in the art *(see,* for example, Mullis et al., Cold Spring Harbor Symp. Quant. Biol. 51:263, 1987; Erlich ed., PCR Technology, Stockton Press, NY, 1989), and software well known in the art may also be employed. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (*see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Additional techniques include capture PCR (Lagerstrom et al., PCR Methods Applic. 1:111-19, 1991) and walking PCR (Parker et al., Nucl. Acids. Res. 19:3055-60, 1991). Transcription-Mediated Amplification, or TMA is another method that may be utilized for the amplification of DNA, rRNA, or mRNA, as described in Patent No. PCT/US91/03184. This autocatalytic and isothermic non-PCR based method utilizes two primers and two enzymes: RNA polymerase and reverse transcriptase. One primer contains a promoter sequence for RNA polymerase. In the first amplification, the promoter-primer hybridizes to the target rRNA at a defined site. Reverse transcriptase creates a DNA copy of the target rRNA by extension from the 3'end of the promoter-primer. The RNA in the resulting complex is degraded and a second primer binds to the DNA copy. A new strand of DNA is synthesized from the end of the primer by reverse transcriptase creating double stranded DNA. RNA polymerase recognizes the promoter sequence in the DNA template and initiates transcription. Each of the newly synthesized RNA amplicons re-enters the TMA process and serves as a template for a new round of replication leading to the expotential expansion of the RNA amplicon. Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g*., NCBI BLAST searches), and such ESTs may be used to generate a contiguous full length sequence. Full length cDNA sequences may also be obtained by analysis of genomic fragments.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis (*see* Adelman et al., DNA 2:183, 1983). Alternatively, RNA molecules may be generated by *in vitro* or *in vivo* transcription of DNA sequences encoding a *Chlamydial* protein, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo* (*e.g.,* by transfecting antigen-presenting cells, such as dendritic cells, with a cDNA construct encoding a *Chlamydial* polypeptide, and administering the transfected cells to the patient).

A portion of a sequence complementary to a coding sequence (*i.e*., an antisense polynucleotide) may also be used as a probe or to modulate gene expression. cDNA constructs that can be transcribed into antisense RNA may also be introduced into cells of tissues to facilitate the production of antisense RNA. An antisense polynucleotide may be used, as described herein, to inhibit expression of a *Chlamydial* protein. Antisense technology can be used to control gene expression through triple-helix formation, which compromises the ability of the double helix to open sufficiently for the binding of polymerases, transcription factors or regulatory molecules *(see* Gee et al., In Huber and Carr, Molecular and Immunologic Approaches, Futura Publishing Co. (Mt. Kisco, NY; 1994)). Alternatively, an antisense molecule may be designed to hybridize with a control region of a gene (*e.g*., promoter, enhancer or transcription initiation site), and block transcription of the gene; or to block translation by inhibiting binding of a transcript to ribosomes.

A portion of a coding sequence, or of a complementary sequence, may also be designed as a probe or primer to detect gene expression. Probes may be labeled with a variety of reporter groups, such as radionuclides and enzymes, and are preferably at least 10 nucleotides in length, more preferably at least 20 nucleotides in length and still more preferably at least 30 nucleotides in length. Primers, as noted above, are preferably 22-30 nucleotides in length.

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Synthetic polypeptides having fewer than about 100 amino acids, and generally fewer than about 50 amino acids, may be generated using techniques well known in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division, Foster City, CA, and may be operated according to the manufacturer's instructions.

As noted above, immunogenic portions of *Chlamydia* antigens may be prepared and identified using well known techniques, such as those summarized in Paul, Fundamental Immunology, 3d ed., Raven Press, 1993, pp.243-247 and references cited therein. Such techniques include screening polypeptide portions of the native antigen for immunogenic properties. The representative ELISAs described herein may generally be employed in these screens. An immunogenic portion of a polypeptide is a portion that, within such representative assays, generates a signal in such assays that is substantially similar to that generated by the full length antigen. In other words, an immunogenic portion of a *Chlamydia* antigen generates at least about 20%, and preferably about 100%, of the signal induced by the full length antigen in a model ELISA as described herein.

Portions and other variants of *Chlamydia* antigens may be generated by synthetic or recombinant means. Variants of a native antigen may generally be prepared using standard mutagenesis techniques, such as oligonucleotide-directed site-specific mutagenesis. Sections of the polynucleotide sequence may also be removed using standard techniques to permit preparation of truncated polypeptides.

Recombinant polypeptides containing portions and/or variants of a native antigen may be readily prepared from a polynucleotide sequence encoding the polypeptide using a variety of techniques well known to those of ordinary skill in the art. For example, supernatants from suitable host/vector systems which secrete recombinant protein into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant protein.

Any of a variety of expression vectors known to those of ordinary skill in the art may be employed to express recombinant polypeptides as described herein. Expression may be achieved in any appropriate host cell that has been transformed or transfected with an expression vector containing a polynucleotide molecule that encodes a recombinant polypeptide. Suitable host cells include prokaryotes, yeast and higher eukaryotic cells. Preferably, the host cells employed are *E. coli,* yeast or a mammalian cell line, such as COS or CHO. The DNA sequences expressed in this manner may encode naturally occurring antigens, portions of naturally occurring antigens, or other variants thereof.

In general, regardless of the method of preparation, the polypeptides disclosed herein are prepared in an isolated, substantially pure, form. Preferably, the polypeptides are at least about 80% pure, more preferably at least about 90% pure and most preferably at least about 99% pure.

Within certain specific embodiments, a polypeptide may be a fusion protein that comprises multiple polypeptides as described herein, or that comprises at least one polypeptide as described herein and an unrelated sequence, such as a known *Chlamydial* protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein. A DNA sequence encoding a fusion protein of the present invention may be constructed using known recombinant DNA techniques to assemble separate DNA sequences encoding, for example, the first and second polypeptides, into an appropriate expression vector. The 3' end of a DNA sequence encoding the first polypeptide is ligated, with or without a peptide linker, to the 5' end of a DNA sequence encoding the second polypeptide so that the reading frames of the sequences are in phase to permit mRNA translation of the two DNA sequences into a single fusion protein that retains the biological activity of both the first and the second polypeptides.

A peptide linker sequence may be employed to separate the first and the second polypeptides by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8562, 1986; U.S. Patent No. 4,935,233 and U.S. Patent No. 4,751,180. The linker sequence may be from 1 to about 50 amino acids in length. As an alternative to the use of a peptide linker sequence (when desired), one can utilize non-essential N-terminal amino acid regions (when present) on the first and second polypeptides to separate the functional domains and prevent steric hindrance.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins *(see,* for example, Stoute et al. New Engl. J. Med., 336:86-91, 1997).

Within preferred embodiments, an immunological fusion partner is derived from protein D, a surface protein of the gram-negative bacterium Haemophilus influenza B (WO 91/18926). Preferably, a protein D derivative comprises approximately the first third of the protein (*e.g*., the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in E. *coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae*, which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (*see* Biotechnology 10:795-798, 1992). Within a preferred embodiment, a repeat portion of LYTA may be incorporated into a fusion protein. A repeat portion is found in the C-terminal region starting at residue 178. A particularly preferred repeat portion incorporates residues 188-305. Additionally, the fusion protein Ra12 may be linked to the inventive polynucleotides to facilitate protein expression.

In another aspect, the present invention provides methods for using one or more of the above polypeptides or fusion proteins (or polynucleotides encoding such polypeptides or fusion proteins) to induce protective immunity against Chlamydial infection in a patient. As used herein, a "patient" refers to any warm-blooded animal, preferably a human. A patient may be afflicted with a disease, or may be free of detectable disease and/or infection. In other words, protective immunity may be induced to prevent or treat Chlamydial infection.

In this aspect, the polypeptide, fusion protein or polynucleotide molecule is generally present within a pharmaceutical composition or a vaccine. Pharmaceutical compositions may comprise one or more polypeptides, each of which may contain one or more of the above sequences (or variants thereof), and a physiologically acceptable carrier. Vaccines may comprise one or more of the above polypeptides and an immunostimulant, such as an adjuvant or a liposome (into which the polypeptide is incorporated). Such pharmaceutical compositions and vaccines may also contain other *Chlamydia* antigens, either incorporated into a combination polypeptide or present within a separate polypeptide.

Alternatively, a vaccine may contain polynucleotides encoding one or more polypeptides or fusion proteins as described above, such that the polypeptide is generated *in situ.* In such vaccines, the polynucleotides may be present within any of a variety of delivery systems known to those of ordinary skill in the art, including nucleic acid expression systems, bacterial and viral expression systems. Appropriate nucleic acid expression systems contain the necessary polynucleotide sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface. In a preferred embodiment, the polynucleotides may be introduced using a viral expression system (*e.g*., vaccinia or other pox virus, retrovirus, or adenovirus), which may involve the use of a non-pathogenic (defective) virus. Techniques for incorporating polynucleotides into such expression systems are well known to those of ordinary skill in the art. The polynucleotides may also be administered as "naked" plasmid vectors as described, for example, in Ulmer et al., Science 259:1745-1749, 1993 and reviewed by Cohen, Science 259:1691-1692, 1993.Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (*i.e.,* an artificial membrane vesicle). The uptake of naked polynucleotides may be increased by incorporating the polynucleotides into and/or onto biodegradable beads, which are efficiently transported into the cells. The preparation and use of such systems is well known in the art.

In a related aspect, a polynucleotide vaccine as described above may be administered simultaneously with or sequentially to either a polypeptide of the present invention or a known *Chlamydia* antigen. For example, administration of polynucleotides encoding a polypeptide of the present invention, either "naked" or in a delivery system as described above, may be followed by administration of an antigen in order to enhance the protective immune effect of the vaccine.

Polypeptides and polynucleotides disclosed herein may also be employed in adoptive immunotherapy for the treatment of *Chlamydial* infection. Adoptive immunotherapy may be broadly classified into either active or passive immunotherapy. In active immunotherapy, treatment relies on the *in vivo* stimulation of the endogenous host immune system with the administration of immune response-modifying agents (for example, vaccines, bacterial adjuvants, and/or cytokines).

In passive immunotherapy, treatment involves the delivery of biologic reagents with established immune reactivity (such as effector cells or antibodies) that can directly or indirectly mediate anti-*Chlamydia* effects and does not necessarily depend on an intact host immune system. Examples of effector cells include T lymphocytes (for example, CD8+ cytotoxic T-lymphocyte, CD4+ T-helper), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Patent No. 4,918,164), for passive immunotherapy.

The predominant method of procuring adequate numbers of T-cells for adoptive immunotherapy is to grow immune T-cells *in vitro.* Culture conditions for expanding single antigen-specific T-cells to several billion in number with retention of antigen recognition *in vivo* are well known in the art. These *in vitro* culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage, monocyte, fibroblast, or B-cells, may be pulsed with immunoreactive polypeptides, or polynucleotide sequence(s) may be introduced into antigen presenting cells, using a variety of standard techniques well known in the art. For example, antigen presenting cells may be transfected or transduced with a polynucleotide sequence, wherein said sequence contains a promoter region appropriate for increasing expression, and can be expressed as part of a recombinant virus or other expression system. Several viral vectors may be used to transduce an antigen presenting cell, including pox virus, vaccinia virus, and adenovirus; also, antigen presenting cells may be transfected with polynucleotide sequences disclosed herein by a variety of means, including gene-gun technology, lipid-mediated delivery, electroporation, osmotic shock, and particlate delivery mechanisms, resulting in efficient and acceptable expression levels as determined by one of ordinary skill in the art. For cultured T-cells to be effective in therapy, the cultured T-cells must be able to grow and distribute widely and to survive long term *in vivo.* Studies have demonstrated that cultured T-cells can be induced to grow *in vivo* and to survive long term in substantial numbers by repeated stimulation with antigen supplemented with IL-2 (see, for example, Cheever, M., et al, "Therapy With Cultured T Cells: Principles Revisited, " Immunological Reviews, 157:177, 1997).

The polypeptides disclosed herein may also be employed to generate and/or isolate chlamydial-reactive T-cells, which can then be administered to the patient. In one technique, antigen-specific T-cell lines may be generated by *in vivo* immunization with short peptides corresponding to immunogenic portions of the disclosed polypeptides. The resulting antigen specific CD8+ or CD4+ T-cell clones may be isolated from the patient, expanded using standard tissue culture techniques, and returned to the patient.

Alternatively, peptides corresponding to immunogenic portions of the polypeptides may be employed to generate *Chlamydia* reactive T cell subsets by selective in *vitro* stimulation and expansion of autologous T cells to provide antigen-specific T cells which may be subsequently transferred to the patient as described, for example, by Chang et al, (Crit. Rev. Oncol. Hematol., 22(3), 213, 1996). Cells of the immune system, such as T cells, may be isolated from the peripheral blood of a patient, using a commercially available cell separation system, such as Isolex™ System, available from Nexell Therapeutics, Inc. Irvine, CA. The separated cells are stimulated with one or more of the immunoreactive polypeptides contained within a delivery vehicle, such as a microsphere, to provide antigen-specific T cells. The population of antigen-specific T cells is then expanded using standard techniques and the cells are administered back to the patient.

In other embodiments, T-cell and/or antibody receptors specific for the polypeptides disclosed herein can be cloned, expanded, and transferred into other vectors or effector cells for use in adoptive immunotherapy. In particular, T cells may be transfected with the appropriate genes to express the variable domains from chlamydia specific monoclonal antibodies as the extracellular recognition elements and joined to the T cell receptor signaling chains, resulting in T cell activation, specific lysis, and cytokine release. This enables the T cell to redirect its specificity in an MHC-independent manner. See for example, Eshhar, Z., Cancer Immunol Immunother, 45(3-4):131-6, 1997 and Hwu, P., et al, Cancer Res, 55(15):3369-73, 1995. Another embodiment may include the transfection of chlamydia antigen specific alpha and beta T cell receptor chains into alternate T cells, as in Cole, DJ, et al, Cancer Res, 55(4):748-52, 1995.

In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T cells to provide antigen-specific T cells which may, in turn, be administered to a patient. The use of peptide-pulsed dendritic cells to generate antigen-specific T cells and the subsequent use of such antigen-specific T cells to eradicate disease in a murine model has been demonstrated by Cheever et al, Immunological Reviews, 157:177, 1997). Additionally, vectors expressing the disclosed polynucleotides may be introduced into stem cells taken from the patient and clonally propagated *in vitro* for autologous transplant back into the same patient.

Within certain aspects, polypeptides, polynucleotides, T cells and/or binding agents disclosed herein may be incorporated into pharmaceutical compositions or immunogenic compositions (*i*.*e*., vaccines). Pharmaceutical compositions comprise one or more such compounds and a physiologically acceptable carrier. Vaccines may comprise one or more such compounds and an immunostimulant. An immunostimulant may be any substance that enhances or potentiates an immune response to an exogenous antigen. Examples of immunostimulants include adjuvants, biodegradable microspheres (*e.g*., polylactic galactide) and liposomes (into which the compound is incorporated; *see e.g.,* Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other *Chlamydial* antigens may be present, either incorporated into a fusion polypeptide or as a separate compound, within the composition or vaccine.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface or secretes such an epitope. In a preferred embodiment, the DNA may be introduced using a viral expression system (*e.g*., vaccinia or other pox virus, retrovirus, or adenovirus), which may involve the use of a non-pathogenic (defective), replication competent virus. Suitable systems are disclosed, for example, in Fisher-Hoch et al., Proc. Natl. Acad. Sci. USA 86:317-321, 1989; Flexner et al., Ann. N.Y. Acad. Sci. 569:86-103, 1989; Flexner et al., Vaccine 8:17-21, 1990; U.S. Patent Nos.4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g*., polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (*e.g*., neutral buffered saline or phosphate buffered saline), carbohydrates (*e.g*., glucose, mannose, sucrose or dextrans), mannitol, proteins, polypeptides or amino acids such as glycine, antioxidants, chelating agents such as EDTA or glutathione, adjuvants (*e.g*., aluminum hydroxide) and/or preservatives. Alternatively, compositions of the present invention may be formulated as a lyophilizate. Compounds may also be encapsulated within liposomes using well known technology.

Any of a variety of immunostimulants may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Within the vaccines provided herein, under select circumstances, the adjuvant composition may be designed to induce an immune response predominantly of the Th1 type or Th2 type. High levels of Th1-type cytokines (*e.g*., IFN-γ, TNFα, IL-2 and IL-12) tend to favor the induction of cell mediated immune responses to an administered antigen. In contrast, high levels of Th2-type cytokines (*e.g*., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Ribi ImmunoChem Research Inc. (Hamilton, MT) (*see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555. Another preferred adjuvant is a saponin, preferably QS21, which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210. Any vaccine provided herein may be prepared using well known methods that result in a combination of antigen, immune response enhancer and a suitable carrier or excipient.

The compositions described herein may be administered as part of a sustained release formulation (*i*.*e*., a formulation such as a capsule, sponge or gel (composed of polysaccharides, for example) that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets *Chlamydia*-infected cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have *anti-Chlamydia* effects *per se* and/or to be immunologically compatible with the receiver (*i*.*e*., matched HLA haplotype). APCs may generally be isolated from any of a variety of biological fluids and organs, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic immunity (*see* Timmerman and Levy, Ann. Rev. Med. 50:507-529, 1999). In general, dendritic cells may be identified based on their typical shape (stellate *in situ*, with marked cytoplasmic processes (dendrites) visible *in vitro*), their ability to take up, process and present antigens with high efficiency, and their ability to activate naïve T cell responses. Dendritic cells may, of course, be engineered to express specific cell-surface receptors or ligands that are not commonly found on dendritic cells *in vivo* or *ex vivo*, and such modified dendritic cells are contemplated by the present invention. As an alternative to dendritic cells, secreted vesicles antigen-loaded dendritic cells (called exosomes) may be used within a vaccine *(see* Zitvogel et al., Nature Med. 4:594-600, 1998).

Dendritic cells and progenitors may be obtained from peripheral blood, bone marrow, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g*., CD54 and CD11) and costimulatory molecules (*e.g*., CD40, CD80, CD86 and 4-1BB).

APCs may generally be transfected with a polynucleotide encoding a *Chlamydial* protein (or portion or other variant thereof) such that the *Chlamydial* polypeptide, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the *Chlamydial* polypeptide, DNA (naked or within a plasmid vector) or RNA; or with antigen-expressing recombinant bacterium or viruses (*e.g*., vaccinia, fowlpox, adenovirus or lentivirus vectors). Prior to loading, the polypeptide may be covalently conjugated to an immunological partner that provides T cell help (*e.g*., a carrier molecule). Alternatively, a dendritic cell may be pulsed with a nonconjugated immunological partner, separately or in the presence of the polypeptide.

Routes and frequency of administration of pharmaceutical compositions and vaccines, as well as dosage, will vary from individual to individual. In general, the pharmaceutical compositions and vaccines may be administered by injection (*e*.*g*., intracutaneous, intramuscular, intravenous or subcutaneous), intranasally (*e*.*g*., by aspiration) or orally. Between 1 and 3 doses may be administered for a 1-36 week period. Preferably, 3 doses are administered, at intervals of 3-4 months, and booster vaccinations may be given periodically thereafter. Alternate protocols may be appropriate for individual patients. A suitable dose is an amount of polypeptide or DNA that, when administered as described above, is capable of raising an immune response in an immunized patient sufficient to protect the patient from *Chlamydial* infection for at least 1-2 years. In general, the amount of polypeptide present in a dose (or produced *in situ* by the DNA in a dose) ranges from about 1 pg to about 100 mg per kg of host, typically from about 10 pg to about 1 mg, and preferably from about 100 pg to about 1 µg. Suitable dose sizes will vary with the size of the patient, but will typically range from about 0.1 mL to about 5 mL.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g*., polylactic galactide) may also be employed as carriers for the pharmaceutical compositions of this invention. Suitable biodegradable microspheres are disclosed, for example, in U.S. Patent Nos. 4,897,268 and 5,075,109.

In general, an appropriate dosage and treatment regimen provides the active compound(s) in an amount sufficient to provide therapeutic and/or prophylactic benefit. Such a response can be monitored by establishing an improved clinical outcome in treated patients as compared to non-treated patients. Increases in preexisting immune responses to a *Chlamydial* protein generally correlate with an improved clinical outcome. Such immune responses may generally be evaluated using standard proliferation, cytotoxicity or cytokine assays, which may be performed using samples obtained from a patient before and after treatment.

In another aspect, the present invention provides methods for using the polypeptides described above to diagnose Chlamydial infection. In this aspect, methods are provided for detecting Chlamydial infection in a biological sample, using one or more of the above polypeptides, either alone or in combination. For clarity, the term "polypeptide" will be used when describing specific embodiments of the inventive diagnostic methods. However, it will be clear to one of skill in the art that the fusion proteins of the present invention may also be employed in such methods.

As used herein, a "biological sample" is any antibody-containing sample obtained from a patient. Preferably, the sample is whole blood, sputum, serum, plasma, saliva, cerebrospinal fluid or urine. More preferably, the sample is a blood, serum or plasma sample obtained from a patient. The polypeptides are used in an assay, as described below, to determine the presence or absence of antibodies to the polypeptide(s) in the sample, relative to a predetermined cut-off value. The presence of such antibodies indicates previous sensitization to *Chlamydia* antigens which may be indicative of *Chlamydia*-infection.

In embodiments in which more than one polypeptide is employed, the polypeptides used are preferably complementary (*i.e.*, one component polypeptide will tend to detect infection in samples where the infection would not be detected by another component polypeptide). Complementary polypeptides may generally be identified by using each polypeptide individually to evaluate serum samples obtained from a series of patients known to be infected with *Chlamydia.* After determining which samples test positive (as described below) with each polypeptide, combinations of two or more polypeptides may be formulated that are capable of detecting infection in most, or all, of the samples tested.

A variety of assay formats are known to those of ordinary skill in the art for using one or more polypeptides to detect antibodies in a sample. *See, e.g.,* Harlow and Lane, Antibodies: A Laboratory Manual, Cold Spring Harbor Laboratory, 1988, which is incorporated herein by reference. In a preferred embodiment, the assay involves the use of polypeptide immobilized on a solid support to bind to and remove the antibody from the sample. The bound antibody may then be detected using a detection reagent that contains a reporter group. Suitable detection reagents include antibodies that bind to the antibody/polypeptide complex and free polypeptide labeled with a reporter group (*e.g*., in a semi-competitive assay). Alternatively, a competitive assay may be utilized, in which an antibody that binds to the polypeptide is labeled with a reporter group and allowed to bind to the immobilized antigen after incubation of the antigen with the sample. The extent to which components of the sample inhibit the binding of the labeled antibody to the polypeptide is indicative of the reactivity of the sample with the immobilized polypeptide.

The solid support may be any solid material known to those of ordinary skill in the art to which the antigen may be attached. For example, the solid support may be a test well in a microtiter plate, or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681.

The polypeptides may be bound to the solid support using a variety of techniques known to those of ordinary skill in the art. In the context of the present invention, the term "bound" refers to both noncovalent association, such as adsorption, and covalent attachment (which may be a direct linkage between the antigen and functional groups on the support or may be a linkage by way of a cross-linking agent). Binding by adsorption to a well in a microtiter plate or to a membrane is preferred. In such cases, adsorption may be achieved by contacting the polypeptide, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of polypeptide ranging from about 10 ng to about 1 µg, and preferably about 100 ng, is sufficient to bind an adequate amount of antigen.

Covalent attachment of polypeptide to a solid support may generally be achieved by first reacting the support with a bifunctional reagent that will react with both the support and a functional group, such as a hydroxyl or amino group, on the polypeptide. For example, the polypeptide may be bound to supports having an appropriate polymer coating using benzoquinone or by condensation of an aldehyde group on the support with an amine and an active hydrogen on the polypeptide (*see*, *e.g.,* Pierce Immunotechnology Catalog and Handbook, 1991, at A12-A13).

In certain embodiments, the assay is an enzyme linked immunosorbent assay (ELISA). This assay may be performed by first contacting a polypeptide antigen that has been immobilized on a solid support, commonly the well of a microtiter plate, with the sample, such that antibodies to the polypeptide within the sample are allowed to bind to the immobilized polypeptide. Unbound sample is then removed from the immobilized polypeptide and a detection reagent capable of binding to the immobilized antibody-polypeptide complex is added. The amount of detection reagent that remains bound to the solid support is then determined using a method appropriate for the specific detection reagent.

More specifically, once the polypeptide is immobilized on the support as described above, the remaining protein binding sites on the support are typically blocked. Any suitable blocking agent known to those of ordinary skill in the art, such as bovine serum albumin (BSA) or Tween 20™ (Sigma Chemical Co., St. Louis, MO) may be employed. The immobilized polypeptide is then incubated with the sample, and antibody is allowed to bind to the antigen. The sample may be diluted with a suitable dilutent, such as phosphate-buffered saline (PBS) prior to incubation. In general, an appropriate contact time (*i.e*., incubation time) is that period of time that is sufficient to detect the presence of antibody within an HGE-infected sample. Preferably, the contact time is sufficient to achieve a level of binding that is at least 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. Detection reagent may then be added to the solid support. An appropriate detection reagent is any compound that binds to the immobilized antibody-polypeptide complex and that can be detected by any of a variety of means known to those in the art. Preferably, the detection reagent contains a binding agent (such as, for example, Protein A, Protein G, immunoglobulin, lectin or free antigen) conjugated to a reporter group. Preferred reporter groups include enzymes (such as horseradish peroxidase), substrates, cofactors, inhibitors, dyes, radionuclides, luminescent groups, fluorescent groups and biotin. The conjugation of binding agent to reporter group may be achieved using standard methods known to those of ordinary skill in the art. Common binding agents may also be purchased conjugated to a variety of reporter groups from many commercial sources (*e.g*., Zymed Laboratories, San Francisco, CA, and Pierce, Rockford, IL).

The detection reagent is then incubated with the immobilized antibody-polypeptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of anti-*Chlamydia* antibodies in the sample, the signal detected from the reporter group that remains bound to the solid support is generally compared to a signal that corresponds to a predetermined cut-off value. In one preferred embodiment, the cut-off value is the average mean signal obtained when the immobilized antigen is incubated with samples from an uninfected patient. In general, a sample generating a signal that is three standard deviations above the predetermined cut-off value is considered positive for *Chlamydia*-infection. In an alternate preferred embodiment, the cut-off value is determined using a Receiver Operator Curve, according to the method of Sackett et al., Clinical Epidemiology: A Basic Science for Clinical Medicine, Little Brown and Co., 1985, pp. 106-107. Briefly, in this embodiment, the cut-off value may be determined from a plot of pairs of true positive rates (*i*.*e*., sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for Chlamydial infection.

In a related embodiment, the assay is performed in a rapid flow-through or strip test format, wherein the antigen is immobilized on a membrane, such as nitrocellulose. In the flow-through test, antibodies within the sample bind to the immobilized polypeptide as the sample passes through the membrane. A detection reagent (*e.g*., protein A-colloidal gold) then binds to the antibody-polypeptide complex as the solution containing the detection reagent flows through the membrane. The detection of bound detection reagent may then be performed as described above. In the strip test format, one end of the membrane to which polypeptide is bound is immersed in a solution containing the sample. The sample migrates along the membrane through a region containing detection reagent and to the area of immobilized polypeptide. Concentration of detection reagent at the polypeptide indicates the presence of anti-*Chlamydia* antibodies in the sample. Typically, the concentration of detection reagent at that site generates a pattern, such as a line, that can be read visually. The absence of such a pattern indicates a negative result. In general, the amount of polypeptide immobilized on the membrane is selected to generate a visually discernible pattern when the biological sample contains a level of antibodies that would be sufficient to generate a positive signal in an ELISA, as discussed above. Preferably, the amount of polypeptide immobilized on the membrane ranges from about 25 ng to about 1 µg, and more preferably from about 50 ng to about 500 ng. Such tests can typically be performed with a very small amount (*e.g*., one drop) of patient serum or blood.

Of course, numerous other assay protocols exist that are suitable for use with the polypeptides of the present invention. The above descriptions are intended to be exemplary only. One example of an alternative assay protocol which may be usefully employed in such methods is a Western blot, wherein the proteins present in a biological sample are separated on a gel, prior to exposure to a binding agent. Such techniques are well known to those of skill in the art.

The present invention further provides agents, such as antibodies and antigen-binding fragments thereof, that specifically bind to a *Chlaniydial* protein. As used herein, an antibody, or antigen-binding fragment thereof, is said to "specifically bind" to a *Chlamydial* protein if it reacts at a detectable level (within, for example, an ELISA) with a *Chlamydial* protein, and does not react detectably with unrelated proteins under similar conditions. As used herein, "binding" refers to a noncovalent association between two separate molecules such that a complex is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ L/mol. The binding constant may be determined using methods well known in the art.

Binding agents may be further capable of differentiating between patients with and without a *Chlamydial* infection using the representative assays provided herein. In other words, antibodies or other binding agents that bind to a *Chlamydial* protein will generate a signal indicating the presence of a *Chlamydial* infection in at least about 20% of patients with the disease, and will generate a negative signal indicating the absence of the disease in at least about 90% of individuals without infection. To determine whether a binding agent satisfies this requirement, biological samples (*e.g*., blood, sera, sputum urine and/or tissue biopsies ) from patients with and without *Chlamydial* infection (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e.g*., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J Immunol. 6:511-519, 1976, and improvements thereto. Briefly, these methods involve the preparation of immortal cell lines capable of producing antibodies having the desired specificity (*i.e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e*.*g*., covalently bonded) to a suitable monoclonal antibody either directly or indirectly (*e.g*., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e*.*g*., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g*., U.S. Patent No. 4,489,710, to Spitler), by irradiation of a photolabile bond (*e.g*., U.S. Patent No. 4,625,014, to Senter et al.), by hydrolysis of derivatized amino acid side chains (*e.g*., U.S. Patent No. 4,638,045, to Kohn et al.), by serum complement-mediated hydrolysis (*e.g*., U.S. Patent No. 4,671,958, to Rodwell et al.), and acid-catalyzed hydrolysis (*e.g*., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (*e.g*., U.S. Patent No. 4,507,234, to Kato et al.), peptides and polysaccharides such as aminodextran (*e.g*., U.S. Patent No. 4,699,784, to Shih et al.). A carrier may also bear an agent by noncovalent bonding or by encapsulation, such as within a liposome vesicle (*e.g*., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in site-specific regions by appropriate methods. It will be evident that the precise dose of the antibody/immunoconjugate will vary depending upon the antibody used, the antigen density, and the rate of clearance of the antibody.

Antibodies may be used in diagnostic tests to detect the presence of *Chlamydia* antigens using assays similar to those detailed above and other techniques well known to those of skill in the art, thereby providing a method for detecting Chlamydial infection in a patient.

Diagnostic reagents of the present invention may also comprise DNA sequences encoding one or more of the above polypeptides, or one or more portions thereof. For example, at least two oligonucleotide primers may be employed in a polymerase chain reaction (PCR) based assay to amplify *Chlamydia*-specific cDNA derived from a biological sample, wherein at least one of the oligonucleotide primers is specific for a DNA molecule encoding a polypeptide of the present invention. The presence of the amplified cDNA is then detected using techniques well known in the art, such as gel electrophoresis. Similarly, oligonucleotide probes specific for a DNA molecule encoding a polypeptide of the present invention may be used in a hybridization assay to detect the presence of an inventive polypeptide in a biological sample.

As used herein, the term "oligonucleotide primer/probe specific for a DNA molecule" means an oligonucleotide sequence that has at least about 80%, preferably at least about 90% and more preferably at least about 95%, identity to the DNA molecule in question. Oligonucleotide primers and/or probes which may be usefully employed in the inventive diagnostic methods preferably have at least about 10-40 nucleotides. In a preferred embodiment, the oligonucleotide primers comprise at least about 10 contiguous nucleotides of a DNA molecule encoding one of the polypeptides disclosed herein. Preferably, oligonucleotide probes for use in the inventive diagnostic methods comprise at least about 15 contiguous oligonucleotides of a DNA molecule encoding one of the polypeptides disclosed herein. Techniques for both PCR based assays and hybridization assays are well known in the art (see, for example, Mullis *et al. Ibid*; Ehrlich, *Ibid*). Primers or probes may thus be used to detect *Chlamydia*-specific sequences in biological samples. DNA probes or primers comprising oligonucleotide sequences described above may be used alone or in combination with each other.

The following Examples are offered by way of illustration and not by way of limitation.

### EXAMPLE 1

### ISOLATION OF DNA SEQUENCES ENCODING CHLAMYDIA ANTIGENS

*Chlamydia* antigens of the present invention were isolated by expression cloning of a genomic DNA library of *Chlamydia trachomatis* LGV II essentially as described by Sanderson et al. (J. Exp. Med., 1995, 182:1751-1757) and were shown to induce PBMC proliferation and IFN-γ in an immunoreactive T cell line.

A *Chlamydia*-specific T cell line was generated by stimulating PBMCs from a normal donor with no history of chlamydial genital tract infection with elementary bodies of *Chlamydia trachomatis* LGV II. This T cell line, referred to as TCL-8, was found to recognize both *Chlamydia trachomatis* and *Chlamydia pneumonia* infected monocyte-derived dendritic cells.

A randomly sheared genomic library of *Chlamydia trachomatis* LGV II was constructed in Lambda ZAP (Stratagene, La Jolla, CA) and the amplified library plated out in 96 well microtiter plates at a density of 30 clones/well. Bacteria were induced to express recombinant protein in the presence of 2 mM IPTG for 3 h, then pelleted and resuspended in 200 µl of RPMI 10% FBS. 10 µl of the induced bacterial suspension was transferred to 96 well plates containing autologous monocyte-derived dendritic cells. After a 2 h incubation, dendritic cells were washed to remove free *E. coli* and *Chlamydia*-specific T cells were added. Positive *E. coli* pools were identified by determining IFN-γ production and proliferation of the T cells in response to the pools.

Four positive pools were identified, which were broken down to yield four pure clones (referred to as 1-B1-66, 4-D7-28, 3-G3-10 and 10-C10-31), with insert sizes of 481 bp, 183 bp, 110 bp and 1400 bp, respectively. The determined DNA sequences for 1-B1-66, 4-D7-28, 3-G3-10 and 10-C10-31 are provided in SEQ ID NO: 1-4, respectively. Clone 1-B1-66 is approximately in region 536690 of the C. *trachomatis* genome (NCBI C. *trachomatis* database). Within clone 1-B1-66, an open reading frame (ORF) has been identified (nucleotides 115 - 375) that encodes a previously identified 9 kDa protein (Stephens, et al. Genbank Accession No. AE001320), the sequence of which is provided in SEQ ID NO: 5). Clone 4-D7-28 is a smaller region of the same ORF (amino acids 22-82 of 1-B1-66). Clone 3-G3-10 is approximately in region 74559 of the C. *trachomatis* genome. The insert is cloned in the antisense orientation with respect to its orientation in the genome. The clone 10-C10-31 contains an open reading frame that corresponds to a previously published sequence for S 13 ribosomal protein from *Chlamydia trachomatis* (Gu, L. et al. J. Bacteriology, 177:2594-2601, 1995). The predicted protein sequences for 4-D7-28 and 10-C10-31 are provided in SEQ ID NO: 6 and 12, respectively. Predicted protein sequences for 3-G3-10 are provided in SEQ ID NO: 7-11.

In a related series of screening studies, an additional T cell line was used to screen the genomic DNA library of *Chlamydia trachomatis* LGV II described above. A *Chlamydia*-specific T cell line (TCT-1) was derived from a patient with a chlamydial genital tract infection by stimulating patient PBMC with autologous monocyte-derived dendritic cells infected with elementary bodies of *Chlamydia trachomatis* LGV II. One clone, 4C9-18 (SEQ ID NO: 21), containing a 1256 bp insert, elicited a specific immune response, as measured by standard proliferation assays, from the *Chlamydia*-specific T cell line TCT-1. Subsequent analysis revealed this clone to contain three known sequences: lipoamide dehydrogenase (Genbank Accession No. AE001326), disclosed in SEQ ID NO: 22; a hypothetical protein CT429 (Genbank Accession No. AE001316), disclosed in SEQ ID NO: 23; and part of an open reading frame of ubiquinone methyltransferase CT428 (Genbank Accession No. AE001316), disclosed in SEQ ID NO: 24.

In further studies involving clone 4C9-18 (SEQ ID NO: 21), the full-length amino acid sequence for lipoamide dehydrognase (SEQ ID NO: 22) from C. *trachomatis* (LGV II) was expressed in clone CtL2-LPDA-FL, as disclosed in SEQ ID NO: 90.

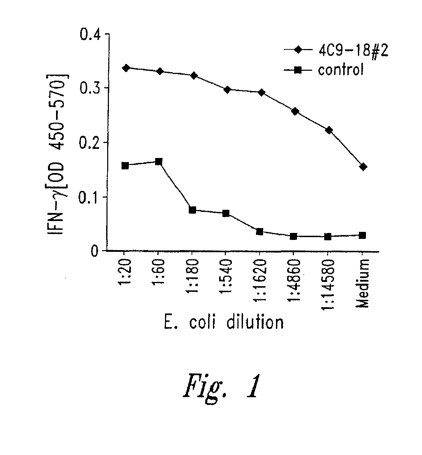

To further characterize the open reading frame containing the T cell stimulating epitope(s), a cDNA fragment containing nucleotides 1-695 of clone 4C9-18 with a cDNA sequence encoding a 6X-Histidine tag on the amino terminus was subcloned into the NdeI/EcoRI site of the pET17b vector (Novagen, Madison, WI), referred to as clone 4C9-18#2 BL21 pLysS (SEQ ID NO: 25, with the corresponding amino acid sequence provided in SEQ ID NO: 26) and transformed into E. *coli.* Selective induction of the transformed E. *coli* with 2 mM IPTG for three hours resulted in the expression of a 26 kDa protein from clone 4C9-18#2 BL21 pLysS, as evidenced by standard Coomassie-stained SDS-PAGE. To determine the immunogenicity of the protein encoded by clone 4C9-18#2 BL21 pLysS, E. *coli* expressing the 26 kDa protein were titered onto 1 x 10⁴ monocyte-derived dendritic cells and incubated for two hours. The dendritic cell cultures were washed and 2.5 x 10⁴ T cells (TCT-1) added and allowed to incubate for an additional 72 hours, at which time the level of IFN-γ in the culture supernatant was determined by ELISA. As shown in Fig. 1, the T-cell line TCT-1 was found to respond to induced cultures as measured by IFN-g, indicating a *Chlamydia*-specific T-cell response against the lipoamide dehydrogenase sequence. Similarly, the protein encoded by clone 4C9-18#2 BL21 pLysS was shown to stimulate the TCT-1 T-cell line by standard proliferation assays.

Subsequent studies to identify additional *Chlamydia trachomatis* antigens using the above-described CD4+ T-cell expression cloning technique yielded additional clones. The TCT-1 and TCL-8 *Chlamydia*-specific T-cell lines, as well as the TCP-21 T-cell line were utilized to screen the *Chlamydia trachomatis* LGVII genomic library. The TCP-2 T-cell line was derived from a patient having a humoral immune response to *Chlamydia pnuemoniae.* The TCT-1 cell line identified 37 positive pools, the TCT-3 cell line identified 41 positive pools and the TCP-21 cell line identified 2 positive pools. The following clones were derived from 10 of these positive pools. Clone 11-A3-93 (SEQ ID NO: 64), identified by the TCP-21 cell line, is a 1339 bp genomic fragment sharing homology to the HAD superfamily (CT103). The second insert in the same clone shares homology with the fab I gene (CT104) present on the complementary strand. Clone 11-C12-91 (SEQ ID NO: 63), identified using the TCP-21 cell line, has a 269 bp insert that is part of the OMP2 gene (CT443) and shares homology with the 60 kDa cysteine rich outer membrane protein of C. *pnuemoniae.*

Clone 11-G10-46, (SEQ ID NO: 62), identified using the TCT-3 cell line, contains a 688 bp insert that shares homology to the hypothetical protein CT610. Clone 11-G1-34, (SEQ ID NO: 61), identified using the TCT-3 cell line, has two partial open reading frames (ORF) with an insert size of 1215 bp. One ORF shares homology to the malate dehydrogenase gene (CT376), and the other ORF shares homology to the glycogen hydrolase gene (CT042). Clone 11-H3-68, (SEQ ID NO: 60), identified using the TCT-3 cell line, has two ORFs with a total insert size of 1180 bp. One partial ORF encodes the plasmid-encoded PGP6-D virulence protein while the second ORF is a complete ORF for the L1 ribosomal gene (CT318). Clone 11-H4-28, (SEQ ID NO: 59), identified using the TCT-3 cell line, has an insert size of 552 bp and is part of the ORF for the dnaK gene (CT396). Clone 12-B3-95, (SEQ ID NO: 58), identified using the TCT-1 cell line, has an insert size of 463 bp and is a part of the ORF for for the lipoamide dehydrogenase gene (CT557). Clones 15-G1-89 and 12-B3-95 are identical, (SEQ ID NO: 55 and 58, respectively), identified using the TCT-1 cell line, has an insert size of 463 bp and is part of the ORF for the lipoamide dehydrogenase gene (CT557). Clone 12-G3-83, (SEQ ID NO: 57), identified using the TCT-1 cell line, has an insert size of 1537 bp and has part of the ORF for the hypothetical protein CT622.

Clone 23-G7-68, (SEQ ID NO: 79), identified using the TCT-3 cell line, contains a 950 bp insert and contains a small part of the L11 ribosomal ORF, the entire ORF for L1 ribosomal protein and a part of the ORF for L10 ribosomal protein. Clone 22-F8-91, (SEQ ID NO: 80), identified using the TCT-1 cell line, contains a 395 bp insert that contains a part of the pmpC ORF on the complementary strand of the clone. Clone 21-E8-95, (SEQ ID NO: 81), identified using the TCT-3 cell line, contains a 2,085 bp insert which contains part of CT613 ORF, the complete ORF for CT612, the complete ORF for CT611 and part of the ORF for CT610. Clone 19-F12-57, (SEQ ID NO: 82), identified using the TCT-3 cell line, contains a 405 bp insert which contains part of the CT 858 ORF and a small part of the recA ORF. Clone 19-F12-53, (SEQ ID NO: 83), identified using the TCT-3 cell line, contains a 379 bp insert that is part of the ORF for CT455 encoding glutamyl tRNA synthetase. Clone 19-A5-54, (SEQ ID NO: 84), identified using the TCT-3 cell line, contains a 715 bp insert that is part of the ORF3 (complementary strand of the clone) of the cryptic plasmid. Clone 17-E11-72, (SEQ ID NO: 85), identified using the TCT-1 cell line, contains a 476 bp insert that is part of the ORF for Opp_2 and pmpD. The pmpD region of this clone is covered by the pmpD region of clone 15-H2-76. Clone 17-C1-77, (SEQ ID NO: 86), identified using the TCT-3 cell line, contains a 1551 bp insert that is part of the CT857 ORF, as well as part of the CT858 ORF. Clone 15-H2-76, (SEQ ID NO: 87), identified using the TCT-1 cell line, contains a 3,031 bp insert that contains a large part of the pmpD ORF, part of the CT089 ORF, as well as part of the ORF for SycE. Clone 15-A3-26, (SEQ ID NO: 88), contains a 976 bp insert that contains part of the ORF for CT858. Clone 17-G4-36, (SEQ ID NO: 267), identified using the TCT-10 cell line, contains a 680 bp insert that is in frame with beta-gal in the plasmid and shares homology to part of the ORF for DNA-directed RNA polymerase beta subunit (CT315 in SerD).

Several of the clones described above share homology to various polymorphic membrane proteins. The genomic sequence of *Chlamydia trachomatis* contains a family of nine polymorphic membrane protein genes, referred to as pmp. These genes are designated pmpA, pmpB, pmpc, pmpD, pmpE, pmpF, pmpG, pmpH and pmpI. Proteins expressed from these genes are believed to be of biological relevance in generating a protective immune response to a *Chlamydial* infection. In particular, pmpC, pmpD, pmpE and pmpI contain predictable signal peptides, suggesting they are outer membrane proteins, and therefore, potential immunological targets.

Based on the *Chlamydia trachomatis* LGVII serovar sequence, primer pairs were designed to PCR amplify the full-length fragments of pmpC, pmpD, pmpE, pmpG, pmpH and pmpI. The resulting fragments were subcloned into the DNA vaccine vector JA4304 or JAL, which is JA4304 with a modified linker (SmithKline Beecham, London, England). Specifically, PmpC was subcloned into the JAL vector using the 5' oligo GAT AGG CGC GCC GCA ATC ATG AAA TTT ATG TCA GCT ACT GCT G and the 3' oligo CAG AAC GCG TTT AGA ATG TCA TAC GAG CAC CGC A, as provided in SEQ ID NO: 197 and 198, respectively. PCR amplification of the gene under conditions well known in the art and ligation into the 5' ASCI/3' MluI sites of the JAL vector was completed after inserting the short nucleotide sequence GCAATC (SEQ ID NO: 199) upstream of the ATG to create a Kozak-like sequence. The resulting expression vector contained the full-length pmpC gene comprising 5325 nucleotides (SEQ ID NO: 173) containing the hypothetical signal sequence, which encodes a 187 kD protein (SEQ ID NO: 179). The pmpD gene was subcloned into the JA4304 vaccine vector following PCR amplification of the gene using the following oligos: 5' oligo- TGC AAT CAT GAG TTC GCA GAA AGA TAT AAA AAG C (SEQ ID NO: 200) and 3' oligo- CAG AGC TAG CTT AAA AGA TCA ATC GCA ATC CAG TAT TC (SEQ ID NO: 201). The gene was ligated into the a 5' blunted HIII/3' MIul site of the JA4304 vaccine vector using standard techniques well known in the art. The CAATC (SEQ ID NO: 202) was inserted upstream of the ATG to create a Kozak-like sequence. This clone is unique in that the last threonine of the HindIII site is missing due to the blunting procedure, as is the last glycine of the Kozak-like sequence. The insert, a 4593 nucleotide fragment (SEQ ID NO: 172) is the full-length gene for pmpD containing the hypothetical signal sequence, which encodes a 161 kD protein (SEQ ID NO: 178). PmpE was subcloned into the JA4304 vector using the 5' oligo- TGC AAT CAT GAA AAA AGC GTT TTT CTT TTT C (SEQ ID NO: 203), and the 3' oligo- CAG AAC GCG TCT AGA ATC GCA GAG CAA TTT C (SEQ ID NO: 204). Following PCR amplification, the gene was ligated into the 5' blunted HIII/3' MluI site of JA4304. To facilitate this, a short nucleotide sequence, TGCAATC (SEQ ID NO: 293), was added upstream of the initiation codon for creating a Kozak-like sequence and reconstituting the HindIII site. The insert is the full-length pmpE gene (SEQ ID NO: 171) containing the hypothetical signal sequence. The pmpE gene encodes a 105 kD protein (SEQ ID NO: 177). The pmpG gene was PCR amplified using the 5' oligo- GTG CAA TCA TGA TTC CTC AAG GAA TTT ACG ( SEQ ID NO: 205), and the 3' oligo- CAG AAC GCG TTT AGA ACC GGA CTT TAC TTC C (SEQ ID NO: 206) and subcloned into the JA4304 vector. Similar cloning strategies were followed for the pmpI and pmpK genes. In addition, primer pairs were designed to PCR amplify the full-length or overlapping fragments of the pmp genes, which were then subcloned for protein expression in the pET17b vector (Novagen, Madison, WI) and transfected into E. coli BL21 pLysS for expression and subsequent purification utilizing the histidine-nickel chromatographic methodology provided by Novagen. Several of the genes encoding the recombinant proteins, as described below, lack the native signal sequence to facilitate expression of the protein. Full-length protein expression of pmpC was accomplished through expression of two overlapping fragments, representing the amino and carboxy termini. Subcloning of the pmpC-amino terminal portion, which lacks the signal sequence, (SEQ ID NO: 187, with the corresponding amino acid sequence provided in SEQ ID NO: 195) used the 5' oligo- CAG ACA TAT GCA TCA CCA TCA CCA TCA CGA GGC GAG CTC GAT CCA AGA TC (SEQ ID NO: 207), and the 3' oligo- CAG AGG TAC CTC AGA TAG CAC TCT CTC CTA TTA AAG TAG G (SEQ ID NO: 208) into the 5' NdeI/3' KPN cloning site of the vector. The carboxy terminus portion of the gene, pmpC-carboxy terminal fragment (SEQ ID NO: 186, with the corresponding amino acid sequence provided in SEQ ID NO: 194), was subcloned into the 5' Net/3' KPN cloning site of the expression vector using the following primers: 5' oligo- CAG AGC TAG CAT GCA TCA CCA TCA CCA TCA CGT TAA GAT TGA GAA CTT CTC TGG C (SEQ ID NO: 209), and 3' oligo- CAG AGG TAC CTT AGA ATG TCA TAC GAG CAC CGC AG (SEQ ID NO: 210). PmpD was also expressed as two overlapping proteins. The pmpD-amino terminal portion, which lacks the signal sequence, (SEQ ID NO: 185, with the corresponding amino acid sequence provided in SEQ ID NO: 193) contains the initiating codon of the pET17b and is expressed as a 80 kD protein. For protein expression and purification purposes, a six-histidine tag follows the initiation codon and is fused at the 28^{th} amino acid (nucleotide 84) of the gene. The following primers were used, 5' oligo, CAG ACA TAT GCA TCA CCA TCA CCA TCA CGG GTT AGC (SEQ ID NO: 211), and the 3' oligo-CAG AGG TAC CTC AGC TCC TCC AGC ACA CTC TCT TC (SEQ ID NO: 212), to splice into the 5' Ndel/3' KPN cloning site of the vector. The pmpD-carboxy terminus portion (SEQ ID NO: 184) was expressed as a 92 kD protein (SEQ ID NO: 192). For expression and subsequent purification, an additional methionine, alanine and serine was included, which represent the initiation codon and the first two amino acids from the pET17b vector. A six-histidine tag downstream of the methionine, alanine and serine is fused at the 691^{st} amino acid (nucleotide 2073) of the gene. The 5' oligo- CAG AGC TAG CCA TCA CCA TCA CCA TCA CGG TGC TAT TTC TTG CTT ACG TGG (SEQ ID NO: 213) and the 3' oligo- CAG AGG TAC TTn AAA AGA TCA ATC GCA ATC CAG TAT TCG (SEQ ID NO: 214) were used to subclone the insert into the 5' NheI/3' KPN cloning site of the expression vector. PmpE was expressed as a 106kD protein (SEQ ID NO: 183 with the corresponding amino acid sequence provided in SEQ ID NO: 191). The pmpE insert also lacks the native signal sequence. PCR amplification of the gene under conditions well known in the art was performed using the following oligo primers: 5' oligo- CAG AGG ATC CAC ATC ACC ATC ACC ATC ACG GAC TAG CTA GAG AGG TTC (SEQ ID NO: 215), and the 3' oligo- CAG AGA ATT CCT AGA ATC GCA GAG CAA TTT C (SEQ ID NO: 216), and the amplified insert was ligated into a 5' BamHI/3' EcoRI site of JA4304. The short nucleotide sequence, as provided in SEQ ID NO: 217, was inserted upstream of the initiation codon for creating the Kozak-like sequence and reconstituting the HindIII site. The expressed protein contains the initiation codon and the downstream 21 amino acids from the pET17b expression vector, i.e., MASMTGGQQMGRDSSLVPSSDP (SEQ ID NO: 218). In addition, a six-histidine tag is included upstream of the sequence described above and is fused at the 28^{th} amino acid (nucleotide 84) of the gene, which eliminates the hypothetical signal peptide. The sequences provided in SEQ ID NO: 183 with the corresponding amino acid sequence provided in SEQ ID NO: 191 do not include these additional sequences. The pmpG gene (SEQ ID NO: 182, with the corresponding amino acid sequence provided in SEQ ID No; 190) was PCR amplified under conditions well known in the art using the following oligo primers: 5' oligo- CAG AGG TAC CGC ATC ACC ATC ACC ATC ACA TGA TTC CTC AAG GAA TTT ACG (SEQ ID NO: 219), and the 3' oligo- CAG AGC GGC CGC TTA GAA CCG GAC TTT ACT TCC (SEQ ID NO: 220), and ligated into the 5' KPN/3' NotI cloning site of the expression vector. The expressed protein contains an additional amino acid sequence at the amino end, namely, MASMTGGQQNGRDSSLVPHHHHHH (SEQ ID NO: 221), which comprises the initiation codon and additional sequence from the pET17b expression vector. The pmpI gene (SEQ ID NO: 181, with the corresponding amino acid sequence provided in SEQ ID No; 189) was PCR amplified under conditions well known in the art using the following oligo primers: 5' oligo- CAG AGC TAG CCA TCA CCA TCA CCA TCA CCT CTT TGG CCA GGA TCC C (SEQ ID NO: 222), and the 3' oligo- CAG AAC TAG TCT AGA ACC TGT AAG TGG TCC (SEQ ID NO: 223), and ligted into the expression vector at the 5' NheI/3' SpeI cloning site. The 95 kD expressed protein contains the initiation codon plus an additional alanine and serine from the pET17b vector at the amino end of the protein. In addition, a six-histidine tag is fused at the 21^{st} amino acid of the gene, which eliminates the hypothetical signal peptide.

Clone 14H1-4, (SEQ ID NO: 56), identified using the TCT-3 cell line, contains a complete ORF for the TSA gene, thiol specific antioxidant - CT603 (the CT603 ORF is a homolog of CPn0778 from C. *pnuemoniae*). The TSA open reading frame in clone 14-H1-4 was amplified such that the expressed protein possess an additional methionine and a 6x histidine tag (amino terminal end). This amplified insert was sub-cloned into the Nde/EcoRI sites of the pET17b vector. Upon induction of this clone with IPTG, a 22.6 kDa protein was purified by Ni-NTA agarose affinity chromatography. The determined amino acid sequence for the 195 amino acid ORF of clone 14-H1-4 encoding the TSA gene is provided in SEQ ID NO: 65. Further analysis yielded a full-length clone for the TSA gene, referred to as CTL2-TSA-FL, with the full-length amino acid sequence provided in SEQ ID NO: 92.

Further studies yielded 10 additional clones identified by the TCT-1 and TCT-3 T-cell lines, as described above. The clones identified by the TCT-1 line are: 16-D4-22, 17-C5-19, 18-C5-2, 20-G3-45 and 21-C7-66; clones identified by the TCT-3 cell line are: 17-C10-31, 17-E2-9, 22-A1-49 and 22-B3-53. Clone 21-G12-60 was recognized by both the TCT-1 and TCT-3 T cell lines. Clone 16-D4-22 (SEQ ID NO: 119), identified using the TCT-1 cell line contains a 953 bp insert that contains two genes, parts of open reading frame 3 (ORF3) and ORF4 of the C. *trachomatis* plasmid for growth within mammalian cells. Clone 17-C5-19 (SEQ ID NO: 118), contains a 951 bp insert that contains part of the ORF for DT431, encoding for clpP_1 protease and part of the ORF for CT430 (diaminopimelate epimerase). Clone 18-C5-2 (SEQ ID NO: 117) is part of the ORF for S1 ribosomal protein with a 446 bp insert that was identified using the TCT-1 cell line. Clone 20-G3-45 (SEQ ID NO: 116), identified by the TCT-1 cell line, contains a 437 bp insert that is part of the pmpB gene (CT413). Clone 21-C7-66 (SEQ ID NO: 115), identified by the TCT-1 line, contains a 995bp insert that encodes part of the dnaK like protein. The insert of this clone does not overlap with the insert of the TCT-3 clone 11-H4-28 (SEQ ID NO: 59), which was shown to be part of the dnaK gene CT396 Clone 17-C10-31 (SEQ ID NO: 114), identified by the TCT-3 cell line, contains a 976 bp insert. This clone contains part of the ORF for CT858, a protease containing IRBP and DHR domains. Clone 17-E2-9 (SEQ ID NO: 113) contains part of ORFs for two genes, CT611 and CT610, that span a 1142 bp insert. Clone 22-A1-49 (SEQ ID NO: 112), identified using the TCT-3 line, also contains two genes in a 698 bp insert. Part of the ORF for CT660 (DNA gyrase{gyrA_2}) is present on the top strand where as the complete ORF for a hypothetical protein CT659 is present on the complementary strand. Clone 22-B3-53 (SEQ ID NO: 111), identified by the TCT-1 line, has a 267 bp insert that encodes part of the ORF for GroEL (CT110). Clone 21-G12-60 (SEQ ID NO: 110), identified by both the TCT-1 and TCT-3 cell lines contains a 1461 bp insert that contains partial ORFs for hypothetical proteins CT875, CT229 and CT228.

Additional *Chlamydia* antigens were obtained by screening a genomic expression library of *Chlamydia trachomatis* (LGV II serovar) in Lambda Screen-1 vector (Novagen, Madison, WI) with sera pooled from several *Chlamydia*-infected individuals using techniques well known in the art. The following immuno-reactive clones were identified and the inserts containing *Chlamydia* genes sequenced: CTL2#1 (SEQ ID NO: 71); CTL2#2 (SEQ ID NO: 70); CTL2#3-5' (SEQ ID NO: 72, a first determined genomic sequence representing the 5' end); CTL2#3-3' (SEQ ID NO: 73, a second determined genomic sequence representing the 3' end); CTL2#4 (SEQ ID NO: 53); CTL2#5 (SEQ ID NO: 69); CTL2#6 (SEQ ID NO: 68); CTL2#7 (SEQ ID NO: 67); CTL2#8b (SEQ ID NO: 54); CTL2#9 (SEQ ID NO: 66); CTL2#10-5' (SEQ ID NO: 74, a first determined genomic sequence representing the 5' end); CTL2#10-3' (SEQ ID NO: 75, a second determined genomic sequence representing the 3' end); CTL2#11-5' (SEQ ID NO: 45, a first determined genomic sequence representing the 5' end); CTL2#11-3' (SEQ ID NO: 44, a second determined genomic sequence representing the 3' end); CTL2#12 (SEQ ID NO: 46); CT2#16-5' (SEQ ID NO: 47); CTL2#18-5' (SEQ ID NO: 49, a first determined genomic sequence representing the 5' end); CTL2#18-3' (SEQ ID NO: 48, a second determined genomic sequence representing the 3' end); CTL2#19-5' (SEQ ID NO: 76, the determined genomic sequence representing the 5' end); CTL2#21 (SEQ ID NO: 50); CTL2#23 (SEQ ID NO: 51; and CTL2#24 (SEQ ID NO: 52).

Additional *Chlamydia trachomatis* antigens were identified by serological expression cloning. These studies used sera pooled from several *Chlamydia*-infected individuals, as described above, but, IgA,and IgM antibodies were used in addition to IgG as a secondary antibody. Clones screened by this method enhance detection of antigens recognized by an early immune response to a *Chlamydial* infection, that is a mucosal humoral immune response. The following immunoreactive clones were characterized and the inserts containing *Chlamydia* genes sequenced: CTL2gam-1 (SEQ ID NO: 290), CTL2gam-2 (SEQ ID NO: 289), CTL2gam-5 (SEQ ID NO: 288), CTL2gam-6-3' (SEQ ID NO: 287, a second determined genomic sequence representing the 3' end), CTL2gam-6-5' (SEQ ID NO: 286, a first determined genomic sequence representing the 5' end), CTL2gam-8 (SEQ ID NO: 285), CTL2gam-10 (SEQ ID NO: 284), CTL2gam-13 (SEQ ID NO: 283), CTL2gam-15-3' (SEQ ID NO: 282, a second determined genomic sequence representing the 3' end), CTL2gam-15-5' (SEQ ID NO: 281, a first determined genomic sequence representing the 5' end), CTL2gam-17 (SEQ ID NO: 280), CTL2gam-18 (SEQ ID NO: 279), CTL2gam-21 (SEQ ID NO: 278), CTL2gam-23 (SEQ ID NO: 277), CTL2gam-24 (SEQ ID NO: 276), CTL2gam-26 (SEQ ID NO: 275), CTL2gam-27 (SEQ ID NO: 274), CTL2gam-28 (SEQ ID NO: 273), CTL2gam-30-3' (SEQ ID NO: 272, a second determined genomic sequence representing the 3' end) and CTL2gam-30-5' (SEQ ID NO: 271, a first determined genomic sequence representing the 5' end).

### EXAMPLE 2

### INDUCTION OF T CELL PROLIFERATION AND INTERFERON-γ PRODUCTION BY CHLAMYDIA TRACHOMATIS ANTIGENS

The ability of recombinant *Chlamydia trachomatis* antigens to induce T cell proliferation and interferon-γ production is determined as follows.

Proteins are induced by IPTG and purified by Ni-NTA agarose affinity chromatograph (Webb et al., *J. Immunology 157*:5034-5041, 1996). The purified polypeptides are then screened for the ability to induce T-cell proliferation in PBMC preparations. PBMCs from C. *trachomatis* patients as well as from normal donors whose T-cells are known to proliferate in response to *Chlamydia* antigens, are cultured in medium comprising RPMI 1640 supplemented with 10% pooled human serum and 50 µg/ml gentamicin. Purified polypeptides are added in duplicate at concentrations of 0.5 to 10 µ g/mL. After six days of culture in 96-well round-bottom plates in a volume of 200 µl, 50 µl of medium is removed from each well for determination of IFN-γ levels, as described below. The plates are then pulsed with 1 µCi/well of tritiated thymidine for a further 18 hours, harvested and tritium uptake determined using a gas scintillation counter. Fractions that result in proliferation in both replicates three fold greater than the proliferation observed in cells cultured in medium alone are considered positive.

IFN-γ is measured using an enzyme-linked immunosorbent assay (ELISA). ELISA plates are coated with a mouse monoclonal antibody directed to human IFN-γ (PharMingen, San Diego, CA) in PBS for four hours at room temperature. Wells are then blocked with PBS containing 5% (W/V) non-fat dried milk for 1 hour at room temperature. The plates are washed six times in PBS/0.2% TWEEN-20 and samples diluted 1:2 in culture medium in the ELISA plates are incubated overnight at room temperature. The plates are again washed and a polyclonal rabbit anti-human IFN-γ serum diluted 1:3000 in PBS/10% normal goat serum is added to each well. The plates are then incubated for two hours at room temperature, washed and horseradish peroxidase-coupled anti-rabbit IgG (Sigma Chemical So., St. Louis, MO) is added at a 1:2000 dilution in PBS/5% non-fat dried milk. After a further two hour incubation at room temperature, the plates are washed and TMB substrate added. The reaction is stopped after 20 min with 1 N sulfuric acid. Optical density is determined at 450 nm using 570 nm as a reference wavelength. Fractions that result in both replicates giving an OD two fold greater than the mean OD from cells cultured in medium alone, plus 3 standard deviations, are considered positive.

Using the above methodology, recombinant 1B1-66 protein (SEQ ID NO: 5) as well as two synthetic peptides corresponding to amino acid residues 48-67 (SEQ ID NO: 13; referred to as 1-B1-66/48-67) and 58-77 (SEQ ID NO: 14, referred to as 1B1-66/58-77), respectively, of SEQ ID NO: 5, were found to induce a proliferative response and IFN-γ production in a Chlamydia-specific T cell line used to screen a genomic library of C. *trachomatis* LGV II.

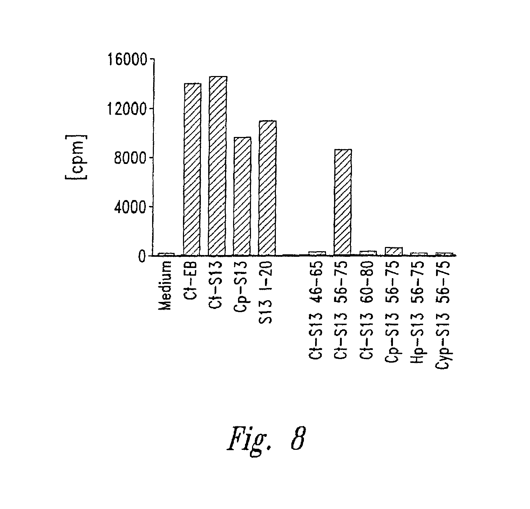

Further studies have identified a C. *trachomatis*-specific T-cell epitope in the ribosomal S 13 protein. Employing standard epitope mapping techniques well known in the art, two T-cell epitopes in the ribosomal S13 protein (rS13) were identified with a *Chlamydia*-specific T-cell line from donor CL-8 (T-cell line TCL-8 EB/DC). Fig. 8 illustrates that the first peptide, rS13 1-20 (SEQ ID NO: 106), is 100% identical with the corresponding C. *pneumoniae* sequence, explaining the cross-reactivity of the T-cell line to recombinant *C. trachomatis-* and *C. pneumoniae*-rS13. The response to the second peptide rS13 56-75 (SEQ ID NO: 108) is C. *trachomatis*-specific, indicating that the rS13 response in this healthy asymptomatic donor was elicited by exposure to C. *trachomatis* and not to C. *pneumoniae*, or any other microbial infection.

As described in Example 1, Clone 11-C12-91 (SEQ ID NO: 63), identified using the TCP-21 cell line, has a 269 bp insert that is part of the OMP2 gene (CT443) and shares homology with the 60 kDa cysteine rich outer membrane protein of C. *pneumoniae,* referred to as OMCB. To further define the reactive epitope(s), epitope mapping was performed using a series of overlapping peptides and the immunoassay previously described. Briefly, proliferative responses were determined by stimulating 2.5 x 10⁴ TCP-21 T-cells in the presence of 1 x 10⁴ monocyte-derived dendritic cells with either non-infectious elementary bodies derived from C. *trachomatis* and C. *pneumoniae,* or peptides derived from the protein sequence of C. *trachomatis* or *C. pneumoniae* OMCB protein (0.1 µg/ml). The TCP-21 T-cells responded to epitopes CT-OMCB #167-186, CT-OMCB #171-190, CT-OMCB #171-186, and to a lesser extent, CT-OMCB #175-186 (SEQ ID NO: 249-252, respectively). Notably, the TCP-21 T-cell line also gave a proliferative response to the homologous C. *pneumoniae* peptide CP-OMCB #171-186 (SEQ ID NO: 253), which was equal to or greater than the response to the C. *trachomatis* peptides. The amino acid substitutions in position two (i.e., Asp for Glu) and position four (i.e., Cys for Ser) did not alter the proliferative response of the T-cells and therefore demonstrating this epitope to be a cross-reactive epitope between C. *trachomatis* and C. *pneumoniae.*

To further define the epitope described above, an additional T-cell line, TCT-3, was used in epitope mapping experiments. The immunoassays were performed as described above, except that only peptides from C. *trachomatis* were tested. The T-cells gave a proliferative response to two peptides, CT-OMCB #152-171 and CT-OMCB #157-176 (SEQ ID NO: 246 and 247, respectively), thereby defining an additional immunogenic epitope in the cysteine rich outer membrane protein of *C. trachomatis.*

Clone 14H1-4, (SEQ ID NO: 56, with the corresponding full-length amino acid sequence provided in SEQ ID NO: 92), was identified using the TCT-3 cell line in the CD4 T-cell expression cloning system previously described, and was shown to contain a complete ORF for the, thiol specific antioxidant gene (CT603), referred to as TSA. Epitope mapping immunoassays were performed, as described above, to further define the epitope. The TCT-3 T-cells line exhibited a strong proliferative response to the overlapping peptides CT-TSA #96-115, CT-TSA #101-120 and CT-TSA #106-125 (SEQ ID NO: 254-256, respectively) demonstrating an immunoreactive epitope in the thiol specific antioxidant gene of *C. trachomatis* serovar LGVII.

### EXAMPLE 3

### PREPARATION OF SYNTHETIC POLYPEPTIDES

Polypeptides may be synthesized on a Millipore 9050 peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugating or labeling of the peptide. Cleavage of the peptides from the solid support may be carried out using the following cleavage mixture: trifluoroacetic acid:ethanedithiolahioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0-60% acetonitrile (containing 0.1% TFA) in water (containing 0.1% TFA) may be used to elute the peptides. Following lyophilization of the pure fractions, the peptides may be characterized using electrospray mass spectrometry and by amino acid analysis.

### EXAMPLE 4

### ISOLATION AND CHARACTERIZATION OF DNA SEQUENCES ENCODING CHLAMYDIA ANTIGENS USING RETROVIRAL EXPRESSION VECTOR SYSTEMS AND SUBSEQUENT IMMUNOLOGICAL ANALYSIS

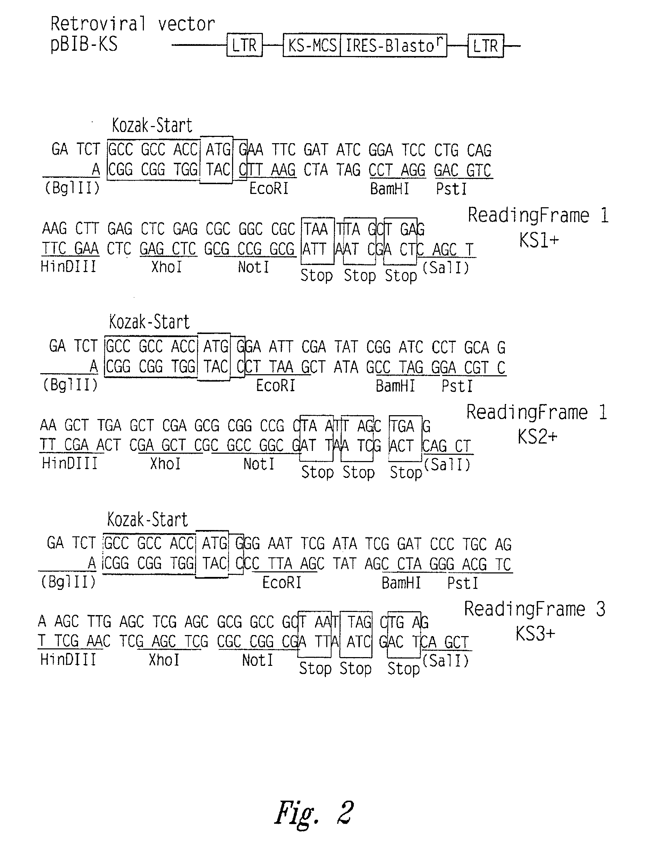

A genomic library of *Chlamydia trachomatis* LGV II was constructed by limited digests using BamHI, BglII, BstYi and MboI restriction enzymes. The restriction digest fragments were subsequently ligated into the BamHI site of the retroviral vectors pBIB-KS1,2,3. This vector set was modified to contain a Kosak translation initiation site and stop codons in order to allow expression of proteins from short DNA genomic fragments, as shown in Fig. 2. DNA pools of 80 clones were prepared and transfected into the retroviral packaging line Phoenix-Ampho, as described in Pear, W.S., Scott, M.L. and Nolan, G.P., Generation of High Titre, Helper-free Retroviruses by Transient Transfection. Methods in Molecular Medicine: Gene Therapy Protocols, Humana Press, Totowa, NJ, pp. 41-57. The *Chlamydia* library in retroviral form was then transduced into H2-Ld expressing P815 cells, which were then used as target cells to stimulate an antigen specific T-cell line.

A *Chlamydia*-specific, murine H2^{d} restricted CD8+ T-cell line was expanded in culture by repeated rounds of stimulation with irradiated C. *trachomatis*-infected J774 cells and irradiated syngeneic spleen cells, as described by Starnbach, M., in J. Immunol., 153:5183, 1994. This *Chlamydia*-specific T-cell line was used to screen the above *Chlamydia* genomic library expressed by the retrovirally-transduced P815 cells. Positive DNA pools were identified by detection of IFN-γ production using Elispot analysis (*SEE* Lalvani et al., J. Experimental Medicine 186:859-865, 1997).

Two positive pools, referred to as 2C7 and 2E10, were identified by IFN-γ Elispot assays. Stable transductants of P815 cells from pool 2C7 were cloned by limiting dilution and individual clones were selected based upon their capacity to elicit IFN-γ production from the *Chlamydia*-specific CTL line. From this screening process, four positive clones were selected, referred to as 2C7-8, 2C7-9, 2C7-19 and 2C7-21. Similarly, the positive pool 2E10 was further screened, resulting in a an additional positive clone, which contains three inserts. The three inserts are fragments of the CT016, tRNA syntase and clpX genes (SEQ ID NO: 268-270, respectively).

Transgenic DNA from these four positive 2C7.8 clones were PCR amplified using pBIB-KS specific primers to selectively amplify the *Chlamydia* DNA insert. Amplified inserts were gel purified and sequenced. One immunoreactive clone, 2C7-8 (SEQ ID NO: 15, with the predicted amino acid sequence provided in SEQ ID NO: 32), is a 160 bp fragment with homology to nucleotides 597304-597145 of *Chlamydia trachomatis,* serovar D (NCBI, BLASTN search; SEQ ID NO: 33, with the predicted amino acid sequence provided in SEQ ID NO: 34). The sequence of clone 2C7-8 maps within two putative open reading frames from the region of high homology described immediately above, and in particular, one of these putative open reading frames, consisting of a 298 amino acid fragment (SEQ ID NO: 16, with the predicted amino acid sequence provided in SEQ ID NO: 17), was demonstrated to exhibit immunological activity.

Full-length cloning of the 298 amino acid fragment (referred to as CT529 and/or the Cap1 gene) from serovar L2 was obtained by PCR amplification using 5'-ttttgaagcaggtaggtgaatatg (forward) (SEQ ID NO: 159) and 5'-ttaagaaatttaaaaaatccctta (reverse) (SEQ ID NO: 160) primers, using purified C. *trachomatis* L2 genomic DNA as template. This PCR product was gel-purified, cloned into pCRBlunt (Invitrogen, Carlsbad, CA) for sequencing, and then subcloned into the *Eco*RI site of pBIB-KMS, a derivative of pBIB-KS for expression. The *Chlamydia pnuemoniae* homlogue of CT529 is provided in SEQ ID NO: 291, with the corresponding amino acid sequence provided in SEQ ID NO: 292.

Full-length DNA encoding various CT529 serovars were amplified by PCR from bacterial lysates containing 10⁵ IFU, essentially as described (Denamur, E., C. Sayada, A. Souriau, J. Orfila, A. Rodolakis and J. Elion. 1991. J. Gen. Microbiol. 137: 2525). The following serovars were amplified as described: Ba (SEQ ID NO: 134, with the corresponding predicted amino acid sequence provided in SEQ ID NO: 135); E (BOUR) and E (MTW447) (SEQ ID NO: 122, with the corresponding predicted amino acid sequence provided in SEQ ID NO: 123); F (NI1) (SEQ ID NO: 128, with the corresponding predicted amino acid sequence provided in SEQ ID NO: 129); G; (SEQ ID NO: 126, with the corresponding predicted amino acid sequence provided in SEQ ID NO: 127); Ia (SEQ ID NO: 124, with the corresponding predicted amino acid sequence provided in SEQ ID NO: 125); L1 (SEQ ID NO: 130, with the corresponding predicted amino acid sequence provided in SEQ ID NO: 131); L3 (SEQ ID NO: 132, with the corresponding predicted amino acid sequence provided in SEQ ID NO: 133); I (SEQ ID NO: 263, with the corresponding predicted amino acid sequence provided in SEQ ID NO: 264); K (SEQ ID NO: 265, with the corresponding predicted amino acid sequence provided in SEQ ID NO: 266); and MoPn (SEQ ID NO: 136, with the corresponding predicted amino acid sequence provided in SEQ ID NO: 137). PCR reactions were performed with Advantage Genomic PCR Kit (Clontech, Palo Alto, CA) using primers specific for serovar L2 DNA (external to the ORF). Primers sequences were 5'-ggtataatatctctctaaattttg (forward-SEQ ID NO: 161) and 5'-agataaaaaaggctgtttc' (reverse-SEQ ID NO: 162) except for MoPn which required 5'-ttttgaagcaggtaggtgaatatg (forward-SEQ ID NO: 163) and 5'-tttacaataagaaaagctaagcactttgt (reverse-SEQ ID NO: 164). PCR amplified DNA was purified with QIAquick PCR purification kit (Qiagen, Valencia, CA) and cloned in pCR2.1 (Invitrogen, Carlsbad, CA) for sequencing.

Sequencing of DNA derived from PCR amplified inserts of immunoreactive clones was done on an automated sequencer (ABI 377) using both a pBIB-KS specific forward primer S'-ccttacacagtcctgctgac (SEQ ID NO: 165) and a reverse primer 3'-gtttccgggccctcacattg (SEQ ID NO: 166). PCRBlunt cloned DNA coding for CT529 serovar L2 and pCR2.1 cloned DNA coding for CT529 serovar Ba, E (BOUR), E (MTW447), F (N11), G, Ia, K, L1, L3 and MoPn were sequenced using T7 promoter primer and universal M13 forward and M13 reverse primers.

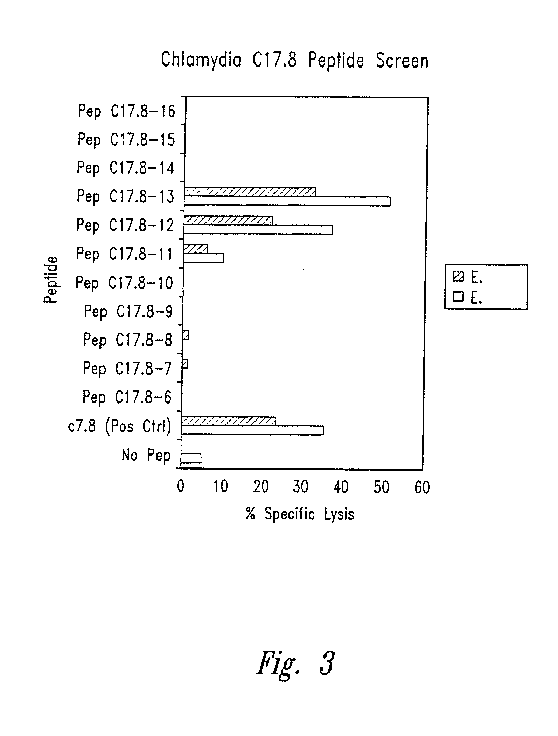

To determine if these two putative open reading frames (SEQ ID NO: 16 and 20) encoded a protein with an associated immunological function, overlapping peptides (17-20 amino acid lengths) spanning the lengths of the two open reading frames were synthesized, as described in Example 3. A standard chromium release assay was utilized to determine the per cent specific lysis of peptide-pulsed H2^{d} restricted target cells. In this assay, aliquots of P815 cells (H2^{d}) were labeled at 37° C for one hour with 100 µCi of ⁵¹Cr in the presence or absence of 1 µg/ml of the indicated peptides. Following this incubation, labeled P815 cells were washed to remove excess ⁵¹Cr and peptide, and subsequently plated in duplicate in microculture plates at a concentration of 1,000 cells/well. Effector CTL (*Chlamydia*-specific CD8 T cells) were added at the indicated effector:target ratios. Following a 4 hour incubation, supernatants were harvested and measured by gamma-counter for release of ⁵¹Cr into the supernatant. Two overlapping peptides from the 298 amino acid open reading frame did specifically stimulate the CTL line. The peptides represented in SEQ ID NO: 138-156 were synthsized, representing the translation of the L2 homologue of the serovar D open reading frame for CT529 (Cap1 gene) and 216 amino acid open reading frame.As shown in Fig. 3, peptides CtC7.8-12 (SEQ ID NO: 18, also referred to as Capl#132-147, SEQ ID NO: 139 ) and CtC7.8-13 (SEQ ID NO: 19, also referred to as Capl#138-155, SEQ ID NO: 140) were able to elicit 38 to 52% specific lysis, respectively, at an effector to target ratio of 10:1. Notably, the overlap between these two peptides contained a predicted H2^{d} (K^{d} and L^{d} ) binding peptide. A 10 amino acid peptide was synthesized to correspond to this overlapping sequence (SEQ ID NO: 31) and was found to generate a strong immune response from the anti-*Chlamydia* CTL line by elispot assay. Significantly, a search of the most recent Genbank database revealed no proteins have previously been described for this gene. Therefore, the putative open reading frame encoding clone 2C7-8 (SEQ ID NO: 15) defines a gene which encompasses an antigen from *Chlamydia* capable of stimulating antigen-specific CD8+ T-cells in a MHC-I restricted manner, demonstrating this antigen could be used to develop a vaccine against *Chlamydia.*

To confirm these results and to further map the epitope, truncated peptides (SEQ ID NO: 138-156) were made and tested for recognition by the T-cells in an IFN-g ELISPOT assay. Truncations of either Ser139 (Capl#140-147, SEQ ID NO: 146) or Leu147 (Capl#138-146, SEQ ID NO: 147) abrogate T-cell recognition. These results indicate that the 9-mer peptide Capl#139-147 (SFIGGITYL, SEQ ID NO: 145) is the minimal epitope recognized by the *Chlamydia*-specific T-cells.

Sequence alignments of Cap1 (CT529) from selected serovars of C. *trachomatis* (SEQ ID NO: 121, 123, 125, 127, 129, 131, 133, 135, 137 and 139) shows one of the amino acid differences is found in position 2 of the proposed epitope. The homologous serovar D peptide is SIIGGITYL (SEQ ID NO: 168). The ability of SFIGGITYL and SIIGGITYL to target cells for recognition by the *Chlamydia* specific T-cells was compared. Serial dilutions of each peptide were incubated with P815 cells and tested for recognition by the T-cells in a ⁵¹Cr release assay, as described above. The *Chlamydia*-specific T-cells recognize the serovar L2 peptide at a minimum concentration of 1 nM and the serovar D peptide at a minimum concentration of 10 nM.

Further studies have shown that a Cap1#139-147-specific T-cell clone recognizes C. *trachomatis* infected cells. To confirm that Cap1₁₃₉₋₁₄₇ is presented on the surface of *Chlamydia* infected cells, Balb-3T3 (H-2^{d}) cells were infected with C. *trachomatis* serovar L2 and tested to determine whether these cells are recognized by a CD8+ T-cell clone specific for Cap1#139-147 epitope (SEQ ID NO: 145). The T-cell clone specific for Cap1#139-147 epitope was obtained by limiting dilution of the line 69 T-cells. The T-cell clone specifically recognized the *Chlamydia* infected cells. In these experiments, target cells were C. *trachomatis* infected (positive control) or uninfected Balb/3T3 cells, showing 45%, 36% and 30% specific lysis at 30:1, 10:1 and 3:1 effector to target ratios, respectively; or Capl#139-147 epitope (SEQ ID NO: 145) coated, or untreated P815 cells, showing 83%, 75% and 58% specific lysis at 30:1, 10:1 and 3:1 effector to target ratios, respectively (negative controls having less than 5% lysis in all cases). This data suggests that the epitope is presented during infection.

In vivo studies show Cap1#139-147 epitope-specific T-cells are primed during murine infection with C. *trachomatis.* To determine if infection with C. *trachomatis* primes a Cap1#139-147 epitope-specific T-cell response, mice were infected i.p. with 10⁸ IFU of C. *trachomatis* serovar L2. Two weeks after infection, the mice were sacrificed and spleen cells were stimulated on irradiated syngeneic spleen cells pulsed with Cap1#139-147 epitope peptide. After 5 days of stimulation, the cultures were used in a standard ⁵¹Cr release assay to determine if there were Cap1#139-147 epitope-specific T-cells present in the culture. Specifically, spleen cells from a C. *trachomatis* serovar L2 immunized mouse or a control mouse injected with PBS after a 5 days culture with Cap1#139-147 peptide-coated syngeneic spleen cells and CD8+ T-cells able to specifically recognize Cap1#139-147 epitope gave 73%, 60% and 32% specific lysis at a30:1, 10:1 and 3:1 effector to target ratios, respectively. The control mice had a percent lysis of approximately 10% at a 30:1 effector to target ratio, and steadily declining with lowering E:T ratios. Target cells were Cap1#139-147 peptide-coated, or untreated P815 cells. These data suggest that Cap1#139-147 peptide-specific T-cells are primed during murine infection with C. *trachomatis.*

### EXAMPLE 5

### GENERATION OF ANTIBODY AND T-CELL RESPONSES IN MICE IMMUNIZED WITH CHLAMYDIA ANTIGENS

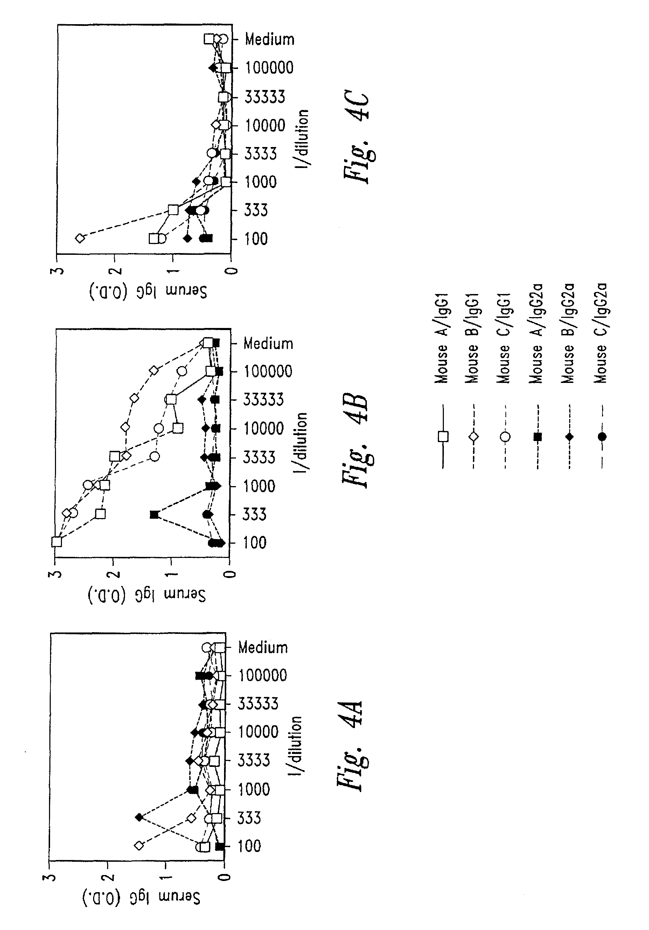

Immunogenicity studies were conducted to determine the antibody and CD4+ T cell responses in mice immunized with either purified SWIB or S13 proteins formulated with Montanide adjuvant, or DNA-based immunizations with pcDNA-3 expression vectors containing the DNA sequences for SWIB or S13. SWIB is also referred to as clone 1-B1-66 (SEQ ID NO: 1, with the corresponding amino acid sequence provided in SEQ ID NO: 5), and S13 ribosomal protein is also referred to as clone 10-C10-31 (SEQ ID NO: 4, with the corresponding amino acid sequence provided in SEQ ID NO: 12). In the first experiment, groups of three C57BL/6 mice were immunized twice and monitored for antibody and CD4+ T-cell responses. DNA immunizations were intradermal at the base of the tail and polypeptide immunizations were administered by subcutaneous route. Results from standard ³H-incorporation assays of spleen cells from immunized mice shows a strong proliferative response from the group immunized with purified recombinant SWIB polypeptide (SEQ ID NO: 5). Further analysis by cytokine induction assays, as previously described, demonstrated that the group immunized with SWIB polypeptide produced a measurable IFN-γ and IL-4 response. Subsequent ELISA-based assays to determine the predominant antibody isotype response in the experimental group immunized with the SWIB polypeptide were performed. Fig. 4 illustrates the SWIB-immunized group gave a humoral response that was predominantly IgG1.

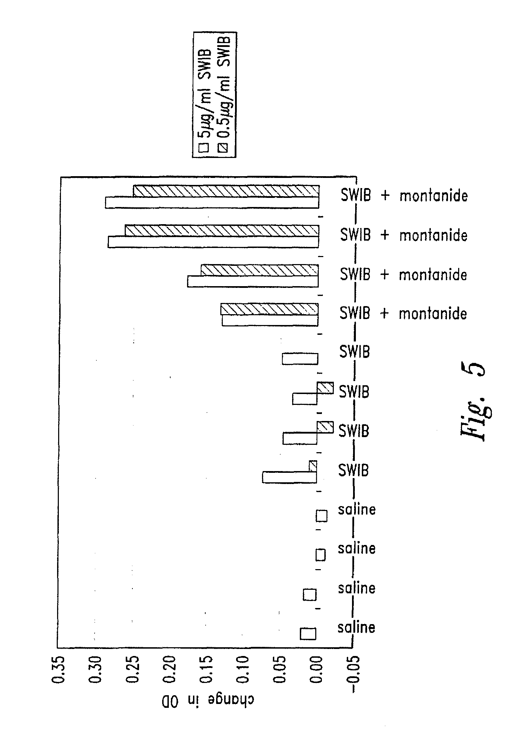

In a second experiment, C3H mice were immunized three times with 10 µg purified SWIB protein (also referred to as clone 1-B1-66, SEQ ID NO: 5) formulated in either PBS or Montanide at three week intervals and harvested two weeks after the third immunization. Antibody titers directed against the SWIB protein were determined by standard ELISA-based techniques well known in the art, demonstrating the SWIB protein formulated with Montanide adjuvant induced a strong humoral immune response. T-cell proliferative responses were determined by a XTT-based assay (Scudiero, et al, Cancer Research, 1988, 48:4827). As shown in Fig. 5, splenocytes from mice immunized with the SWIB polypeptide plus Montanide elicited an antigen specific proliferative response. In addition, the capacity of splenocytes from immunized animals to secrete IFN-γ in response to soluble recombinant SWIB polypeptide was determined using the cytokine induction assay previously described. The splenocytes from all animals in the group immunized with SWIB polypeptide formulated with montanide adjuvant secreted IFN-γ in response to exposure to the SWIB Chlamydia antigen, demonstrating an *Chlamydia*-specific immune response.

In a further experiment, C3H mice were immunized at three separate time points at the base of the tail with 10 µg of purified SWIB or S13 protein (C. *trachomatis,* SWIB protein, clone 1-B1-66, SEQ ID NO: 5, and S13 protein, clone 10-C10-31, SEQ ID NO: 4) formulated with the SBAS2 adjuvant (SmithKline Beecham, London, England). Antigen-specific antibody titers were measured by ELISA, showing both polypeptides induced a strong IgG response, ranging in titers from 1 x10⁴ to 1 x10⁻⁵. The IgG1 and IgG2a components of this response were present in fairly equal amounts. Antigen-specific T-cell proliferative responses, determined by standard ³H-incorporation assays on spleen cells isolated from immunized mice, were quite strong for SWIB (50,000 cpm above the negative control) and even stronger for s13 (100,000 cpm above the negative control). The IFNγ production was assayed by standard ELISA techniques from supernatant from the proliferating culture. *In vitro* restimulation of the culture with S13 protein induced high levels of IFNγ production, approximately 25 ng/ml versus 2 ng/ml for the negative control. Restimulation with the SWIB protein also induced IFNγ, although to a lesser extent.

In a related experiment, C3H mice were immunized at three separate time points with 10 µg of purified SWIB or S13 protein (C. *trachomatis,* SWIB protein, clone 1-B1-66, SEQ ID NO: 5, and S 13 protein, clone 10-C10-31, SEQ ID NO: 4) mixed with 10 µg of Cholera Toxin. Mucosal immunization was through intranasal inoculation. Antigen-specific antibody responses were determined by standard ELISA techniques. Antigen-specific IgG antibodies were present in the blood of SWIB-immunized mice, with titers ranging from 1 x10⁻³ to 1 x10⁻⁴, but non-detectable in the S13-immunized animals. Antigen-specific T-cell responses from isolated splenocytes, as measured by IFNγ production, gave similar results to those described immediately above for systemic immunization.

An animal study was conducted to determine the immunogenicity of the CT529 serovar LGVII CTL epitope, defined by the CT529 10mer consensus peptide (CSFIGGITYL - SEQ ID NO: 31), which was identified as an H2-Kd restricted CTL epitope. BALB/c mice (3 mice per group) were immunized three times with 25 µg of peptide combined with various adjuvants. The peptide was administered systemically at the base of the tail in either SKB Adjuvant System SBAS-2", SBAS-7 (SmithKline Beecham, London, England) or Montanide. The peptide was also administered intranasally mixed with 10ug of Cholera Toxin (CT). Naive mice were used as a control. Four weeks after the 3rd immunization, spleen cells were restimulated with LPS-blasts pulsed with 10ug/ml CT529 10mer consensus peptide at three different effector to LPS-blasts ratios : 6, 1.5 and 0.4 at 1x10⁶ cell/ml. After 2 restimulations, effector cells were tested for their ability to lyse peptide pulsed P815 cells using a standard chromium release assay. A non-relevant peptide from chicken egg ovalbumin was used as a negative control. The results demonstrate that a significant immune response was elicited towards the CT529 10mer consensus peptide and that antigen-specific T-cells capable of lysing peptide-pulsed targets were elicited in response to immunization with the peptide. Specifically, antigen-specific lytic activities were found in the SBAS-7 and CT adjuvanted group while Montanide and SBAS-2" failed to adjuvant the CTL epitope immunization.

### EXAMPLE 6

### EXPRESSION AND CHARACTERIZATION OF CHLAMYDIA PNEUMONIAE GENES

The human T-cell line, TCL-8, described in Example 1, recognizes *Chlamydia trachomatis* as well as *Chlamydia pneumonia* infected monocyte-derived dendritic cells, suggesting *Chlamydia trachomatis* and *pneumonia* may encode cross-reactive T-cell epitopes. To isolate the *Chlamydia pneumonia* genes homologous to *Chlamydia trachomatis* LGV II clones 1B1-66, also referred to as SWIB (SEQ ID NO: 1) and clone 10C10-31, also referred to as S13 ribosomal protein (SEQ ID NO: 4), HeLa 229 cells were infected with C. *pneumonia* strain TWAR (CDC/CWL-029). After three days incubation, the C. *pneumonia-*infected HeLa cells were harvested, washed and resuspended in 200 µl water and heated in a boiling water bath for 20 minutes. Ten microliters of the disrupted cell suspension was used as the PCR template.

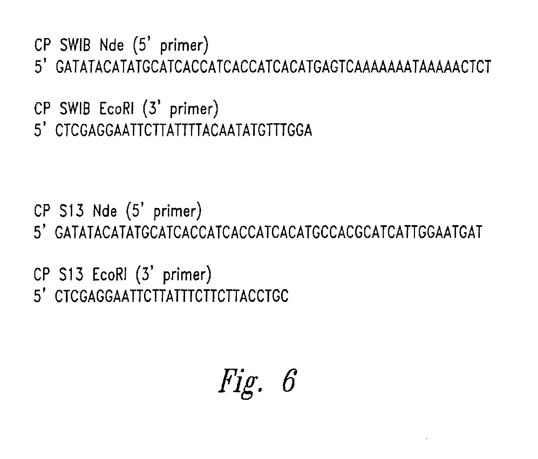

*C. pneumonia* specific primers were designed for clones 1B1-66 and 10C10-31 such that the 5' end had a 6X-Histidine tag and a Nde I site inserted, and the 3' end had a stop codon and a BamHI site included (Fig. 6). The PCR products were amplified and sequenced by standard techniques well known in the art. The C. *pneumonia*-specific PCR products were cloned into expression vector pET17B (Novagen, Madison, WI) and transfected into E. coli BL21 pLysS for expression and subsequent purification utilizing the histidine-nickel chromatographic methodology provided by Novagen. Two proteins from C. *pneumonia* were thus generated, a 10-11 kDa protein referred to as CpSWIB (SEQ ID NO: 27, and SEQ ID NO: 78 having a 6X His tag, with the corresponding amino acid sequence provided in SEQ ID NO: 28, respectively), a 15 kDa protein referred to as CpS13 (SEQ ID NO: 29, and SEQ ID NO: 77, having a 6X His tag, with the corresponding amino acid sequence provided in SEQ ID NO: 30 and 91, respectively).

### EXAMPLE 7

### INDUCTION OF T CELL PROLIFERATION AND INTERFERON-γ PRODUCTION BY CHLAMYDIA PNEUMONIAE ANTIGENS

The ability of recombinant *Chlamydia pneumoniae* antigens to induce T cell proliferation and interferon-γ production is determined as follows.

Proteins are induced by IPTG and purified by Ni-NTA agarose affinity chromatography (Webb et al., J Immunology 157:5034-5041, 1996). The purified polypeptides are then screened for the ability to induce T-cell proliferation in PBMC preparations. PBMCs from C. *pneumoniae* patients as well as from normal donors whose T-cells are known to proliferate in response to *Chlamydia* antigens, are cultured in medium comprising RPMI 1640 supplemented with 10% pooled human serum and 50 µg/ml gentamicin. Purified polypeptides are added in duplicate at concentrations of 0.5 to 10 µ g/mL. After six days of culture in 96-well round-bottom plates in a volume of 200 µl, 50 µl of medium is removed from each well for determination of IFN-γ levels, as described below. The plates are then pulsed with 1 µCi/well of tritiated thymidine for a further 18 hours, harvested and tritium uptake determined using a gas scintillation counter. Fractions that result in proliferation in both replicates three fold greater than the proliferation observed in cells cultured in medium alone are considered positive.

IFN-γ was measured using an enzyme-linked immunosorbent assay (ELISA). ELISA plates are coated with a mouse monoclonal antibody directed to human IFN-γ (PharMingen, San Diego, CA) in PBS for four hours at room temperature. Wells are then blocked with PBS containing 5% (W/V) non-fat dried milk for 1 hour at room temperature. The plates are washed six times in PBS/0.2% TWEEN-20 and samples diluted 1:2 in culture medium in the ELISA plates are incubated overnight at room temperature. The plates are again washed and a polyclonal rabbit anti-human IFN-γ serum diluted 1:3000 in PBS/10% normal goat serum is added to each well. The plates are then incubated for two hours at room temperature, washed and horseradish peroxidase-coupled anti-rabbit IgG (Sigma Chemical So., St. Louis, MO) is added at a 1:2000 dilution in PBS/5% non-fat dried milk. After a further two hour incubation at room temperature, the plates are washed and TMB substrate added. The reaction is stopped after 20 min with 1 N sulfuric acid. Optical density is determined at 450 nm using 570 nm as a reference wavelength. Fractions that result in both replicates giving an OD two fold greater than the mean OD from cells cultured in medium alone, plus 3 standard deviations, are considered positive.

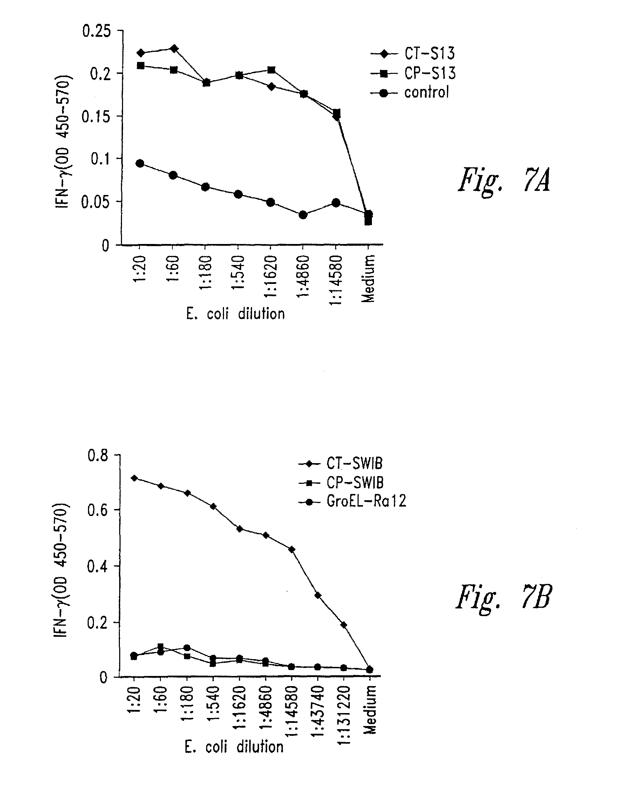

A human anti-*Chlamydia* T-cell line (TCL-8) capable of cross-reacting to *C*. *trachomatis* and *C. pneumonia* was used to determine whether the expressed proteins described in the example above, (i.e., CpSWIB, SEQ ID NO: 27, and SEQ ID NO: 78 having a 6X His tag, with the corresponding amino acid sequence provided in SEQ ID NO: 28, respectively, and the 15 kDa protein referred to as CpS13 SEQ ID NO: 29, and SEQ ID NO: 77, having a 6X His tag, with the corresponding amino acid sequence provided in SEQ ID NO: 30 and 91, respectively), possessed T-cell epitopes common to both *C. trachomatis* and *C. pneumonia.* Briefly, *E*. *coli* expressing *Chlamydial* proteins were titered on 1 x 10⁴ monocyte-derived dendritic cells. After two hours, the dendritic_cells cultures were washed and 2.5 x 10⁴ T cells (TCL-8) added and allowed to incubate for an additional 72 hours. The amount of INF-γ in the culture supernatant was then determined by ELISA. As shown in Figs. 7A and 7B, the TCL-8 T-cell line specifically recognized the S13 ribosomal protein from both C. *trachomatis* and C. *pneumonia* as demonstrated by the antigen-specific induction of IFN-γ, whereas only the SWIB protein from *C*. *trachomatis* was recognized by the T-cell line. To validate these results, the T cell epitope of C. *trachomatis* SWIB was identified by epitope mapping using target cells pulsed with a series of overlapping peptides and the T-cell line TCL-8. 3H-thymidine incorporation assays demonstrated that the peptide, referred to as C.t.SWIB 52-67, of SEQ ID NO: 39 gave the strongest proliferation of the TCL-8 line. The homologous peptides corresponding to the SWIB of C. *pneumoniae* sequence (SEQ ID NO: 40), the topoisomerase-SWIB fusion of *C. pneumoniae* (SEQ ID NO: 43) and *C. trachomatis* (SEQ ID NO: 42) as well as the human SWI domain (SEQ ID NO: 41) were synthesized and tested in the above assay. The T-cell line TCL-8 only recognized the *C. trachomatis* peptide of SEQ ID NO: 39 and not the corresponding C. *pneumoniae* peptide (SEQ ID NO: 40), or the other corresponding peptides described above (SEQ ID NO; 41-43).

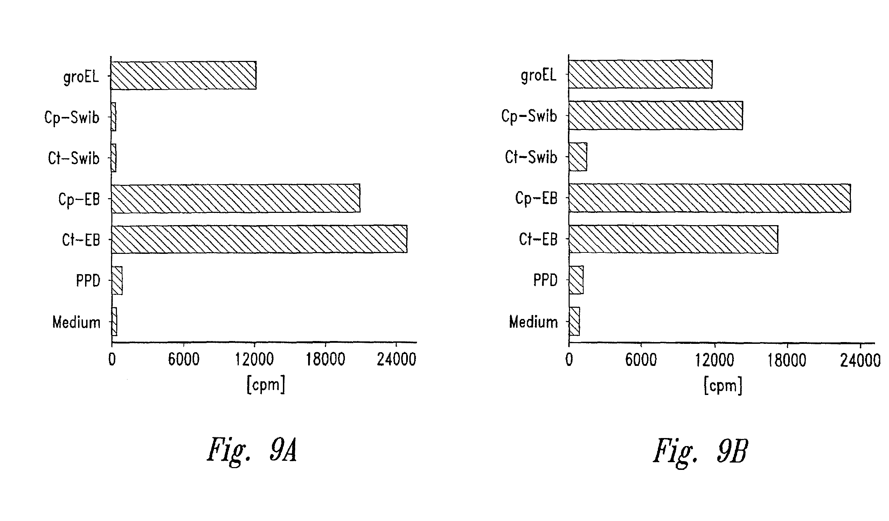

Chlamydia-specific T cell lines were generated from donor CP-21 with a positive serum titer against C. *pneumoniae* by stimulating donor PBMC with either C. *trachomatis* or *C*. *pneumoniae*-infected monocyte-derived dendritic cells, respectively. T-cells generated against C. *pneumoniae* responded to recombinant *C. pneumoniae*-SWIB but not C. *trachomatis-SWIB,* whereas the T-cell line generated against *C*. *trachomatis* did not respond to either *C*. *trachomatis-* or *C*. *pneumoniae*-SWIB (see Fig. 9). The *C*. *pneumoniae-*SWIB specific immune response of donor CP-21 confirms the *C*. *pneumoniae* infection and indicates the elicitation of *C*. *pneumoniae*-SWIB specific T-cells during *in vivo C. pneumoniae* infection.

Epitope mapping of the T-cell response to *C*. *pneumoniae*-SWIB has shown that Cp-SWIB-specific T-cells responded to the overlapping peptides Cp-SWIB 32-51 (SEQ ID NO: 101) and Cp-SWIB 37-56 (SEQ ID NO: 102), indicating a *C. pneumoniae*-SWIB-specific T-cell epitope Cp-SWIB 37-51 (SEQ ID NO: 100).

In additional experiments, T-cell lines were generated from donor CP1, also a *C. pneumoniae* seropositive donor, by stimulating PBMC with non-infectious elementary bodies from *C. trachomatis* and *C. pneumoniae,* respectively. In particular, proliferative responses were determined by stimulating 2.5 x 10⁴ T-cells in the presence of 1 x 10⁴ monocyte-derived dendritic cells and non-infectious elementary bodies derived from *C*. *trachomatis* and *C. pneumoniae,* or either recombinant *C*. *trachomatis* or *C*. *pneumoniae* SWIB protein. The T-cell response against SWIB resembled the data obtained with T-cell lines from CP-21 in that *C*. *pneumoniae-SWIB,* but not *C*. *trachomatis*-SWIB elicited a response by the *C. pneumoniae* T-cell line. In addition, the *C*. *trachomatis* T-cell line did not proliferate in response to either *C*. *trachomatis* or *C. pneumoniae* SWIB, though it did proliferate in response to both CT and CP elementary bodies.As described in Example 1, Clone 11-C12-91 (SEQ ID NO: 63), identified using the TCP-21 cell line, has a 269 bp insert that is part of the OMP2 gene (CT443) and shares homology with the 60 kDa cysteine rich outer membrane protein of *C*. *pneumoniae,* referred to as OMCB. To further define the reactive epitope(s), epitope mapping was performed using a series of overlapping peptides and the immunoassay previously described. Briefly, proliferative responses were determined by stimulating 2.5 x 10⁴ TCP-21 T-cells in the presence of 1 x 10⁴ monocyte-derived dendritic cells with either non-infectious elementary bodies derived from *C*. *trachomatis* and *C. pneumoniae,* or peptides derived from the protein sequence of *C*. *trachomatis* or *C. pneumoniae* OMCB protein (0.1 µg/ml). The TCP-21 T-cells responded to epitopes CT-OMCB #167-186, CT-OMCB #171-190, CT-OMCB #171-186, and to a lesser extent, CT-OMCB #175-186 (SEQ ID NO: 249-252, respectively). Notably, the TCP-21 T-cell line also gave a proliferative response to the homologous *C. pneumoniae* peptide CP-OMCB #171-186 (SEQ ID NO: 253), which was equal to or greater than the response to the to the *C*. *trachomatis* peptides. The amino acid substitutions in position two (i.e., Asp for Glu) and position four (i.e., Cys for Ser) did not alter the proliferative response of the T-cells and therefore demonstrating this epitope to be a cross-reactive epitope between *C*. *trachomatis* and *C. pneumoniae.*

### EXAMPLE 8

### IMMUNE RESPONSES OF HUMAN PBMC AND T-CELL LINES AGAINST CHLAMYDIA ANTIGENS

The examples provided herein suggest that there is a population of healthy donors among the general population that have been infected with *C*. *trachomatis* and generated a protective immune response controlling the *C*. *trachomatis* infection. These donors remained clinically asymptomatic and seronegative for *C*. *trachomatis.* To characterize the immune responses of normal donors against *chlamydial* antigens which had been identified by CD4 expression cloning, PBMC obtained from 12 healthy donors were tested against a panel of recombinant *chlamydial* antigens including *C*. *trachomatis-, C. pneumoniae*-SWIB and *C*. *trachomatis-, C. pneumoniae*-S13. The data are summarized in Table I below. All donors were seronegative for *C*. *trachomatis,* whereas 6/12 had a positive *C. pneumoniae* titer. Using a stimulation index of >4 as a positive response, 11/12 of the subjects responded to *C*. *trachomatis* elementary bodies and 12/12 responded to *C. pneumoniae* elementary bodies. One donor, AD104, responded to recombinant *C. pneumoniae*-S13 protein, but not to recombinant *C*. *trachomatis*-S13 protein, indicating a *C*. *pneumoniae*-specific response. Three out of 12 donors had a *C*. *trachomatis*-SWIB, but not a *C. pneumoniae*-SWIB specific response, confirming a *C*. *trachomatis* infection. *C*. *trachomatis* and *C. pneumoniae-* S13 elicited a response in 8/12 donors suggesting a chlamydial infection. These data demonstrate the ability of SWIB and S13 to elicit a T-cell response in PBMC of normal study subjects.

**Table I.**

| Immune response of normal subjects against *Chlomydia* | | | | | | | | | | |
|---|---|---|---|---|---|---|---|---|---|---|
| onor | Sex | *Chlamydia* IgGtiter EB | CT | CP EB | CT Swib | CP Swib | CT S13 | CP S13 | CT 1pdA | CT TSA |
| D100 | male | negative | ++ | +++ | + | - | ++ | ++ | - | n.t. |
| D104 | female | negative | +++ | ++ | - | - | - | ++ | - | n.t. |
| D108 | male | CP1:256 | ++ | ++ | + | +/- | + | + | + | n.t. |
| D112 | female | negative | ++ | ++ | + | - | + | - | +/- | n.t. |
| D120 | male | negative | - | + | - | - | - | - | - | n.t. |
| D124 | female | CP1:128 | ++ | ++ | - | - | - | - | - | n.t. |
| D128 | male | CP1:512 | + | ++ | - | - | ++ | + | ++ | - |
| D132 | famale | negative | ++ | ++ | - | - | + | + | - | - |
| D136 | female | CP1:128 | + | ++ | - | - | +/- | - | - | - |
| D140 | male | CP1:256 | ++ | ++ | - | - | + | + | - | - |
| D142 | female | CP1:512 | ++ | ++ | - | - | + | + | + | - |
| D146 | female | negative | ++ | ++ | - | - | ++ | + | + | - |

| | | | | | | | | | | |
|---|---|---|---|---|---|---|---|---|---|---|
| CT= *Chlamydia trachomatis*; CP= *Chlamydia pneumoniae;* EB= *Chlamydia* elementary bodies; Swib= recombinant *Chlamydia* Swib protein; S13= recombinant *Chlamydia* S13 protein; lpdA= recombinant *Chlamydia* lpdA protein; TSA= recombinant *Chlamydia* TSA protein.Values represent results from standard proliferation assays. Proliferative responses were determined by stimulating 3 x 10⁵ PBMC with 1 x 10⁴ monocyte-derived dendritic cells pre-incubated with the respective recombinant antigens or elementary bodies (EB). Assays were harvested after 6 days with a ³H-thymidine pulse for the last 18h. | | | | | | | | | | |

### SI: Stimulation index

| | | |
|---|---|---|
| +/-: | SI ∼ | 4 |
| +: | SI > | 4 |
| ++: | SI | 10-30 |
| +++: | SI > | 30 |

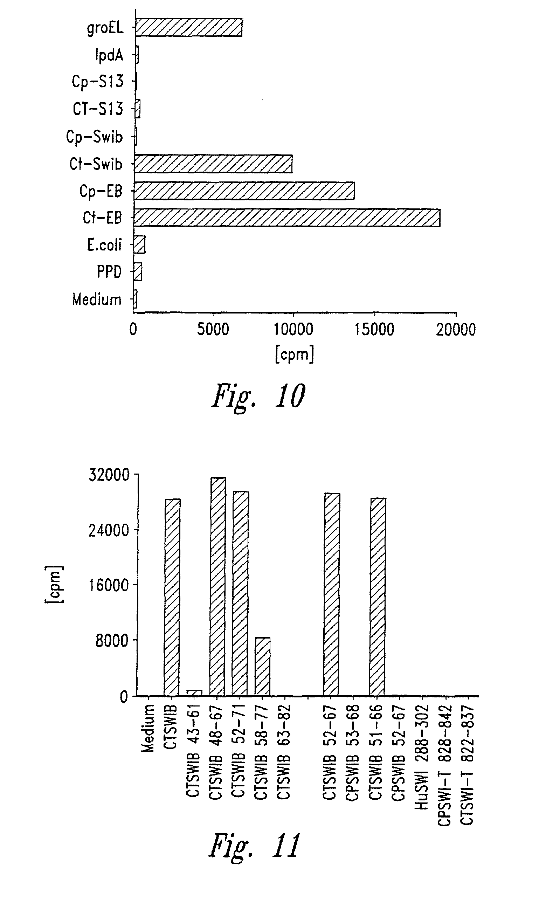

In a first series of experiments, T-cell lines were generated from a healthy female individual (CT-10) with a history of genital exposure to *C*. *trachomatis* by stimulating T-cells with *C*. *trachomatis* LGV II elementary bodies as previously described. Although the study subject was exposed to *C. trachomatis,* she did not seroconvert and did not develop clinical symptoms, suggesting donor CT-10 may have developed a protective immune response against *C*. *trachomatis.* As shown in Fig. 10, a primary *Chlamydia*-specific T-cell line derived from donor CT-10 responded to *C*. *trachomatis*-SWIB, but not *C. pneumoniae-*SWIB recombinant proteins, confirming the exposure of CT-10 to *C. trachomatis.* Epitope mapping of the T-cell response to *C*. *trachomatis*-SWIB showed that this donor responded to the same epitope Ct-SWIB 52-67 (SEQ ID NO: 39) as T-cell line TCL-8, as shown in Fig. 11.

Additional T-cell lines were generated as described above for various *C*. *trachomatis* patients. A summary of the patients' clinical profile and proliferative responses to various *C*. *trachomatis* and *C. pneumoniae* elementary bodies and recombinant proteins are summarized in Table II.

| **Proliferative response of *C. trachomatis* patients** | | | | | | | | | | |
|---|---|---|---|---|---|---|---|---|---|---|
| **atients** | **Clinical manifestation** | **IgG titer** | **CT EB** | **CP EB** | **CT Swib** | **CP Swib** | **CT S13** | **CP S13** | **CT IpdA** | **CT TSA** |
| **CT-1** | **NGU** | **negative** | **+** | **+** | **-** | **-** | **++** | **++** | **++** | **+** |
| **CT-2** | **NGU** | **negative** | **++** | **++** | **-** | **-** | **+** | **+/-** | **-** | **-** |
| **CT-3** | **asymptomatic shed Eb Dx was HPV** | **Ct 1:512 Cp 1:1024 Cps 1:256** | **+** | **+** | **-** | **-** | **+** | **-** | **+** | **-** |
| **CT-4** | **asymptomatic shed Eb** | **Ct 1:1024** | **+** | **+** | **-** | **-** | **-** | **-** | **-** | **-** |
| **CT-5** | **BV** | **Ct 1:256 Cp 1:256** | **++** | **++** | **-** | **-** | **+** | **-** | **-** | **-** |
| **CT-6** | **perinial rash discharge** | **Cp 1:1024** | **+** | **+** | **-** | **-** | **-** | **-** | **-** | **-** |
| **CT-7** | **BV genital ulcer** | **Ct 1:512 Cp 1:1024** | **+** | **+** | **-** | **-** | **+** | **+** | **+** | **-** |
| **CT-8** | **Not known** | **Not tested** | **++** | **++** | **-** | **-** | **-** | **-** | **-** | **-** |
| **CT-9** | **asymptomatic** | **Ct 1:128 Cp 1:128** | **+++** | **++** | **-** | **-** | **++** | **+** | **+** | **-** |
| **CT-10** | **Itch mild vulvar** | **negative** | **++** | **++** | **-** | **-** | **-** | **-** | **-** | **-** |
| **CT-11** | **BV, abnormal pap** | **Ct 1: 512** | **+++** | **+++** | **-** | **-** | **+++** | **+/-** | **++** | **+** |
| **CT-12** | **asymptomatic** | **Cp 1:512** | **++** | **++** | **-** | **-** | **++** | **+** | **+** | **-** |

| | | | | | | | | | | |
|---|---|---|---|---|---|---|---|---|---|---|
| NGU= Non-Gonococcal Urethritis; BV= Bacterial Vaginosis; CT= *Chlamydia trachomatis*; CP= *Chlamydia pneumoniae;* EB= *Chlamydia* elementary bodies; Swib= recombinant *Chlamydia* Swib protein; S13= recombinant *Chlamydia* S13 protein; lpdA= recombinant *Chlamydia* lpdA protein; TSA= recombinant *Chlamydia* TSA protein Values represent results from standard proliferation assays. Proliferative responses were determined by stimulating 3 x 10⁵ PBMC with 1 x 10⁴ monocyte-derived dendritic cells pre-incubated with the respective recombinant antigens or elementary bodies (EB). Assays were harvested after 6 days with a ³H-thymidine pulse for the last 18 hours. | | | | | | | | | | |

### SI: Stimulation index

| | | |
|---|---|---|
| +/-: | SI ∼ | 4 |
| +: | SI > | 4 |
| ++: | SI | 10-30 |
| +++; | SI > | 30 |

Using the panel of asymptomatic (as defined above) study subjects and *C*. *trachomatis* patients, as summarized in Tables I and II, a comprehensive study of the immune responses of PBMC derived from the two groups was conducted. Briefly, PBMCs from *C*. *pneumoniae* patients as well as from normal donors are cultured in medium comprising RPMI 1640 supplemented with 10% pooled human serum and 50 µg/ml gentamicin. Purified polypeptides, a panel of recombinant *chlamydial* antigens including *C*. *trachomatis-, C. pneumoniae*-SWIB and S13, as well as. *C*. *trachomatis* lpdA and TSA are added in duplicate at concentrations of 0.5 to 10 µg/mL. After six days of culture in 96-well round-bottom plates in a volume of 200 µl, 50 µl of medium is removed from each well for determination of IFN-γ levels, as described below. The plates are then pulsed with 1 µCi/well of tritiated thymidine for a further 18 hours, harvested and tritium uptake determined using a gas scintillation counter. Fractions that result in proliferation in both replicates three fold greater than the proliferation observed in cells cultured in medium alone are considered positive.

Proliferative responses to the recombinant *Chlamydiae* antigens demonstrated that the majority of asymptomatic donors and *C*. *trachomatis* patients recognized the *C*. *trachomatis* S13 antigen (8/12) and a majority of the *C*. *trachomatis* patients recognized the *C. pneumonia* S13 antigen (8/12), with 4/12 asymptomatic donors also recognizing the *C*. *pneumonia* S13 antigen. Also, six out of twelve of the *C*. *trachomatis* patients and four out of twelve of the asymptomatic donors gave a proliferative response to the lpdA antigen of *C*. *trachomatis.* These results demonstrate that the *C*. *trachomatis* and *C. pneumonia* S13 antigen, *C*. *trachomatis* Swib antigen and the *C*. *trachomatis* lpdA antigen are recognized by the asymptomatic donors, indicating these antigens were recognized during exposure to *Chlamydia* and an immune response elicited against them. This implies these antigens may play a role in conferring protective immunity in a human host. In addition, the *C*. *trachomatis* and *C*. *pneumonia* S13 antigen is recognized equally well among the *C*. *trachomatis* patients, therefore indicating there may be epitopes shared between *C*. *trachomatis* and *C*. *pneumonia* in the S13 protein. Table III summarizes the results of these studies.

**Table III.**

| **Antigen** | **Normal Donors** | **C.t. Patients** |
|---|---|---|
| C.t.-Swib | 3/12 | 0/12 |
| C.p.-Swib | 0/12 | 0/12 |
| C.t.-S13 | 8/12 | 8/12 |
| C.p.-S13 | 4/12 | 8/12 |
| lpdA | 4/12 | 6/12 |
| TSA | 0/12 | 2/12 |

A series of studies were initiated to determine the cellular immune response to short-term T-cell lines generated from .asymptomatic donors and *C*. *trachomatis* patients. Cellular immune responses were measured by standard proliferation assays and IFN-γ, as described in Example 7. Specifically, the majority of the antigens were in the form of single *E. coli* clones expressing Chlamydial antigens, although some recombinant proteins were also used in the assays. The single *E*. *coli* clones were titered on 1 x 10⁴ monocyte-derived dendritic cells and after two hours, the culture was washed and 2.5 x 10⁴ T-cells were added. The assay using the recombinant proteins were performed as previously described. Proliferation was determined after four days with a standard ³H-thymidine pulse for the last 18 hours. Induction of IFN-γ was determined from culture supernatants harvested after four days using standard ELISA assays, as described above. The results show that all the *C*. *trachomatis* antigens tested, except for C.T. Swib, elicited a proliferative response from one or more different T-cell lines derived form *C*. *trachomatis* patients. In addition, proliferative responses were elicited from both the *C*. *trachomatis* patients and asymptomatic donors for the following *Chlamydia* genes, CT622, groEL, pmpD, CT610 and rS13.

The 12G3-83 clone also contains sequences to CT734 and CT764 in addition to CT622, and therefore these gene sequence may also have immunoreactive epitopes. Similarly, clone 21G12-60 contains sequences to the hypothetical protein genes CT229 and CT228 in addition to CT875; and 15H2-76 also contains sequences from CT812 and CT088, as well as sharing homology to the sycE gene. Clone 11H3-61 also contains sequences sharing homology to the PGP6-D virulence protein.

**Table IV.**

| **Clone** | **C. t. Antigen** (putative*) | **TCL from Asymp. Donors** | **TCL from C. t. Patients** | **SEQ ID NO::** |
|---|---|---|---|---|
| 1B 1-66 _{(E. coli)} | Swib | 2/2 | 0/4 | 5 |
| 1B1-66 ₍ₚᵣₒₜₑᵢₙ₎ | Swib | 2/2 | 0/4 | 5 |
| 12G3-83 _{(E. coli)} | CT622* | 2/2 | 4/4 | 57 |
| 22B3-53 _{(E. coli)} | groEL | 1/2 | 4/4 | 111 |
| 22B3-53 ₍ₚᵣₒₜₑᵢₙ₎ | groEL | 1/2 | 4/4 | 111 |
| 15H2-76 _{(E. coli)} | PmpD* | 1/2 | 3/4 | 87 |
| 11H3-61 _{(E. coli)} | rL1* | 0/2 | 3/4 | 60 |
| 14H1-4 _{(E. coli)} | TSA | 0/2 | 3/4 | 56 |
| 14H1-4 ₍ₚᵣₒₜₑᵢₙ₎ | TSA | 0/2 | 3/4 | 56 |
| 11G10-46 _{(E. coli)} | CT610 | 1/2 | 1/4 | 62 |
| 10C10-17 _{(E. coli)} | rS13 | 1/2 | 1/4 | 62 |
| 10C10-17 ₍ₚᵣₒₜₑᵢₙ₎ | rS13 | 1/2 | 1/4 | 62 |
| 21G12-60 _{(E. coli)} | CT875* | 0/2 | 2/4 | 110 |
| 11H4-32 _{(E. coli)} | dnaK | 0/2 | 2/4 | 59 |
| 21C7-8 _{(E. coli)} | dnaK | 0/2 | 2/4 | 115 |
| 17C10-31 _{(E. coli)} | CT858 | 0/2 | 2/4 | 114 |

### EXAMPLE 9

### PROTECTION STUDIES USING CHLAMYDIA ANTIGENS

Protection studies were conducted in mice to determine whether immunization with chlamydial antigens can impact on the genital tract disease resulting from chlamydial inoculation. Two models were utilized; a model of intravaginal inoculation that uses a human isolate containing a strain of *Chlamydia psittaci* (MTW447), and a model of intrauterine inoculation that involves a human isolate identified as *Chlamydia trachomatis,* serovar F (strain NI1). Both strains induce inflammation in the upper genital tract, which resemble endometritis and salpingitis caused by *Chlamydia trachomatis* in women. In the first experiment, C3H mice (4 mice per group) were immunized three times with 100 µg of pcDNA-3 expression vector containing *C*. *trachomatis* SWIB DNA (SEQ ID NO: 1, with the corresponding amino acid sequence provided in SEQ ID NO: 5). Inoculations were at the base of the tail for systemic immunization. Two weeks after the last immunization, animals were progesterone treated and infected, either thru the vagina or by injection of the inoculum in the uterus. Two weeks after infection, the mice were sacrificed and genital tracts sectioned, stained and examined for histopathology. Inflammation level was scored (from + for very mild, to +++++ for very severe). Scores attributed to each single oviduct /ovary were summed and divided by the number of organs examined to get a mean score of inflammation for the group. In the model of uterine inoculation, negative control-immunized animals receiving empty vector showed consistent inflammation with an ovary /oviduct mean inflammation score of 6.12, in contrast to 2.62 for the DNA-immunized group. In the model of vaginal inoculation and ascending infection, negative control-immunized mice had an ovary /oviduct mean inflammation score of 8.37, versus 5.00 for the DNA-immunized group. Also, in the later model, vaccinated mice showed no signs of tubal occlusion while negative control vaccinated groups had inflammatory cells in the lumen of the oviduct

In a second experiment, C3H mice (4 mice per group) were immunized three times with 50 µg of pcDNA-3 expression vector containing *C*. *trachomatis* SWIB DNA (SEQ ID NO: 1, with the corresponding amino acid sequence provided in SEQ ID NO: 5) encapsulated in Poly Lactide co-Glycolide microspheres (PLG); immunizations were made intra-peritoneally. Two weeks after the last immunization, animal were progesterone treated and infected by inoculation of *C*. *psittaci* in the vagina. Two weeks after infection, mice were sacrificed and genital tracts sectioned, stained and examined for histopathology. Inflammation level was scored as previously described. Scores attributed to each single oviduct /ovary were summed and divided by the number of examined organs to get a mean of inflammation for the group. Negative control-immunized animals receiving PLG-encapsulated empty vector showed consistent infammation with an ovary /oviduct mean inflammation score of 7.28, versus 5.71 for the PLG-encapsulated DNA immunized group. Inflammation in the peritoneum was 1.75 for the vaccinated group versus 3. 75 for the control.

In a third experiment, C3H mice (4 per group) were immunized three times with 10 µg of purified recombinant protein, either SWIB (SEQ ID NO: 1, with the corresponding amino acid sequence provided in SEQ ID NO: 5, or S13 (SEQ ID NO: 4, with the corresponding amino acid sequence provided in SEQ ID NO: 12) mixed with Cholera Toxin (CT); the preparation was administred intranasally upon anaesthesia in a 20 uL volume. Two weeks after the last immunization, animal were progesterone treated and infected, either by vaginal inoculation of *C. psittaci* or by injection of *C*. *trachomatis* serovar F in the uterus. Two weeks after infection, the mice were sacrificed and genital tracts sectioned, stained and examined for histopathology. The degree of inflammation was scored as described above. Scores attributed to each single oviduct /ovary were summed and divided by the number of examined organs to get a mean score of inflammation for the group. In the model of uterine inoculation, negative control- immunized animals receiving cholera toxin alone showed an ovary /oviduct mean inflammation score of 4.25 (only 2 mice analyzed ; 2 other died) versus 5.00 for the s13 plus cholera toxin-immunized group, and 1.00 for the SWIB plus cholera toxin. Untreated infected animals had an ovary /oviduct mean inflammation score of 7. In the model of vaginal inoculation and ascending infection, negative control-immunized mice had an ovary /oviduct mean inflammation score of 7.37 versus 6.75 for the s13 plus cholera toxin-immunized group and 5.37 for the SWIB plus cholera toxin-immunized group. Untreated infected animals had an ovary /oviduct mean inflammation score of 8.

The three experiments described above suggest that SWIB-specific protection is obtainable. This protective effect is more marked in the model of homologous infection but is still present when in a heterologous challenge infection with *C*. *psittaci.*

Although the present invention has been described in some detail by way of illustration and example for purposes of clarity of understanding, changes and modifications can be carried out without departing from the scope of the invention which is intended to be limited only by the scope of the appended claims.

### Numbered paragraphs

The invention may be further defined according to the following numbered paragraphs.
1. An isolated polypeptide comprising an immunogenic portion of a *Chlamydia* antigen, wherein said antigen comprises an amino acid sequence encoded by a polynucleotide sequence selected from the group consisting of: (a) sequences recited in SEQ ID NO: 169-174, 181-188, 263, 265 and 267-290 ; (b) sequences complementary to a sequence of (a); and (c) polynucleotide sequences that hybridize to a sequence of (a) or (b) under moderately stringent conditions.
2. The polypeptide of paragraph 1 wherein the polypeptide comprises a sequence selected from the group consisting of SEQ ID NO: 175-180, 189-196, 264 and 266.
3. An isolated polynucleotide molecule comprising a nucleotide sequence encoding a polypeptide according to any one of paragraphs 1 and 2.
4. A recombinant expression vector comprising a polynucleotide molecule according to paragraph 3.
5. A host cell transformed with an expression vector according to paragraph 4.
6. The host cell of paragraph 5 wherein the host cell is selected from the group consisting of *E. coli,* yeast and mammalian cells.
7. A fusion protein comprising a polypeptide according to any one of paragraphs 1 and 2.
8. A fusion protein according to paragraph 7, wherein the fusion protein comprises an expression enhancer that increases expression of the fusion protein in a host cell transfected with a polynucleotide encoding the fusion protein.
9. A fusion protein according to paragraph 7, wherein the fusion protein comprises a T helper epitope that is not present within the polypeptide of paragraph 1.
10. A fusion protein according to paragraph 7, wherein the fusion protein comprises an affinity tag.
11. An isolated polynucleotide encoding a fusion protein according to paragraph 7.
12. An isolated monoclonal antibody, or antigen-binding fragment thereof, that specifically binds to a Chlamydia protein that comprises an amino acid sequence that is encoded by a polynucleotide sequence according to paragraph 1, or a complement of any of the foregoing polynucleotide sequences.
13. A pharmaceutical composition comprising a polypeptide according to paragraph 1, and a physiologically acceptable carrier.
14. A pharmaceutical composition comprising a polynucleotide molecule according to paragraph 3 and a physiologically acceptable carrier.
15. A pharmaceutical composition comprising a polypeptide and a physiologically acceptable carrier, wherein the polypeptide is encoded by polynucleotide molecule selected from the group consisting of: (a) sequences recited in SEQ ID NO: 169-174, 181-188, 263, 265 and 267-291; (b) sequences complementary to a sequence of (a); and (c) sequences that hybridize to a sequence of (a) or (b) under moderately stringent conditions.
16. A pharmaceutical composition comprising a polynucleotide molecule and a physiologically acceptable carrier, wherein the polynucleotide molecule comprises a sequence selected from the group consisting of: (a) sequences recited in SEQ ID NO: 169-174, 181-188, 263, 265 and 267-291; (b) sequences complementary to a sequence of (a); and (c) sequences that hybridize to a sequence of (a) or (b) under moderately stringent conditions.
17. A pharmaceutical composition comprising a physiologically acceptable carrier and at least one component selected from the group consisting of:
   (a) a fusion protein according to paragraph 7;
   (b) a polynucleotide according to paragraph 11; and
   (c) an antibody according to paragraph 12.
18. A vaccine comprising a polypeptide according to paragraph 1, and an immunostimulant.
19. A vaccine comprising a polynucleotide molecule according to paragraph 3 and an immunostimulant.
20. A vaccine comprising a polypeptide and an immunostimulant, wherein the polypeptide is encoded by a sequence selected from the group consisting of: (a) sequences recited in SEQ ID NO: 169-174, 181-188, 263, 265 and 267-291 ; (b) sequences complementary to a sequence of (a); and (c) sequences that hybridize to a sequence of (a) or (b) under moderately stringent conditions.
21. A vaccine comprising a DNA molecule and an immunostimulant, wherein the DNA molecule comprises a sequence selected from the group consisting of: (a) sequences recited in SEQ ID NO: 169-174, 181-188, 263, 265 and 267-291; (b) sequences complementary to a sequence of (a); and (c) sequences that hybridize to a sequence of (a) or (b) under moderately stringent conditions.
22. A vaccine comprising an immunostimulant and at least one component selected from the group consisting of:
   (a) a fusion protein according to paragraph 7;
   (b) a polynucleotide according to paragraph 11; and
   (c) an antibody according to paragraph 12.
23. The vaccine of any one of paragraphs 18-22 wherein the immunostimulant is an adjuvant.
24. A method for inducing protective immunity in a patient, comprising administering to a patient a pharmaceutical composition according to any one of paragraphs 13-17.
25. A method for inducing protective immunity in a patient, comprising administering to a patient a vaccine according to any one of paragraphs 18-22.
26. An isolated polyclonal antibody, or antigen-binding fragment thereof, that specifically binds to a Chlamydia protein that comprises an amino acid sequence that is encoded by a polynucleotide sequence according to paragraph 1, or a complement of any of the foregoing polynucleotide sequences.
27. A method for detecting *Chlamydia* infection in a patient, comprising:
   (a) obtaining a biological sample from the patient;
   (b) contacting the sample with a polypeptide comprising an immunogenic portion of a *Chlamydia* antigen, wherein said antigen comprises an amino acid sequence encoded by a polynucleotide sequence selected from the group consisting of: (i) a sequence recited in SEQ ID NO: 169-174, 181-188, 263, 265 and 267-291. (ii) sequences complementary to a sequence of (i), and (c) polynucleotide sequences that hybridize to a sequence of (i) or (ii) under moderately stringent conditions; and
   (c) detecting the presence of antibodies that bind to the polypeptide.
28. A method for detecting *Chlamydia* infection in a patient, comprising:
   (a) obtaining a biological sample from the patient;
   (b) contacting the sample with a fusion protein comprising a polypeptide, the polypeptide comprising an immunogenic portion of a *Chlamydia* antigen, wherein said antigen comprises an amino acid sequence encoded by a polynucleotide sequence selected from the group consisting of (i) a sequence recited in SEQ ID NO: 169-174, 181-188, 263, 265 and 267-291 (ii) sequences complementary to a sequence of (i), and (c) polynucleotide sequences that hybridize to a sequence of (i) or (ii) under moderately stringent conditions; and
   (c) detecting the presence of antibodies that bind to the fusion protein.
29. The method of any one of paragraphs 27 and 28 wherein the biological sample is selected from the group consisting of whole blood, serum, plasma, saliva, cerebrospinal fluid and urine.
30. A method for detecting *Chlamydia* infection in a biological sample, comprising:
   (a) contacting the sample with at least two oligonucleotide primers in a polymerase chain reaction, wherein at least one of the oligonucleotide primers is specific for a polynucleotide molecule comprising a sequence of SEQ ID NO: 169-174, 181-188, 263, 265 and 267-291; and
   (b) detecting in the sample a polynucleotide sequence that amplifies in the presence of the oligonucleotide primers, thereby detecting *Chlamydia* infection.
31. The method of paragraph 30, wherein at least one of the oligonucleotide primers comprises at least about 10 contiguous nucleotides of a polynucleotide sequence of SEQ ID NO: 169-174, 181-188, 263, 265 and 267-291.
32. A method for detecting *Chlamydia* infection in a biological sample, comprising:
   (a) contacting the sample with one or more oligonucleotide probes specific for a polynucleotide molecule comprising a sequence of SEQ ID NO: 169-174, 181-188, 263, 265 and 267-291; and
   (b) detecting in the sample a polynucleotide sequence that hybridizes to the oligonucleotide probe, thereby detecting *Chlamydia* infection.
33. The method of paragraph 32 wherein the probe comprises at least about 15 contiguous nucleotides of a polynucleotide sequence of SEQ ID NO: 169-174, 181-188, 263, 265 and 267-291.
34. A method for detecting *Chlamydia* infection in a biological sample, comprising:
   (a) contacting the biological sample with a binding agent which is capable of binding to a polypeptide comprising an immunogenic portion of a *Chlamydia* antigen, wherein said antigen comprises an amino acid sequence encoded by a polynucleotide sequence selected from the group consisting of: (i) a sequence recited in SEQ ID NO: 169-174, 181-188, 263, 265 and 267-291, (ii) sequences complementary to a sequence of (i), and (c) polynucleotide sequences that hybridize to a sequence of (i) or (ii) under moderately stringent conditions; and
   (b) detecting in the sample a polypeptide that binds to the binding agent, thereby detecting *Chlamydia* infection in the biological sample.
35. A method of detecting *Chlamydia* infection in a biological sample, comprising:
   (a) contacting the biological sample with a binding agent which is capable of binding to a fusion protein comprising a polypeptide, the polypeptide comprising an immunogenic portion of a *Chlamydia* antigen, wherein said antigen comprises an amino acid sequence encoded by a polynucleotide sequence selected from the group consisting of: (i) a sequence recited in SEQ ID NO: 169-174, 181-188, 263, 265 and 267-291, (ii) sequences complementary to a sequence of (i), and (c) polynucleotide sequences that hybridize to a sequence of (i) or (ii) under moderately stringent conditions; and
   (b) detecting in the sample a polypeptide that binds to the binding agent, thereby detecting *Chlamydia* infection in the biological sample.
36. The method of any one of paragraphs 34 and 35 wherein the binding agent is a monoclonal antibody.
37. The method of any one of paragraphs 34 and 35 wherein the binding agent is a polyclonal antibody.
38. The method of any one of paragraphs 34 and 35 wherein the biological sample is selected from the group consisting of whole blood, sputum, serum, plasma, saliva, cerebrospinal fluid and urine.
39. A diagnostic kit comprising:
   (a) a polypeptide comprising an immunogenic portion of a *Chlamydia* antigen, wherein said antigen comprises an amino acid sequence encoded by a polynucleotide sequence selected from the group consisting of: (i) a sequence recited in SEQ ID NO: 169-174, 181-188, 263, 265 and 267-291, (ii) sequences complementary to a sequence of (i), and (c) polynucleotide sequences that hybridize to a sequence of (i) or (ii) under moderately stringent conditions; and
   (b) a detection reagent.
40. A diagnostic kit comprising:
   (a) a fusion protein comprising a polypeptide, the polypeptide comprising an immunogenic portion of a *Chlamydia* antigen, wherein said antigen comprises an amino acid sequence encoded by a polynucleotide sequence selected from the group consisting of: (i) a sequence recited in SEQ ID NO: 169-174, 181-188, 263, 265 and 267-291 (ii) sequences complementary to a sequence of (i), and (c) polynucleotide sequences that hybridize to a sequence of (i) or (ii) under moderately stringent conditions; and
   (b) a detection reagent.
41. The kit of paragraphs 39 or 40 wherein the polypeptide is immobilized on a solid support.
42. The kit of paragraphs 39 or 40 wherein the detection reagent comprises a reporter group conjugated to a binding agent.
43. The kit of paragraph 42 wherein the binding agent is selected from the group consisting of anti-immunoglobulins, Protein G, Protein A and lectins.
44. The kit of paragraph 42 wherein the reporter group is selected from the group consisting of radioisotopes, fluorescent groups, luminescent groups, enzymes, biotin and dye particles.
45. A diagnostic kit comprising at least two oligonucleotide primers, at least one of the oligonucleotide primers being specific for a polynucleotide molecule comprising a polynucleotide sequence of SEQ ID NO: 169-174, 181-188, 263, 265 and 267-291.
46. A diagnostic kit according to paragraph 43, wherein at least one of the oligonucleotide primers comprises at least about 10 contiguous nucleotides of a sequence of SEQ ID NO: 169-174, 181-188, 263, 265 and 267-291.
47. A diagnostic kit comprising at least one oligonucleotide probe, the oligonucleotide probe being specific for a polynucleotide molecule comprising a sequence of SEQ ID NO: 169-174, 181-188, 263, 265 and 267-291.
48. A kit according to paragraph 47, wherein the oligonucleotide probe comprises at least about 15 contiguous nucleotides of a polynucleotide sequence of SEQ ID NO: 169-174, 181-188, 263, 265 and 267-291.
49. A diagnostic kit comprising:
   (a) at least one antibody, or antigen-binding fragment thereof, according to paragraph 22; and
   (b) a detection reagent.
50. A method for treating *Chlamydia* infection in a patient, comprising the steps of:
   (a) obtaining peripheral blood cells from the patient;
   (b) incubating the cells in the presence of at least one polypeptide, the polypeptide comprising an immunogenic portion of a *Chlamydia* antigen, wherein said antigen comprises an amino acid sequence encoded by a polynucleotide sequence selected from the group consisting of: (i) a sequence recited in SEQ ID NO: 169-174, 181-188, 263, 265 and 267-291 (ii) sequences complementary to a sequence of (i), and (c) polynucleotide sequences that hybridize to a sequence of (i) or (ii) under moderately stringent conditions, such that T cells proliferate; and
   (c) administering to the patient the proliferated T cells.
51. A method for treating *Chlamydia* infection in a patient, comprising the steps of:
   (a) obtaining peripheral blood cells from the patient;
   (b) incubating the cells in the presence of at least one polynucleotide, comprises a polynucleotide sequence selected from the group consisting of: (i) a sequence recited in SEQ ID NO: 169-174, 181-188, 263, 265 and 267-291 (ii) sequences complementary to a sequence of (i), and (c) polynucleotide sequences that hybridize to a sequence of (i) or (ii) under moderately stringent conditions, such that T cells proliferate; and
   (c) administering to the patient the proliferated T cells.
52. The method of any one of paragraphs 50 and 51 wherein the step of incubating the T cells is repeated one or more times.
53. The method of any one of paragraphs 50 and 51 wherein step (a) further comprises separating T cells from the peripheral blood cells, and the cells incubated in step (b) are the T cells.
54. The method of any one of paragraphs 50 and 51 wherein step (a) further comprises separating CD4+ cells or CD8+ T cells from the peripheral blood cells, and the cells proliferated in step (b) are CD4+ or CD8+ T cells.
55. The method of any one of paragraphs 50 and 51 wherein step (a) further comprises separating gamma/delta T lymphocytes from the peripheral blood cells, and the cells proliferated in step (b) are gamma/delta T lymphocytes.
56. The method of any one of paragraphs 50 and 51 wherein step (b) further comprises cloning one or more T cells that proliferated in the presence of the polypeptide.
57. A pharmaceutical composition for the treatment of *Chlamydia* infection in a patient, comprising T cells proliferated in the presence of a polypeptide of paragraph 1, in combination with a physiologically acceptable carrier.
58. A pharmaceutical composition for the treatment of *Chlamydia* infection in a patient, comprising T cells proliferated in the presence of a polynucleotide of paragraph 3, in combination with a physiologically acceptable carrier.
59. A method for treating *Chlamydia* infection in a patient, comprising the steps of:
   (a) incubating antigen presenting cells in the presence of at least one polypeptide of paragraph 1;
   (b) administering to the patient the incubated antigen presenting cells.
60. A method for treating *Chlamydia* infection in a patient, comprising the steps of:
   (a) introducing at least one polynucleotide of paragraph 3 into antigen presenting cells;
   (b) administering to the patient the antigen presenting cells.
61. The method of paragraphs 59 or 60 wherein the antigen presenting cells are selected from the group consisting of dendritic cells. macrophage cells, B cells fibroblast cells, monocyte cells, and stem cells.
62. A pharmaceutical composition for the treatment of *Chlamydia* infection in a patient, comprising antigen presenting cells incubated in the presence of a polypeptide of paragraph 1, in combination with a physiologically acceptable carrier.
63. A pharmaceutical composition for the treatment if *Chlamydia* infection in a patient, comprising antigen presenting cells incubated in the presence of a polynucleotide of paragraph 3, in combination with a physiologically acceptable carrier.
64. A polypeptide comprising an immunogenic portion of a *Chlamydia* antigen, wherein said immunogenic portion comprises a sequence of SEQ ID NO: 246, 247 and 254-256.
65. An immunogenic epitope of a *Chlamydia* antigen, comprising a sequence of SEQ ID NO: 246, 247 or 254-256.
66. An isolated polypeptide comprising a sequence recited in any one of SEQ ID NO: 224-262, 246, 247, 254-256, 292 and 294-305.
67. A recombinant fusion polypeptide comprising a an amino acid sequence of a Ra12 polypeptide and an amino acid sequence of a Chlamydial polypeptide.
68. The recombinant polypeptide of paragraphs 67, wherein the Chlamydial polypeptide is a Pmp polypeptide.
69. The recombinant polypeptide of paragraphs 67, wherein the Chlamydial polypeptide is a PmpA, PmpF, PmpH, PmpB, or PmpC.
70. The recombinant polypeptide of paragraphs 67, wherein the amino acid sequence of the fusion polypeptide is a sequence selected from the group consisting of SEQ ID NOs: 309, 313, 317, 321, 325, 329, 333, 337, 341, 345, 349, 353 and 357.
71. A recombinant DNA molecule encoding a fusion polypeptide according to paragraph 67.

## Claims

1. A composition comprising a physiologically acceptable carrier, and an isolated polypeptide comprising:
(i) an amino acid sequence having at least 90% identity to the sequence encoded by SEQ ID No: 172;
(ii) an immunogenic fragment of the amino acid sequence encoded by SEQ ID No: 172;
(iii) the amino acid sequence of SEQ ID No: 178 or a variant thereof having at least 90% identity thereto; or
(iv) an immunogenic fragment of the amino acid sequence of SEQ ID No: 178.

2. A composition according to claim 1, wherein the isolated polypeptide consists of the amino acid sequence set forth in SEQ ID NO: 178.

3. A composition according to claim 1, wherein the isolated polypeptide consists of the amino acid sequence encoded by SEQ ID No: 172.

4. A composition according to claim 1, wherein the immunogenic fragment of the amino acid sequence encoded by SEQ ID No: 172 comprises an amino acid sequence encoded by SEQ ID NO. 184 or 185.

5. A composition according to claim 1, wherein the wherein the immunogenic fragment of the amino acid sequence encoded by SEQ ID No: 172 comprises the amino acid sequence of SEQ ID NO. 192 or 193.

6. A composition comprising a physiologically acceptable carrier and an isolated polynucleotide, said isolated polynucleotide comprising:
(i) a polynucleotide sequence encoding the polypeptide sequence set forth in SEQ ID NO: 178 or a variant thereof having at least 90% identity thereto; or
(ii) a polynucleotide sequence encoding an immunogenic fragment of the amino acid sequence of SEQ ID No: 178;
(iii) the polynucleotide sequence of SEQ ID NO. 172, or a variant thereof having at least 90% identity thereto; or
(iv) a fragment of the polynucleotide sequence of SEQ ID NO. 172.

7. A composition according to claim 17, wherein the isolated polynucleotide consists of the polynucleotide sequence of SEQ ID No: 172.

8. A composition according to claim 6, wherein the fragment of the polynucleotide of SEQ ID NO. 172 comprises SEQ ID NO. 184 or 185.

9. A composition according to claim 6, wherein the fragment of the polynucleotide of SEQ ID NO. 172 encodes an amino acid sequence comprising SEQ ID NO. 192 or 193.

10. A composition comprising a physiologically acceptable carrier and a fusion protein, said fusion protein comprising a polypeptide as defined in any of claims 1-5.

11. A composition according to any one of claims 1 to 10, wherein the composition is a pharmaceutical composition.

12. A composition according to any one of claims 1 to 10, wherein the composition is a vaccine composition, which further comprises an immunostimulant.

13. A vaccine composition according to claim 12, wherein the immunostimulant is an adjuvant.

14. A pharmaceutical composition according to claim 11 for use in inducing protective immunity in a patient.

15. A vaccine composition according to claim 12 or 13 for use in inducing protective immunity in a patient.
